(19)

Europäisches Patentamt
European Patent Office
Office européen des brevets

(11) **EP 4 591 882 A2**

(12) **EUROPEAN PATENT APPLICATION**

(43) Date of publication:
**30.07.2025 Bulletin 2025/31**

(21) Application number: **25177102.8**

(22) Date of filing: **24.02.2021**

(51) International Patent Classification (IPC):
**A61K 39/395** $^{(2006.01)}$

(52) Cooperative Patent Classification (CPC):
**C07K 16/2809;** C07K 2317/21; C07K 2317/34;
C07K 2317/565; C07K 2317/622; C07K 2317/70;
C07K 2317/74; C07K 2317/92

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.02.2020 GB 202002581**

(62) Document number(s) of the earlier application(s) in
accordance with Art. 76 EPC:
**21709758.3 / 4 110 387**

(71) Applicant: **GammaDelta Therapeutics LTD
London W2 6BD (GB)**

(72) Inventors:
- **NUSSBAUMER, Oliver
London W12 7FQ (GB)**
- **POLYAKOVA, Oxana
London W12 7FQ (GB)**

- **HAYDAY, Adrian
London WC2R 2LS (GB)**
- **VANTOUROUT, Pierre
London WC2R 2LS (GB)**

(74) Representative: **Jones, Nicholas Andrew
Withers & Rogers LLP
2 London Bridge
London SE1 9RA (GB)**

Remarks:
•The complete document including Reference
Table(s) and the Sequence Listing(s) can be
downloaded from the EPO website
•This application was filed on 16-05-2025 as a
divisional application to the application mentioned
under INID code 62.

(54) **EX VIVO GAMMA DELTA T CELL POPULATIONS**

(57) The invention relates to *ex vivo* methods of modulating gamma variable 4 (Vγ4) T cells using antibodies or fragments thereof, which specifically bind to a Vγ4 chain of a γδ T cell receptor (TCR) and not to a gamma variable 2 (Vγ2) chain of a γδ TCR. Methods of treatment and other uses of expanded populations of Vγ4 T cells produced by said methods are also provided.

FIGURE 2A

**Description**

**FIELD OF THE INVENTION**

The invention relates to populations of gamma delta T cells contacted with anti-TCR gamma variable 4 (anti-Vγ4) antibodies.

**BACKGROUND OF THE INVENTION**

The growing interest in T cell immunotherapy for cancer has focused on the evident capacity of subsets of CD8+ and CD4+ alpha beta ($\alpha\beta$) T cells to recognize cancer cells and to mediate host-protective functional potentials, particularly when de-repressed by clinically mediated antagonism of inhibitory pathways exerted by PD-1, CTLA-4, and other receptors. However, $\alpha\beta$ T cells are MHC-restricted which can lead to graft versus host disease.

Gamma delta T cells ($\gamma\delta$ T cells) represent a subset of T cells that express on their surface a distinct, defining $\gamma\delta$ T-cell receptor (TCR). This TCR is made up of one gamma ($\gamma$) and one delta ($\delta$) chain, each of which undergoes chain rearrangement but have a limited number of V genes as compared to $\alpha\beta$ T cells. The main TRVG gene segments encoding V$\gamma$ are TRGV2, TRGV3, TRGV4, TRGV5, TRGV8, TRGV9 and non-functional genes TRGV10, TRGV11, TRGVA and TRGVB. The most frequent TRDV gene segments encode V$\delta$1, V$\delta$2, and V$\delta$3, plus several V segments that have both V$\delta$ and V$\alpha$ designation (Adams et al., 296:30-40 (2015) Cell Immunol.). Human $\gamma\delta$ T cells can be broadly classified based on their TCR chains, as certain $\gamma$ and $\delta$ types are found on cells more prevalently, though not exclusively, in one or more tissue types. For example, most blood-resident $\gamma\delta$ T cells express a V$\delta$2 TCR, commonly V$\gamma$9V$\delta$2, whereas this is less common among tissue-resident $\gamma\delta$ T cells such as those in the skin, which more frequently use the V$\delta$1 TCR paired with gamma chains, for example often paired with V$\gamma$4 in the gut.

However to date, due to high homology between V$\gamma$4 TCR and other TRGV family members such as the V$\gamma$2 TCR, modalities capable of targeting only the V$\gamma$4 TCR have not been possible. Therefore there is an unmet need for antibodies specific for V$\gamma$4, including such specific antibodies that specifically bind or modulate the V$\gamma$4 TCR.

**SUMMARY OF THE INVENTION**

According to a first aspect of the invention, there is provided an *ex vivo* method of modulating gamma variable 4 (V$\gamma$4) T cells comprising administering an antibody or fragment thereof, which specifically binds to a V$\gamma$4 chain of a $\gamma\delta$ T cell receptor (TCR) and not to a gamma variable 2 (V$\gamma$2) chain of a $\gamma\delta$ TCR, to a cell population comprising V$\gamma$4 T cells. It should be understood that this is with reference to a V$\gamma$4 chain and a V$\gamma$2 from the same species. Preferably, according to all aspects and embodiments described herein, the species is *Homo sapiens* (human) and therefore the invention also provides an *ex vivo* method of modulating gamma variable 4 (V$\gamma$4) T cells comprising administering an antibody or fragment thereof, which specifically binds to a human gamma variable 4 (V$\gamma$4) chain of a $\gamma\delta$ T cell receptor (TCR) and not to a human gamma variable 2 (V$\gamma$2) chain of a $\gamma\delta$ TCR. For instance, the human V$\gamma$4 chain may have a sequence according to amino acids 1-99 of SEQ ID NO. 1 and/or the human V$\gamma$2 chain may have a sequence according to SEQ ID NO. 335. In other species, the antibody or fragment thereof, specifically binds to the species-specific ortholog of the human gamma variable 4 (V$\gamma$4) chain of a $\gamma\delta$ T cell receptor (TCR) and not to the species-specific ortholog of the human gamma variable 2 (V$\gamma$2) chain of a $\gamma\delta$ TCR. Thus, the antibody or fragment thereof, may specifically bind to a human gamma variable 4 (V$\gamma$4) chain of a $\gamma\delta$ T cell receptor (TCR) having a sequence corresponding to amino acids 1-99 of SEQ ID NO. 1 or non-human ortholog thereof and not to a human gamma variable 2 (V$\gamma$2) chain of a $\gamma\delta$ TCR having a sequence corresponding to SEQ ID NO. 335 or non-human ortholog thereof. Ortholog in this context may mean a gamma chain sequence with the highest sequence similarity to the reference sequence, or preferably one which possesses the same function (e.g. interaction with orthologous cognate ligands *in vivo*). For instance, in mouse, the protein designated under the Heilig & Tonegave nomenclature as V$\gamma$7 is functionally most closely related to human V$\gamma$4 (Barros et al. (2016) Cell, 167:203-218.e17).

This is a significant advancement to the field. For instance, in humans, the V$\gamma$4 chain and V$\gamma$2 chain are highly homologous (sequence identity of 91%), differing in respect of only 9 amino acids. Three of these nine changes map across CDR1 and CDR2, whilst four of these nine changes map to a sub-region of framework region 3 (FR3) - amino acids 67-82 of SEQ ID NO: 1. Due to the very high sequence similarity between the V$\gamma$4 chain and V$\gamma$2 chain, it was previously thought that it would not be possible to develop an antibody or fragment thereof able to specifically distinguish between the human V$\gamma$4 chain and V$\gamma$2 chain of a $\gamma\delta$ TCR. Surprisingly and contrary to the prevailing view in the art, the present inventors have been able to develop such antibodies using the methods described in more detail herein. Thus, the invention provides *ex vivo* methods of using antibodies and fragments thereof which are able to specifically modulate V$\gamma$4-containing $\gamma\delta$ TCRs.

The antibodies or fragments thereof described herein may bind to an epitope of the human V$\gamma$4 chain of the $\gamma\delta$ TCR comprising one or more amino acid residues within amino acid region 67-82 of SEQ ID NO: 1.

According to a further aspect of the invention, there is provided an *ex vivo* method of modulating gamma variable 4 (Vγ4) T cells comprising administering an anti-Vγ4 antibody or fragment thereof, which comprises one or more of:

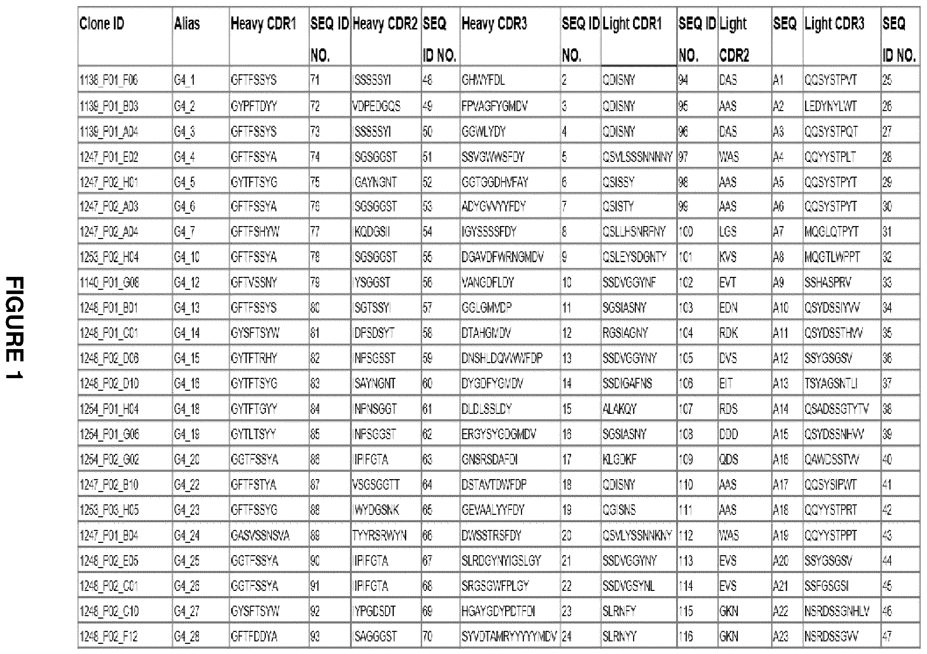

a CDR3 comprising a sequence having at least 80% sequence identity with any one of SEQ ID NOs: 2-47, preferably with SEQ ID NO: 10 and/or 33;
a CDR2 comprising a sequence having at least 80% sequence identity with any one of SEQ ID NOs: 48-70 and SEQUENCES: A1-A23 (of Figure 1), preferably with SEQ ID NO: 56 and/or A9; and/or
a CDR1 comprising a sequence having at least 80% sequence identity with any one of SEQ ID NOs: 71-116, preferably with SEQ ID NO: 79 and/or 102
to a cell population comprising Vγ4 T cells.

In some embodiments, the anti-Vγ4 antibody or fragment thereof may comprise one or more of:

a heavy chain CDR3 (HCDR3) comprising a sequence having at least 80% sequence identity with any one of SEQ ID NOs: 2-24, preferably with SEQ ID NO: 10;
a heavy chain CDR2 (HCDR2) comprising a sequence having at least 80% sequence identity with any one of SEQ ID NOs: 48-70, preferably with SEQ ID NO: 56 ; and/or
a heavy chain CDR1 (HCDR1) comprising a sequence having at least 80% sequence identity with any one of SEQ ID NOs: 71-93, preferably with SEQ ID NO: 79.

Alternatively, or in addition to, the anti-Vγ4 antibody or fragment thereof may comprise one or more of:

a light chain CDR3 (LCDR3) comprising a sequence having at least 80% sequence identity with any one of SEQ ID NOs: 25-47, preferably with SEQ ID NO: 33;
a light chain CDR2 (LCDR2) comprising a sequence having at least 80% sequence identity with any one of SEQUENCES: A1-A23 (of Figure 1), preferably with SEQ ID NO: A9; and/or
a light chain CDR1 (LCDR1) comprising a sequence having at least 80% sequence identity with any one of SEQ ID NOs: 94-116, preferably with SEQ ID NO: 102.

In some embodiments, the anti-Vγ4 antibody or fragment thereof comprises an amino acid sequence having at least 80% sequence identity with any one of SEQ ID NOs: 117-162. In some embodiments, the anti-Vγ4 antibody or fragment thereof may comprise a heavy chain variable (VH) amino acid sequence having at least 80% sequence identity with any one of SEQ ID NOs: 117-139, preferably with SEQ ID NO: 125. Alternatively, or in addition to, the anti-Vγ4 antibody or fragment thereof may comprise a light chain variable (VL) amino acid sequence having at least 80% sequence identity with any one of SEQ ID NOs: 140-162, preferably with SEQ ID NO: 148.

In some embodiments, the anti-Vγ4 antibody or fragment thereof comprises one or more of:

(a) a VH comprising a HCDR1 having SEQ ID NO: 79, a HCDR2 having SEQ ID NO: 56 and a HCDR3 having SEQ ID NO: 10, optionally wherein the VH comprises SEQ ID NO: 125; and
a VL comprising a LCDR1 having SEQ ID NO: 102, a LCDR2 having SEQUENCE A9 (of Figure 1) and a LCDR3 having SEQ ID NO: 33, optionally wherein the VL comprises SEQ ID NO: 148;
(b) a VH comprising a HCDR1 having SEQ ID NO: 86, a HCDR2 having SEQ ID NO: 63 and a HCDR3 having SEQ ID NO: 17, optionally wherein the VH comprises SEQ ID NO: 132; and
a VL comprising a LCDR1 having SEQ ID NO: 109, a LCDR2 having SEQUENCE A16 (of Figure 1) and a LCDR3 having SEQ ID NO: 40, optionally wherein the VL comprises SEQ ID NO: 155;
(c) a VH comprising a HCDR1 having SEQ ID NO: 73, a HCDR2 having SEQ ID NO: 50 and a HCDR3 having SEQ ID NO: 4, optionally wherein the VH comprises SEQ ID NO: 119; and
a VL comprising a LCDR1 having SEQ ID NO: 96, a LCDR2 having SEQUENCE A3 (of Figure 1) and a LCDR3 having SEQ ID NO: 27, optionally wherein the VL comprises SEQ ID NO: 142;
(d) a VH comprising a HCDR1 having SEQ ID NO: 83, a HCDR2 having SEQ ID NO: 60 and a HCDR3 having SEQ ID NO: 14, optionally wherein the VH comprises SEQ ID NO: 129; and
a VL comprising a LCDR1 having SEQ ID NO: 106, a LCDR2 having SEQUENCE A13 (of Figure 1) and a LCDR3 having SEQ ID NO: 37, optionally wherein the VL comprises SEQ ID NO: 152;
(e) a VH comprising a HCDR1 having SEQ ID NO: 84, a HCDR2 having SEQ ID NO: 61 and a HCDR3 having SEQ ID NO: 15, optionally wherein the VH comprises SEQ ID NO: 130; and
a VL comprising a LCDR1 having SEQ ID NO: 107, a LCDR2 having SEQUENCE A14 (of Figure 1) and a LCDR3 having SEQ ID NO: 38, optionally wherein the VL comprises SEQ ID NO: 153;
(f) a VH comprising a HCDR1 having SEQ ID NO: 88, a HCDR2 having SEQ ID NO: 65 and a HCDR3 having SEQ ID NO: 19, optionally wherein the VH comprises SEQ ID NO: 134; and

a VL comprising a LCDR1 having SEQ ID NO: 111, a LCDR2 having SEQUENCE A18 (of Figure 1) and a LCDR3 having SEQ ID NO: 42, optionally wherein the VL comprises SEQ ID NO: 157;

(g) a VH comprising a HCDR1 having SEQ ID NO: 92, a HCDR2 having SEQ ID NO: 69 and a HCDR3 having SEQ ID NO: 23, optionally wherein the VH comprises SEQ ID NO: 138; and

a VL comprising a LCDR1 having SEQ ID NO: 115, a LCDR2 having SEQUENCE A22 (of Figure 1) and a LCDR3 having SEQ ID NO: 46, optionally wherein the VL comprises SEQ ID NO: 161;

(h) a VH comprising a HCDR1 having SEQ ID NO: 71, a HCDR2 having SEQ ID NO: 48 and a HCDR3 having SEQ ID NO: 2, optionally wherein the VH comprises SEQ ID NO: 117; and

a VL comprising a LCDR1 having SEQ ID NO: 94, a LCDR2 having SEQUENCE A1 (of Figure 1) and a LCDR3 having SEQ ID NO: 25, optionally wherein the VL comprises SEQ ID NO: 140;

(i) a VH comprising a HCDR1 having SEQ ID NO: 72, a HCDR2 having SEQ ID NO: 49 and a HCDR3 having SEQ ID NO: 3, optionally wherein the VH comprises SEQ ID NO: 118; and

a VL comprising a LCDR1 having SEQ ID NO: 95, a LCDR2 having SEQUENCE A2 (of Figure 1) and a LCDR3 having SEQ ID NO: 26, optionally wherein the VL comprises SEQ ID NO: 141;

(j) a VH comprising a HCDR1 having SEQ ID NO: 74, a HCDR2 having SEQ ID NO: 51 and a HCDR3 having SEQ ID NO: 5, optionally wherein the VH comprises SEQ ID NO: 120; and

a VL comprising a LCDR1 having SEQ ID NO: 97, a LCDR2 having SEQUENCE A4 (of Figure 1) and a LCDR3 having SEQ ID NO: 28, optionally wherein the VL comprises SEQ ID NO: 143;

(k) a VH comprising a HCDR1 having SEQ ID NO: 75, a HCDR2 having SEQ ID NO: 52 and a HCDR3 having SEQ ID NO: 6, optionally wherein the VH comprises SEQ ID NO: 121; and

a VL comprising a LCDR1 having SEQ ID NO: 98, a LCDR2 having SEQUENCE A5 (of Figure 1) and a LCDR3 having SEQ ID NO: 29, optionally wherein the VL comprises SEQ ID NO: 144;

(l) a VH comprising a HCDR1 having SEQ ID NO: 76, a HCDR2 having SEQ ID NO: 53 and a HCDR3 having SEQ ID NO: 7, optionally wherein the VH comprises SEQ ID NO: 122; and

a VL comprising a LCDR1 having SEQ ID NO: 99, a LCDR2 having SEQUENCE A6 (of Figure 1) and a LCDR3 having SEQ ID NO: 30, optionally wherein the VL comprises SEQ ID NO: 145;

(m) a VH comprising a HCDR1 having SEQ ID NO: 77, a HCDR2 having SEQ ID NO: 54 and a HCDR3 having SEQ ID NO: 8, optionally wherein the VH comprises SEQ ID NO: 123; and

a VL comprising a LCDR1 having SEQ ID NO: 100, a LCDR2 having SEQUENCE A7 (of Figure 1) and a LCDR3 having SEQ ID NO: 31, optionally wherein the VL comprises SEQ ID NO: 146;

(n) a VH comprising a HCDR1 having SEQ ID NO: 78, a HCDR2 having SEQ ID NO: 55 and a HCDR3 having SEQ ID NO: 9, optionally wherein the VH comprises SEQ ID NO: 124; and

a VL comprising a LCDR1 having SEQ ID NO: 101, a LCDR2 having SEQUENCE A8 (of Figure 1) and a LCDR3 having SEQ ID NO: 32, optionally wherein the VL comprises SEQ ID NO: 147;

(o) a VH comprising a HCDR1 having SEQ ID NO: 80, a HCDR2 having SEQ ID NO: 57 and a HCDR3 having SEQ ID NO: 11, optionally wherein the VH comprises SEQ ID NO: 126; and

a VL comprising a LCDR1 having SEQ ID NO: 103, a LCDR2 having SEQUENCE A10 (of Figure 1) and a LCDR3 having SEQ ID NO: 34, optionally wherein the VL comprises SEQ ID NO: 149;

(p) a VH comprising a HCDR1 having SEQ ID NO: 81, a HCDR2 having SEQ ID NO: 58 and a HCDR3 having SEQ ID NO: 12, optionally wherein the VH comprises SEQ ID NO: 127; and

a VL comprising a LCDR1 having SEQ ID NO: 104, a LCDR2 having SEQUENCE A11 (of Figure 1) and a LCDR3 having SEQ ID NO: 35, optionally wherein the VL comprises SEQ ID NO: 150;

(q) a VH comprising a HCDR1 having SEQ ID NO: 82, a HCDR2 having SEQ ID NO: 59 and a HCDR3 having SEQ ID NO: 13, optionally wherein the VH comprises SEQ ID NO: 128; and

a VL comprising a LCDR1 having SEQ ID NO: 105, a LCDR2 having SEQUENCE A12 (of Figure 1) and a LCDR3 having SEQ ID NO: 36, optionally wherein the VL comprises SEQ ID NO: 151;

(r) a VH comprising a HCDR1 having SEQ ID NO: 85, a HCDR2 having SEQ ID NO: 62 and a HCDR3 having SEQ ID NO: 16, optionally wherein the VH comprises SEQ ID NO: 131; and

a VL comprising a LCDR1 having SEQ ID NO: 108, a LCDR2 having SEQUENCE A15 (of Figure 1) and a LCDR3 having SEQ ID NO: 39, optionally wherein the VL comprises SEQ ID NO: 154;

(s) a VH comprising a HCDR1 having SEQ ID NO: 87, a HCDR2 having SEQ ID NO: 64 and a HCDR3 having SEQ ID NO: 18, optionally wherein the VH comprises SEQ ID NO: 133; and

a VL comprising a LCDR1 having SEQ ID NO: 110, a LCDR2 having SEQUENCE A17 (of Figure 1) and a LCDR3 having SEQ ID NO: 41, optionally wherein the VL comprises SEQ ID NO: 156;

(t) a VH comprising a HCDR1 having SEQ ID NO: 89, a HCDR2 having SEQ ID NO: 66 and a HCDR3 having SEQ ID NO: 20, optionally wherein the VH comprises SEQ ID NO: 135; and

a VL comprising a LCDR1 having SEQ ID NO: 112, a LCDR2 having SEQUENCE A19 (of Figure 1) and a LCDR3 having SEQ ID NO: 43, optionally wherein the VL comprises SEQ ID NO: 158;

(u) a VH comprising a HCDR1 having SEQ ID NO: 90, a HCDR2 having SEQ ID NO: 67 and a HCDR3 having SEQ ID NO: 21, optionally wherein the VH comprises SEQ ID NO: 136; and

a VL comprising a LCDR1 having SEQ ID NO: 113, a LCDR2 having SEQUENCE A20 (of Figure 1) and a LCDR3 having SEQ ID NO: 44, optionally wherein the VL comprises SEQ ID NO: 159;

(v) a VH comprising a HCDR1 having SEQ ID NO: 91, a HCDR2 having SEQ ID NO: 68 and a HCDR3 having SEQ ID NO: 22, optionally wherein the VH comprises SEQ ID NO: 137; and

a VL comprising a LCDR1 having SEQ ID NO: 114, a LCDR2 having SEQUENCE A21 (of Figure 1) and a LCDR3 having SEQ ID NO: 45, optionally wherein the VL comprises SEQ ID NO: 160;

and/or

(w) a VH comprising a HCDR1 having SEQ ID NO: 93, a HCDR2 having SEQ ID NO: 70 and a HCDR3 having SEQ ID NO: 24, optionally wherein the VH comprises SEQ ID NO: 139; and

a VL comprising a LCDR1 having SEQ ID NO: 116, a LCDR2 having SEQUENCE A23 (of Figure 1) and a LCDR3 having SEQ ID NO: 47, optionally wherein the VL comprises SEQ ID NO: 162.

In some embodiments, the anti-Vγ4 antibody or fragment thereof comprises an amino acid sequence having at least 80% sequence identity with any one of SEQ ID NOs: 163-185.

In some embodiments, the anti-Vγ4 antibody comprises an amino acid sequence having at least 80% sequence identity with any one of SEQ ID NOs: 233-255. In a related embodiment, the anti-Vγ4 antibody comprises or consists of a heavy chain amino acid sequence having at least 80% sequence identity with any one of SEQ ID NOs: 284-306 and/or a light chain amino acid sequence having at least 80% sequence identity with any one of SEQ ID NOs: 307-329.

In some embodiments, the anti-Vγ4 antibody or fragment thereof specifically binds to a Vγ4 chain of a γδ T cell receptor (TCR) and competes with binding to the Vγ4 chain of a γδ T cell receptor (TCR) with an antibody or fragment thereof as defined herein.

According to a further aspect of the invention, there is provided a Vγ4 T cell population obtained by the *ex vivo* method as defined herein.

According to a further aspect of the invention, there is provided a composition comprising the Vγ4 T cell population as defined herein.

According to a further aspect of the invention, there is provided a pharmaceutical composition comprising the Vγ4 T cell population as defined herein, optionally together with a pharmaceutically acceptable diluent or carrier.

According to a further aspect of the invention, there is provided the pharmaceutical composition of the invention as defined herein, for use as a medicament. Similarly, there is provided a method of treating a disease or disorder (e.g. cancer, an infectious disease or an inflammatory disease) in a subject in need thereof, comprising administering to the subject a therapeutically effective amount of the Vγ4 T cell population of the invention or the pharmaceutical composition of the invention as defined herein.

## BRIEF DESCRIPTION OF THE FIGURES

**Figure 1: Complementarity Determining Region (CDR) sequences of exemplary anti-Vγ4 antibodies.** Shown are the CDR sequences for exemplary anti-Vγ4 antibodies described herein. The corresponding SEQ ID NO. is shown to the right of each sequence.

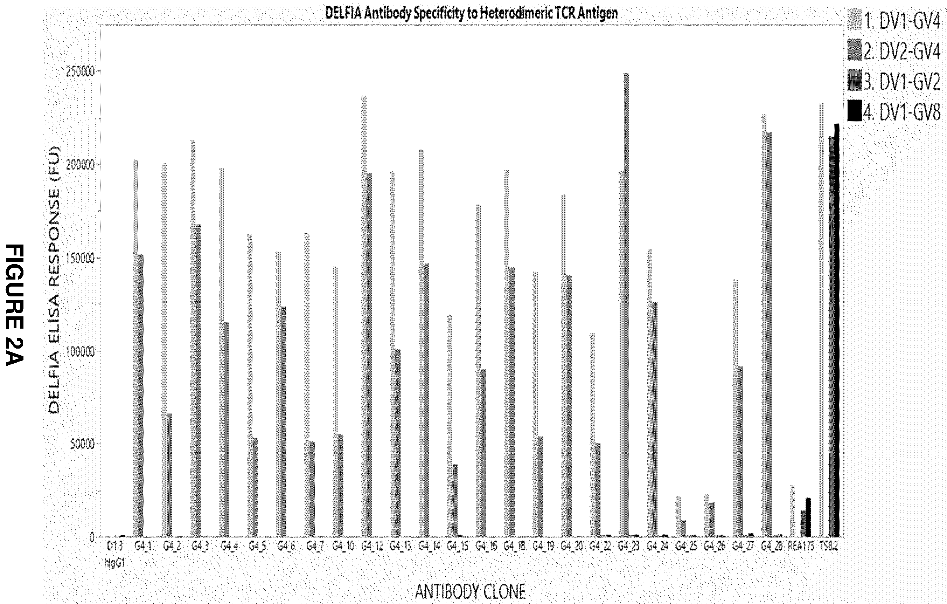

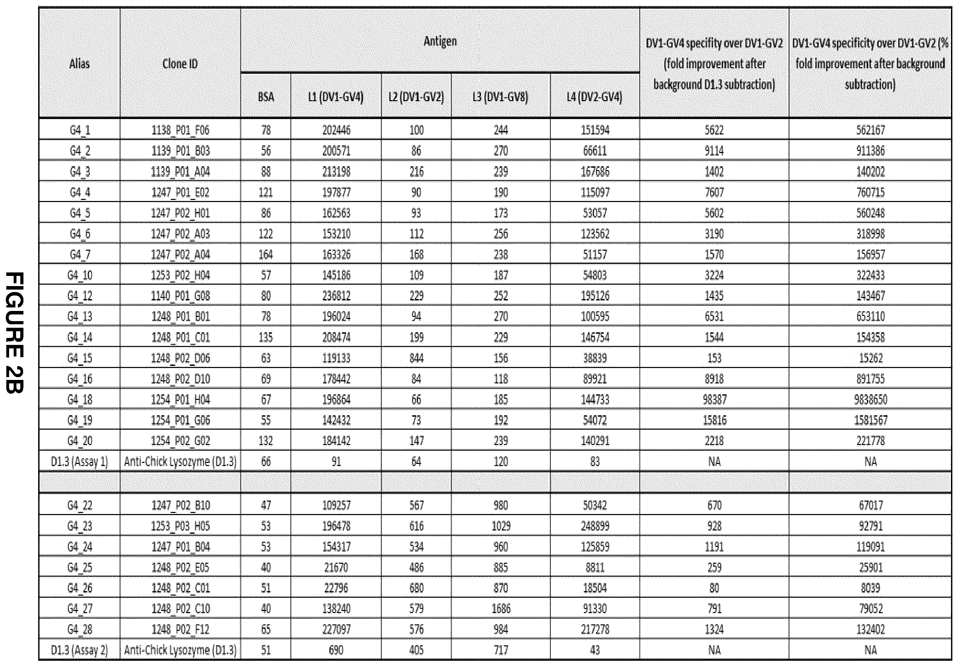

**Figure 2: Antibody specificity against heterodimeric TCR antigens via DELFIA Elisa Assay.** (A) Presented are the results for all antibodies that passed QC assessment (Analytical SEC-HPLC) and which also exhibited specificity for human Vγ4 chain. These antibodies (X-axis) were tested for binding against four different recombinant heterodimeric human TCRs respectively (DV1-GV4; DV2-GV4; DV1-GV2; DV1-GV8). Controls include the isotype controlled anti-chicken lysozyme D1.3 antibody (in-house, far left) plus anti-Vδ1 antibodies REA173 (Miltenyi) and TS8.2 (Fisher) - far right. (B) Quantification of the data shown in (A) and further showing the fold-change increase in binding of each example clone to the human Vγ4 chain versus the human Vγ2 chain.

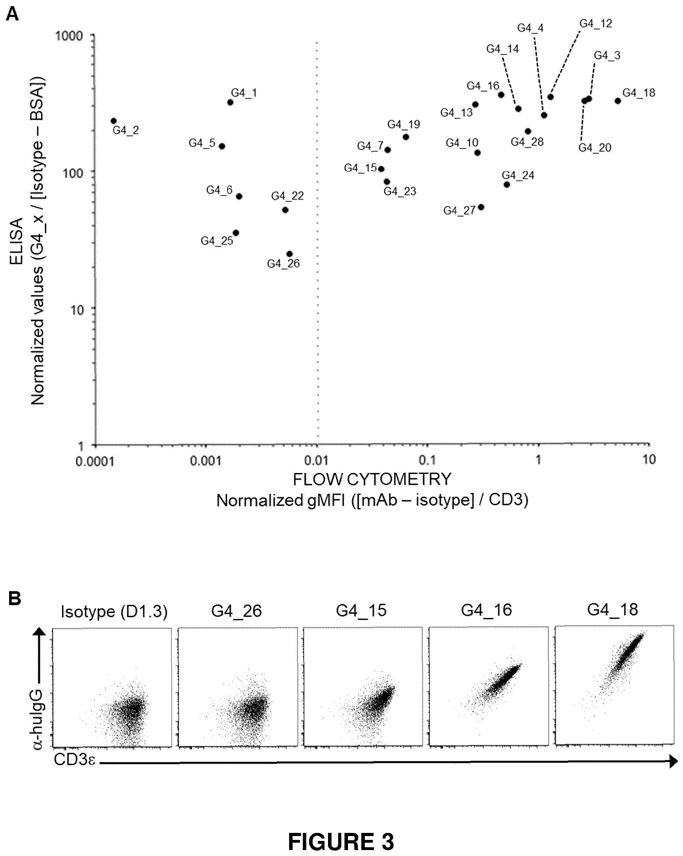

**Figure 3: A comparison of antibody binding to Vγ4Vδ1 TCRs presented as either recombinant antigens or as recombinant cell surface receptors.** (A) Normalized and log transformed X/Y plot of antibody binding to either the DV1-GV4 antigen via Delfia ELISA (Y-axis) or to JRT3-hu17 cells (X-axis). Vertical grey dotted line indicates the cut-off for mAbs considered negative (left) and positive (right) for JRT3-hu17 binding in this study. X-axis gMFI signal was normalized to CD3 to account for the variation in TCR expression between each construct. (B) Flow data plot to further illustrate the negative/positive cut-off. Antibody G4_26 (mid-left panel) exhibits the highest normalized gMFI value among the negative group and exhibits a similar plot to the negative isotype control (D1.3; far left panel). G4_15 (middle panel) has the lowest normalized gMFI value among the positive group and exhibits a clear, albeit weak, staining enhancement when compared to the D1.3 isotype control. Examples of intermediate (G4_16; mid-right) and strong (G4_18; far right) signals are also provided for reference.

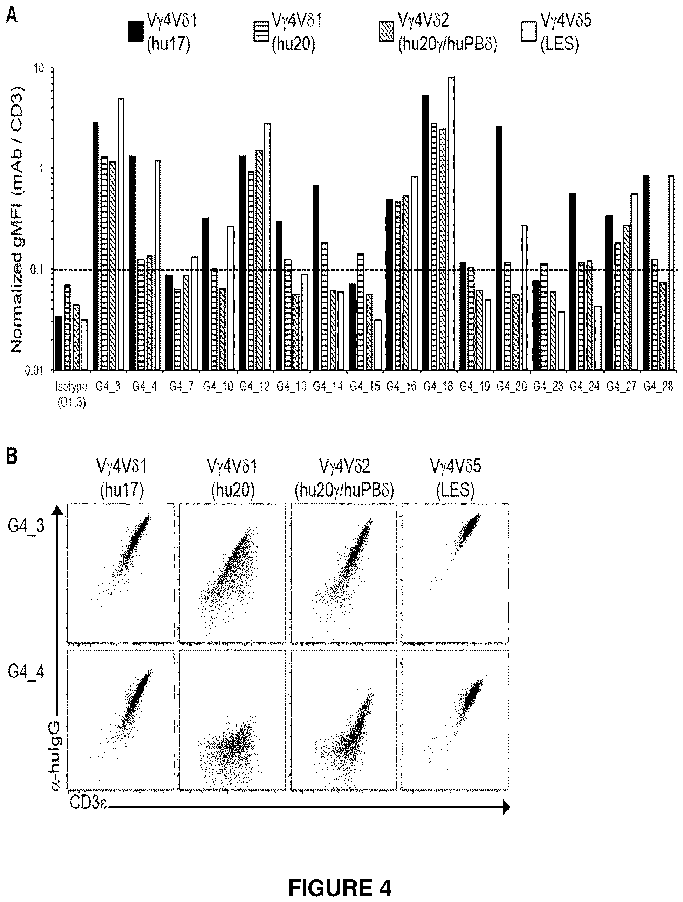

**Figure 4: Antibody binding to a panel of recombinantly expressed γ4 TCRs containing differing CDR3 sequences and/or paired with differing delta chains.** (A) Histogram representation of antibody binding signals generated against recombinant TCRs expressed on Jurkat cells. Sequential analysis presented as follows: Antibody binding signal against Vγ4Vδ1-hu17 (black bars); antibody binding signal against Vγ4Vδ1-hu20 (horizontal striped bars); antibody binding signal against Vγ4Vδ2 hu20γ-PBδ (diagonal striped bars); antibody binding signal against Vγ4Vδ5-LES (white bars). All binding signals normalized to CD3 to account for the variation in TCR expression between differing TCR constructs in JRT3 cells. (B) Example flow data for two of the lead antibodies in this study to further illustrate the difference between an exemplar antibody (G4_3) shown positive for all Vγ4 TCRs versus another lead antibody (G4_4) shown positive for only some of the Vγ4 TCRs.

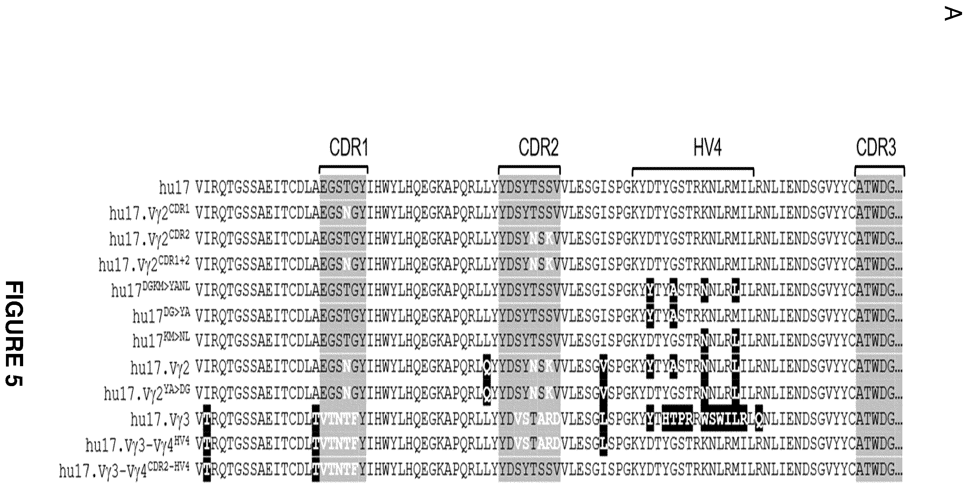

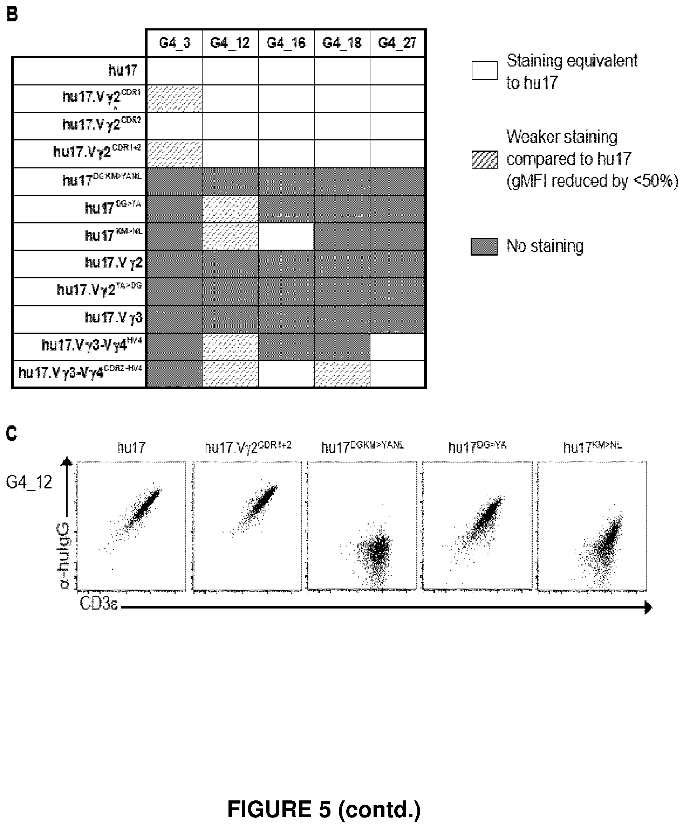

**Figure 5: Antibody binding and epitope mapping against chimeric hu17 TCRs expressed on JRT3 cells.** (A) Alignment of the germ line-encoded variable gamma regions of the indicated chimeric hu17 constructs is presented. Note that due to space limitations, the first 10 amino acids of the mature Vγ2/3/4 sequences (amino acids 1-10 of SEQ ID NO: 256 [SSNLEGRTKS]) are omitted but are identical across all constructs. Amino acids that are different from the reference hu17 sequence (wild-type Vγ4 TCR) are indicated. (B) Summary table of the reactivity of each antibody to the indicated chimeric TCR constructs. Results highlight the relative binding specificity of each indicated antibody to the individual TCRs expressed on JRT3 cells. (C) Example flow data of epitope mapping to illustrate the differential binding signals observed in this study.

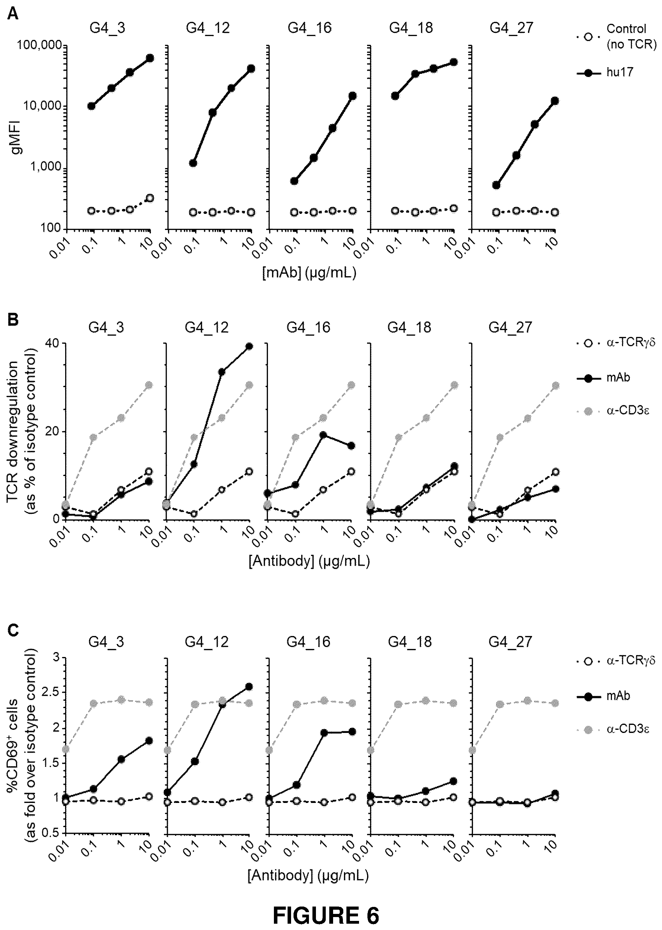

**Figure 6: Example antibody binding and conferred function on Vγ4 TCR (hu17) expressing cells.** (A) Titrated binding of antibodies to JRT3-hu17 showing all example antibodies bound to JRT3-hu17 cells. Non-transduced JRT3 cells (no TCR) employed as a negative control demonstrating that expression of hu17 was essential for antibody binding. (B) Conferred TCR downregulation by titrated antibodies versus downregulation conferred by positive control antibodies: anti-CD3ε (UCHT-1, Biolegend) or anti-pan-TCRγδ (B1, Biolegend). (C) Conferred CD69 upregulation by titrated antibodies versus upregulation observed by comparator antibodies: anti-CD3ε (UCHT-1, Biolegend) or anti-pan-TCRγδ (B1, Biolegend).

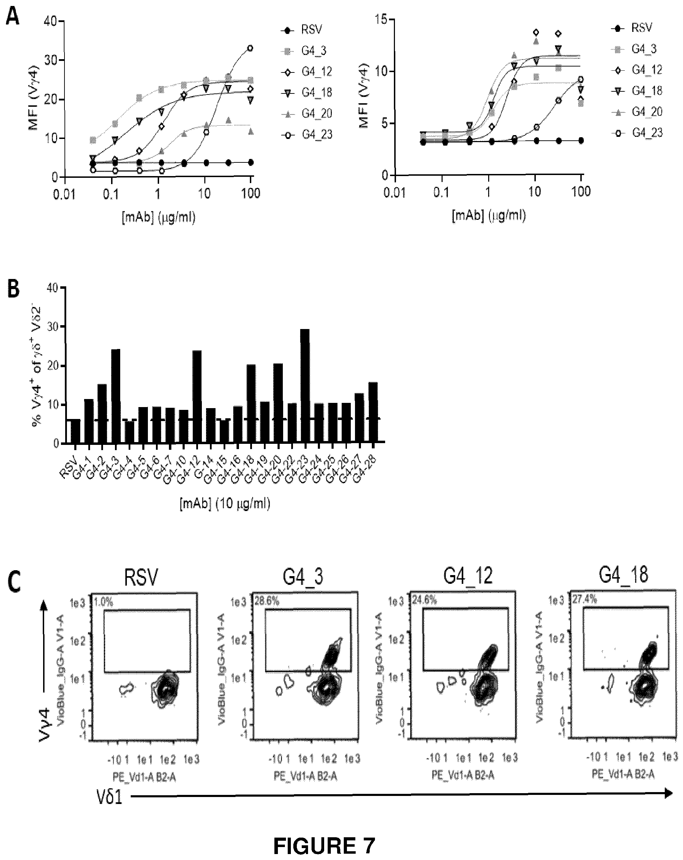

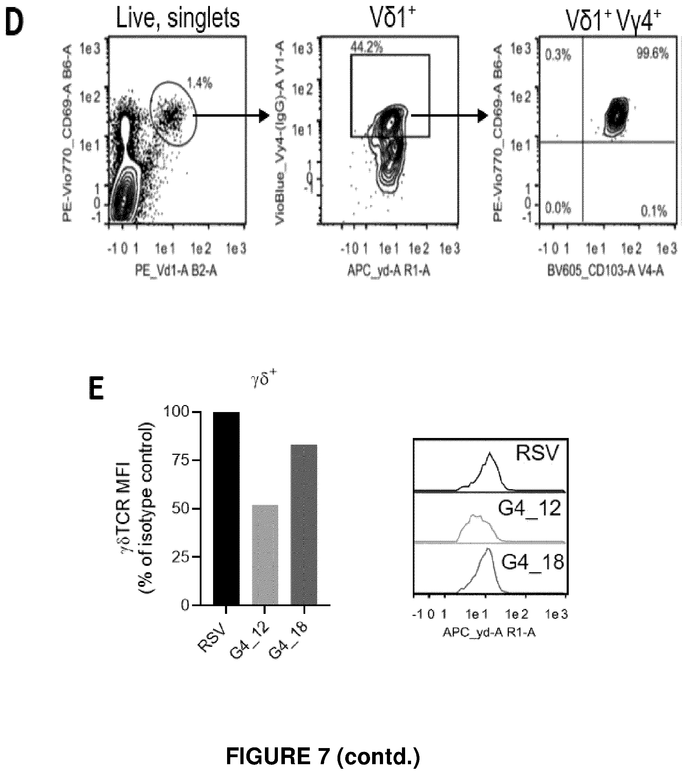

**Figure 7: Example antibody targeting and modulation of primary Vγ4-positive cells.** (A) Titrated binding of anti-Vγ4 antibodies to primary Vγ4+ T cells expanded from the skin of two individual donors, showing that all example antibodies could bind to primary skin-derived Vγ4+ T cells in a dose-dependent manner. Isotype control was employed as a negative control demonstrating the specificity for Vγ4 of the example antibodies. (B) Binding of anti-Vγ4 antibodies to Vγ4+ T cells derived from peripheral blood mononuclear cells (PBMCs), showing that substantially all of the example antibodies could bind to primary blood-derived Vγ4+ T cells. RSV isotype control was employed as a negative control. (C) Binding of the anti-Vγ4 antibodies G4_3, G4_12 and G4_18 to gut-derived intraepithelial lymphocytes (IELs) from colorectal cancer (CRC) patients, showing binding of all three example antibodies to this cell population. Cells were gated as single, live, γδ+, IgG1+ (Vγ4)+. (D) Phenotyping of Vγ4+ γδ T cells in the gut digest before stimulation with anti-Vγ4 antibodies, showing that example antibody, G4_18, could be used to identify Vγ4+ cells. 1.4% of live, single cells were Vδ1+. Of these, 44.2% were paired with Vγ4, and these displayed markers of tissue residency (CD69+ CD103+). (E) Conferred TCR downregulation by example antibodies, G4_12 and G4_18, respectively versus downregulation conferred by isotype negative control, accompanied by representative FACS plots.

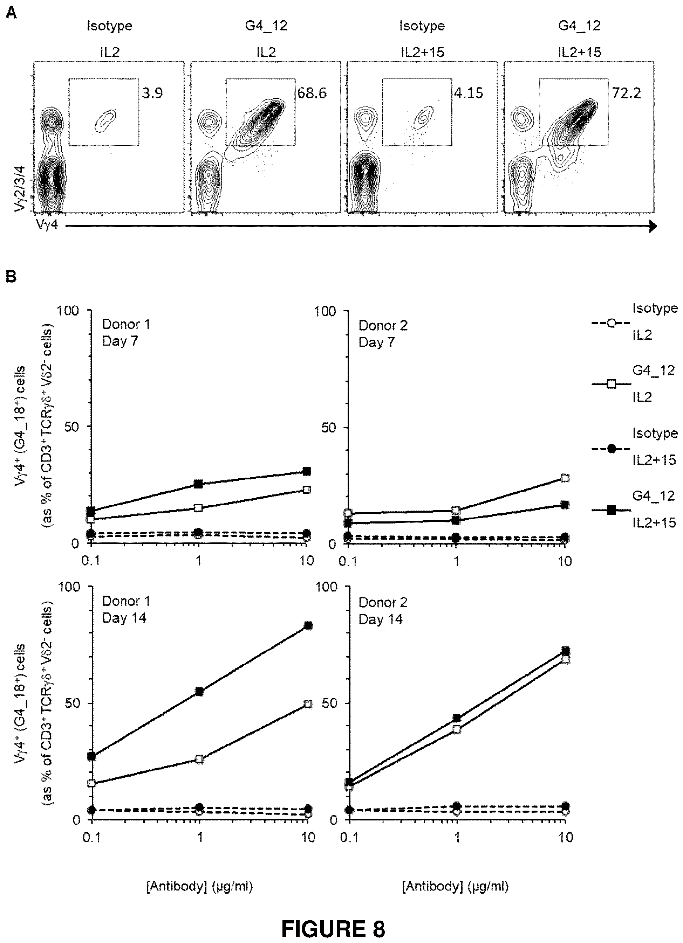

**Figure 8: Use of Vγ4-specific antibodies to increase the number of primary human Vγ4 T cells.** (A) Example flow data to illustrate the increase in Vγ4 T cells (as determined by staining with clone G4_18) following a 14 day culture of PBMC with plate-bound anti-Vγ4 clone G4_12 compared to isotype control, in the presence of IL-2 or IL-2 + IL-15. (B) Summary of the increase in Vγ4 T cells (as determined by staining with clone G4_18) after 7 days (top row) and 14 days (bottom row) cultures of PBMC from two donors with plate-bound anti-Vγ4 clone G4_12 compared to isotype control, in the presence of IL-2 or IL-2 + IL-15.

**DETAILED DESCRIPTION OF THE INVENTION**

## Definitions

Unless defined otherwise, all technical and scientific terms used herein have the meaning commonly understood by a person skilled in the art to which this invention belongs. As used herein, the following terms have the meanings ascribed to them below.

Gamma delta ($\gamma\delta$) T cells represent a small subset of T cells that express on their surface a distinct, defining T Cell Receptor (TCR). This TCR is made up of one gamma ($\gamma$) and one delta ($\delta$) chain. Each chain contains a variable (V) region, a constant (C) region, a transmembrane region and a cytoplasmic tail. The V region contains an antigen binding site. There are two major sub-types of human $\gamma\delta$ T cells: one that is dominant in the peripheral blood and one that is dominant in non-haematopoietic tissues. The two sub-types may be defined by the type of $\delta$ and/or $\gamma$ present on the cells. For example, most blood-resident $\gamma\delta$ T cells express a V$\delta$2 TCR, for example V$\gamma$9V$\delta$2, whereas this is less common among tissue-resident $\gamma\delta$ T cells, which more frequently use V$\delta$1 for example in skin and V$\gamma$4 in the gut. References to "V$\gamma$4 T cells" refer to $\gamma\delta$ T cells with a V$\gamma$4 chain, i.e. V$\gamma$4+ cells.

References to "gamma variable 4" may also be referred to as V$\gamma$4 or Vg4. A gamma variable 4 polypeptide, or a nucleotide encoding a TCR chain containing this region, or the TCR protein complex comprising this region, may be referred to as "TRGV4". Antibodies or fragments thereof which interact with the V$\gamma$4 chain of a $\gamma\delta$ TCR, are all effectively antibodies or fragments thereof which bind to V$\gamma$4 and may referred to as "anti-TCR gamma variable 4 antibodies or fragments thereof" or "anti-V$\gamma$4 antibodies or fragments thereof". Reference to a human V$\gamma$4 polypeptide may mean a polypeptide having an amino acid sequence corresponding to amino acids 1-99 of SEQ ID NO. 1. This 99 amino-acid sequence also corresponds to SEQ ID NO: 334. Therefore, it should be understood that reference herein to amino acids 1-99 of SEQ ID NO. 1 may be used interchangeably with reference to SEQ ID NO: 334, according to all aspects and embodiments of the invention. For instance, reference herein to amino acid region 67-82 of SEQ ID NO: 1 is equivalent with amino acid region 67-82 of SEQ ID NO: 334 and may be used interchangeably herein.

References to "delta variable 1" may also be referred to as V$\delta$1 or Vd1. A delta variable 1 polypeptide, or a nucleotide encoding a TCR chain containing this region, , or the TCR protein complex comprising this region, may be referred to as "TRDV1". Antibodies or fragments thereof which interact with the V$\delta$1 chain of a $\gamma\delta$ TCR, are all effectively antibodies or fragments thereof which bind to V$\delta$1 and may referred to as "anti-TCR delta variable 1 antibodies or fragments thereof" or "anti-V$\delta$1 antibodies or fragments thereof". Reference to a human V$\delta$1 polypeptide may mean a polypeptide having an amino acid sequence corresponding to SEQ ID NO. 337.

References to "gamma variable 2" may also be referred to as V$\gamma$2 or Vg2. A gamma variable 2 polypeptide, or a nucleotide encoding a TCR chain containing this region, , or the TCR protein complex comprising this region, may be referred to as "TRGV2". Antibodies or fragments thereof which interact with the V$\gamma$2 chain of a $\gamma\delta$ TCR, are all effectively antibodies or fragments thereof which bind to V$\gamma$2 and may referred to as "anti-TCR gamma variable 2 antibodies or fragments thereof" or "anti-V$\gamma$2 antibodies or fragments thereof". Reference to a human V$\gamma$2 polypeptide may mean a polypeptide having an amino acid sequence corresponding to SEQ ID NO. 335.

References to "gamma variable 8" may also be referred to as V$\gamma$8 or Vg8. A gamma variable 8 polypeptide, or a nucleotide encoding a TCR chain containing this region, , or the TCR protein complex comprising this region, may be referred to as "TRGV8". Antibodies or fragments thereof which interact with the V$\gamma$8 chain of a $\gamma\delta$ TCR, are all effectively antibodies or fragments thereof which bind to V$\gamma$8 and may referred to as "anti-TCR gamma variable 8 antibodies or fragments thereof" or "anti-V$\gamma$8 antibodies or fragments thereof". Reference to a human V$\gamma$8 polypeptide may mean a polypeptide having an amino acid sequence corresponding to SEQ ID NO. 336.

The term "antibody" includes any antibody protein construct comprising at least one antibody variable domain comprising at least one antigen binding site (ABS). Antibodies include, but are not limited to, immunoglobulins of types IgA, IgG, IgE, IgD, IgM (as well as subtypes thereof). The overall structure of Immunoglobulin G (IgG) antibodies assembled from two identical heavy (H)-chain and two identical light (L)-chain polypeptides is well established and highly conserved in mammals (Padlan (1994) Mol. Immunol. 31:169-217).

A conventional antibody or immunoglobulin (Ig) is a protein comprising four polypeptide chains: two heavy (H) chains and two light (L) chains. Each chain is divided into a constant region and a variable domain. The heavy (H) chain variable domains are abbreviated herein as VH, and the light (L) chain variable domains are abbreviated herein as VL. These domains, domains related thereto and domains derived therefrom, may be referred to herein as immunoglobulin chain variable domains. The VH and VL domains (also referred to as VH and VL regions) can be further subdivided into regions, termed "complementarity determining regions" ("CDRs"), interspersed with regions that are more conserved, termed "framework regions" ("FRs"). The framework and complementarity determining regions have been precisely defined (Kabat et al. Sequences of Proteins of Immunological Interest, Fifth Edition U.S. Department of Health and Human Services, (1991) NIH Publication Number 91-3242). There are also alternative numbering conventions for CDR sequences, for example those set out in Chothia et al. (1989) Nature 342: 877-883. In a conventional antibody, each VH and VL is composed of three CDRs and four FRs, arranged from amino-terminus to carboxy-terminus in the following order: FR1, CDR1, FR2, CDR2, FR3, CDR3, FR4. The conventional antibody tetramer of two heavy immunoglobulin chains and two light immunoglobulin chains is formed with the heavy and the light immunoglobulin chains interconnected by *e.g.* disulphide bonds, and the heavy chains similarly connected. The heavy chain constant region includes three domains, CH1, CH2 and CH3. The light chain constant region is comprised of one domain, CL. The variable domain of the heavy chains and the variable domain of the light chains are binding domains that interact with an antigen. The constant regions of the antibodies typically mediate the binding of the antibody to host tissues or factors, including various cells of the immune system (*e.g.* effector cells) and the first component (C1q) of the classical complement system.

A fragment of the antibody (which may also be referred to as "antibody fragment", "immunoglobulin fragment", "antigen-binding fragment" or "antigen-binding polypeptide") as used herein refers to a portion of an antibody (or constructs that contain said portion) that specifically binds to the target, the gamma variable 4 (Vγ4) chain of a γδ T cell receptor (*e.g.* a molecule in which one or more immunoglobulin chains is not full length, but which specifically binds to the target). Examples of binding fragments encompassed within the term antibody fragment include:

(i) a Fab fragment (a monovalent fragment consisting of the VL, VH, CL and CH1 domains);
(ii) a F(ab')2 fragment (a bivalent fragment consisting of two Fab fragments linked by a disulphide bridge at the hinge region);
(iii) a Fd fragment (consisting of the VH and CH1 domains);
(iv) a Fv fragment (consisting of the VL and VH domains of a single arm of an antibody);
(v) a single chain variable fragment, scFv (consisting of VL and VH domains joined, using recombinant methods, by a synthetic linker that enables them to be made as a single protein chain in which the VL and VH regions pair to form monovalent molecules);
(vi) a VH (an immunoglobulin chain variable domain consisting of a VH domain);
(vii) a VL (an immunoglobulin chain variable domain consisting of a VL domain);
(viii) a domain antibody (dAb, consisting of either the VH or VL domain);
(ix) a minibody (consisting of a pair of scFv fragments which are linked via CH3 domains); and
(x) a diabody (consisting of a noncovalent dimer of scFv fragments that consist of a VH domain from one antibody connected by a small peptide linker to a VL domain from another antibody).

"Human antibody" refers to antibodies having variable and constant regions derived from human germline immunoglobulin sequences. Human subjects administered with said human antibodies do not generate cross-species antibody responses (for example termed HAMA responses - human-anti-mouse antibody) to the primary amino acids contained within said antibodies. Said human antibodies may include amino acid residues not encoded by human germline immunoglobulin sequences (e.g. mutations introduced by random or site-specific mutagenesis or by somatic mutation), for example in the CDRs and in particular CDR3. However, the term is not intended to include antibodies in which CDR sequences derived from the germline of another mammalian species, such as a mouse, have been grafted onto human framework sequences. Human antibodies that are prepared, expressed, created or isolated by recombinant means, such as antibodies expressed using a recombinant expression vector transfected into a host cell, antibodies isolated from a recombinant, combinatorial human antibody library, antibodies isolated from an animal (e.g., a mouse) that is transgenic for human immunoglobulin genes or antibodies prepared, expressed, created or isolated by any other means that involves splicing of human immunoglobulin gene sequences to other DNA sequences, may also be referred to as "recombinant human antibodies".

Substituting at least one amino acid residue in the framework region of a non-human immunoglobulin variable domain with the corresponding residue from a human variable domain is referred to as "humanisation". Humanisation of a variable domain may reduce immunogenicity in humans.

"Specificity" refers to the number of different types of antigens or antigenic determinants to which a particular antibody or fragment thereof can bind. The specificity of an antibody is the ability of the antibody to recognise a particular antigen as a unique molecular entity and distinguish it from another. An antibody that "specifically binds" to an antigen or an epitope is a term well understood in the art. A molecule is said to exhibit "specific binding" if it reacts more frequently, more rapidly, with greater duration and/or with greater affinity with a particular target antigen or epitope, than it does with alternative targets. An antibody "specifically binds" to a target antigen or epitope if it binds with greater affinity, avidity, more readily, and/or with greater duration than it binds to other substances. An antibody (or fragment thereof) may be considered to specifically bind to a target if the binding is statistically significant compared to a non-relevant binder.

"Affinity", represented by the equilibrium constant for the dissociation of an antigen with an antigen-binding polypeptide (KD), is a measure of the binding strength between an antigenic determinant and an antigen-binding site on the antibody (or fragment thereof): the lesser the value of the KD, the stronger the binding strength between an antigenic determinant and the antigen-binding polypeptide. Alternatively, the affinity can also be expressed as the affinity constant (KA), which is 1/KD. Affinity can be determined by known methods, depending on the specific antigen of interest. For example. KD may be determined by surface plasmon resonance.

Any KD value less than $10^{-6}$ is considered to indicate binding. Specific binding of an antibody, or fragment thereof, to an antigen or antigenic determinant can be determined in any suitable known manner, including, for example, Scatchard analysis and/or competitive binding assays, such as radioimmunoassays (RIA), enzyme immunoassays (EIA) and sandwich competition assays, equilibrium dialysis, equilibrium binding, gel filtration, ELISA, surface plasmon resonance, or spectroscopy (e.g. using a fluorescence assay) and the different variants thereof known in the art.

"Avidity" is the measure of the strength of binding between an antibody, or fragment thereof, and the pertinent antigen. Avidity is related to both the affinity between an antigenic determinant and its antigen binding site on the antibody and the number of pertinent binding sites present on the antibody.

"Human tissue Vγ4+ cells," and "haemopoietic and blood Vγ4+ cells" and "tumour infiltrating lymphocyte (TIL) Vγ4+ cells," are defined as Vγ4+ cells contained in or derived from either human tissue or the haemopoietic blood system or human tumours respectively. All said cell types can be identified by their (i) location or from where they are derived and (ii) their expression of the Vγ4+ TCR.

Suitably, the antibody or fragment thereof (*i.e.* polypeptide) is isolated. An "isolated" polypeptide is one that is removed from its original environment. The term "isolated" may be used to refer to an antibody that is substantially free of other antibodies having different antigenic specificities (*e.g.* an isolated antibody that specifically binds Vγ4, or a fragment thereof, is substantially free of antibodies that specifically bind antigens other than Vγ4). The term "isolated" may also be used to refer to preparations where the isolated antibody is sufficiently pure to be administered therapeutically when formulated as an active ingredient of a pharmaceutical composition, or at least 70-80% (w/w) pure, more preferably, at least 80-90% (w/w) pure, even more preferably, 90-95% pure; and, most preferably, at least 95%, 96%, 97%, 98%, 99%, or 100% (w/w) pure.

Suitably, the polynucleotides used in the present invention are isolated. An "isolated" polynucleotide is one that is removed from its original environment. For example, a naturally-occurring polynucleotide is isolated if it is separated from some or all of the coexisting materials in the natural system. A polynucleotide is considered to be isolated if, for example, it is cloned into a vector that is not a part of its natural environment or if it is comprised within cDNA.

The antibody or fragment thereof may be a "functionally active variant" which also includes naturally occurring allelic variants, as well as mutants or any other non-naturally occurring variants. As is known in the art, an allelic variant is an alternate form of a (poly)peptide that is characterized as having a substitution, deletion, or addition of one or more amino acids that essentially does not alter the biological function of the polypeptide. By way of non-limiting example, said functionally active variants may still function when the frameworks containing the CDRs are modified, when the CDRs themselves are modified, when said CDRs are grafted to alternate frameworks, or when N- or C-terminal extensions are incorporated. Further, CDR-containing binding domains may be paired with differing partner chains such as those shared with another antibody. Upon sharing with so called 'common' light or 'common' heavy chains, said binding domains may still function. Further, said binding domains may function when multimerized. Further, 'antibodies or fragments thereof' may also comprise functional variants wherein the VH or VL or constant domains have been modified away or towards a different canonical sequence (for example as listed at IMGT.org) and which still function.

For the purposes of comparing two closely-related polypeptide sequences, the "% sequence identity" between a first polypeptide sequence and a second polypeptide sequence may be calculated using NCBI BLAST v2.0, using standard settings for polypeptide sequences (BLASTP). For the purposes of comparing two closely-related polynucleotide sequences, the "% sequence identity" between a first nucleotide sequence and a second nucleotide sequence may be calculated using NCBI BLAST v2.0, using standard settings for nucleotide sequences (BLASTN).

Polypeptide or polynucleotide sequences are said to be the same as or "identical" to other polypeptide or polynucleotide sequences, if they share 100% sequence identity over their entire length. Residues in sequences are numbered from left to right, *i.e.* from N- to C- terminus for polypeptides; from 5' to 3' terminus for polynucleotides.

In some embodiments, any specified % sequence identity of a sequence is calculated without the sequences of all 6 CDRs of the antibody. For example, the anti-Vγ4 antibody or antigen-binding fragment thereof may comprise a variable heavy chain region sequence having at least 70%, 75%, 80%, 85%, 90%, 91%, 92%, 93%, 94%, 95%, 96%, 97%, 98% or 99% to a specified variable heavy chain region sequence and/or a variable light chain region sequence having at least 70%, 75%, 80%, 85%, 90%, 91%, 92%, 93%, 94%, 95%, 96%, 97%, 98% or 99% identity to a specified variable light chain region sequence, wherein any amino acid variations occur only in the framework regions of the variable heavy and light chain region sequences. In such embodiments, the anti-Vγ4 antibody or fragment thereof having certain sequence identities retain the complete heavy and light chain CDR1, CDR2 and CDR3 sequences of the corresponding anti-Vγ4 antibody or fragment thereof.

A "difference" between sequences refers to an insertion, deletion or substitution of a single amino acid residue in a position of the second sequence, compared to the first sequence. Two polypeptide sequences can contain one, two or more such amino acid differences. Insertions, deletions or substitutions in a second sequence which is otherwise identical (100% sequence identity) to a first sequence result in reduced % sequence identity. For example, if the identical sequences are 9 amino acid residues long, one substitution in the second sequence results in a sequence identity of 88.9%. If first and second polypeptide sequences are 9 amino acid residues long and share 6 identical residues, the first and second polypeptide sequences share greater than 66% identity (the first and second polypeptide sequences share 66.7% identity).

Alternatively, for the purposes of comparing a first, reference polypeptide sequence to a second, comparison polypeptide sequence, the number of additions, substitutions and/or deletions made to the first sequence to produce the second sequence may be ascertained. An "addition" is the addition of one amino acid residue into the sequence of the first polypeptide (including addition at either terminus of the first polypeptide). A "substitution" is the substitution of one amino acid residue in the sequence of the first polypeptide with one different amino acid residue. Said substitution may be conservative or non-conservative. A "deletion" is the deletion of one amino acid residue from the sequence of the first polypeptide (including deletion at either terminus of the first polypeptide).

Using the three letter and one letter codes, the naturally occurring amino acids may be referred to as follows: glycine (G or Gly), alanine (A or Ala), valine (V or Val), leucine (L or Leu), isoleucine (I or Ile), proline (P or Pro), phenylalanine (F or Phe), tyrosine (Y or Tyr), tryptophan (W or Trp), lysine (K or Lys), arginine (R or Arg), histidine (H or His), aspartic acid (D or Asp), glutamic acid (E or Glu), asparagine (N or Asn), glutamine (Q or Gln), cysteine (C or Cys), methionine (M or Met), serine (S or Ser) and Threonine (T or Thr). Where a residue may be aspartic acid or asparagine, the symbols Asx or B may be used. Where a residue may be glutamic acid or glutamine, the symbols Glx or Z may be used. References to aspartic acid include aspartate, and glutamic acid include glutamate, unless the context specifies otherwise.

A "conservative" amino acid substitution is an amino acid substitution in which an amino acid residue is replaced with another amino acid residue of similar chemical structure and which is expected to have little influence on the function, activity or other biological properties of the polypeptide. Such conservative substitutions suitably are substitutions in which one amino acid within the following groups is substituted by another amino acid residue from within the same group:

| Group | Amino acid residue |
|---|---|
| Non-polar aliphatic | Glycine |
| | Alanine |
| | Valine |
| | Methionine |
| | Leucine |
| | Isoleucine |
| Aromatic | Phenylalanine |
| | Tyrosine |
| | Tryptophan |
| Polar uncharged | Serine |
| | Threonine |
| | Cysteine |
| | Proline |
| | Asparagine |
| | Glutamine |
| Negatively charged | Aspartate |
| | Glutamate |
| Positively charged | Lysine |
| | Arginine |
| | Histidine |

Suitably, a hydrophobic amino acid residue is a non-polar amino acid. More suitably, a hydrophobic amino acid residue is selected from V, I, L, M, F, W or C. In some embodiments, a hydrophobic amino acid residue is selected from glycine, alanine, valine, methionine, leucine, isoleucine, phenylalanine, tyrosine, or tryptophan.

As used herein, numbering of polypeptide sequences and definitions of CDRs and FRs are as defined according to the Kabat system (Kabat *et al.,* 1991, herein incorporated by reference in its entirety). A "corresponding" amino acid residue between a first and second polypeptide sequence is an amino acid residue in a first sequence which shares the same position according to the Kabat system with an amino acid residue in a second sequence, whilst the amino acid residue in the second sequence may differ in identity from the first. Suitably corresponding residues will share the same number (and letter) if the framework and CDRs are the same length according to Kabat definition. Alignment can be achieved manually or by using, for example, a known computer algorithm for sequence alignment such as NCBI BLAST v2.0 (BLASTP or BLASTN) using standard settings.

References herein to an "epitope" refer to the portion of the target which is specifically bound by the antibody or fragment thereof. Epitopes may also be referred to as "antigenic determinants". An antibody binds "essentially the same epitope" as another antibody when they both recognize identical or sterically overlapping epitopes. Commonly used methods to determine whether two antibodies bind to identical or overlapping epitopes are competition assays, which can be configured in a number of different formats (*e.g.* well plates using radioactive or enzyme labels, or flow cytometry on antigen-expressing cells) using either labelled antigen or labelled antibody. An antibody binds "the same epitope" as another antibody when they both recognize identical epitopes (i.e. all contact points between the antigen and the antibody are the same). For example, an antibody may bind the same epitope as another antibody when all contact points across a specified region of an antigen are identified as the same with the aid of a characterization method such as antibody/antigen cross-linking-coupled MS , HDX, X-ray crystallography, cryo-EM, or mutagenesis.

Further, with aid of such characterization methods, it is also possible to characterize antibodies which bind essentially the same epitope by recognizing some but not all of the identical contact points. Specifically, such antibodies may share a sufficient number of identical contact points in a specified antigenic region to deliver a broadly equivalent technical effect and/or equivalent antigen interaction selectivity. Additionally, in some instances whereby antibodies recognize essentially the same epitope and confer a broadly equivalent technical effect and/or interaction selectivity, it can also be useful to define the epitope binding footprint by the totality of antigen contacts inclusive of the most N-terminal antigen contact point through to the most C-terminal antigen contact point.

Epitopes found on protein targets may be defined as "linear epitopes" or "conformational epitopes". Linear epitopes are formed by a continuous sequence of amino acids in a protein antigen. Conformational epitopes are formed of amino acids that are discontinuous in the protein sequence, but which are brought together upon folding of the protein into its three-dimensional structure.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian and yeast vectors). Other vectors (*e.g.* non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, other forms of expression vectors are also included, such as viral vectors (*e.g.* replication defective retroviruses, adenoviruses and adeno-associated viruses), which serve equivalent functions, and also bacteriophage and phagemid systems. The term "recombinant host cell" (or simply "host cell"), as used herein, is intended to refer to a cell into which a recombinant expression vector has been introduced. Such terms are intended to refer not only to the particular subject cell but to the progeny of such a cell, for example, when said progeny are employed to make a cell line or cell bank which is then optionally stored, provided, sold, transferred, or employed to manufacture an antibody or fragment thereof as described herein.

References to "subject", "patient" or "individual" refer to a subject, in particular a mammalian subject, to be treated. Mammalian subjects include humans, non-human primates, farm animals (such as cows), sports animals, or pet animals, such as dogs, cats, guinea pigs, rabbits, rats or mice. In some embodiments, the subject is a human. In alternative embodiments, the subject is a non-human mammal, such as a mouse.

The term "sufficient amount" means an amount sufficient to produce a desired effect. The term "therapeutically effective amount" is an amount that is effective to ameliorate a symptom of a disease or disorder. A therapeutically effective amount can be a "prophylactically effective amount" as prophylaxis can be considered therapy.

A disease or disorder is "ameliorated" if the severity of a sign or symptom of the disease or disorder, the frequency with which such a sign or symptom is experienced by a subject, or both, is reduced.

As used herein, "treating a disease or disorder" means reducing the frequency and/or severity of at least one sign or symptom of the disease or disorder experienced by a subject.

"Cancer," as used herein, refers to the abnormal growth or division of cells. Generally, the growth and/or life span of a cancer cell exceeds, and is not coordinated with, that of the normal cells and tissues around it. Cancers may be benign, pre-malignant or malignant. Cancer occurs in a variety of cells and tissues.

"Inflammation" refers to a chronic or acute triggering of the immune system resulting in an inflamed cell, cell type, tissue, or organ.

As used herein, the term "about" includes up to and including 10% greater and up to and including 10% lower than the value specified, suitably up to and including 5% greater and up to and including 5% lower than the value specified, especially the value specified. The term "between", includes the values of the specified boundaries.

## Methods of modulating γδ T cells

According to a first aspect of the invention, there is provided an *ex vivo* method of modulating gamma variable 4 chain (Vγ4) T cells comprising administering an anti-Vγ4 antibody or fragment thereof as defined herein to a cell population comprising Vγ4 T cells. It will be understood that "administering" the antibody or fragment thereof includes "contacting" the Vγ4 T cells.

Modulation of Vγ4 T cells may include:

- expansion of the Vγ4 T cells, *e.g.* by selectively increasing the number of Vγ4 T cells or promotion of survival of Vγ4 T cells;
- stimulation of the Vγ4 T cells, *e.g.* by increasing Vγ4 T cell potency, *i.e.* increasing target cell killing;
- prevention of Vγ4 T cell exhaustion, *e.g.* by increasing persistence of the Vγ4 T cells;
- degranulation of Vγ4 T cells;
- immunosuppression of the Vγ4 T cells, *e.g.* by downregulation of Vγ4 TCR cell surface expression, *i.e.* by causing Vγ4 TCR internalisation or reduced expression of Vγ4 TCR protein, or blocking the Vγ4 TCR from binding;
- reducing Vγ4 T cell number, *e.g.* by inhibition of Vγ4 T cell proliferation or by inducing Vγ4 T cell death (*i.e.* killing Vγ4 T cells).

Such modulation of Vγ4 T cells may include, for example, Vγ4 T cell activation or Vγ4 T cell inhibition. In one embodiment, the Vγ4 T cells are activated by administering an anti-Vγ4 antibody or fragment thereof as defined herein. In an alternative embodiment, the Vγ4 T cells are inhibited by administering an anti-Vγ4 antibody or fragment thereof as defined herein. In an alternative embodiment, the Vγ4 T cells are not inhibited upon administration of an anti-Vγ4 antibody or fragment thereof as defined herein.

In one embodiment, the modulation of Vγ4 T cells comprises administering an anti-TCR gamma 4 variable antibody or fragment thereof to Vγ4 T cells in a culture *(i.e. in vitro* or *ex vivo)*. The Vγ4 T cells may be present in a mixed cell population, *e.g.* in a cell population comprising other lymphocyte cell types (*e.g.* αβ T cells or NK cells).

In one embodiment, the cell population comprising Vγ4 T cells is isolated (*i.e.* from a sample as described herein) prior to administration of the anti-Vγ4 antibody or fragment thereof. In a further embodiment, the cell population is enriched for T cells prior to administration of the anti-Vγ4 antibody or fragment thereof. In a yet further embodiment, the cell population is enriched for γδ T cells prior to administration of the anti-Vγ4 antibody or fragment thereof.

The method may also be performed on a cell population comprising a purified fraction of γδ T cells. In such embodiments, the cell population is depleted of cells types other than γδ T cells present in the sample, such as αβ T cells and/or NK cells, prior to administration of the anti-Vγ4 antibody or fragment thereof. The cell population may additionally, or alternatively, be enriched of cells types which may contain Vγ4, such as T cells and/or γδ cells, prior to administration of the anti-Vγ4 antibody or fragment thereof. For example, prior to culturing the sample, the sample may be enriched for T cells, or enriched for γδ T cells, or depleted of αβ T cells or depleted of non-γδ T cells. In one embodiment, the sample is first depleted of αβ T cells and then enriched for CD3+ cells. Enrichment or depletion may be achieved using techniques known in the art, such as using magnetic beads coated with antibodies that bind to molecules on the cell surface relevant to the phenotype to be enriched/depleted.

The presence of cell types other than lymphocytes in cell culture, may inhibit Vγ4 cell expansion. Such cells, e.g. stromal, epithelial, tumour and/or feeder cells, may be removed prior to culture. Thus, in one embodiment, the cell population is not in direct contact with stromal cells during culture. Examples of stromal cells include fibroblasts, pericytes, mesenchymal cells, keratinocytes, endothelial cells and non-haematological tumour cells. Preferably, the lymphocytes are not in direct contact with fibroblasts during culture. In one embodiment, the cell population is not in direct contact with epithelial cells during culture. In one embodiment, the cell population is not in direct contact with tumour cells and/or feeder cells during culture.

In one embodiment, the method comprises culturing the Vγ4 T cells in the absence of substantial stromal cell contact. In a further embodiment, the method comprises culturing the Vγ4 T cells in the absence of substantial fibroblast cell contact.

In one embodiment, the method comprises culturing the Vγ4 T cells in media which is substantially free of serum (*e.g.* serum-free media or media containing a serum-replacement (SR)). Thus, in one embodiment, the method comprises culturing in serum-free media. Such serum free medium may also include serum replacement medium, where the serum replacement is based on chemically defined components to avoid the use of human or animal derived serum. In an alternative embodiment, the method comprises culturing in media which contains serum (*e.g.* human AB serum or fetal bovine serum (FBS)). In one embodiment, the media contains serum-replacement. In one embodiment, the media contains no animal-derived products.

It will be appreciated that a sample cultured in serum-free media has the advantage of avoiding issues with filtration, precipitation, contamination and supply of serum. Furthermore, animal derived products are not favoured for use in clinical grade manufacturing of human therapeutics.

In one embodiment, the anti-Vγ4 antibody or fragment thereof is in a soluble or immobilized form. For example, the antibody or fragment thereof may be administered to the Vγ4 T cells in a soluble form. Alternatively, the antibody or fragment thereof may be administered to the Vγ4 T cells when the antibody or fragment thereof is bound or covalently linked to a surface, such as a bead or plate (*i.e.* in an immobilized form). In one embodiment, the antibody is immobilized on a surface, such as Fc-coated wells. Alternatively, the antibody or fragment thereof is bound to the surface of a cell (*e.g.* immobilized on the surface of an antigen presenting cell (APC)). In another embodiment, the antibody is not immobilized on a surface when the cell population is contacted with the antibody.

The cell population contacted by the anti-Vγ4 antibody or fragment thereof may be obtained from a variety of sample types (methods of isolation are further described below). In one embodiment, the sample is a non-haematopoietic tissue sample. References herein to "non-haematopoietic tissues" or "non-haematopoietic tissue sample" include skin (*e.g.* human skin) and gut (*e.g.* human gut). Non-haematopoietic tissue is a tissue other than blood, bone marrow, lymphoid tissue, lymph node tissue, or thymus tissue. In one embodiment, the non-haematopoietic tissue sample is skin (*e.g.* human skin). In some embodiments, the cell population (*e.g.* γδ T cells) is not obtained from particular types of samples of biological fluids, such as blood or synovial fluid. In some embodiments, the cell population (*e.g.* γδ T cells) is obtained from skin (*e.g.* human skin), which can be obtained by methods known in the art. For example, the cell population may be obtained from the non-haematopoietic tissue sample by culturing the non-haematopoietic tissue sample on a synthetic scaffold configured to facilitate cell egress from the non-haematopoietic tissue sample. Alternatively, the methods can be applied to a cell population (*e.g.* γδ T cells) obtained from the gastrointestinal tract (*e.g.* colon or gut), mammary gland, lung, prostate, liver, spleen, pancreas, uterus, vagina and other cutaneous, mucosal or serous membranes.

In an alternative embodiment, the sample is a haematopoietic sample or fraction thereof (*i.e.* the cell population is obtained from a haematopoietic sample or a fraction thereof). References herein to "haematopoietic sample" or "haematopoietic tissue sample" include blood (such as peripheral blood or umbilical cord blood), bone marrow, lymphoid tissue, lymph node tissue, thymus tissue, and fractions or enriched portions thereof. The sample is preferably blood including peripheral blood or umbilical cord blood or fractions thereof, including buffy coat cells, leukapheresis products, peripheral blood mononuclear cells (PBMCs) and low density mononuclear cells (LDMCs). In some embodiments the sample is human blood or a fraction thereof. The cells may be obtained from a sample of blood using techniques known in the art such as density gradient centrifugation. For example, whole blood may be layered onto an equal volume of FICOLL-HYPAQUE followed by centrifugation at 400xg for 15-30 minutes at room temperature. The interface material will contain low density mononuclear cells which can be collected and washed in culture medium and centrifuged at 200xg for 10 minutes at room temperature.

The cell population may be obtained from a cancer tissue sample (*i.e.* the γδ T cells may also be resident in cancer tissue samples), *e.g.* tumours of the gut, breast or prostate. In some embodiments, the cell population may be from human cancer tissue samples (*e.g.* solid tumour tissues). In other embodiments, the cell population may be from a sample other than human cancer tissue (*e.g.* a tissue without a substantial number of tumour cells). For example, the cell population may be from a region of skin (*e.g.* healthy skin) separate from a nearby or adjacent cancer tissue. Thus, in some embodiments, the cell population is not obtained from cancer tissue (*e.g.* human cancer tissue).

The cell population may be obtained from human or non-human animal tissue. Therefore, the method may additionally comprise a step of obtaining a cell population from human or non-human animal tissue. In one embodiment the sample has been obtained from a human. In an alternative embodiment, the sample has been obtained from a non-human animal subject.

Expansion of γδ T cells

In one embodiment, the modulation comprises activation of the Vγ4 T cells, in particular expansion of the Vγ4 T cells. Therefore, according to an aspect of the invention, there is provided an *ex vivo* method of expanding Vγ4 T cells comprising administering an anti-Vγ4 antibody or fragment thereof as defined herein to a cell population comprising Vγ4 T cells. Such expansion of Vγ4 T cells may be achieved through the selective increase in number of Vγ4 T cells and/or through the promotion of survival of Vγ4 T cells. In one embodiment, the expansion of Vγ4 T cells comprises administering an anti-TCR gamma 4 variable antibody or fragment thereof to Vγ4 T cells in a culture (*i.e. in vitro* or *ex vivo*). The Vγ4 T cells may be present in a mixed cell population, *e.g.* in a cell population comprising other lymphocyte cell types (*e.g.* αβ T cells or NK cells).

The invention therefore provides *ex vivo* methods for producing an enriched γδ T cell (*e.g.* Vγ4 T cell) population. The enriched population can be produced from an isolated mixed cell populations (*e.g.* obtained from samples taken from patients/donors) by a method comprising contacting the mixed cell population, or a purified fraction thereof, with the antibody or fragment thereof. Said antibody (or fragment thereof) selectively expands Vγ4 T cells by binding to an epitope specific to a Vγ4 chain of a γδ TCR.

Also provided is an expanded Vγ4 T cell population obtained according to the method as defined herein. According to this aspect of the invention, it will be appreciated that such an expanded population of Vγ4 T cells may be obtained and/or expanded *in vitro* or *ex vivo.* In one aspect, there is provided an expanded Vγ4 population obtained according to the method as defined herein, wherein the Vγ4 population is isolated and expanded *in vitro* or *ex vivo.*

Antibodies or fragments thereof as described herein may be used in methods of expanding γδ T cells (*e.g.* Vγ4 T cells). These methods may be carried out *in vitro.* If the expansion methods are carried out *in vitro,* the antibodies (or fragments thereof) may be applied to isolated γδ T cells (*e.g.* Vγ4 T cells) obtained as described above. In some embodiments, the γδ T cells are expanded from a cell population that has been isolated from a non-haematopoietic tissue sample. In an alternative embodiment, the γδ T cells are expanded from a cell population that has been isolated from a haematopoietic tissue sample, such as a blood sample.

Expansion of γδ T cells (*e.g.* Vγ4 T cells) may comprise culturing the sample in the presence of the antibody or fragment thereof as described herein, and a cytokine. Cytokines may include interleukins, lymphokines, interferons, colony stimulating factors and chemokines. In one embodiment, the cytokine is selected from the group consisting of interleukin-2 (IL-2), interleukin-4 (IL-4), interleukin-6 (IL-6), interleukin-7 (IL-7), interleukin-8 (IL-8), interleukin-9 (IL-9), interleukin-12 (IL-12), interleukin-18 (IL-18), interleukin-21 (IL-21), interleukin-33 (IL-33), insulin-like growth factor 1 (IGF-1), interleukin-1β (IL-1β), interferon-γ (IFN-γ) and stromal cell-derived factor-1 (SDF-1). It will be understood that references to the cytokines as described herein, may include any compound that has the same activity as said cytokine with respect to its ability to promote similar physiological effects on Vγ4 T cells in culture and includes, but is not limited to, mimetics, or any functional equivalent thereof.

In one embodiment, said cytokine is a common cytokine receptor gamma-chain (γc) family of cytokines. In a further embodiment, the γc-cytokine is selected from: IL-2, IL 4, IL-7, IL-9, IL-12, IL-15, IL-21 or mixtures thereof.

The cytokine (*e.g.* an interleukin) used may be of human or animal origin, preferably of human origin. It may be a wild-type protein or any biologically active fragment or variant, that is, to say, capable of binding its receptor. Such binding may induce activation of γδ T cells in the conditions of a method according to the invention. More preferably, the cytokines may be in soluble form, fused or complexed with another molecule, such as for example a peptide, polypeptide or biologically active protein. Preferably, a human recombinant cytokine is used. More preferably, the range of interleukin concentration could vary between 1-10000 U/ml, even more preferably between 100-1000 U/ml.

In a further embodiment, the cytokine is a chemokine. It will be further appreciated that the chemokine will vary and be selected depending on the sample used to obtain the γδ T cells.

In one embodiment, the method comprises culturing the cell population in the presence of IL-2, IL-9 and/or IL-15. In a further embodiment, the method comprises culturing the cell population in the presence of IL-2. In a further embodiment, the method comprises culturing the cell population in the presence of IL-2 and/or IL-15 (*i.e.* IL 2, IL-15 or a combination thereof). In an alternative embodiment, the method comprises culturing the cell population in the presence of IL-9 and/or IL-15 (*i.e.* IL 9, IL-15 or a combination thereof). In one embodiment, the method comprises the cell population in the presence of IL-2, IL-9 and/or IL-15, and an additional growth factor (for example, IL-21). In other embodiments, the method comprises culturing a cell population in a medium devoid of growth factors other than IL-2 and/or IL-15. In alternative embodiments, the method comprises culturing a cell population in a medium devoid of growth factors other than IL-9 and/or IL-15. In a further embodiment, the method comprises culturing a cell population in a medium which consists of a basal medium supplemented with IL-2, IL-9 and/or IL-15. In a further embodiment, the method comprises culturing a cell population in a medium which consists of a basal medium supplemented with IL-2 and/or IL-15.

In one embodiment, the method comprises culturing the cell population in the presence of IL-15 and a factor selected from the group consisting of IL-2, IL-4, IL-21, IL-6, IL-7, IL-8, IL-9, IL-12, IL-18, IL-33, IGF-1, IL-1β, IFN-γ, human platelet lysate (HPL), and stromal cell-derived factor-1 (SDF-1).

Expansion of γδ T cells may comprise culturing the sample in the presence of at least one further T cell mitogen. The term "a T cell mitogen" (which may also be referred to as "a γδ TCR agonist") means any agent that can stimulate T cells through TCR signalling including, but not limited to, plant lectins such as phytohemagglutinin (PHA) and concanavalin A (ConA) and lectins of non-plant origin. In one embodiment, the T cell mitogen is an anti-CD3 monoclonal antibody (mAb). Other mitogens include phorbol 12-myristate-13-acetate (TPA) and its related compounds, such as mezerein, or bacterial compounds (*e.g.* Staphylococcal enterotoxin A (SEA) and Streptococcal protein A). The T cell mitogen may be soluble or immobilized and more than one T cell mitogen may be used in the method of expansion.

As used herein, references to "expanded" or "expanded population of γδ T cells" includes populations of cells which are larger or contain a larger number of cells than a non-expanded population. Such populations may be large in number, small in number or a mixed population with the expansion of a proportion or particular cell type within the population. It will be appreciated that the term "expansion method" refers to processes which result in expansion or an expanded population. Thus, expansion or an expanded population may be larger in number or contain a larger number of cells compared to a population which has not had an expansion step performed or prior to any expansion step. It will be further appreciated that any numbers indicated herein to indicate expansion (*e.g.* fold-increase or fold-expansion) are illustrative of an increase in the number or size of a population of cells or the number of cells and are indicative of the amount of expansion.

In one embodiment, the method comprises culturing the cell population for at least 5 days (*e.g.* at least 6 days, at least 7 days, at least 8 days, at least 9 days, at least 10 days, at least 11 days, at least 12 days, at least 13 days, at least 14 days, at least 18 days, at least 21 days, at least 28 days, or longer, *e.g.* from 5 days to 40 days, from 7 days to 35 days, from 14 days to 28 days, from 14 days to 21 days or about 14 days). In a further embodiment, the method comprises culturing the cell population for at least 7 days, such as at least 11 days or at least 14 days.

In further embodiments, method comprises culturing the cell population for a duration (*e.g.* at least 5 days, at least 6 days, at least 7 days, at least 8 days, at least 9 days, at least 10 days, at least 11 days, at least 12 days, at least 13 days, at least 14 days, at least 18 days, at least 21 days, at least 28 days, or longer, *e.g.* from 5 days to 40 days, from 7 days to 35 days, from 14 days to 28 days, from 14 days to 21 days or about 14 days) in an amount effective to produce an expanded population of γδ T cells.

In one embodiment, the cell population is cultured for a period of 5 to 60 days, such as at least 7 to 45 days, 7 to 21 days, from 7 days to 18 days or 7 to 14 days. If the method includes an isolation culture period (e.g. of 1 to 40 days, such as 14 to 21 days), the isolation and expansion steps, in some embodiments, can last between 21 and 39 days.

The method may comprise regular addition of the anti-Vγ4 antibody or fragment thereof and/or growth factor during culturing. For example, the anti-Vγ4 antibody or fragment thereof and/or growth factor could be added every 2 to 5 days, more preferably every 3 to 4 days. In one embodiment, the anti-Vγ4 antibody or fragment thereof and/or growth factor is added after 7 days of culture and every 2 to 3 days thereafter.

Methods of expansion provide an expanded population of γδ T cells that is greater in number than a reference population. In some embodiments, the expanded population of γδ T cells (*e.g.* Vγ4 T cells) is greater in number than the isolated population of γδ T cells prior to the expansion step (*e.g.* at least 2-fold in number, at least 5-fold in number, at least 10-fold in number, at least 25-fold in number, at least 50-fold in number, at least 60-fold in number, at least 70-fold in number, at least 80-fold in number, at least 90-fold in number, at least 100-fold in number, at least 200-fold in number, at least 300-fold in number, at least 400-fold in number, at least 500-fold in number, at 600-fold in number, at least 1,000-fold in number, or more relative to the isolated population of γδ T cells prior to the expansion step). In one embodiment, the expanded population of γδ T cells (*e.g.* Vγ4 T cells) is greater in number than a population cultured for the same length of time without the presence of the antibody or fragment thereof. In one embodiment, the expanded population of γδ T cells (*e.g.* Vγ4 T cells) is greater in number than a population cultured for the same length of time in the presence of an isotype control, such as human IgG1.

Methods of expansion provide an expanded population of Vγ4 T cells that has a higher percentage of Vγ4 T cells than a reference population. In some embodiments, the expanded population of Vγ4 T cells contains greater than about 50% Vγ4 T cells, such as greater than about 55%, 60%, 65%, 70%, 75%, 80%, 85%, 87%, 90%, 91%, 92%, 93%, 94% or 95% Vγ4 T cells. In a further embodiment, the expanded population of Vγ4 T cells contains greater than about 60% Vγ4 T cells, such as greater than about 70% Vγ4 T cells.

Numerous basal culture media suitable for use in the proliferation of γδ T cells are available, in particular medium, such as AIM-V, Iscoves medium and RPMI-1640 (Life Technologies), EXVIVO-10, EXVIVO-15 or EXVIVO-20 (Lonza), in the presence of serum or plasma. The medium may be supplemented with other media factors as defined herein, such as serum, serum proteins and selective agents, such as antibiotics. For example, in some embodiments, RPMI-1640 medium containing 2 mM glutamine, 10% FBS, 10 mM HEPES, pH 7.2, 1% penicillin-streptomycin, sodium pyruvate (1 mM; Life Technologies), non-essential amino acids (*e.g.* 100 μM Gly, Ala, Asn, Asp, Glu, Pro and Ser; 1X MEM non-essential amino acids (Life Technologies)), and/or 10 μl/L β-mercaptoethanol. In some embodiments, the media comprises RPMI-1640 supplemented with 5% human AB serum, Sodium Pyruvate (1 mM; Life Technologies) and penicillin/streptomycin. In an alternative embodiment, AIM-V medium may be supplemented with CTS Immune serum replacement and amphotericin B. In certain embodiments, the media may be further supplemented with IL-2, IL-4, IL-9 and/or IL 15 as described herein. Conveniently, cells are cultured at 37°C in a humidified atmosphere containing 5% $CO_2$ in a suitable culture medium during isolation and/or expansion.

Addition of other factors in the expansion culture of γδ T cells may also be used. In one embodiment, such factors are used in the expansion which selectively promote the expansion of γδ T cells. For example, expansion may additionally comprise addition of exogenous cytokines to the expansion culture, such as interleukins. Such expansion may comprise culturing the γδ T cells in the presence of IL-2 and IL-15. Alternatively, expansion may comprise culturing the γδ T cells in the presence of IL-9 and IL-15. It will be appreciated that any expansion step is performed for a duration of time effective to produce an expanded population of γδ T cells.

Methods of expanding γδ T cells may comprise a population doubling time of less than 5 days (*e.g.* less than 4.5 days, less than 4.0 days, less than 3.9 days, less than 3.8 days, less than 3.7 days, less than 3.6 days, less than 3.5 days, less than 3.4 days, less than 3.3 days, less than 3.2 days, less than 3.1 days, less than 3.0 days, less than 2.9 days, less than 2.8 days, less than 2.7 days, less than 2.6 days, less than 2.5 days, less than 2.4 days, less than 2.3 days, less than 2.2 days, less than 2.1 days, less than 2.0 days, less than 46 hours, less than 42 hours, less than 38 hours, less than 35 hours, less than 32 hours).

**Methods of isolating γδ T cells**

As described herein, antibodies (or fragments thereof) may be applied to γδ T cells in culture, *i.e.* γδ T cells, which have been obtained from a sample. In one embodiment, the cell population is isolated from a sample prior to administering the anti-Vγ4 antibody or fragment thereof. Therefore, there is provided a method of modulating (in particular, expanding) Vγ4 T cells comprising administering an anti-Vγ4 antibody or fragment thereof as defined herein to a population of γδ T cells (*e.g.* a cell population comprising Vγ4 T cells) isolated from a sample.

References herein to "isolation" or "isolating" of cells, in particular of γδ T cells, refer to methods or processes wherein cells are removed, separated, purified, enriched or otherwise taken out from a tissue or a pool of cells. It will be appreciated that such references include the terms "separated", "removed", "purified", "enriched" and the like. Isolation of γδ T cells includes the isolation or separation of cells from an intact non-haematopoietic tissue sample or from the stromal cells of the non-haematopoietic tissue (*e.g.* fibroblasts or epithelial cells). Such isolation may alternatively or additionally comprise the isolation or separation of γδ T cells from other haematopoietic cells (*e.g.* αβ T cells or other lymphocytes). Isolation may be for a defined period of time, for example starting from the time the tissue explant or biopsy is placed in the isolation culture and ending when the cells are collected from culture, such as by centrifugation or other means for transferring the isolated cell population to expansion culture or used for other purposes, or the original tissue explant or biopsy is removed from the culture. The isolation step may be for at least about 3 days to about 45 days. In one embodiment, the isolation step is for at least about 10 days to at least 28 days. In a further embodiment, the isolation step is for at least 14 days to at least 21 days. The isolation step may therefore be for at least 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, about 35 days, about 40 days, or about 45 days. It can be appreciated that although cell proliferation may not be substantial during this isolation step, it is not necessarily absent. Indeed for someone skilled in the art it is recognized that isolated cells may also start to divide to generate a plurality of such cells within the isolation vessel containing the sample.

Thus, references herein to "isolated γδ T cells", "isolated γδ T cell population" or "isolated population of γδ T cells" will be appreciated to refer to γδ cells that have been isolated, separated, removed, purified or enriched from the sample, such as a non-haematopoietic tissue sample of origin, such that the cells are out of substantial contact with cells contained within the intact (non-haematopoietic tissue) sample. References herein to "isolated Vγ4 T cells", "isolated Vγ4 T cell population", "isolated population of Vγ4 T cells", "separated Vγ4 T cells", "separated Vγ4 T cell population" or "separated population of Vγ4 T cells" will be appreciated to refer to Vγ4 T cells that have been isolated, separated, removed, purified or enriched from the sample, such as a non-haematopoietic tissue sample of origin, such that the cells are out of substantial contact with cells contained within the intact (non-haematopoietic tissue) sample.

Non-haematopoietic tissue resident lymphocytes can be harvested and separated from stromal cells, such as dermal fibroblasts, *e.g.* by firm pipetting. The lymphocyte harvest may further be washed through a 40μm nylon mesh in order to retain fibroblast aggregates that may have become loose during the process. Lymphocytes may also be isolated using fluorescent or magnetic associated cell sorting using, for example, CD45 antibodies.

Isolation of γδ T cells may comprise culturing the sample in the presence of at least one cytokine. For example, the method may comprise culturing the sample in the presence of at least agent, such as a chemokine. It will be further appreciated that chemokines will be selected depending on the γδ T cells being isolated. Furthermore, the chemokines will vary and be selected depending on the sample used for isolation of the γδ T cells.

Isolation of γδ T cells may comprise further culturing the sample in the presence of at least one cytokine. Said cytokine may be different to the cytokine used in the initial culture.

Isolation methods may comprise culturing the sample. References herein to "culturing" include the addition of the sample, including isolated, separated, removed, purified or enriched cells from the sample, to media comprising growth factors and/or essential nutrients required and/or preferred by the cells and/or sample. It will be appreciated that such culture conditions may be adapted according to the cells or cell population to be isolated from the sample or may be adapted according to the cells or cell population to be isolated and expanded from the sample.

In certain embodiments, culturing of the sample is for a duration of time sufficient for the isolation of γδ T cells from the sample. In certain embodiments, the duration of culture is at least 14 days. In certain embodiments, the duration of culture is less than 45 days, such as less than 30 days, such as less than 25 days. In a further embodiment, the duration of culture is between 14 days and 35 days, such as between 14 days and 21 days. In a yet further embodiment, the duration of culture is about 21 days.

In particular embodiments, the γδ T are collected from the culture after culturing of the sample. Collection of the γδ T cells may include the physical collection of γδ T cells from the culture, isolation of the γδ T cells from other lymphocytes (*e.g.* αβ T cells and/or NK cells) or isolation and/or separation of the γδ T cells from other cells present in the sample, *e.g.* stromal cells such as fibroblasts. In one embodiment, γδ T cells are collected by mechanical means (*e.g.* pipetting). In a further embodiment, γδ T cells are collected by means of magnetic separation and/or labelling. In a yet further embodiment, the γδ T cells are collected by flow cytometric techniques such as FACS. Thus, in certain embodiments, the γδ T cells are collected by means of specific labelling the γδ T cells. It will be appreciated that such collection of γδ T cells may include the physical removal from the culture of the sample, transfer to a separate culture vessel or to separate or different culture conditions.

It will be appreciated that such collecting of γδ T cells is performed after a duration of time sufficient to achieve an isolated population of γδ T cells from the sample. In certain embodiments, the γδ T cells are collected after at least one week, at least 10 days, at least 11 days, at least 12 days, at least 13 days or at least 14 days of culturing of the sample. Suitably, the γδ T cells are collected after 40 days or less, such as 38 days or less, 36 days or less, 34 days or less, 32 days or less, 30 days or less, 28 days or less, 26 days or less or 24 days or less. In one embodiment, the γδ T cells are collected after at least 14 days of culturing of the sample. In a further embodiment, the γδ T cells are collected after 14 to 21 days of culturing of the sample.

In one embodiment, the sample is cultured in media which is substantially free of serum (*e.g.* serum-free media or media containing a serum-replacement (SR)). Thus, in one embodiment, the sample is cultured in serum-free media. Such serum free medium may also include serum replacement medium, where the serum replacement is based on chemically defined components to avoid the use of human or animal derived serum. In one embodiment, the media contains no animal-derived products. In an alternative embodiment, the sample is cultured in media which contains serum (*e.g.* human AB serum or fetal bovine serum (FBS)).

Culture media may additionally include other ingredients that can assist in the growth and expansion of the γδ T cells. Examples of other ingredients that may be added, include, but are not limited to, plasma or serum, purified proteins such as albumin, a lipid source such as low density lipoprotein (LDL), vitamins, amino acids, steroids and any other supplements supporting or promoting cell growth and/or survival.

### Antibodies or fragments thereof

Provided herein are antibodies or fragments thereof, which specifically bind to a variable gamma 4 (Vγ4) chain of a γδ T cell receptor (TCR). In particular, the antibody or fragment thereof does not bind to (or cross react with) a variable gamma 2 (Vγ2) chain of a γδ TCR. It should be understood that this is with reference to a Vγ4 chain and a Vγ2 from the same species. Preferably, the species is *Homo sapiens* (human) and therefore the antibody or fragment thereof, may specifically bind to a human gamma variable 4 (Vγ4) chain of a γδ T cell receptor (TCR) and not to a human gamma variable 2 (Vγ2) chain of a γδ TCR. For instance, the human Vγ4 chain may have a sequence according to amino acids 1-99 of SEQ ID NO. 1 and/or the human Vγ2 chain may have a sequence according to SEQ ID NO. 335. In other species, the antibody or fragment thereof, specifically binds to the species-specific ortholog of the human gamma variable 4 (Vγ4) chain of a γδ T cell receptor (TCR) and not to the species-specific ortholog of the human gamma variable 2 (Vγ2) chain of a γδ TCR. Thus, the antibody or fragment thereof may specifically bind to a human gamma variable 4 (Vγ4) chain of a γδ T cell receptor (TCR) having a sequence corresponding to amino acids 1-99 of SEQ ID NO. 1 or non-human ortholog thereof and not to a human gamma variable 2 (Vγ2) chain of a γδ TCR having a sequence corresponding to SEQ ID NO. 335 or non-human ortholog thereof. Ortholog in this context may mean a gamma chain sequence with the highest sequence similarity to the reference sequence, or preferably one which possesses the same function (e.g. interaction with orthologous cognate ligands *in vivo*). For instance, in mouse, the protein designated under the Heilig & Tonegave nomenclature as Vγ7 is functionally most closely related to human Vγ4 (Barros et al. (2016) Cell, 167:203-218.e17).

This development is profound. In humans, for example, the Vγ4 and Vγ2 chains share 91% sequence identity (they only differ by nine amino acids). Therefore this has made it difficult to obtain antibodies which bind to (human) Vγ4 and not to (human) Vγ2 and it was not expected in the art to be possible to produce such antibodies.

When referring to an antibody or fragment thereof which specifically binds to a Vγ4 chain of a γδ TCR, this generally means that binding of the antibody or fragment thereof to the Vγ4 chain is statistically significantly increased relative to a negative control antibody and/or a negative control antigen (e.g. as measured via binding in an ELISA assay, optionally a DELFIA ELISA assay, or SPR). The level detected in respect of the negative control antibody and/or negative control antigen may be considered the background level for the assay used, representing "noise" in the assay system as would be well-understood by the skilled person. In particular embodiments, signal levels above a pre-determined threshold relative to the background level may be considered to represent detection of binding (e.g. about 1-fold, 2-fold, 3-fold, 4-fold, 5-fold or more above the background level). For instance, in a DELFIA ELISA assay a signal level 5-fold or more above the background level may be considered to indicate binding of the antibody to the antigen. The skilled person is well able to determine a suitable threshold based on the assay system being used. Conversely, when referring to an antibody or fragment thereof which does not bind to (or cross react with) a Vγ2 chain of a γδ TCR, this generally means that binding of the antibody or fragment thereof to the Vγ2 chain is not statistically significantly increased relative to a negative control antibody and/or a negative control antigen (e.g. as measured via binding in an ELISA assay, optionally a DELFIA ELISA assay, or SPR). This is demonstrated, for example, in **Figure 2A** and discussed in **Example 4.** According to all aspects and embodiments of the invention disclosed herein, this property may also be expressed as the fold-change difference in detected binding levels (e.g. as measured via binding in an ELISA assay, optionally a DELFIA ELISA assay, or SPR) between the antibody or fragment thereof and the Vγ4 chain versus the antibody or fragment thereof and the Vγ2 chain. For instance, the antibody or fragment thereof may show an at least about 50-fold, 60-fold, 70-fold, 80-fold, 90-fold, 100-fold, 150-fold, 200-fold, 300-fold, 400-fold, 500-fold, 600-fold, 700-fold, 800-fold, 900-fold, 1000-fold, 2000-fold, 3000-fold, 4000-fold, 5000-fold, 6000-fold, 7000-fold, 8000-fold, 9000-fold, 10000-fold, 15000-fold, 25000-fold, 50000-fold, 75000-fold, 95000-fold or more increase in binding to the Vγ4 chain as compared against binding to the Vγ2 chain. This is demonstrated, for example, in **Figure 2B** and discussed in **Example 4.** However these fold increases are deemed conservative inasmuch to calculate them it has been assumed all Vγ2 signal above controls is not background noise. However, and as discussed previously, a skilled person may instead exclude low signal above background in such DELFIA ELISA assays as assay noise (e.g. about 1-fold, 2-fold, 3-fold, 4-fold, 5-fold or more above the background level) and so consider signal below these thresholds as non-specific background binding.

In one embodiment, the antibody or fragment thereof is an scFv, Fab, Fab', F(ab')2, Fv, variable domain (*e.g.* VH or VL), diabody, minibody or monoclonal antibody. In a particular embodiment, the antibody or fragment thereof is an scFv. In another particular embodiment, the antibody is a monoclonal antibody.

Antibodies described herein can be of any class, *e.g.* IgG, IgA, IgM, IgE, IgD, or isotypes thereof, and can comprise a kappa or lambda light chain. In one embodiment, the antibody is an IgG antibody, for example, at least one of isotypes, IgG1, IgG2, IgG3 or IgG4. In on embodiment, the antibody is an IgG1. In a further embodiment, the antibody may be in a format, such as an IgG format, that has been modified to confer desired properties, such as having the Fc mutated to reduce effector function, extend half life, alter ADCC, or improve hinge stability. Such modifications are well known in the art and exemplary embodiments are described herein. For instance, an antibody or fragment thereof may comprise an IgG1 constant domain comprising an amino acid sequence according to SEQ ID NO: 332 or 333.

In one embodiment, the antibody or fragment thereof is human. Thus, the antibody or fragment thereof may be derived from a human immunoglobulin (Ig) sequence. The CDR, framework and/or constant region of the antibody (or fragment thereof) may be derived from a human Ig sequence, in particular a human IgG sequence. The CDR, framework and/or constant region may be substantially identical for a human Ig sequence, in particular a human IgG sequence. An advantage of using human antibodies is that they have low or no immunogenicity in humans.

An antibody or fragment thereof can also be chimeric, for example a mouse-human antibody chimera.

Alternatively, the antibody or fragment thereof is derived from a non-human species, such as a mouse. Such non-human antibodies can be modified to increase their similarity to antibody variants produced naturally in humans, thus the antibody or fragment thereof can be partially or fully humanised. Therefore, in one embodiment, the antibody or fragment thereof is humanised.

Antibodies targeted to epitopes

Provided herein are antibodies (or fragments thereof) which bind to an epitope of the Vγ4 chain of a γδ TCR. Binding of the epitope on the Vγ4 chain may optionally have an effect on γδ TCR activity, such as activation or inhibition. The antibodies (or fragments thereof) may have a blocking effect by prevention of the binding or interaction of another antibody or molecule. The antibodies are specific for the Vγ4 chain of a γδ TCR, and do not bind epitopes of other antigens, such as the Vγ2 chain of a γδ TCR or the Vγ8 chain of a γδ TCR, as defined herein.

In one embodiment, the epitope may be an activating epitope of a γδ T cell. An "activating" epitope can include, for example, modulation of a TCR-associated function, such as TCR downregulation, degranulation of the cell, cytoxicity, proliferation, mobilisation, increased survival or resistance to exhaustion, intracellular signaling, cytokine or growth factor secretion, phenotypic change, or a change in gene expression. For example, the binding of the activating epitope may stimulate expansion (*i.e.* proliferation) of the γδ T cell population, preferably the Vγ4+ T cell population. Accordingly, these antibodies can be used to modulate γδ T cell activation, and, thereby, to modulate the immune response. Therefore, in one embodiment, binding of the activating epitope downregulates the γδ TCR. In an additional or alternative embodiment, binding of the activating epitope activates degranulation of the γδ T cell. In a further additional or alternative embodiment, binding of the activating epitope activates the γδ T cell to kill target cells (e.g. cancer cells).

In one embodiment, the antibodies or fragments thereof block Vγ4 and prevent TCR binding (*e.g.* through steric hinderance). By blocking Vγ4, the antibody may prevent TCR activation and/or signalling. The epitope may therefore be an inhibitory epitope of a γδ T cell. An "inhibitory" epitope can include, for example, blocking TCR function, thereby inhibiting TCR activation.

The epitope is preferably comprised of at least one extracellular, soluble, hydrophilic, external or cytoplasmic portion of the Vγ4 chain of a γδ TCR.

In particular embodiments, the epitope does not comprise an epitope found in a non-germline encoded region of the Vγ4 chain of the γδ TCR, in particular CDR3 of the Vγ4 chain. In a preferred embodiment, the epitope is within a framework region of the Vγ4 chain of the γδ TCR, which may be the hypervariable 4 region of framework region 3. It will be appreciated that such binding allows for the unique recognition of the Vγ4 chain in general without the restriction to the sequences of the TCR which are highly variable between Vγ4 chains (in particular CDR3). As such, it will be appreciated that any Vγ4 chain-comprising γδ TCR may be recognised using the antibodies or fragments thereof as defined herein, irrespective of the specificity of the γδ TCR.

It is possible that the γδ receptor can bind a variety of modulating ligands independently and via spatially distinct domains. Consistent with such multi-modal ligand binding, recent studies by Melandri et al. (2018) Nat. Immunol. 19: 1352-1365 have shown that human TCR binding to the endogenous BTNL3 ligand is via a discrete domain located N-terminal of CDR3 on the γ4 chain. The authors highlight that because BTNL3 binding is mediated via this specific germline region of the TCR, the more C-terminal, somatically recombined CDR3 loop remains free to bind other ligands independently. Furthermore, this sub-region of framework region 3 (FR3) (which may also be referred to as 'hypervariable region 4' (HV4)) differs from the human γ2 chain by four amino acids. However, no specific anti-Vγ4 antibodies were disclosed in Melandri et al. nor was it suggested how such antibodies could be derived. Indeed, the prevailing view was that this would not be possible due to the significant sequence homology shared between the human Vγ4 and Vγ2 chains (91% sequence identity).

An antibody which binds within the HV4 region may allow the CDR3 region of the γ4 chain to still bind, with the added advantage of providing a binder which is specific to γ4 over γ2. Furthermore, as the HV4 is germline-encoded, some antibodies targeting this region may recognise all Vγ4 chains, while other antibodies that recognise Vγ4 may be specific for certain Vγ4 chains.

The disclosure now provides antibodies and fragments thereof which may specifically bind to the HV4 region of the Vγ4 chain. Therefore, in one embodiment, the antibody or fragment thereof binds to an epitope of the HV4 region of the Vγ4 chain. The HV4 region comprises amino acids 67 to 82 of SEQ ID NO: 1. Therefore, in one embodiment, the epitope comprises one or more amino acid residues within amino acid region 67-82 of SEQ ID NO: 1, e.g. the portion of the Vγ4 chain which is not part of the CDR1, CDR2 and/or CDR3 sequences. In so doing, the antibody or fragment thereof may modulate the interaction between the Vγ4+ TCR and BTNL3/8. In one embodiment, the epitope does not comprise amino acid residues within amino acid region 96-106 (CDR3) of SEQ ID NO: 1. In one embodiment, the epitope does not comprise amino acid residues within amino acid region 50-57 (CDR2) of SEQ ID NO: 1. In one embodiment, the epitope does not comprise amino acid residues within amino acid region 27-32 (CDR1) of SEQ ID NO: 1.

In particular embodiments, the antibody or fragment thereof may, upon binding to one or more of amino acids 67 to 82 of SEQ ID NO: 1, activate the Vγ4+ TCR

In a similar manner to the well characterised αβ T cells, γδ T cells utilize a distinct set of somatically rearranged variable (V), diversity (D) (for β and δ only), joining (J), and constant (C) genes, although γδ T cells contain fewer V, D, and J segments than αβ T cells. In one embodiment, the epitope bound by the antibodies (or fragments thereof) does not comprise an epitope found in the J region of the Vγ4 chain. The antibody or fragment may therefore only bind in the V region of the Vγ4 chain. Thus, in one embodiment, the epitope consists of an epitope in the V region of the γδ TCR (e.g. amino acid residues 1-99 of SEQ ID NO: 1).

Reference to the epitope are made in relation to the Vγ4 sequence described in Luoma et al. (2013) Immunity 39: 1032-1042, and RCSB Protein Data Bank entry: 4MNH, shown as SEQ ID NO: 1:

SSNLEGRTKSVIRQTGSSAEITCDLAEGSTGYIHWYLHQEGKAPQRLLYYDSYTSSVVLESGIS

PGKYDTYGSTRKNLRMILRNLIENDSGVYYCATWDEKYYKKLFGSGTTLVVTEDLKNVFPPEV

AVFEPSEAEISHTQKATLVCLATGFYPDHVELSWWVNGKEVHSGVCTDPQPLKEQPALNDSR

YALSSRLRVSATFWQNPRNHFRCQVQFYGLSENDEWTQDRAKPVTQIVSAEAWGRADSRGG

LEVLFQ (SEQ ID NO: 1)

SEQ ID NO: 1 represents a soluble TCR comprising a V region (also referred to as the variable domain) and a J region. The V region comprises amino acid residues 1-99, the J region comprises amino acid residues 102-116 and the constant region from TCRβ comprises amino acid residues 117-256. Within the V region, CDR1 is defined as amino acid residues 27 to 32 of SEQ ID NO: 1, CDR2 is defined as amino acid residues 50 to 57 of SEQ ID NO: 1, and CDR3 is defined as amino acid residues 96 to 106 of SEQ ID NO: 1.

The inventors have identified that amino acids K76 (i.e. lysine at position 76) and M80 (i.e. methionine at position 80) of SEQ ID NO: 1 may be particularly important for binding to the HV4 region of the (human) Vγ4 chain **(Example 6).**

Thus, the epitope may comprise, or consist of, K76 and/or M80 of SEQ ID NO: 1.

The inventors have further identified that amino acids within the amino acid region 71-79 of SEQ ID NO: 1 may be particularly important for binding to the HV4 region of the (human) Vγ4 chain. Thus, in a further embodiment, the epitope comprises one or more amino acid residues within amino acid region 71-79 of SEQ ID NO: 1.

In one embodiment, the epitope comprises one or more, such as two, three, four, five, six, seven, eight, nine, ten or more amino acid residues within the described region.

In one embodiment, the epitope comprises one or more (such as 5 or more, such as 10 or more) amino acid residues within amino acid region 67-82 of SEQ ID NO: 1. In a further embodiment the epitope comprises one or more (such as 3 or more, such as 5 or more) amino acid residues within amino acid region 71-79 of SEQ ID NO: 1.

It will be further understood that said antibody (or fragment thereof) does not need to bind to all amino acids within the defined range. Such epitopes may be referred to as linear epitopes. For example, an antibody which binds to an epitope comprising amino acid residues within amino acid region 67-82 of SEQ ID NO: 1, may only bind with one or more of the amino acid residues in said range, *e.g.* the amino acid residues at each end of the range (*i.e.* amino acids 67 and 82), optionally including amino acids within the range (*i.e.* amino acids 71, 73, 75, 76 and 79).

For instance, the inventors have found that amino acid residues 71, 73, 75, 76 and 79 of SEQ ID NO: 1 may form the epitope to which the anti-Vγ4 antibody or fragment thereof binds **(Example 8).** Thus, in one embodiment, the epitope comprises at least one of amino acid residues 71, 73, 75, 76 and 79 of SEQ ID NO: 1. In further embodiments, the epitope comprises one, two, three, four or five (in particular four or five) amino acids selected from amino acid residues 71, 73, 75, 76 and 79 of SEQ ID NO: 1.

In a further embodiment, the epitope consists of one or more amino acid residues within amino acid regions: 67-82 of SEQ ID NO: 1. In a further embodiment, the epitope consists of one or more amino acid residues within amino acid regions: 71-79 of SEQ ID NO: 1.

In a further embodiment, the epitope comprises amino acid residues: 71-79 of SEQ ID NO: 1, or suitably consists of amino acid residues: 71-79 of SEQ ID NO: 1. In a yet further embodiment, the epitope comprises amino acid residues: 71, 73, 75, 76 and 79 of SEQ ID NO: 1, or suitably consists of amino acid residues: 71, 73, 75, 76 and 79 of SEQ ID NO: 1.

Various techniques are known in the art to establish which epitope is bound by an antibody. Exemplary techniques include, for example, routine cross-blocking assays, alanine scanning mutational analysis, peptide blot analysis, peptide cleavage analysis crystallographic studies and NMR analysis. In addition, methods such as epitope excision, epitope extraction and chemical modification of antigens can be employed. Another method that can be used to identify the amino acids within a polypeptide with which an antibody interacts is hydrogen/deuterium exchange detected by mass spectrometry (as described in **Example 8).** In general terms, the hydrogen/deuterium exchange method involves deuterium-labelling the protein of interest, followed by binding the antibody to the deuterium-labelled protein. Next, the protein/antibody complex is transferred to water and exchangeable protons within amino acids that are protected by the antibody complex undergo deuterium-to-hydrogen back-exchange at a slower rate than exchangeable protons within amino acids that are not part of the interface. As a result, amino acids that form part of the protein/antibody interface may retain deuterium and therefore exhibit relatively higher mass compared to amino acids not included in the interface. After dissociation of the antibody, the target protein is subjected to protease cleavage and mass spectrometry analysis, thereby revealing the deuterium-labelled residues which correspond to the specific amino acids with which the antibody interacts.

In addition, or as an alternative, antigen chimerization & mutagenesis studies can be used to identify the amino acids within a polypeptide with which an antibody interacts (as described in **Example 6).** In general terms, this method involves creating a series of one or more chimeric antigens wherein the amino acid sequence of a first reference antigen may be systematically altered based on the amino acid sequence of a second reference antigen in order to substitute one or more of the amino acids in the first reference antigen with respective amino acids from the second reference antigen. "Respective amino acids" in this context means amino acids in equivalent positions within the sequence of the first reference antigen and second reference antigen upon sequence alignment thereof. Binding of the test antibody to each of the first reference antigen, second reference antigen and/or series of one or more chimeric antigens is then measured. Loss/gain of binding to each antigen can then be attributed to specific amino acid changes made relative to the first reference sequence and/or second reference sequence. It may be already known whether or not the antibody is capable of binding or not to the first reference antigen and/or the second reference antigen. For instance, as described in **Example 6,** the first reference antigen may be a human Vγ4 chain and the second reference antigen may be a human Vγ2 chain, with the series of chimeric antigens made by replacing one or more of the amino acids in the Vγ4 chain sequence with the respective one or more amino acids in the Vγ2 chain sequence.

Antibody sequences

The anti-Vγ4 antibodies, or fragments thereof, may be described with reference to their CDR sequences.

Therefore, in one embodiment, the anti-Vγ4 antibody or fragment thereof, which comprises one or more of:

a CDR3 comprising a sequence having at least 80% sequence identity with any one of SEQ ID NOs: 2-47, preferably with SEQ ID NO: 10 and/or 33;

a CDR2 comprising a sequence having at least 80% sequence identity with any one of SEQ ID NOs: 48-70 and SEQUENCES: A1-A23 (of Figure 1), preferably with SEQ ID NO: 56 and/or A9; and/or

a CDR1 comprising a sequence having at least 80% sequence identity with any one of SEQ ID NOs: 71-116, preferably with SEQ ID NO: 79 and/or 102.

In one embodiment, the anti-Vγ4 antibody or fragment thereof comprises a CDR3 comprising a sequence having at least 80% sequence identity with any one of SEQ ID NOs: 2-47. In one embodiment, the antibody or fragment thereof comprises a CDR2 comprising a sequence having at least 80% sequence identity with any one of SEQ ID NOs: 48-70 and SEQUENCES: A1-A23 (of Figure 1). In one embodiment, the antibody or fragment thereof comprises a CDR1 comprising a sequence having at least 80% sequence identity with any one of SEQ ID NOs: 71-116.

In some embodiments, the anti-Vγ4 antibody or fragment thereof may comprise one or more of:

a heavy chain CDR3 (HCDR3) comprising a sequence having at least 80% sequence identity with any one of SEQ ID NOs: 2-24, preferably with SEQ ID NO: 10;

a heavy chain CDR2 (HCDR2) comprising a sequence having at least 80% sequence identity with any one of SEQ ID NOs: 48-70, preferably with SEQ ID NO: 56 ; and/or

a heavy chain CDR1 (HCDR1) comprising a sequence having at least 80% sequence identity with any one of SEQ ID NOs: 71-93, preferably with SEQ ID NO: 79.

Alternatively, or in addition to, the anti-Vγ4 antibody or fragment thereof may comprise one or more of:

a light chain CDR3 (LCDR3) comprising a sequence having at least 80% sequence identity with any one of SEQ ID NOs: 25-47, preferably with SEQ ID NO: 33;

a light chain CDR2 (LCDR2) comprising a sequence having at least 80% sequence identity with any one of SEQUENCES: A1-A23 (of Figure 1), preferably with SEQ ID NO: A9; and/or

a light chain CDR1 (LCDR1) comprising a sequence having at least 80% sequence identity with any one of SEQ ID NOs: 94-116, preferably with SEQ ID NO: 102.

In one embodiment, the antibody or fragment thereof comprises a CDR3 comprising a sequence having at least 85%, 90%, 95%, 97%, 98% or 99% sequence identity with any one of SEQ ID NOs: 2-47. In one embodiment, the antibody or fragment thereof comprises a CDR2 comprising a sequence having at least 85%, 90%, 95%, 97%, 98% or 99% sequence identity with any one of SEQ ID NOs: 48-70 and SEQUENCES: A1-A23 (of Figure 1). In one embodiment, the antibody or fragment thereof comprises a CDR1 comprising a sequence having at least 85%, 90%, 95%, 97%, 98% or 99% sequence identity with any one of SEQ ID NOs: 71-116.

In one embodiment, the antibody or fragment thereof comprises a CDR3 consisting of a sequence having at least 85%, 90%, 95%, 97%, 98% or 99% sequence identity with any one of SEQ ID NOs: 2-47. In one embodiment, the antibody or fragment thereof comprises a CDR2 consisting of a sequence having at least 85%, 90%, 95%, 97%, 98% or 99% sequence identity with any one of SEQ ID NOs: 48-70 and SEQUENCES: A1-A23 (of Figure 1). In one embodiment, the antibody or fragment thereof comprises a CDR1 consisting of a sequence having at least 85%, 90%, 95%, 97%, 98% or 99% sequence identity with any one of SEQ ID NOs: 71-116.

In one embodiment, the antibody or fragment thereof comprises a VH region comprising a CDR3 comprising a sequence having at least 80% sequence identity with any one of SEQ ID NOs: 2-24 and/or a VL region comprising a CDR3 comprising a sequence having at least 80% sequence identity with any one of SEQ ID NOs: 25-47. In one embodiment, the antibody or fragment thereof comprises a VH region comprising a CDR3 consisting of a sequence having at least 80% sequence identity with any one of SEQ ID NOs: 2-24 and/or a VL region comprising a CDR3 consisting of a sequence having at least 80% sequence identity with any one of SEQ ID NOs: 25-47.

In one embodiment, the antibody or fragment thereof comprises a VH region comprising a CDR3 comprising a sequence having at least 90% sequence identity with any one of SEQ ID NOs: 2-24 and/or a VL region comprising a CDR3 comprising a sequence having at least 90% sequence identity with any one of SEQ ID NOs: 25-47. In one embodiment, the antibody or fragment thereof comprises a VH region comprising a CDR3 consisting of a sequence having at least 90% sequence identity with any one of SEQ ID NOs: 2-24 and/or a VL region comprising a CDR3 consisting of a sequence having at least 90% sequence identity with any one of SEQ ID NOs: 25-47.

In one embodiment, the antibody or fragment thereof comprises a VH region comprising a CDR3 comprising a sequence having at least 95% sequence identity with any one of SEQ ID NOs: 2-24 and/or a VL region comprising a CDR3 comprising a sequence having at least 95% sequence identity with any one of SEQ ID NOs: 25-47. In one embodiment, the antibody or fragment thereof comprises a VH region comprising a CDR3 consisting of a sequence having at least 95% sequence identity with any one of SEQ ID NOs: 2-24 and/or a VL region comprising a CDR3 consisting of a sequence having at least 95% sequence identity with any one of SEQ ID NOs: 25-47.

In one embodiment, the antibody or fragment thereof comprises a VH region comprising a CDR3 comprising a sequence having at least 80% sequence identity with any one of SEQ ID NOs: 2-24 and a VL region comprising a CDR3 comprising a sequence having at least 80% sequence identity with any one of SEQ ID NOs: 25-47. In one embodiment, the antibody or fragment thereof comprises a VH region comprising a CDR3 consisting of a sequence having at least 80% sequence identity with any one of SEQ ID NOs: 2-24 and a VL region comprising a CDR3 consisting of a sequence having at least 80% sequence identity with any one of SEQ ID NOs: 25-47.

Embodiments which refer herein to "at least 80%" or "80% or greater", will be understood to include all values equal to or greater than 80%, such as 85%, 90%, 95%, 97%, 98%, 99% or 100% sequence identity. In one embodiment, the antibody or fragment comprises at least 85%, such as at least 90%, at least 95%, at least 97%, at least 98% or at least 99% sequence identity to the specified sequence.

Instead of percentage sequence identity, the embodiments may also be defined with one or more amino acid changes, for examples one or more additions, substitutions and/or deletions. In one embodiment, the sequence may comprise up to five amino acid changes, such as up to three amino acid changes, in particular up to two amino acid changes. For example, the sequence may comprise up to five amino acid substitutions, such as up to three amino acid substitutions, in particular up to one or two amino acid substitutions. For example, CDR3 of the antibody or fragment thereof may comprise or more suitably consist of a sequence having no more than 2, more suitably no more than 1 substitution(s) compared to any one of SEQ ID NOs: 2-47.

Suitably any residues of CDR1, CDR2 or CDR3 differing from their corresponding residues in SEQ ID NO: 2-116 and SEQUENCES: A1-A23 are conservative substitutions with respect to their corresponding residues. For example, any residues of CDR3 differing from their corresponding residues in SEQ ID NOs: 2-47 are conservative substitutions with respect to their corresponding residues.

In one embodiment, the antibody or fragment thereof comprises:

(i) a VH region comprising a CDR3 comprising a sequence having at least 80% sequence identity with any one of SEQ ID NOs: 2-24;
(ii) a VH region comprising a CDR2 comprising a sequence having at least 80% sequence identity with any one of SEQ ID NOs: 48-70;
(iii) a VH region comprising a CDR1 comprising a sequence having at least 80% sequence identity with any one of SEQ ID NOs: 71-93;
(iv) a VL region comprising a CDR3 comprising a sequence having at least 80% sequence identity with any one of SEQ ID NOs: 25-47;
(v) a VL region comprising a CDR2 comprising a sequence having at least 80% sequence identity with any one of SEQUENCES: A1-A23 (of Figure 1); and/or
(vi) a VL region comprising a CDR1 comprising a sequence having at least 80% sequence identity with any one of SEQ ID NOs: 94-116.

In one embodiment, the antibody or fragment thereof comprises a heavy chain with:

(i) a VH region comprising a CDR3 comprising a sequence having at least 80% sequence identity with any one of SEQ ID NOs: 2-24;
(ii) a VH region comprising a CDR2 comprising a sequence having at least 80% sequence identity with any one of SEQ ID NOs: 48-70; and
(iii) a VH region comprising a CDR1 comprising a sequence having at least 80% sequence identity with any one of SEQ ID NOs: 71-93.

In one embodiment, the antibody or fragment thereof comprises a light chain with:

(i) a VL region comprising a CDR3 comprising a sequence having at least 80% sequence identity with any one of SEQ ID NOs: 25-47;
(ii) a VL region comprising a CDR2 comprising a sequence having at least 80% sequence identity with any one of SEQUENCES: A1-A23 (of Figure 1); and
(iii) a VL region comprising a CDR1 comprising a sequence having at least 80% sequence identity with any one of SEQ ID NOs: 94-116.

In one embodiment, the antibody or fragment thereof comprises (or consists of) a VH region comprising a CDR3 comprising a sequence having at least 80% sequence identity with any one of SEQ ID NOs: 2-24, such as SEQ ID NOs: 10, 4, 14, 15, 17, 19 or 23. In one embodiment, the antibody or fragment thereof comprises (or consists of) a VH region comprising a CDR2 comprising a sequence having at least 80% sequence identity with any one of SEQ ID NOs: 48-70, such as SEQ ID NOs: 56, 50, 60, 61, 63, 65 or 69. In one embodiment, the antibody or fragment thereof comprises (or consists of) a VH region comprising a CDR1 comprising a sequence having at least 80% sequence identity with any one of SEQ ID NOs: 71-93, such as SEQ ID NOs: 79, 73, 83, 84, 86, 88 or 92.

In one embodiment, the VH region comprises a CDR3 comprising a sequence of SEQ ID NO: 10, a CDR2 comprising a sequence of SEQ ID NO: 56, and a CDR1 comprising a sequence of SEQ ID NO: 79. In one embodiment, the CDR3 consists of a sequence of SEQ ID NO: 10, the CDR2 consists of a sequence of SEQ ID NO: 56, and the CDR1 consists of a sequence of SEQ ID NO: 79.

In one embodiment, the VH region comprises a CDR3 comprising a sequence of SEQ ID NO: 4, a CDR2 comprising a sequence of SEQ ID NO: 50, and a CDR1 comprising a sequence of SEQ ID NO: 73. In one embodiment, the CDR3 consists of a sequence of SEQ ID NO: 4, the CDR2 consists of a sequence of SEQ ID NO: 50, and the CDR1 consists of a sequence of SEQ ID NO: 73.

In one embodiment, the VH region comprises a CDR3 comprising a sequence of SEQ ID NO: 14, a CDR2 comprising a sequence of SEQ ID NO: 60, and a CDR1 comprising a sequence of SEQ ID NO: 83. In one embodiment, the CDR3 consists of a sequence of SEQ ID NO: 14, the CDR2 consists of a sequence of SEQ ID NO: 60, and the CDR1 consists of a sequence of SEQ ID NO: 83.

In one embodiment, the VH region comprises a CDR3 comprising a sequence of SEQ ID NO: 15, a CDR2 comprising a sequence of SEQ ID NO: 61, and a CDR1 comprising a sequence of SEQ ID NO: 84. In one embodiment, the CDR3 consists of a sequence of SEQ ID NO: 15, the CDR2 consists of a sequence of SEQ ID NO: 61, and the CDR1 consists of a sequence of SEQ ID NO: 84.

In one embodiment, the VH region comprises a CDR3 comprising a sequence of SEQ ID NO: 17, a CDR2 comprising a sequence of SEQ ID NO: 63, and a CDR1 comprising a sequence of SEQ ID NO: 86. In one embodiment, the CDR3 consists of a sequence of SEQ ID NO: 17, the CDR2 consists of a sequence of SEQ ID NO: 63, and the CDR1 consists of a sequence of SEQ ID NO: 86.

In one embodiment, the VH region comprises a CDR3 comprising a sequence of SEQ ID NO: 19, a CDR2 comprising a sequence of SEQ ID NO: 65, and a CDR1 comprising a sequence of SEQ ID NO: 88. In one embodiment, the CDR3 consists of a sequence of SEQ ID NO: 19, the CDR2 consists of a sequence of SEQ ID NO: 65, and the CDR1 consists of a sequence of SEQ ID NO: 88.

In one embodiment, the VH region comprises a CDR3 comprising a sequence of SEQ ID NO: 23, a CDR2 comprising a sequence of SEQ ID NO: 69, and a CDR1 comprising a sequence of SEQ ID NO: 92. In one embodiment, the CDR3 consists of a sequence of SEQ ID NO: 23, the CDR2 consists of a sequence of SEQ ID NO: 69, and the CDR1 consists of a sequence of SEQ ID NO: 92.

In one embodiment, the antibody or fragment thereof comprises (or consists of) a VL region comprising a CDR3 comprising a sequence having at least 80% sequence identity with any one of SEQ ID NOs: 25-47, such as SEQ ID NOs: 33, 27, 37, 38, 40, 42 or 46. In one embodiment, the antibody or fragment thereof comprises (or consists of) a VL region comprising a CDR2 comprising a sequence having at least 80% sequence identity with any one of SEQUENCES: A1-A23 (of Figure 1), such as SEQUENCES: A9, A3, A13, A14, A16, A18 or A22. In one embodiment, the antibody or fragment thereof comprises (or consists of) a VL region comprising a CDR1 comprising a sequence having at least 80% sequence identity with any one of SEQ ID NOs: 94-116, such as SEQ ID NOs: 102, 96, 106, 107, 109, 111 or 115.

In one embodiment, the VL region comprises a CDR3 comprising a sequence of SEQ ID NO: 33, a CDR2 comprising a sequence of SEQUENCE: A9, and a CDR1 comprising a sequence of SEQ ID NO: 102. In one embodiment, the CDR3 consists of a sequence of SEQ ID NO: 33, the CDR2 consists of a sequence of SEQUENCE: A9, and the CDR1 consists of a sequence of SEQ ID NO: 102.

In one embodiment, the VL region comprises a CDR3 comprising a sequence of SEQ ID NO: 27, a CDR2 comprising a sequence of SEQUENCE: A3, and a CDR1 comprising a sequence of SEQ ID NO: 96. In one embodiment, the CDR3 consists of a sequence of SEQ ID NO: 27, the CDR2 consists of a sequence of SEQUENCE: A3, and the CDR1 consists of a sequence of SEQ ID NO: 96.

In one embodiment, the VL region comprises a CDR3 comprising a sequence of SEQ ID NO: 37, a CDR2 comprising a sequence of SEQUENCE: A13, and a CDR1 comprising a sequence of SEQ ID NO: 106. In one embodiment, the CDR3 consists of a sequence of SEQ ID NO: 37, the CDR2 consists of a sequence of SEQUENCE: A13, and the CDR1 consists of a sequence of SEQ ID NO: 106.

In one embodiment, the VL region comprises a CDR3 comprising a sequence of SEQ ID NO: 38, a CDR2 comprising a sequence of SEQUENCE: A14, and a CDR1 comprising a sequence of SEQ ID NO: 107. In one embodiment, the CDR3 consists of a sequence of SEQ ID NO: 38, the CDR2 consists of a sequence of SEQUENCE: A14, and the CDR1 consists of a sequence of SEQ ID NO: 107.

In one embodiment, the VL region comprises a CDR3 comprising a sequence of SEQ ID NO: 40, a CDR2 comprising a sequence of SEQUENCE: A16, and a CDR1 comprising a sequence of SEQ ID NO: 109. In one embodiment, the CDR3 consists of a sequence of SEQ ID NO: 40, the CDR2 consists of a sequence of SEQUENCE: A16, and the CDR1 consists of a sequence of SEQ ID NO: 109.

In one embodiment, the VL region comprises a CDR3 comprising a sequence of SEQ ID NO: 42, a CDR2 comprising a sequence of SEQUENCE: A18, and a CDR1 comprising a sequence of SEQ ID NO: 111. In one embodiment, the CDR3 consists of a sequence of SEQ ID NO: 42, the CDR2 consists of a sequence of SEQUENCE: A18, and the CDR1 consists of a sequence of SEQ ID NO: 111.

In one embodiment, the VL region comprises a CDR3 comprising a sequence of SEQ ID NO: 46, a CDR2 comprising a sequence of SEQUENCE: A22, and a CDR1 comprising a sequence of SEQ ID NO: 115. In one embodiment, the CDR3 consists of a sequence of SEQ ID NO: 46, the CDR2 consists of a sequence of SEQUENCE: A22, and the CDR1 consists of a sequence of SEQ ID NO: 115.

In one embodiment, the antibody or fragment thereof comprises one or more CDR sequences as described in **Figure 1.** In a further embodiment, the antibody or fragment thereof comprises one or more (such as all) CDR sequences of clone 1140_P01_G08 [G4_12] or clone 1139_P01_A04 **[G4_03]** as described in **Figure 1.**

Thus, the anti-V$\gamma$4 antibody or fragment thereof may comprise one or more of:

(a) a VH comprising a HCDR1 having SEQ ID NO: 79, a HCDR2 having SEQ ID NO: 56 and a HCDR3 having SEQ ID NO: 10, optionally wherein the VH comprises or consists of SEQ ID NO: 125; and

a VL comprising a LCDR1 having SEQ ID NO: 102, a LCDR2 having SEQUENCE A9 (of Figure 1) and a LCDR3 having SEQ ID NO: 33, optionally wherein the VL comprises or consists of SEQ ID NO: 148;

(b) a VH comprising a HCDR1 having SEQ ID NO: 86, a HCDR2 having SEQ ID NO: 63 and a HCDR3 having SEQ ID NO: 17, optionally wherein the VH comprises or consists of SEQ ID NO: 132; and

a VL comprising a LCDR1 having SEQ ID NO: 109, a LCDR2 having SEQUENCE A16 (of Figure 1) and a LCDR3 having SEQ ID NO: 40, optionally wherein the VL comprises or consists of SEQ ID NO: 155;

(c) a VH comprising a HCDR1 having SEQ ID NO: 73, a HCDR2 having SEQ ID NO: 50 and a HCDR3 having SEQ ID NO: 4, optionally wherein the VH comprises or consists of SEQ ID NO: 119; and

a VL comprising a LCDR1 having SEQ ID NO: 96, a LCDR2 having SEQUENCE A3 (of Figure 1) and a LCDR3 having SEQ ID NO: 27, optionally wherein the VL comprises or consists of SEQ ID NO: 142;

(d) a VH comprising a HCDR1 having SEQ ID NO: 83, a HCDR2 having SEQ ID NO: 60 and a HCDR3 having SEQ ID NO: 14, optionally wherein the VH comprises or consists of SEQ ID NO: 129; and

a VL comprising a LCDR1 having SEQ ID NO: 106, a LCDR2 having SEQUENCE A13 (of Figure 1) and a LCDR3 having SEQ ID NO: 37, optionally wherein the VL comprises or consists of SEQ ID NO: 152;

(e) a VH comprising a HCDR1 having SEQ ID NO: 84, a HCDR2 having SEQ ID NO: 61 and a HCDR3 having SEQ ID NO: 15, optionally wherein the VH comprises or consists of SEQ ID NO: 130; and

a VL comprising a LCDR1 having SEQ ID NO: 107, a LCDR2 having SEQUENCE A14 (of Figure 1) and a LCDR3 having SEQ ID NO: 38, optionally wherein the VL comprises or consists of SEQ ID NO: 153;

(f) a VH comprising a HCDR1 having SEQ ID NO: 88, a HCDR2 having SEQ ID NO: 65 and a HCDR3 having SEQ ID NO: 19, optionally wherein the VH comprises or consists of SEQ ID NO: 134; and

a VL comprising a LCDR1 having SEQ ID NO: 111, a LCDR2 having SEQUENCE A18 (of Figure 1) and a LCDR3 having SEQ ID NO: 42, optionally wherein the VL comprises or consists of SEQ ID NO: 157;

(g) a VH comprising a HCDR1 having SEQ ID NO: 92, a HCDR2 having SEQ ID NO: 69 and a HCDR3 having SEQ ID NO: 23, optionally wherein the VH comprises or consists of SEQ ID NO: 138; and

a VL comprising a LCDR1 having SEQ ID NO: 115, a LCDR2 having SEQUENCE A22 (of Figure 1) and a LCDR3 having SEQ ID NO: 46, optionally wherein the VL comprises or consists of SEQ ID NO: 161;

(h) a VH comprising a HCDR1 having SEQ ID NO: 71, a HCDR2 having SEQ ID NO: 48 and a HCDR3 having SEQ ID NO: 2, optionally wherein the VH comprises or consists of SEQ ID NO: 117; and

a VL comprising a LCDR1 having SEQ ID NO: 94, a LCDR2 having SEQUENCE A1 (of Figure 1) and a LCDR3 having SEQ ID NO: 25, optionally wherein the VL comprises or consists of SEQ ID NO: 140;

(i) a VH comprising a HCDR1 having SEQ ID NO: 72, a HCDR2 having SEQ ID NO: 49 and a HCDR3 having SEQ ID NO: 3, optionally wherein the VH comprises or consists of SEQ ID NO: 118; and

a VL comprising a LCDR1 having SEQ ID NO: 95, a LCDR2 having SEQUENCE A2 (of Figure 1) and a LCDR3 having SEQ ID NO: 26, optionally wherein the VL comprises or consists of SEQ ID NO: 141;

(j) a VH comprising a HCDR1 having SEQ ID NO: 74, a HCDR2 having SEQ ID NO: 51 and a HCDR3 having SEQ ID NO: 5, optionally wherein the VH comprises or consists of SEQ ID NO: 120; and

a VL comprising a LCDR1 having SEQ ID NO: 97, a LCDR2 having SEQUENCE A4 (of Figure 1) and a LCDR3 having SEQ ID NO: 28, optionally wherein the VL comprises or consists of SEQ ID NO: 143;

(k) a VH comprising a HCDR1 having SEQ ID NO: 75, a HCDR2 having SEQ ID NO: 52 and a HCDR3 having SEQ ID NO: 6, optionally wherein the VH comprises or consists of SEQ ID NO: 121; and

a VL comprising a LCDR1 having SEQ ID NO: 98, a LCDR2 having SEQUENCE A5 (of Figure 1) and a LCDR3 having SEQ ID NO: 29, optionally wherein the VL comprises or consists of SEQ ID NO: 144;

(l) a VH comprising a HCDR1 having SEQ ID NO: 76, a HCDR2 having SEQ ID NO: 53 and a HCDR3 having SEQ ID NO: 7, optionally wherein the VH comprises or consists of SEQ ID NO: 122; and

a VL comprising a LCDR1 having SEQ ID NO: 99, a LCDR2 having SEQUENCE A6 (of Figure 1) and a LCDR3 having SEQ ID NO: 30, optionally wherein the VL comprises or consists of SEQ ID NO: 145;

(m) a VH comprising a HCDR1 having SEQ ID NO: 77, a HCDR2 having SEQ ID NO: 54 and a HCDR3 having SEQ ID NO: 8, optionally wherein the VH comprises or consists of SEQ ID NO: 123; and

a VL comprising a LCDR1 having SEQ ID NO: 100, a LCDR2 having SEQUENCE A7 (of Figure 1) and a LCDR3 having SEQ ID NO: 31, optionally wherein the VL comprises or consists of SEQ ID NO: 146;

(n) a VH comprising a HCDR1 having SEQ ID NO: 78, a HCDR2 having SEQ ID NO: 55 and a HCDR3 having SEQ ID NO: 9, optionally wherein the VH comprises or consists of SEQ ID NO: 124; and

a VL comprising a LCDR1 having SEQ ID NO: 101, a LCDR2 having SEQUENCE A8 (of Figure 1) and a LCDR3 having SEQ ID NO: 32, optionally wherein the VL comprises or consists of SEQ ID NO: 147;

(o) a VH comprising a HCDR1 having SEQ ID NO: 80, a HCDR2 having SEQ ID NO: 57 and a HCDR3 having SEQ ID NO: 11, optionally wherein the VH comprises or consists of SEQ ID NO: 126; and

a VL comprising a LCDR1 having SEQ ID NO: 103, a LCDR2 having SEQUENCE A10 (of Figure 1) and a LCDR3 having SEQ ID NO: 34, optionally wherein the VL comprises or consists of SEQ ID NO: 149;

(p) a VH comprising a HCDR1 having SEQ ID NO: 81, a HCDR2 having SEQ ID NO: 58 and a HCDR3 having SEQ ID NO: 12, optionally wherein the VH comprises or consists of SEQ ID NO: 127; and

a VL comprising a LCDR1 having SEQ ID NO: 104, a LCDR2 having SEQUENCE A11 (of Figure 1) and a LCDR3 having SEQ ID NO: 35, optionally wherein the VL comprises or consists of SEQ ID NO: 150;

(q) a VH comprising a HCDR1 having SEQ ID NO: 82, a HCDR2 having SEQ ID NO: 59 and a HCDR3 having SEQ ID NO: 13, optionally wherein the VH comprises or consists of SEQ ID NO: 128; and

a VL comprising a LCDR1 having SEQ ID NO: 105, a LCDR2 having SEQUENCE A12 (of Figure 1) and a LCDR3 having SEQ ID NO: 36, optionally wherein the VL comprises or consists of SEQ ID NO: 151;

(r) a VH comprising a HCDR1 having SEQ ID NO: 85, a HCDR2 having SEQ ID NO: 62 and a HCDR3 having SEQ ID NO: 16, optionally wherein the VH comprises or consists of SEQ ID NO: 131; and

a VL comprising a LCDR1 having SEQ ID NO: 108, a LCDR2 having SEQUENCE A15 (of Figure 1) and a LCDR3 having SEQ ID NO: 39, optionally wherein the VL comprises or consists of SEQ ID NO: 154;

(s) a VH comprising a HCDR1 having SEQ ID NO: 87, a HCDR2 having SEQ ID NO: 64 and a HCDR3 having SEQ ID NO: 18, optionally wherein the VH comprises or consists of SEQ ID NO: 133; and

a VL comprising a LCDR1 having SEQ ID NO: 110, a LCDR2 having SEQUENCE A17 (of Figure 1) and a LCDR3 having SEQ ID NO: 41, optionally wherein the VL comprises or consists of SEQ ID NO: 156;

(t) a VH comprising a HCDR1 having SEQ ID NO: 89, a HCDR2 having SEQ ID NO: 66 and a HCDR3 having SEQ ID NO: 20, optionally wherein the VH comprises or consists of SEQ ID NO: 135; and

a VL comprising a LCDR1 having SEQ ID NO: 112, a LCDR2 having SEQUENCE A19 (of Figure 1) and a LCDR3 having SEQ ID NO: 43, optionally wherein the VL comprises or consists of SEQ ID NO: 158;

(u) a VH comprising a HCDR1 having SEQ ID NO: 90, a HCDR2 having SEQ ID NO: 67 and a HCDR3 having SEQ ID NO: 21, optionally wherein the VH comprises or consists of SEQ ID NO: 136; and

a VL comprising a LCDR1 having SEQ ID NO: 113, a LCDR2 having SEQUENCE A20 (of Figure 1) and a LCDR3 having SEQ ID NO: 44, optionally wherein the VL comprises or consists of SEQ ID NO: 159;

(v) a VH comprising a HCDR1 having SEQ ID NO: 91, a HCDR2 having SEQ ID NO: 68 and a HCDR3 having SEQ ID NO: 22, optionally wherein the VH comprises or consists of SEQ ID NO: 137; and

a VL comprising a LCDR1 having SEQ ID NO: 114, a LCDR2 having SEQUENCE A21 (of Figure 1) and a LCDR3 having SEQ ID NO: 45, optionally wherein the VL comprises or consists of SEQ ID NO: 160; and/or

(w) a VH comprising a HCDR1 having SEQ ID NO: 93, a HCDR2 having SEQ ID NO: 70 and a HCDR3 having SEQ ID NO: 24, optionally wherein the VH comprises or consists of SEQ ID NO: 139; and

a VL comprising a LCDR1 having SEQ ID NO: 116, a LCDR2 having SEQUENCE A23 (of Figure 1) and a LCDR3 having SEQ ID NO: 47, optionally wherein the VL comprises or consists of SEQ ID NO: 162.

Suitably the VH and VL regions recited above each comprise four framework regions (FR1-FR4). In one embodiment, the antibody or fragment thereof comprises a framework region (e.g. FR1, FR2, FR3 and/or FR4) comprising a sequence having at least 80% sequence identity with the framework region in any one of SEQ ID NOs: 117-162. In one embodiment, the antibody or fragment thereof comprises a framework region (e.g. FR1, FR2, FR3 and/or FR4) comprising a sequence having at least 90%, such as at least 95%, 97% or 99% sequence identity with the framework region in any one of SEQ ID NOs: 117-162. In one embodiment, the antibody or fragment thereof comprises a framework region (e.g. FR1, FR2, FR3 and/or FR4) comprising a sequence in any one of SEQ ID NOs: 117-162. In one embodiment, the antibody or fragment thereof comprises a framework region (e.g. FR1, FR2, FR3 and/or FR4) consisting of a sequence in any one of SEQ ID NOs: 117-162.

The antibodies described herein may be defined by their full light chain and/or heavy chain variable sequences. Therefore, in one embodiment, the anti-Vγ4 antibody or fragment thereof comprises an amino acid sequence having at least 80% sequence identity with any one of SEQ ID NOs: 117-162. In one embodiment, the anti-Vγ4 antibody or fragment thereof consists of an amino acid sequence having at least 80% sequence identity with any one of SEQ ID NOs: 117-162.

In one embodiment, the antibody or fragment thereof comprises a VH region comprising an amino acid sequence having at least 80% sequence identity with any one of SEQ ID NOs: 117-139. In one embodiment, the antibody or fragment thereof comprises a VH region consisting of an amino acid sequence having at least 80% sequence identity with any one of SEQ ID NOs: 117-139. In a further embodiment, the VH region comprises an amino acid sequence having at least 80% sequence identity with any one of SEQ ID NOs: 125, 119, 129, 130, 132, 134 or 138. In a further embodiment, the VH region consists of an amino acid sequence having at least 80% sequence identity with any one of SEQ ID NOs: 125, 119, 129, 130, 132, 134 or 138.

In one embodiment, the antibody or fragment thereof comprises a VL region comprising an amino acid sequence having at least 80% sequence identity with any one of SEQ ID NOs: 140-162. In one embodiment, the antibody or fragment thereof comprises a VL region consisting of an amino acid sequence having at least 80% sequence identity with any one of SEQ ID NOs: 140-162. In a further embodiment, the VL region comprises an amino acid sequence having at least 80% sequence identity with any one of SEQ ID NOs: 148, 142, 152, 153, 155, 157 or 161. In a further embodiment, the VL region consists of an amino acid sequence having at least 80% sequence identity with any one of SEQ ID NOs: 148, 142, 152, 153, 155, 157 or 161.

In a further embodiment, the antibody or fragment thereof comprises a VH region comprising an amino acid sequence having at least 80% sequence identity with any one of SEQ ID NOs: 117-139 and a VL region comprising an amino acid sequence having at least 80% sequence identity with any one of SEQ ID NOs: 140-162. In a further embodiment, the antibody or fragment thereof comprises a VH region consisting of an amino acid sequence having at least 80% sequence identity with any one of SEQ ID NOs: 117-139 and a VL region consisting of an amino acid sequence having at least 80% sequence identity with any one of SEQ ID NOs: 140-162.

In one embodiment, the antibody or fragment thereof comprises a VH region comprising an amino acid sequence of SEQ ID NO: 125 (1140_P01_G08) [G4_12]. In one embodiment, the antibody or fragment thereof comprises a VH region consisting of an amino acid sequence of SEQ ID NO: 125. In one embodiment, the antibody or fragment thereof comprises a VL region comprising an amino acid sequence of SEQ ID NO: 148 (1140_P01_G08) [G4_12]. In one embodiment, the antibody or fragment thereof comprises a VL region consisting of an amino acid sequence of SEQ ID NO: 148.

In one embodiment, the antibody or fragment thereof comprises a VH region comprising an amino acid sequence of SEQ ID NO: 125 and a VL region comprising an amino acid sequence of SEQ ID NO: 148. In one embodiment, the antibody or fragment thereof comprises a VH region consisting of an amino acid sequence of SEQ ID NO: 125 and a VL region consisting of an amino acid sequence of SEQ ID NO: 148.

In one embodiment, the antibody or fragment thereof comprises a VH region comprising an amino acid sequence of SEQ ID NO: 119 (1139_P01_A04) [G4_3]. In one embodiment, the antibody or fragment thereof comprises a VH region consisting of an amino acid sequence of SEQ ID NO: 119. In one embodiment, the antibody or fragment thereof comprises a VL region comprising an amino acid sequence of SEQ ID NO: 142 (1139_P01_A04) [G4_3]. In one embodiment, the antibody or fragment thereof comprises a VL region consisting of an amino acid sequence of SEQ ID NO: 142.

In one embodiment, the antibody or fragment thereof comprises a VH region comprising an amino acid sequence of SEQ ID NO: 119 and a VL region comprising an amino acid sequence of SEQ ID NO: 142. In one embodiment, the antibody or fragment thereof comprises a VH region consisting of an amino acid sequence of SEQ ID NO: 119 and a VL region consisting of an amino acid sequence of SEQ ID NO: 142.

In one embodiment, the antibody or fragment thereof comprises a VH region comprising an amino acid sequence of SEQ ID NO: 129 (1248_P02_D10) **[G4_16].** In one embodiment, the antibody or fragment thereof comprises a VH region consisting of an amino acid sequence of SEQ ID NO: 129. In one embodiment, the antibody or fragment thereof comprises a VL region comprising an amino acid sequence of SEQ ID NO: 152 (1248_P02_D10) **[G4_16].** In one embodiment, the antibody or fragment thereof comprises a VL region consisting of an amino acid sequence of SEQ ID NO: 152.

In one embodiment, the antibody or fragment thereof comprises a VH region comprising an amino acid sequence of SEQ ID NO: 129 and a VL region comprising an amino acid sequence of SEQ ID NO: 152. In one embodiment, the antibody or fragment thereof comprises a VH region consisting of an amino acid sequence of SEQ ID NO: 129 and a VL region consisting of an amino acid sequence of SEQ ID NO: 152.

In one embodiment, the antibody or fragment thereof comprises a VH region comprising an amino acid sequence of SEQ ID NO: 130 (1254_P01_H04) **[G4_18].** In one embodiment, the antibody or fragment thereof comprises a VH region consisting of an amino acid sequence of SEQ ID NO: 130. In one embodiment, the antibody or fragment thereof comprises a VL region comprising an amino acid sequence of SEQ ID NO: 153 (1254_P01_H04) **[G4_18].** In one embodiment, the antibody or fragment thereof comprises a VL region consisting of an amino acid sequence of SEQ ID NO: 153.

In one embodiment, the antibody or fragment thereof comprises a VH region comprising an amino acid sequence of SEQ ID NO: 130 and a VL region comprising an amino acid sequence of SEQ ID NO: 153. In one embodiment, the antibody or fragment thereof comprises a VH region consisting of an amino acid sequence of SEQ ID NO: 130 and a VL region consisting of an amino acid sequence of SEQ ID NO: 153.

In one embodiment, the antibody or fragment thereof comprises a VH region comprising an amino acid sequence of SEQ ID NO: 132 (1254_P02_G02) **[G4_20].** In one embodiment, the antibody or fragment thereof comprises a VH region consisting of an amino acid sequence of SEQ ID NO: 132. In one embodiment, the antibody or fragment thereof comprises a VL region comprising an amino acid sequence of SEQ ID NO: 155 (1254_P02_G02) **[G4_20].** In one embodiment, the antibody or fragment thereof comprises a VL region consisting of an amino acid sequence of SEQ ID NO: 155.

In one embodiment, the antibody or fragment thereof comprises a VH region comprising an amino acid sequence of SEQ ID NO: 132 and a VL region comprising an amino acid sequence of SEQ ID NO: 155. In one embodiment, the antibody or fragment thereof comprises a VH region consisting of an amino acid sequence of SEQ ID NO: 132 and a VL region consisting of an amino acid sequence of SEQ ID NO: 155.

In one embodiment, the antibody or fragment thereof comprises a VH region comprising an amino acid sequence of SEQ ID NO: 134 (1253_P03_H05) **[G4_23].** In one embodiment, the antibody or fragment thereof comprises a VH region consisting of an amino acid sequence of SEQ ID NO: 134. In one embodiment, the antibody or fragment thereof comprises a VL region comprising an amino acid sequence of SEQ ID NO: 157 (1253_P03_H05) **[G4_23].** In one embodiment, the antibody or fragment thereof comprises a VL region consisting of an amino acid sequence of SEQ ID NO: 157.

In one embodiment, the antibody or fragment thereof comprises a VH region comprising an amino acid sequence of SEQ ID NO: 134 and a VL region comprising an amino acid sequence of SEQ ID NO: 157. In one embodiment, the antibody or fragment thereof comprises a VH region consisting of an amino acid sequence of SEQ ID NO: 134 and a VL region consisting of an amino acid sequence of SEQ ID NO: 157.

In one embodiment, the antibody or fragment thereof comprises a VH region comprising an amino acid sequence of SEQ ID NO: 138 (1248_P02_C10) **[G4_27].** In one embodiment, the antibody or fragment thereof comprises a VH region consisting of an amino acid sequence of SEQ ID NO: 138. In one embodiment, the antibody or fragment thereof comprises a VL region comprising an amino acid sequence of SEQ ID NO: 161 (1248_P02_C10) **[G4_27].** In one embodiment, the antibody or fragment thereof comprises a VL region consisting of an amino acid sequence of SEQ ID NO: 161.

In one embodiment, the antibody or fragment thereof comprises a VH region comprising an amino acid sequence of SEQ ID NO: 138 and a VL region comprising an amino acid sequence of SEQ ID NO: 161. In one embodiment, the antibody or fragment thereof comprises a VH region consisting of an amino acid sequence of SEQ ID NO: 138 and a VL region consisting of an amino acid sequence of SEQ ID NO: 161.

For fragments comprising both the VH and VL regions, these may be associated either covalently (e.g. via disulphide bonds or a linker) or non-covalently. The antibody fragment described herein may comprise an scFv, *i.e.* a fragment comprising a VH region and a VL region joined by a linker. In one embodiment, the VH and VL region are joined by a (*e.g.* synthetic) polypeptide linker. The polypeptide linker may comprise a $(Gly_4Ser)_n$ linker, where n = from 1 to 8, *e.g.* 2, 3, 4, 5 or 7. The polypeptide linker may comprise a $[(Gly_4Ser)_n(Gly_3AlaSer)_m]_p$ linker, where n = from 1 to 8, *e.g.* 2, 3, 4, 5 or 7, m = from 0 to 8, *e.g.* 0, 1, 2 or 3, and p = from 1 to 8, *e.g.* 1, 2 or 3. In a further embodiment, the linker comprises SEQ ID NO: 186. In a further embodiment, the linker consists of SEQ ID NO: 186.

In one embodiment, the antibody or fragment thereof comprises an amino acid sequence having at least 80% sequence identity with any one of SEQ ID NOs: 163-185. In a further embodiment, the antibody or fragment thereof comprises an amino acid sequence of any one of SEQ ID NOs: 163-185. In a yet further embodiment, the antibody or fragment thereof comprises an amino acid sequence of SEQ ID NOs: 171, 165, 175, 176, 178, 180 or 184.

In one embodiment, the antibody or fragment thereof consists of an amino acid sequence having at least 80% sequence identity with any one of SEQ ID NOs: 163-185. In a further embodiment, the antibody or fragment thereof consists of an amino acid sequence of any one of SEQ ID NOs: 163-185. In a yet further embodiment, the antibody or fragment thereof consists of an amino acid sequence of SEQ ID NOs: 171, 165, 175, 176, 178, 180 or 184.

As described herein, the antibodies may be in any format. In a preferred embodiment, the antibody is in an IgG1 format. Therefore, in one embodiment, the antibody or fragment thereof comprises an amino acid sequence having at least 80% sequence identity with any one of SEQ ID NOs: 233-255. In a further embodiment, the antibody or fragment thereof comprises an amino acid sequence of any one of SEQ ID NOs: 233-255. In a yet further embodiment, the antibody or fragment thereof comprises an amino acid sequence of SEQ ID NOs: 235, 241, 245, 246 or 254.

In one embodiment, the antibody or fragment thereof consists of an amino acid sequence having at least 80% sequence identity with any one of SEQ ID NOs: 233-255. In a further embodiment, the antibody or fragment thereof consists of an amino acid sequence of any one of SEQ ID NOs: 233-255. In a yet further embodiment, the antibody or fragment thereof consists of an amino acid sequence of SEQ ID NOs: 235, 241, 245, 246 or 254.

Alternatively, there is provided an antibody or fragment thereof which comprises or consists of a heavy chain amino acid sequence having at least 80% sequence identity with any one of SEQ ID NOs: 284-306 and/or a light chain amino acid sequence having at least 80% sequence identity with any one of SEQ ID NOs: 307-329. Thus, there is provided an antibody or fragment thereof which comprises or consists of a heavy chain amino acid sequence according to any one of SEQ ID NOs: 284-306 and/or a light chain amino acid sequence according to any one of SEQ ID NOs: 307-329. In a particular embodiment, the antibody or fragment thereof comprises or consists of a heavy chain amino acid sequence according to SEQ ID NO: 292 and a light chain amino acid sequence according to SEQ ID NO: 315 (clone **G4_12).** In a further embodiment, the antibody or fragment thereof comprises or consists of a heavy chain amino acid sequence according to SEQ ID NO: 286 and a light chain amino acid sequence according to SEQ ID NO: 309 (clone **G4_3)**. In a further embodiment, the antibody or fragment thereof comprises or consists of a heavy chain amino acid sequence according to SEQ ID NO: 296 and a light chain amino acid sequence according to SEQ ID NO: 319 (clone **G4_16).** In a further embodiment, the antibody or fragment thereof comprises or consists of a heavy chain amino acid sequence according to SEQ ID NO: 297 and a light chain amino acid sequence according to SEQ ID NO: 320 (clone **G4_18).** In a further embodiment, the antibody or fragment thereof comprises or consists of a heavy chain amino acid sequence according to SEQ ID NO: 299 and a light chain amino acid sequence according to SEQ ID NO: 322 (clone **G4_20).** In a further embodiment, the antibody or fragment thereof comprises or consists of a heavy chain amino acid sequence according to SEQ ID NO: 301 and a light chain amino acid sequence according to SEQ ID NO: 324 (clone **G4_23).** In a further embodiment, the antibody or fragment thereof comprises or consists of a heavy chain amino acid sequence according to SEQ ID NO: 305 and a light chain amino acid sequence according to SEQ ID NO: 328 (clone **G4_27).** In other embodiments, the antibody or fragment thereof comprises or consists of:

(a) a heavy chain amino acid sequence according to SEQ ID NO: 284 and a light chain amino acid sequence according to SEQ ID NO: 307 (clone **G4_1**);
(b) a heavy chain amino acid sequence according to SEQ ID NO: 285 and a light chain amino acid sequence according to SEQ ID NO: 308 (clone **G4_2)**;
(c) a heavy chain amino acid sequence according to SEQ ID NO: 287 and a light chain amino acid sequence according to SEQ ID NO: 310 (clone **G4_4**);
(d) a heavy chain amino acid sequence according to SEQ ID NO: 288 and a light chain amino acid sequence according to SEQ ID NO: 311 (clone **G4_5**);
(e) a heavy chain amino acid sequence according to SEQ ID NO: 289 and a light chain amino acid sequence according to SEQ ID NO: 312 (clone **G4_6**);
(f) a heavy chain amino acid sequence according to SEQ ID NO: 290 and a light chain amino acid sequence according to SEQ ID NO: 313 (clone **G4_7**);
(g) a heavy chain amino acid sequence according to SEQ ID NO: 291 and a light chain amino acid sequence according to SEQ ID NO: 314 (clone **G4_10)**;
(h) a heavy chain amino acid sequence according to SEQ ID NO: 293 and a light chain amino acid sequence according to SEQ ID NO: 316 (clone **G4_13)**;
(i) a heavy chain amino acid sequence according to SEQ ID NO: 294 and a light chain amino acid sequence according to SEQ ID NO: 317 (clone **G4_14)**;
(j) a heavy chain amino acid sequence according to SEQ ID NO: 295 and a light chain amino acid sequence according to SEQ ID NO: 318 (clone **G4_15)**;
(k) a heavy chain amino acid sequence according to SEQ ID NO: 298 and a light chain amino acid sequence according to SEQ ID NO: 321 (clone **G4_19)**;
(l) a heavy chain amino acid sequence according to SEQ ID NO: 300 and a light chain amino acid sequence according to SEQ ID NO: 323 (clone **G4_22)**;
(m) a heavy chain amino acid sequence according to SEQ ID NO: 302 and a light chain amino acid sequence according to SEQ ID NO: 325 (clone **G4_24)**;
(n) a heavy chain amino acid sequence according to SEQ ID NO: 303 and a light chain amino acid sequence according to SEQ ID NO: 326 (clone **G4_25)**;
(o) a heavy chain amino acid sequence according to SEQ ID NO: 304 and a light chain amino acid sequence according to SEQ ID NO: 327 (clone **G4_26)**;
or
(p) a heavy chain amino acid sequence according to SEQ ID NO: 306 and a light chain amino acid sequence according to SEQ ID NO: 329 (clone **G4_28).**

Competing antibodies

In one embodiment, the antibody or fragment thereof which specifically binds to a Vγ4 chain of a γδ TCR and not to a Vy2 chain of a γδ TCR binds to the same, or essentially the same, epitope as, or competes with, an antibody or fragment thereof as defined or exemplified herein. One can easily determine whether an antibody binds to the same epitope as, or competes for binding with, a reference anti-Vγ4 antibody by using routine methods known in the art. For example, to determine if a test antibody binds to the same epitope as a reference anti-Vγ4 antibody described herein, the reference antibody is allowed to bind to a Vγ4 protein or peptide under saturating conditions. Next, the ability of a test antibody to bind to the Vγ4 chain is assessed. If the test antibody is able to bind to Vγ4 following saturation binding with the reference anti-Vγ4 antibody, it can be concluded that the test antibody binds to a different epitope than the reference anti-Vγ4 antibody. On the other hand, if the test antibody is not able to bind to the Vγ4 chain following saturation binding with the reference anti-Vγ4 antibody, then the test antibody may bind to the same epitope as the epitope bound by the reference anti-Vγ4 antibody.

The present disclosure also includes anti-Vγ4 antibodies or fragments thereof that compete for binding to Vγ4 with an antibody or fragment thereof as defined herein, or an antibody having the CDR sequences of any of the exemplary antibodies described herein. For example, competitive assays can be performed with the antibodies described herein in order to determine what proteins, antibodies, and other antagonists compete for binding to the Vγ4 chain with the antibody and/or share the epitope. These assays are readily known to those of skill in the art; they evaluate competition between antagonists or ligands for a limited number of binding sites on a protein, *e.g.*, Vγ4. The antibody (or fragment thereof) is immobilized or insolubilized before or after the competition and the sample bound to the Vγ4 chain is separated from the unbound sample, for example, by decanting (where the antibody was pre-insolubilized) or by centrifuging (where the antibody was precipitated after the competitive reaction). Also, the competitive binding may be determined by whether the function is altered by the binding or lack of binding of the antibody to the protein, *e.g.*, whether the antibody molecule inhibits or potentiates the enzymatic activity of, for example, a label. ELISA and other functional assays may be used, as known in the art and described herein.

Two antibodies bind to the same or overlapping epitope if each competitively inhibits (blocks) binding of the other to the target antigen. That is, a 1-, 5-, 10-, 20- or 100-fold or more excess of one antibody inhibits binding of the other by at least 50% but preferably 75%, 90% or even 99% as measured in a competitive binding assay. Alternatively, two antibodies have the same epitope if essentially all amino acid mutations in the target antigen that reduce or eliminate binding of one antibody also reduce or eliminate binding of the other.

Additional routine experimentation (*e.g.*, peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

Antibody sequence modifications

The antibodies and fragments thereof may be modified using known methods. Sequence modifications to antibody molecules described herein can be readily incorporate by those skilled in the art. The following examples are non-limiting.

During antibody discovery and sequence recovery from phage libraries, desired antibody variable domains may be re-formatted into full length IgG by sub-cloning. To accelerate the process, variable domains are often transferred using restriction enzymes. These restriction sites may introduce additional/alternate amino acids and away from the canonical sequence (such canonical sequences may be found, for example, in the international ImMunoGeneTics [IMGT] information system, see http://www.imgt.org). These may be introduced as kappa or lambda light chain sequence modifications.

*Kappa light chain modifications*

The kappa light chain variable sequences may be cloned using restriction sites (*e.g.* Nhe1-Not1) during re-formatting into full length IgG. More specifically, at the kappa light chain N-terminus, an additional Ala-Ser sequence was introduced to support cloning. Preferably, this additional AS sequence is then removed during further development such to generate the canonical N-terminal sequence. Hence, in one embodiment, kappa light chain containing antibodies described herein do not contain an AS sequence at their N-termini, *i.e.* SEQ ID NOs: 140-147 and 156-158 do not comprise the initial AS sequence. It will be understood that this embodiment also applies to other sequences included herein which contain this sequence.

Additional amino acid changes may be made to support cloning. For example, for the antibodies described herein, at the kappa light-chain variable-domain/constant domain border a valine-to-alanine change was introduced to support cloning when preparing full-length sequences. This resulted in a kappa constant domain modification. Specifically this results in the constant domain beginning RTAAAPS (from a NotI restriction site). Preferably, this sequence can be modified during further development to generate the canonical kappa light-chain constant regions which start with RTVAAPS. Such modifications do not change the functional properties of the antibodies. Hence, in one embodiment kappa light chain containing antibodies described herein contain a constant domain starting with the sequence RTV. Therefore, in one embodiment, sequence RTAAAPS of SEQ ID NOs: 233-240, 249-251, 307-314 and 323-325 is replaced with sequence RTVAAPS. In a preferred embodiment comprising a preferred kappa light chain constant domain allotype, the kappa light chain constant domain has an amino acid sequence according to SEQ ID NO: 330 and may be combined with any light chain variable domain disclosed herein.

*Lambda light chain modifications*

Similar to the kappa example above, the lambda light chain variable domains may also be cloned by introducing restriction sites (*e.g.* Nhe1-Not1) during re-formatting into full length IgG. More specifically, at the lambda light chain N-terminus, an additional Ala-Ser sequence may be introduced to support cloning. Preferably, this additional AS sequence is then removed during further development such to generate the canonical N-terminal sequence. Hence, in one embodiment, lambda light chain containing antibodies described herein do not contain an AS sequence at their N-termini *i.e.* SEQ ID NOs: 148-155 and 159-162 do not comprise the initial AS sequence. It will be understood that this embodiment also applies to other sequences included herein which contain this sequence.

As another example, for the antibodies described herein at the lambda light-chain variable-domain/constant domain border a lysine-to-alanine sequence change was introduced to support cloning when preparing full-length sequences. This resulted in a lambda constant domain modification. Specifically this results in the constant domain beginning with GQPAAAPS (from a NotI restriction site). Preferably, this sequence can be modified during further development such to generate the canonical lambda light constant region which starts GQPKAAPS. Such modifications do not change the functional properties of the antibodies. Hence, in one embodiment, lambda light chain containing antibodies described herein contain a constant domain starting with the sequence GQPK. Therefore, in one embodiment, sequence GQPAAAPS of SEQ ID NOs: 241-248, 252-255, 315-322 and 326-329 is replaced with sequence GQPKAAPS. In a preferred embodiment comprising a preferred lambda light chain constant domain allotype, the lambda light chain constant domain has an amino acid sequence according to SEQ ID NO: 331 and may be combined with any light chain variable domain disclosed herein.

*Lambda and Kappa light chain modifications*

In view of the above disclosure regarding removal of the N-terminal AS residues from the lambda and/or kappa light chain variable domains disclosed herein as SEQ ID Nos: 140-162, the anti-V$\gamma$4 antibody or fragment may comprise a light chain variable (VL) amino acid sequence according to any one of SEQ ID NOs: 261-283, which correspond to SEQ ID NOs: 140-162 lacking the N-terminal AS residues. Therefore, any reference in this specification to a VL amino acid sequence according to one or more of SEQ ID NOs: 140-162 may be substituted with a VL amino acid sequence according to SEQ ID NOs: 261-283 respectively, and all such embodiments are hereby disclosed. By way of illustration, reference herein to a light chain variable domain according to SEQ ID NO: 148 (derived from clone **G4_12)** may be substituted with reference to SEQ ID NO: 269.

*Heavy chain modifications*

Typically, human variable heavy chain sequences start with either the basic glutamine (Q) or acidic glutamate (E). However both such sequences are then known to convert to the acidic amino acid residue, pyro-glutamate (pE). The Q to pE conversion results in a charge change to the antibody, whilst a E to pE conversion does not change the charge of the antibody. Hence, to avoid a variable charge-change over time, one option is to modify a starting heavy chain sequence from Q to E in the first instance. Hence, in one embodiment, the heavy chain of antibody described herein having a Q residue at the N-terminus of the heavy chain may contain a Q to E modification at the N-terminus. In particular, the initial residue of any of SEQ ID NOs: 118, 120, 124, 126, 132, 133, 135, 137, 138 and/or 139 may be modified from Q to E. It will be understood that this embodiment also applies to other sequences included herein which contain this sequence (i.e. any embodiment incorporating these sequences, for example into full-length antibodies or fragments thereof). In some embodiments, it may be advantageous to substitute an E residue at the N-terminus of the heavy chain to a Q residue. Accordingly, in some embodiments, the E residue at the N-terminus of any one SEQ ID NOs: 117, 119, 121-123, 125, 127-131, 134 and/or 136 may be substituted with a Q residue.

Furthermore, the C-terminus of the IgG1 constant domain ends with PGK. However the terminal basic lysine (K) is then often cleaved during expression (*e.g.* in CHO cells). This in turn results in a charge change to the antibody through varied loss of the C-terminal lysine residue. Therefore, one option is to remove the lysine in the first instance resulting in a uniform and consistent heavy chain C-terminus sequence ending in PG. Hence, in one embodiment, the heavy chain of an antibody described herein has the terminal K removed from its C-terminus. In particular, the antibody may comprise any one of SEQ ID NOs: 233-255 or 284-306 where the terminal lysine residue has been removed.

*Optional allotype modifications*

During antibody discovery, specific human allotypes may be employed. Optionally, the antibodies can be switched to differing human allotypes during development. By way of non-limiting example, for the kappa chain there are three human allotypes designated Km1, Km1,2 and Km3 which define three Km alleles (using allotype numbering): Km1 correlates with valine153 (IMGT V45.1) and leucine 191 (IMGT L101); Km1,2 correlates with alanine 153 (IMGT A45.1) and leucine 191 (IMGT L101); and Km3 correlates with alanine 153 (IMGT A45.1) and valine 191 (IMGT V101). Optionally, one can therefore modify a sequence from one allotype to another by standard cloning approaches. For example a L191V (IMGT L101V) change will convert a Km1,2 allotype to a Km3 allotype. For further reference on such allotypes see Jefferis and Lefranc (2009) MAbs 1(4):332-8, which is herein incorporated by reference.

Hence in one embodiment an antibody described herein contains amino acid substitutions derived from another human allotype of the same gene. In a further embodiment, the antibody contains a L191V (IMGT L101V) substitution to the kappa chain to convert the c-domain from a km1,2 to a km3 allotype.

In a preferred embodiment comprising a preferred kappa light chain constant domain allotype, the kappa light chain constant domain has an amino acid sequence according to SEQ ID NO: 330 and may be combined with any light chain variable domain disclosed herein. In an alternative preferred embodiment comprising a preferred lambda light chain constant domain allotype, the lambda light chain constant domain has an amino acid sequence according to SEQ ID NO: 331 and may be combined with any light chain variable domain disclosed herein.

Antibody binding

The antibody or fragment thereof may bind to the Vγ4 chain of a γδ TCR with a binding affinity (KD) as measured by surface plasmon resonance of less than $3.0 \times 10^{-7}$ M (*i.e.* 300 nM) or less than $1.5 \times 10^{-7}$ M (*i.e.* 150 nM). In a further embodiment, the KD is $1.3 \times 10^{-7}$ M (*i.e.* 130 nM) or less, such as $1.0 \times 10^{-7}$ M (*i.e.* 100 nM) or less. In a yet further embodiment, the KD is less than $6.0 \times 10^{-8}$ M (*i.e.* 60 nM), such as less than $5.0 \times 10^{-8}$ M (*i.e.* 50 nM), less than $4.0 \times 10^{-8}$ M (*i.e.* 40 nM), less than $3.0 \times 10^{-8}$ M (*i.e.* 30 nM) or less than $2.0 \times 10^{-8}$ M (*i.e.* 20 nM). In further embodiments, the KD may be $1.0 \times 10^{-8}$ M (*i.e.* 10 nM) or less, such as $5.0 \times 10^{-9}$ M (*i.e.* 5 nM) or less, $4.0 \times 10^{-9}$ M (*i.e.* 4 nM) or less, $3.0 \times 10^{-9}$ M (*i.e.* 3 nM) or less, $2.0 \times 10^{-9}$ M (*i.e.* 2 nM) or less, or $1.0 \times 10^{-9}$ M (*i.e.* 1 nM) or less. For example, in one embodiment the (e.g. human) anti-Vγ4 antibody binds to the Vγ4 chain of a γδ TCR with a binding affinity (KD) as measured by surface plasmon resonance of less than $1.5 \times 10^{-7}$ M (*i.e.* 150 nM).

In one embodiment, the antibody or fragment thereof which binds to the Vγ4 chain of a γδ TCR with a binding affinity (KD) as measured by surface plasmon resonance of less than $4.0 \times 10^{-8}$ M (*i.e.* 40 nM), less than $3.0 \times 10^{-8}$ M (*i.e.* 30 nM) or less than $2.0 \times 10^{-8}$ M (*i.e.* 20 nM).

In one embodiment, the binding affinity of the antibody or fragment thereof is established by coating the antibody or fragment thereof directly or indirectly (*e.g.* by capture with an anti-human IgG Fc) onto the surface of a sensor (*e.g.* an amine high capacity chip or equivalent), wherein the target bound by the antibody or fragment thereof (*i.e.* the Vγ4 chain of a γδ TCR) is flowed over the chip to detect binding. In alternative embodiments, the antigen may be directly or indirectly coated onto the surface of the sensor, over which test antibody or a fragment thereof is then flowed. The skilled person is well able to determine suitable test conditions. For example, suitably, a MASS-2 instrument (which may also be referred to as Sierra SPR-32) may be used at 25 °C in PBS + 0.02 % Tween 20 running buffer at 30 µl/min. In other suitable embodiments, a Reichert 4SPR instrument may be used at room temperature (e.g. 25 °C) in PBS + 0.05 % Tween 20 with a flowrate of 25 µl/min.

Antibody functional characterisation

Described herein are assays which may be used to define antibody function. For example, the antibody or fragment thereof described herein may be assessed by γδ TCR engagement, e.g. measuring downregulation of the γδ TCR upon antibody binding and/or upregulation of CD69 surface expression upon antibody binding. Surface expression of the γδ TCR or CD69 following application of the antibody or fragment thereof (optionally presented on the surface of a cell) can be measured, e.g. by flow cytometry.

The antibody or fragment thereof described herein may also be assessed by measuring γδ T cell degranulation. For example, expression of CD107a, a marker for cell degranulation, can be measured following application of the antibody or fragment thereof (optionally presented on the surface of a cell) to γδ T cells, e.g. by flow cytometry. The antibody or fragment thereof described herein may also be assessed by measuring $\gamma\delta$ T cell-mediated killing activity (to test if the antibody has an effect on the killing activity of the $\gamma\delta$ T cell i.e. the ability of the antibody to induce the $\gamma\delta$ T cell to directly or indirectly kill target cells). For example, target cells may be incubated with $\gamma\delta$ T cells in the presence of the antibody or fragment thereof (optionally presented on the surface of a cell). Following incubation, the culture may be stained with a cell viability dye to distinguish between live and dead target cells. The proportion of dead cells can then be measured, *e.g.* by flow cytometry.

As described herein, the antibodies or fragments thereof used in the assays may be presented on a surface, for example the surface of a cell, such as a cell comprising an Fc receptor. For example, the antibodies or fragments thereof may be presented on the surface of THP-1 cells, such as TIB-202™ cells (available from American Type Culture Collection (ATCC)). Alternatively, the antibodies or fragments thereof may be used directly in the assays.

In such functional assays, output may be measured by calculating the half maximal concentration, also referred to as "EC50" or "effective concentration at 50 percent". The term "IC50" refers to the inhibitory concentration. Both EC50 and IC50 may be measured using methods known in the art, such as flow cytometry methods. For the avoidance of doubt, the values of EC50 in the present application are provided using IgG1 formatted antibody. Such values can be easily converted based on the molecular weight of the antibody format for equivalent values as follows:

$$(\mu g/ml) / (MW \text{ in } kDa) = \mu M$$

Millilitres may be denoted as "ml" or "mL" herein and used interchangeably.

The EC50 for downregulation of the $\gamma\delta$ TCR upon antibody (or fragment) binding may be less than 0.5 $\mu$g/ml, such as less than 0.4 $\mu$g/ml, 0.3 $\mu$g/ml, 0.2 $\mu$g/ml, 0.15 $\mu$g/ml, 0.1 $\mu$g/ml or 0.05 $\mu$g/ml. In particular, said EC50 values are when the antibody is measured in an IgG1 format. For example, the EC50 $\gamma\delta$ TCR downregulation value can be measured using flow cytometry.

The EC50 for $\gamma\delta$ T cell degranulation upon antibody (or fragment) binding may be less than 0.05 $\mu$g/ml, such as less than 0.04 $\mu$g/ml, 0.03 $\mu$g/ml, 0.02 $\mu$g/ml, 0.015 $\mu$g/ml, 0.01 $\mu$g/ml or 0.008 $\mu$g/ml. In particular, said EC50 values are when the antibody is measured in an IgG1 format. For example, the $\gamma\delta$ T cell degranulation EC50 value can be measured by detecting CD107a expression (*i.e.* a marker of cell degranulation) using flow cytometry. In one embodiment, CD107a expression is measured using an anti-CD107a antibody, such as anti-human CD107a BV421 (clone H4A3) (BD Biosciences).

The EC50 for $\gamma\delta$ T cell-mediated killing upon the antibody (or fragment) binding may be less than 0.5 $\mu$g/ml, such as less than 0.4 $\mu$g/ml, 0.3 $\mu$g/ml, 0.2 $\mu$g/ml, 0.15 $\mu$g/ml, 0.1 $\mu$g/ml or 0.07 $\mu$g/ml. In particular, said EC50 values are when the antibody is measured in an IgG1 format. For example, the EC50 $\gamma\delta$ T cell-mediated killing value can be measured by detecting proportion of dead cells (*i.e.* using a cell viability dye) using flow cytometry following incubation of the antibody, $\gamma\delta$ T cell and target cells. In one embodiment, death of the target cell is measured using a cell viability dye is Viability Dye eFluor™ 520 (ThermoFisher).

In the assays described in these aspects, the antibody or fragment thereof may be presented on the surface of a cell, such as a THP-1 cell, for example TIB-202™ (ATCC). The THP-1 cells are optionally labelled with a dye, such as CellTracker™ Orange CMTMR (ThermoFisher).

## Polynucleotides and expression vectors

Also provided are polynucleotides encoding the anti-V$\gamma$4 antibodies or fragments described herein. In one embodiment, the anti-V$\gamma$4 antibody or fragment thereof is encoded by a polynucleotide which comprises or consists of a sequence having at least 70%, such as at least 80%, such as at least 90%, such as at least 95%, such as at least 99% sequence identity with SEQ ID NOs: 187-232. In one embodiment, the anti-V$\gamma$4 antibody or fragment thereof is encoded by an expression vector which comprises the VH region of SEQ ID NOs: 187-209. In another embodiment, the anti-V$\gamma$4 antibody or fragment thereof is encoded by an expression vector comprises the VL region of SEQ ID NOs: 210-232. In a further embodiment, the anti-V$\gamma$4 antibody or fragment thereof is encoded by a polynucleotide which comprises or consists of SEQ ID NOs: 187-232. In a further embodiment, there is provided a cDNA comprising said polynucleotide.

In one embodiment, the polynucleotide comprises or consists of a sequence having at least 70%, such as at least 80%, such as at least 90%, such as at least 95%, such as at least 99% sequence identity with SEQ ID NOs: 195, 189, 199, 200, 202, 204, 208, 218, 212, 222, 223, 225, 227 or 231. In one embodiment, the expression vector comprises the VH region of SEQ ID NOs: 195, 189, 199, 200, 202, 204, or 208. In another embodiment, the expression vector comprises the VL region of SEQ ID NOs: 218, 212, 222, 223, 225, 227 or 231. In a further embodiment the polynucleotide comprises or consists of SEQ ID NOs: 195, 189, 199, 200, 202, 204, 208, 218, 212, 222, 223, 225, 227 or 231, in particular SEQ ID NO: 195 and/or 218; or SEQ ID NO: 189 and/or 212. In a further embodiment, there is provided a cDNA comprising said polynucleotide.

In one embodiment, the polynucleotide comprises or consists of a sequence having at least 70%, such as at least 80%, such as at least 90%, such as at least 95%, such as at least 99% sequence identity with any one of the portions of SEQ ID NOs: 187-232 which encodes CDR1, CDR2 and/or CDR3 of the encoded immunoglobulin chain variable domain. In one embodiment, the polynucleotide comprises or consists of a sequence having at least 70%, such as at least 80%, such as at least 90%, such as at least 95%, such as at least 99% sequence identity with any one of the portions of SEQ ID NOs: 195, 189, 199, 200, 202, 204, 208, 218, 212, 222, 223, 225, 227 or 231 which encodes CDR1, CDR2 and/or CDR3 of the encoded immunoglobulin chain variable domain.

In one embodiment, the polynucleotide comprises or consists of a sequence having at least 70%, such as at least 80%, such as at least 90%, such as at least 95%, such as at least 99% sequence identity with any one of the portions of SEQ ID NOs: 187-232 which encodes FR1, FR2, FR3 and/or FR4 of the encoded immunoglobulin chain variable domain. In one embodiment, the polynucleotide comprises or consists of a sequence having at least 70%, such as at least 80%, such as at least 90%, such as at least 95%, such as at least 99% sequence identity with any one of the portions of SEQ ID NOs: 195, 189, 199, 200, 202, 204, 208, 218, 212, 222, 223, 225, 227 or 231 which encodes FR1, FR2, FR3 and/or FR4 of the encoded immunoglobulin chain variable domain.

To express the antibodies, or fragments thereof, polynucleotides encoding partial or full-length light and heavy chains, as described herein, are inserted into expression vectors such that the genes are operatively linked to transcriptional and translational control sequences (which may be termed an 'expression cassette' as well understood in the art). Therefore, also described is an expression vector comprising a polynucleotide sequence as defined herein. In one embodiment, the expression vector comprises the VH sequence of any one of SEQ ID NOs: 187-209, such as any one of SEQ ID NOs: 195, 189, 199, 200, 202, 204 or 208. In another embodiment, the expression vector comprises the VL region of any one of SEQ ID NOs: 210-232, such as any one of SEQ ID NOs: 218, 212, 222, 223, 225, 227 or 231. Such expression vectors or cassettes may be used in pairs, suitably pairing the heavy and light chain variable sequences according to the pairing of various amino acid sequences providing the antibodies disclosed herein. In some embodiments, the expression vectors comprise a sequence having at least 70%, such as at least 80%, such as at least 90%, such as at least 95%, such as at least 99% sequence identity or 100% identity with any one of SEQ ID NOs: 187-209 (encoding a variable heavy region) and further comprises a sequence having at least 70%, such as at least 80%, such as at least 90%, such as at least 95%, such as at least 99% sequence identity or 100% identity with any one of SEQ ID NOs: 210-232 (encoding a variable light region). Again, the sequences may be provided in specific pairs as described herein to encode the antibodies disclosed herein.

The polynucleotides and expression vectors may also be described in reference to the amino acid sequence encoded. Therefore, in one embodiment, the polynucleotide comprises or consists of a sequence encoding the amino acid sequence of any one of SEQ ID NOs: 1 to 186, 233-260.

Mutations can be made to the DNA or cDNA that encode polypeptides which are silent as to the amino acid sequence of the polypeptide, but which provide preferred codons for translation in a particular host. The preferred codons for translation of a nucleic acid in, *e.g., E. coli* and S. *cerevisiae,* as well as mammalian, specifically human, are known.

Mutation of polypeptides can be achieved for example by substitutions, additions or deletions to a nucleic acid encoding the polypeptide. The substitutions, additions or deletions to a nucleic acid encoding the polypeptide can be introduced by many methods, including for example error-prone PCR, shuffling, oligonucleotide-directed mutagenesis, assembly PCR, PCR mutagenesis, *in vivo* mutagenesis, cassette mutagenesis, recursive ensemble mutagenesis, exponential ensemble mutagenesis, site-specific mutagenesis, gene reassembly, artificial gene synthesis, Gene Site Saturation Mutagenesis (GSSM), synthetic ligation reassembly (SLR) or a combination of these methods. The modifications, additions or deletions to a nucleic acid can also be introduced by a method comprising recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, ensemble mutagenesis, chimeric nucleic acid multimer creation, or a combination thereof.

In particular, artificial gene synthesis may be used. A gene encoding a polypeptide can be synthetically produced by, for example, solid-phase DNA synthesis. Entire genes may be synthesized *de novo,* without the need for precursor template DNA. To obtain the desired oligonucleotide, the building blocks are sequentially coupled to the growing oligonucleotide chain in the order required by the sequence of the product. Upon the completion of the chain assembly, the product is released from the solid phase to solution, deprotected, and collected. Products can be isolated by high-performance liquid chromatography (HPLC) to obtain the desired oligonucleotides in high purity.

Expression vectors include, for example, plasmids, retroviruses, cosmids, yeast artificial chromosomes (YACs) and Epstein-Barr virus (EBV) derived episomes. The polynucleotide is ligated into a vector such that transcriptional and translational control sequences within the vector serve their intended function of regulating the transcription and translation of the polynucleotide. Expression and/or control sequences can include promoters, enhancers, transcription terminators, a start codon (i.e. ATG) 5' to the coding sequence, splicing signals for introns and stop codons. The expression vector and expression control sequences are chosen to be compatible with the expression host cell used. Thus, also described is a nucleotide sequence encoding single chain variable fragments according to any one of SEQ ID NOs:

163-185, comprising a VH region and a VL region joined by a synthetic linker (encoding SEQ ID NO: 186). It will be understood that polynucleotides or expression vectors described herein may comprise the VH region, the VL region or both (optionally including the linker). Therefore, polynucleotides encoding the VH and VL regions can be inserted into separate vectors, alternatively sequences encoding both regions are inserted into the same expression vector. The polynucleotide(s) are inserted into the expression vector by standard methods (*e.g.*, ligation of complementary restriction sites on the polynucleotide and vector, or blunt end ligation if no restriction sites are present).

A convenient vector is one that encodes a functionally complete human CH or CL immunoglobulin sequence, with appropriate restriction sites engineered so that any VH or VL sequence can be easily inserted and expressed, as described herein. The expression vector can also encode a signal peptide that facilitates secretion of the antibody (or fragment thereof) from a host cell. The polynucleotide may be cloned into the vector such that the signal peptide is linked in-frame to the amino terminus of the antibody. The signal peptide can be an immunoglobulin signal peptide or a heterologous signal peptide (*i.e.,* a signal peptide from a non-immunoglobulin protein).

A host cell may comprise a first vector encoding the light chain of the antibody or fragment thereof, and a second vector encoding the heavy chain of the antibody or fragment thereof. Alternatively, the heavy and light chains may both be encoded on the same expression vector introduced into the host cell.

In one embodiment, the polynucleotide or expression vector encodes a membrane anchor or transmembrane domain fused to the antibody or fragment thereof, wherein the antibody or fragment thereof is presented on an extracellular surface of the host cell.

Transformation can be by any known method for introducing polynucleotides into a host cell. Methods for introduction of heterologous polynucleotides into mammalian cells are well known in the art and include dextran-mediated transfection, calcium phosphate precipitation, polybrene-mediated transfection, protoplast fusion, electroporation, transduction, encapsulation of the polynucleotide(s) in liposomes, biolistic injection and direct microinjection of the DNA into nuclei. In addition, nucleic acid molecules may be introduced into mammalian cells by viral vectors.

Mammalian cell lines available as hosts for expression are well known in the art and include many immortalized cell lines available from the American Type Culture Collection (ATCC). These include, inter alia, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. Antigen-binding fragments of antibodies such as the scFv and Fv fragments can be isolated and expressed in *E. coli* using methods known in the art.

The antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Antibodies (or fragments) described herein can be obtained and manipulated using the techniques disclosed for example in Green and Sambrook, Molecular Cloning: A Laboratory Manual (2012) 4th Edition Cold Spring Harbour Laboratory Press.

Monoclonal antibodies can be produced using hybridoma technology, by fusing a specific antibody-producing B cell with a myeloma (B cell cancer) cell that is selected for its ability to grow in tissue culture and for an absence of antibody chain synthesis.

A monoclonal antibody directed against a determined antigen can, for example, be obtained by:

a) immortalizing lymphocytes obtained from the peripheral blood of an animal previously immunized with a determined antigen, with an immortal cell and preferably with myeloma cells, in order to form a hybridoma,
b) culturing the immortalized cells (hybridoma) formed and recovering the cells producing the antibodies having the desired specificity.

Alternatively, the use of a hybridoma cell is not required. Antibodies capable of binding to the target antigens as described herein may be isolated from a suitable antibody library via routine practice, for example, using the phage display, yeast display, ribosomal display, or mammalian display technology known in the art. Accordingly, monoclonal antibodies can be obtained, for example, by a process comprising the steps of:

a) cloning into vectors, especially into phages and more particularly filamentous bacteriophages, DNA or cDNA sequences obtained from lymphocytes especially peripheral blood lymphocytes of an animal (suitably previously immunized with determined antigens),
b) transforming prokaryotic cells with the above vectors in conditions allowing the production of the antibodies,
c) selecting the antibodies by subjecting them to antigen-affinity selection,

d) recovering the antibodies having the desired specificity.

**Pharmaceutical compositions**

According to a further aspect of the invention, there is provided a composition comprising the Vγ4 T cell population obtained by a method as defined herein. In one embodiment the Vγ4 T cell population is the expanded Vγ4 T cell population. In such embodiments, the composition may comprise the cells, optionally in combination with other excipients. Also included are compositions comprising one or more additional active agents (e.g. active agents suitable for treating the diseases mentioned herein).

Pharmaceutical compositions may include Vγ4 T cells, in particular expanded Vγ4 T cells, as described herein in combination with one or more pharmaceutically or physiologically acceptable carrier, diluents, or excipients. Such compositions may include buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e.g.*, aluminium hydroxide); and preservatives. Cryopreservation solutions which may be used in the pharmaceutical compositions of the invention include, for example, DMSO. Compositions can be formulated, *e.g.,* for intravenous administration.

In one embodiment, the pharmaceutical composition is substantially free of, *e.g.*, there are no detectable levels of a contaminant, *e.g.,* of endotoxin or mycoplasma.

The preferred mode of administration is parenteral (*e.g.*, intravenous, subcutaneous, intraperitoneal, intramuscular, intrathecal). In a preferred embodiment, the composition is administered by intravenous infusion or injection. In another preferred embodiment, the composition is administered by intramuscular or subcutaneous injection.

It is within the scope of the invention to use the pharmaceutical composition of the invention in therapeutic methods for the treatment of diseases as described herein as an adjunct to, or in conjunction with, other established therapies normally used in the treatment of such diseases.

In a further aspect of the invention, the cell population, composition or pharmaceutical composition is administered sequentially, simultaneously or separately with at least one active agent.

**Treatment methods using cell populations**

According to a further aspect of the invention, there is provided a cell population obtained by a method as defined herein for use as a medicament. According to a further aspect of the invention, there is provided the expanded cell population as defined herein for use as a medicament. References herein to a cell population "for use" as a medicament or in therapy are limited to administration of the cell population to a subject. Such uses do not include administration of the antibody or fragment thereof direct to a patient i.e. wherein said antibody is used as the therapeutic.

In one embodiment, the cell population is for use in the treatment of cancer, an infectious disease or an inflammatory disease. In a further embodiment, the cell population is for use in the treatment of cancer.

In one embodiment, the cell population for use as a medicament comprises more than 50% Vγ4 T cells, such as more than 60%, more than 70%, more than 80%, more than 90%, more than 95% or more than 99% Vγ4 T cells. In a further embodiment, the cell population for use as a medicament consists of Vγ4 T cells.

According to a further aspect of the invention, there is provided the pharmaceutical composition comprising the cell population as defined herein for use as a medicament. In one embodiment, the pharmaceutical composition comprising the cell population is for use in therapy, particularly for use in the treatment of cancer, an infectious disease or an inflammatory disease. In a further embodiment, the pharmaceutical composition comprising the cell population is for use in the treatment of cancer.

According to a further aspect of the invention, there is provided a method of modulating an immune response in a subject in need thereof comprising administering a therapeutically effective amount of the cell population as defined herein.

According to a further aspect of the invention, there is provided a method of treating a cancer, an infectious disease or an inflammatory disease in a subject in need thereof, comprising administering a therapeutically effective amount of the cell population as defined herein. Alternatively, a therapeutically effective amount of the pharmaceutical composition comprising the cell population is administered.

According to further aspects of the invention, there is provided the use of the cell population as defined herein for the manufacture of a medicament, for example in the treatment of cancer, an infectious disease or an inflammatory disease.

Adoptive T cell therapy

Gamma delta T cells obtained by the expansion methods of the invention may be used as a medicament, for example for adoptive T cell therapy. This involves the transfer of γδ T cells into a patient. The therapy may be autologous, *i.e.* the γδ T cells may be transferred back into the same patient from which they were obtained, or the therapy may be allogeneic, *i.e.* the γδ T cells from one person may be transferred into a different patient. In instances involving allogeneic transfer, the γδ T cells may be substantially free of αβ T cells. For example, αβ T cells may be depleted from the γδ T cell population, *e.g.*, after expansion, using any suitable means known in the art (*e.g.,* by negative selection, *e.g.,* using magnetic beads). A method of treatment may include: providing a sample (*e.g.* a non-haematopoietic tissue sample) obtained from a donor individual; culturing γδ T cells obtained from the sample as described herein, *e.g.* to produce an expanded population; and administering the population of γδ T cells to a recipient individual.

The patient or subject to be treated is preferably a human cancer patient (*e.g.*, a human cancer patient being treated for a solid tumour) or a virus-infected patient (*e.g.*, a CMV-infected or HIV infected patient). In some instances, the patient has and/or is being treated for a solid tumour. Because they are normally resident in non-haematopoietic tissues, tissue-resident Vγ4 T cells are also more likely to home to and be retained within tumour masses than their systemic blood-resident counterparts and adoptive transfer of these cells is likely to be more effective at targeting solid tumours and potentially other non-haematopoietic tissue-associated immunopathologies.

As γδ T cells are non-MHC restricted, they do not recognize a host into which they are transferred as foreign, which means that they are less likely to cause graft-versus-host disease. This means that they can be used "off the shelf" and transferred into any recipient, *e.g.*, for allogeneic adoptive T cell therapy.

γδ T cells obtained by methods described herein may express NKG2D and respond to a NKG2D ligand (*e.g.* MICA), which is strongly associated with malignancy. They may also express a cytotoxic profile in the absence of any activation and are therefore likely to be effective at killing tumour cells. For example, the γδ T cells obtained as described herein may express one or more, preferably all of IFN-γ, TNF-α, GM-CSF, CCL4, IL-13, Granulysin, Granzyme A and B, and Perforin in the absence of any activation. IL-17A may not be expressed.

In some embodiments, a method of treatment of an individual with a tumour may include; providing a sample of said tumour obtained from a donor individual, culturing the γδ T cells obtained from the sample as described above, and; administering the population of γδ T cells to the individual with the tumour. In a further embodiment, a method of treatment of an individual with a tumour in a non-haematopoietic tissue may include; providing a sample of said non-haematopoietic tissue obtained from a donor individual, culturing the γδ T cells obtained from the sample as described above, and; administering the population of γδ T cells to the individual with the tumour.

In some instances, a therapeutically effective amount of γδ T cells obtained by the any of the methods described above can be administered in a therapeutically effective amount to a subject (*e.g.,* for treatment of cancer, *e.g.* for treatment of a solid tumour). In some cases, the therapeutically effective amount of γδ T cells (*e.g.,* Vγ4 T cells) is less than $10 \times 10^{12}$ cells per dose (*e.g.*, less than $9 \times 10^{12}$ cells per dose, less than $8 \times 10^{12}$ cells per dose, less than $7 \times 10^{12}$ cells per dose, less than $6 \times 10^{12}$ cells per dose, less than $5 \times 10^{12}$ cells per dose, less than $4 \times 10^{12}$ cells per dose, less than $3 \times 10^{12}$ cells per dose, less than $2 \times 10^{12}$ cells per dose, less than $1 \times 10^{12}$ cells per dose, less than $9 \times 10^{11}$ cells per dose, less than $8 \times 10^{11}$ cells per dose, less than $7 \times 10^{11}$ cells per dose, less than $6 \times 10^{11}$ cells per dose, less than $5 \times 10^{11}$ cells per dose, less than $4 \times 10^{11}$ cells per dose, less than $3 \times 10^{11}$ cells per dose, less than $2 \times 10^{11}$ cells per dose, less than $1 \times 10^{11}$ cells per dose, less than $9 \times 10^{10}$ cells per dose, less than $7.5 \times 10^{10}$ cells per dose, less than $5 \times 10^{10}$ cells per dose, less than $2.5 \times 10^{10}$ cells per dose, less than $1 \times 10^{10}$ cells per dose, less than $7.5 \times 10^{9}$ cells per dose, less than $5 \times 10^{9}$ cells per dose, less than $2.5 \times 10^{9}$ cells per dose, less than $1 \times 10^{9}$ cells per dose, less than $7.5 \times 10^{8}$ cells per dose, less than $5 \times 10^{8}$ cells per dose, less than $2,5 \times 10^{8}$ cells per dose, less than $1 \times 10^{8}$ cells per dose, less than $7.5 \times 10^{7}$ cells per dose, less than $5 \times 10^{7}$ cells per dose, less than $2,5 \times 10^{7}$ cells per dose, less than $1 \times 10^{7}$ cells per dose, less than $7.5 \times 10^{6}$ cells per dose, less than $5 \times 10^{6}$ cells per dose, less than $2,5 \times 10^{6}$ cells per dose, less than $1 \times 10^{6}$ cells per dose, less than $7.5 \times 10^{5}$ cells per dose, less than $5 \times 10^{5}$ cells per dose, less than $2,5 \times 10^{5}$ cells per dose, or less than $1 \times 10^{5}$ cells per dose).

In some embodiments, the therapeutically effective amount of γδ T cells (*e.g.* Vγ4 T cells) is less than $10 \times 10^{12}$ cells over the course of treatment (*e.g.*, less than $9 \times 10^{12}$ cells, less than $8 \times 10^{12}$ cells, less than $7 \times 10^{12}$ cells, less than $6 \times 10^{12}$ cells, less than $5 \times 10^{12}$ cells, less than $4 \times 10^{12}$ cells, less than $3 \times 10^{12}$ cells, less than $2 \times 10^{12}$ cells, less than $1 \times 10^{12}$ cells, less than $9 \times 10^{11}$ cells, less than $8 \times 10^{11}$ cells, less than $7 \times 10^{11}$ cells, less than $6 \times 10^{11}$ cells, less than $5 \times 10^{11}$ cells, less than $4 \times 10^{11}$ cells, less than $3 \times 10^{11}$ cells, less than $2 \times 10^{11}$ cells, less than $1 \times 10^{11}$ cells, less than $9 \times 10^{10}$ cells, less than $7.5 \times 10^{10}$ cells, less than $5 \times 10^{10}$ cells, less than $2.5 \times 10^{10}$ cells, less than $1 \times 10^{10}$ cells, less than $7.5 \times 10^{9}$ cells, less than $5 \times 10^{9}$ cells, less than $2.5 \times 10^{9}$ cells, less than $1 \times 10^{9}$ cells, less than $7.5 \times 10^{8}$ cells, less than $5 \times 10^{8}$ cells, less than $2.5 \times 10^{8}$ cells, less than $1 \times 10^{8}$ cells, less than $7.5 \times 10^{7}$ cells, less than $5 \times 10^{7}$ cells, less than $2.5 \times 10^{7}$ cells, less than $1 \times 10^{7}$ cells, less than $7.5 \times 10^{6}$ cells, less than $5 \times 10^{6}$ cells, less than $2.5 \times 10^{6}$ cells, less than $1 \times 10^{6}$ cells, less than $7.5 \times 10^{5}$ cells, less than $5 \times 10^{5}$ cells, less than $2.5 \times 10^{5}$ cells, or less than $1 \times 10^{5}$ cells over the course of treatment).

In some embodiments, a dose of γδ T cells (*e.g.*, Vγ4 T cells) as described herein comprises about $1 \times 10^{6}$, $1.1 \times 10^{6}$, $2 \times 10^{6}$, $3.6 \times 10^{6}$, $5 \times 10^{6}$, $1 \times 10^{7}$, $1.8 \times 10^{7}$, $2 \times 10^{7}$, $5 \times 10^{7}$, $1 \times 10^{8}$, $2 \times 10^{8}$, or $5 \times 10^{8}$ cells/kg. In some embodiments, a dose of γδ T cells (*e.g.*, Vγ4 T cells) comprises up to about $1 \times 10^{6}$, $1.1 \times 10^{6}$, $2 \times 10^{6}$, $3.6 \times 10^{6}$, $5 \times 10^{6}$, $1 \times 10^{7}$, $1.8 \times 10^{7}$, $2 \times 10^{7}$, $5 \times 10^{7}$, $1 \times 10^{8}$, $2 \times 10^{8}$, or $5 \times 10^{8}$ cells/kg. In some embodiments, a dose of γδ T cells (*e.g.*, Vγ4 T cells) comprises about $1.1 \times 10^{6}$- $1.8 \times 10^{7}$ cells/kg. In some embodiments, a dose of γδ T cells (*e.g.*, Vγ4 T cells) comprises about $1 \times 10^{7}$, $2 \times 10^{7}$, 5

x 10^7, 1 x 10^8, 2 x 10^8, 5 x 10^8, 1 x 10^9, 2 x 10^9, or 5 x 10^9 cells. In some embodiments, a dose of γδ T cells (*e.g.*, Vγ4 T cells) comprises at least about 1 x 10^7, 2 x 10^7, 5 x 10^7, 1 x 10^8, 2 x 10^8, 5 x 10^8, 1 x 10^9, 2 x 10^9, or 5 x 10^9 cells. In some embodiments, a dose of γδ T cells (*e.g.*, Vγ4 T cells) comprises up to about 1 x 10^7, 2 x 10^7, 5 x 10^7, 1 x 10^8, 2 x 10^8, 5 x 10^8, 1 x 10^9, 2 x 10^9, or 5 x 10^9 cells.

In one embodiment, the subject is administered 10^4 to 10^6 γδ T cells (*e.g.*, Vγ4 T cells) per kg body weight of the subject. In one embodiment, the subject receives an initial administration of a population of γδ T cells (*e.g.*, an initial administration of 10^4 to 10^6 γδ T cells per kg body weight of the subject, *e.g.*, 10^4 to 10^5 γδ T cells per kg body weight of the subject), and one or more (*e.g.*, 2, 3, 4, or 5) subsequent administrations of γδ T cells (*e.g.*, one or more subsequent administration of 10^4 to 10^6 γδ T cells per kg body weight of the subject, *e.g.*, 10^4 to 10^5 γδ T cells per kg body weight of the subject). In one embodiment, the one or more subsequent administrations are administered less than 15 days, *e.g.*, 14, 13, 12, 11, 10, 9, 8, 7, 6, 5, 4, 3, or 2 days after the previous administration, *e.g.*, less than 4, 3, or 2 days after the previous administration. In one embodiment, the subject receives a total of about 10^6 γδ T cells per kg body weight of the subject over the course of at least three administrations of a population of γδ T cells, *e.g.*, the subject receives an initial dose of 1 x 10^5 γδ T cells, a second administration of 3 x 10^5 γδ T cells, and a third administration of 6 x 10^5 γδ T cells, and, *e.g.*, each administration is administered less than 4, 3, or 2 days after the previous administration.

In some embodiments, one or more additional therapeutic agents can be administered to the subject. The additional therapeutic agent may be selected from the group consisting of an immunotherapeutic agent, a cytotoxic agent, a growth inhibitory agent, a radiation therapy agent, an anti-angiogenic agent, or a combination of two or more agents thereof. The additional therapeutic agent may be administered concurrently with, prior to, or after administration of the γδ T cells. The additional therapeutic agent may be an immunotherapeutic agent, which may act on a target within the subject's body (*e.g.*, the subject's own immune system) and/or on the transferred γδ T cells.

The administration of the compositions may be carried out in any convenient manner. The compositions described herein may be administered to a patient transarterially, subcutaneously, intradermally, intratumorally, intranodally, intramedullary, intramuscularly, by intravenous injection, or intraperitoneally, *e.g.*, by intradermal or subcutaneous injection. The compositions of γδ T cells may be injected directly into a tumour, lymph node, or site of infection.

Gene Engineering

The γδ T cells obtained by the method of the invention may also be gene engineered for enhanced therapeutic properties, such as for Chimeric Antigen Receptor T cell (CAR-T) therapy. This involves the generation of engineered T cell receptors (TCRs) to re-program the T cell with a new specificity, *e.g.* the specificity of a monoclonal antibody. The engineered TCR may make the T cells specific for malignant cells and therefore useful for cancer immunotherapy. For example, the T cells may recognize cancer cells expressing a tumour antigen, such as a tumour associated antigen that is not expressed by normal somatic cells from the subject tissue. Thus, the CAR-modified T cells may be used for adoptive T cell therapy of, for example, cancer patients.

**Other uses of the antibodies or fragments thereof**

According to a further aspect of the invention, there is provided the use of an anti-Vγ4 antibody or fragment thereof as described herein to study antigen recognition, activation, signal transduction or function of γδ T cells (in particular Vγ4 T cells). As described herein, the antibodies have been shown to be active in assays which can be used to investigate γδ T cell function. Such antibodies may also be useful for inducing the proliferation of γδ T cells, therefore may be used in methods of expanding γδ T cells (such as Vγ4 T cells).

Antibodies which bind to the Vγ4 chain can be used to detect γδ T cells. For example, the antibody may be labelled with a detectable label or reporter molecule or used as a capture ligand to selectively detect and/or isolate Vγ4 T cells in a sample. Labelled antibodies find use in many methods known in the art, for example immunohistochemistry and ELISA.

The detectable label or reporter molecule can be a radioisotope, such as ^3H, ^14C, ^32P, ^35S, or ^125I; a fluorescent or chemiluminescent moiety such as fluorescein isothiocyanate, or rhodamine; or an enzyme such as alkaline phosphatase, β-galactosidase, horseradish peroxidase, or luciferase. Fluorescent labels applied to antibodies of the invention may then be used in fluorescence-activated cell sorting (FACS) methods.

It will be understood that all embodiments described herein may be applied to all aspects of the invention.

**CLAUSES**

A set of clauses defining the invention and its preferred aspects is as follows:

1. An *ex vivo* method of modulating gamma variable 4 (Vγ4) T cells comprising administering an antibody or fragment thereof, which specifically binds to a Vγ4 chain of a γδ T cell receptor (TCR) and not to a gamma variable 2 (Vγ2) chain of a γδ TCR, to a cell population comprising Vγ4 T cells.

2. The method as defined in clause 1, wherein the Vγ4 chain of the γδ TCR is human Vγ4 and the Vy2 chain of the γδ TCR is human Vy2.

3. The method as defined in clause 1 or 2, wherein the antibody or fragment thereof binds to an epitope of the Vγ4 chain of the γδ TCR comprising one or more amino acid residues within amino acid region 67-82 of SEQ ID NO: 1.

4. The method as defined in any one of clauses 1 to 3, wherein the antibody or fragment thereof binds to an epitope of the Vγ4 chain of the γδ TCR comprising one or more amino acid residues within amino acid region 71-79 of SEQ ID NO: 1.

5. The method as defined in clause 4, wherein the epitope comprises at least one of amino acid residues 71, 73, 75, 76, 79 of SEQ ID NO: 1.

6. The method as defined in any one of clauses 1 to 5, wherein the epitope consists of one or more amino acid residues within amino acid region 67-82 of SEQ ID NO: 1.

7. The method as defined in any one of clauses 1 to 6, wherein the epitope comprises or consists of K76 and/or M80 of SEQ ID NO: 1.

8. The method as defined in any one of clauses 1 to 7, wherein the epitope is an activating epitope of a γδ T cell.

9. The method as defined in clause 8, wherein binding of the activating epitope: (i) downregulates the γδ TCR; (ii) activates degranulation of the γδ T cell; (iii) activates γδ T cell-mediated killing; and/or (iv) activates or increases Vγ4 chain-mediated cell signalling.

10. The method as defined in any one of clauses 1 to 9, wherein the antibody or fragment thereof only binds to an epitope in the V region of a Vγ4 chain of a γδ TCR.

11. The method as defined in any one of clauses 1 to 10, wherein the antibody or fragment thereof does not bind to an epitope found in CDR3 of a Vγ4 chain of a γδ TCR.

12. An *ex vivo* method of modulating gamma variable 4 (Vγ4) T cells comprising administering an anti-Vγ4 antibody or fragment thereof, which comprises one or more of:

a CDR3 comprising a sequence having at least 80% sequence identity with any one of SEQ ID NOs: 2-47, preferably with SEQ ID NO: 10 and/or 33;
a CDR2 comprising a sequence having at least 80% sequence identity with any one of SEQ ID NOs: 48-70 and SEQUENCES: A1-A23 (of Figure 1), preferably with SEQ ID NO: 56 and/or SEQUENCE A9; and/or
a CDR1 comprising a sequence having at least 80% sequence identity with any one of SEQ ID NOs: 71-116, preferably with SEQ ID NO: 79 and/or 102,
to a cell population comprising Vγ4 T cells.

13. The method as defined in clause 12, wherein the antibody or fragment thereof comprises a VH region comprising a CDR3 comprising a sequence having at least 80% sequence identity with any one of SEQ ID NOs: 2-24, such as SEQ ID NOs: 10, 4, 14, 15, 17, 19 or 23.

14. The method as defined in clause 12 or clause 13, wherein the antibody or fragment thereof comprises a VH region comprising a CDR2 comprising a sequence having at least 80% sequence identity with any one of SEQ ID NOs: 48-70, such as SEQ ID NOs: 56, 50, 60, 61, 63, 65 or 69.

15. The method as defined in any one of clauses 12 to 14, wherein the antibody or fragment thereof comprises a VH region comprising a CDR1 comprising a sequence having at least 80% sequence identity with any one of SEQ ID NOs: 71-93, such as SEQ ID NOs: 79, 73, 83, 84, 86, 88 or 92.

16. The method as defined in any one of clauses 12 to 15, wherein the antibody or fragment thereof comprises a VH region comprising a CDR3 comprising a sequence of SEQ ID NO: 10, a CDR2 comprising a sequence of SEQ ID NO:

56, and a CDR1 comprising a sequence of SEQ ID NO: 79.

17. The method as defined in any one of clauses 12 to 15, wherein the antibody or fragment thereof comprises a VH region comprising a CDR3 comprising a sequence of SEQ ID NO: 4, a CDR2 comprising a sequence of SEQ ID NO: 50, and a CDR1 comprising a sequence of SEQ ID NO: 73.

18. The method as defined in any one of clauses 12 to 15, wherein the antibody or fragment thereof comprises a VH region comprising a CDR3 comprising a sequence of SEQ ID NO: 14, a CDR2 comprising a sequence of SEQ ID NO: 60, and a CDR1 comprising a sequence of SEQ ID NO: 83.

19. The method as defined in any one of clauses 12 to 15, wherein the antibody or fragment thereof comprises a VH region comprising a CDR3 comprising a sequence of SEQ ID NO: 15, a CDR2 comprising a sequence of SEQ ID NO: 61, and a CDR1 comprising a sequence of SEQ ID NO: 84.

20. The method as defined in any one of clauses 12 to 15, wherein the antibody or fragment thereof comprises a VH region comprising a CDR3 comprising a sequence of SEQ ID NO: 17, a CDR2 comprising a sequence of SEQ ID NO: 63, and a CDR1 comprising a sequence of SEQ ID NO: 86.

21. The method as defined in any one of clauses 12 to 15, wherein the antibody or fragment thereof comprises a VH region comprising a CDR3 comprising a sequence of SEQ ID NO: 19, a CDR2 comprising a sequence of SEQ ID NO: 65, and a CDR1 comprising a sequence of SEQ ID NO: 88.

22. The method as defined in any one of clauses 12 to 15, wherein the antibody or fragment thereof comprises a VH region comprising a CDR3 comprising a sequence of SEQ ID NO: 23, a CDR2 comprising a sequence of SEQ ID NO: 69, and a CDR1 comprising a sequence of SEQ ID NO: 92.

23. The method as defined in any one of clauses 12 to 22, wherein the antibody or fragment thereof comprises a VL region comprising a CDR3 comprising a sequence having at least 80% sequence identity with any one of SEQ ID NOs: 25-47, such as SEQ ID NOs: 33, 27, 37, 38, 40, 42 or 46.

24. The method as defined in any one of clauses 12 to 23, wherein the antibody or fragment thereof comprises a VL region comprising a CDR2 comprising a sequence having at least 80% sequence identity with any one of SEQUENCES: A1-A23 (of Figure 1), such as SEQUENCES: A9, A3, A13, A14, A16, A18 or A22.

25. The method as defined in any one of clauses 12 to 26, wherein the antibody or fragment thereof comprises a VL region comprising a CDR1 comprising a sequence having at least 80% sequence identity with any one of SEQ ID NOs: 94-116, such as SEQ ID NOs: 102, 96, 106, 107, 109, 111 or 115.

26. The method as defined in any one of clauses 12 to 25, wherein the antibody or fragment thereof comprises a VL region comprising a CDR3 comprising a sequence of SEQ ID NO: 33, a CDR2 comprising a sequence of SEQUENCE: A9, and a CDR1 comprising a sequence of SEQ ID NO: 102.

27. The method as defined in any one of clauses 12 to 25, wherein the antibody or fragment thereof comprises a VL region comprising a CDR3 comprising a sequence of SEQ ID NO: 27, a CDR2 comprising a sequence of SEQUENCE: A3, and a CDR1 comprising a sequence of SEQ ID NO: 96.

28. The method as defined in any one of clauses 12 to 25, wherein the antibody or fragment thereof comprises a VL region comprising a CDR3 comprising a sequence of SEQ ID NO: 37, a CDR2 comprising a sequence of SEQUENCE: A13, and a CDR1 comprising a sequence of SEQ ID NO: 106.

29. The method as defined in any one of clauses 12 to 25, wherein the antibody or fragment thereof comprises a VL region comprising a CDR3 comprising a sequence of SEQ ID NO: 38, a CDR2 comprising a sequence of SEQUENCE: A14, and a CDR1 comprising a sequence of SEQ ID NO: 107.

30. The method as defined in any one of clauses 12 to 25, wherein the antibody or fragment thereof comprises a VL region comprising a CDR3 comprising a sequence of SEQ ID NO: 40, a CDR2 comprising a sequence of SEQUENCE: A16, and a CDR1 comprising a sequence of SEQ ID NO: 109.

31. The method as defined in any one of clauses 12 to 25, wherein the antibody or fragment thereof comprises a VL region comprising a CDR3 comprising a sequence of SEQ ID NO: 42, a CDR2 comprising a sequence of SEQUENCE: A18, and a CDR1 comprising a sequence of SEQ ID NO: 111.

32. The method as defined in any one of clauses 12 to 25, wherein the antibody or fragment thereof comprises a VL region comprising a CDR3 comprising a sequence of SEQ ID NO: 46, a CDR2 comprising a sequence of SEQUENCE: A23, and a CDR1 comprising a sequence of SEQ ID NO: 115.

33. An *ex vivo* method of modulating gamma variable 4 (Vγ4) T cells comprising administering an anti-Vγ4 antibody or fragment thereof, which comprises an amino acid sequence having at least 80% sequence identity with any one of SEQ ID NOs: 117-162 or 261-283, to a cell population comprising Vγ4 T cells.

34. The method as defined in clause 33, wherein the antibody or fragment thereof comprises a VH region comprising an amino acid sequence having at least 80% sequence identity with any one of SEQ ID NOs: 117-139.

35. The method as defined in clause 34, wherein the VH region comprises an amino acid sequence having at least 80% sequence identity with any one of SEQ ID NOs: 125, 119, 129, 130, 132, 134, or 138.

36. The method as defined in any one of clauses 33 to 35, wherein the antibody or fragment thereof comprises a VL region comprising an amino acid sequence having at least 80% sequence identity with any one of SEQ ID NOs: 140-162 or 261-283.

37. The method as defined in clause 36, wherein the VL region comprises an amino acid sequence having at least 80% sequence identity with any one of:

> (a) SEQ ID NOs: 148, 142, 152, 153, 155, 157 or 161; or
> (b) SEQ ID NOs: 269, 263, 273, 274, 276, 278 or 282.

38. The method as defined in any one of clauses 33 to 37, wherein the antibody or fragment thereof comprises a VH region comprising an amino acid sequence of SEQ ID NO: 125 and a VL region comprising an amino acid sequence of SEQ ID NO: 148 or 269.

39. The method as defined in any one of clauses 33 to 37, wherein the antibody or fragment thereof comprises a VH region comprising an amino acid sequence of SEQ ID NO: 119 and a VL region comprising an amino acid sequence of SEQ ID NO: 142 or 263.

40. The method as defined in any one of clauses 33 to 37, wherein the antibody or fragment thereof comprises a VH region comprising an amino acid sequence of SEQ ID NO: 129 and a VL region comprising an amino acid sequence of SEQ ID NO: 152 or 273.

41. The method as defined in any one of clauses 33 to 37, wherein the antibody or fragment thereof comprises a VH region comprising an amino acid sequence of SEQ ID NO: 130 and a VL region comprising an amino acid sequence of SEQ ID NO: 153 or 274.

42. The method as defined in any one of clauses 33 to 37, wherein the antibody or fragment thereof comprises a VH region comprising an amino acid sequence of SEQ ID NO: 132 and a VL region comprising an amino acid sequence of SEQ ID NO: 155 or 276.

43. The method as defined in any one of clauses 33 to 37, wherein the antibody or fragment thereof comprises a VH region comprising an amino acid sequence of SEQ ID NO: 134 and a VL region comprising an amino acid sequence of SEQ ID NO: 157 or 278.

44. The method as defined in any one of clauses 33 to 37, wherein the antibody or fragment thereof comprises a VH region comprising an amino acid sequence of SEQ ID NO: 138 and a VL region comprising an amino acid sequence of SEQ ID NO: 161 or 282.

45. An *ex vivo* method of modulating gamma variable 4 (Vγ4) T cells comprising administering an anti-Vγ4 antibody or fragment thereof comprising one or more of:

(a) a VH comprising a HCDR1 having SEQ ID NO: 79, a HCDR2 having SEQ ID NO: 56 and a HCDR3 having SEQ ID NO: 10, optionally wherein the VH comprises SEQ ID NO: 125; and

a VL comprising a LCDR1 having SEQ ID NO: 102, a LCDR2 having SEQUENCE A9 (of Figure 1) and a LCDR3 having SEQ ID NO: 33, optionally wherein the VL comprises SEQ ID NO: 148 or 269;

(b) a VH comprising a HCDR1 having SEQ ID NO: 86, a HCDR2 having SEQ ID NO: 63 and a HCDR3 having SEQ ID NO: 17, optionally wherein the VH comprises SEQ ID NO: 132; and

a VL comprising a LCDR1 having SEQ ID NO: 109, a LCDR2 having SEQUENCE A16 (of Figure 1) and a LCDR3 having SEQ ID NO: 40, optionally wherein the VL comprises SEQ ID NO: 155 or 276;

(c) a VH comprising a HCDR1 having SEQ ID NO: 73, a HCDR2 having SEQ ID NO: 50 and a HCDR3 having SEQ ID NO: 4, optionally wherein the VH comprises SEQ ID NO: 119; and

a VL comprising a LCDR1 having SEQ ID NO: 96, a LCDR2 having SEQUENCE A3 (of Figure 1) and a LCDR3 having SEQ ID NO: 27, optionally wherein the VL comprises SEQ ID NO: 142 or 263;

(d) a VH comprising a HCDR1 having SEQ ID NO: 83, a HCDR2 having SEQ ID NO: 60 and a HCDR3 having SEQ ID NO: 14, optionally wherein the VH comprises SEQ ID NO: 129; and

a VL comprising a LCDR1 having SEQ ID NO: 106, a LCDR2 having SEQUENCE A13 (of Figure 1) and a LCDR3 having SEQ ID NO: 37, optionally wherein the VL comprises SEQ ID NO: 152 or 273;

(e) a VH comprising a HCDR1 having SEQ ID NO: 84, a HCDR2 having SEQ ID NO: 61 and a HCDR3 having SEQ ID NO: 15, optionally wherein the VH comprises SEQ ID NO: 130; and

a VL comprising a LCDR1 having SEQ ID NO: 107, a LCDR2 having SEQUENCE A14 (of Figure 1) and a LCDR3 having SEQ ID NO: 38, optionally wherein the VL comprises SEQ ID NO: 153 or 274;

(f) a VH comprising a HCDR1 having SEQ ID NO: 88, a HCDR2 having SEQ ID NO: 65 and a HCDR3 having SEQ ID NO: 19, optionally wherein the VH comprises SEQ ID NO: 134; and

a VL comprising a LCDR1 having SEQ ID NO: 111, a LCDR2 having SEQUENCE A18 (of Figure 1) and a LCDR3 having SEQ ID NO: 42, optionally wherein the VL comprises SEQ ID NO: 157 or 278;

(g) a VH comprising a HCDR1 having SEQ ID NO: 92, a HCDR2 having SEQ ID NO: 69 and a HCDR3 having SEQ ID NO: 23, optionally wherein the VH comprises SEQ ID NO: 138; and

a VL comprising a LCDR1 having SEQ ID NO: 115, a LCDR2 having SEQUENCE A22 (of Figure 1) and a LCDR3 having SEQ ID NO: 46, optionally wherein the VL comprises SEQ ID NO: 161 or 282;

(h) a VH comprising a HCDR1 having SEQ ID NO: 71, a HCDR2 having SEQ ID NO: 48 and a HCDR3 having SEQ ID NO: 2, optionally wherein the VH comprises SEQ ID NO: 117; and

a VL comprising a LCDR1 having SEQ ID NO: 94, a LCDR2 having SEQUENCE A1 (of Figure 1) and a LCDR3 having SEQ ID NO: 25, optionally wherein the VL comprises SEQ ID NO: 140 or 261;

(i) a VH comprising a HCDR1 having SEQ ID NO: 72, a HCDR2 having SEQ ID NO: 49 and a HCDR3 having SEQ ID NO: 3, optionally wherein the VH comprises SEQ ID NO: 118; and

a VL comprising a LCDR1 having SEQ ID NO: 95, a LCDR2 having SEQUENCE A2 (of Figure 1) and a LCDR3 having SEQ ID NO: 26, optionally wherein the VL comprises SEQ ID NO: 141 or 262;

(j) a VH comprising a HCDR1 having SEQ ID NO: 74, a HCDR2 having SEQ ID NO: 51 and a HCDR3 having SEQ ID NO: 5, optionally wherein the VH comprises SEQ ID NO: 120; and

a VL comprising a LCDR1 having SEQ ID NO: 97, a LCDR2 having SEQUENCE A4 (of Figure 1) and a LCDR3 having SEQ ID NO: 28, optionally wherein the VL comprises SEQ ID NO: 143 or 264;

(k) a VH comprising a HCDR1 having SEQ ID NO: 75, a HCDR2 having SEQ ID NO: 52 and a HCDR3 having SEQ ID NO: 6, optionally wherein the VH comprises SEQ ID NO: 121; and

a VL comprising a LCDR1 having SEQ ID NO: 98, a LCDR2 having SEQUENCE A5 (of Figure 1) and a LCDR3 having SEQ ID NO: 29, optionally wherein the VL comprises SEQ ID NO: 144 or 265;

(l) a VH comprising a HCDR1 having SEQ ID NO: 76, a HCDR2 having SEQ ID NO: 53 and a HCDR3 having SEQ ID NO: 7, optionally wherein the VH comprises SEQ ID NO: 122; and

a VL comprising a LCDR1 having SEQ ID NO: 99, a LCDR2 having SEQUENCE A6 (of Figure 1) and a LCDR3 having SEQ ID NO: 30, optionally wherein the VL comprises SEQ ID NO: 145 or 266;

(m) a VH comprising a HCDR1 having SEQ ID NO: 77, a HCDR2 having SEQ ID NO: 54 and a HCDR3 having SEQ ID NO: 8, optionally wherein the VH comprises SEQ ID NO: 123; and

a VL comprising a LCDR1 having SEQ ID NO: 100, a LCDR2 having SEQUENCE A7 (of Figure 1) and a LCDR3 having SEQ ID NO: 31, optionally wherein the VL comprises SEQ ID NO: 146 or 267;

(n) a VH comprising a HCDR1 having SEQ ID NO: 78, a HCDR2 having SEQ ID NO: 55 and a HCDR3 having SEQ ID NO: 9, optionally wherein the VH comprises SEQ ID NO: 124; and

a VL comprising a LCDR1 having SEQ ID NO: 101, a LCDR2 having SEQUENCE A8 (of Figure 1) and a LCDR3 having SEQ ID NO: 32, optionally wherein the VL comprises SEQ ID NO: 147 or 268;

(o) a VH comprising a HCDR1 having SEQ ID NO: 80, a HCDR2 having SEQ ID NO: 57 and a HCDR3 having SEQ ID NO: 11, optionally wherein the VH comprises SEQ ID NO: 126; and

a VL comprising a LCDR1 having SEQ ID NO: 103, a LCDR2 having SEQUENCE A10 (of Figure 1) and a LCDR3 having SEQ ID NO: 34, optionally wherein the VL comprises SEQ ID NO: 149 or 270;

(p) a VH comprising a HCDR1 having SEQ ID NO: 81, a HCDR2 having SEQ ID NO: 58 and a HCDR3 having SEQ ID NO: 12, optionally wherein the VH comprises SEQ ID NO: 127; and

a VL comprising a LCDR1 having SEQ ID NO: 104, a LCDR2 having SEQUENCE A11 (of Figure 1) and a LCDR3 having SEQ ID NO: 35, optionally wherein the VL comprises SEQ ID NO: 150 or 271;

(q) a VH comprising a HCDR1 having SEQ ID NO: 82, a HCDR2 having SEQ ID NO: 59 and a HCDR3 having SEQ ID NO: 13, optionally wherein the VH comprises SEQ ID NO: 128; and

a VL comprising a LCDR1 having SEQ ID NO: 105, a LCDR2 having SEQUENCE A12 (of Figure 1) and a LCDR3 having SEQ ID NO: 36, optionally wherein the VL comprises SEQ ID NO: 151 or 272;

(r) a VH comprising a HCDR1 having SEQ ID NO: 85, a HCDR2 having SEQ ID NO: 62 and a HCDR3 having SEQ ID NO: 16, optionally wherein the VH comprises SEQ ID NO: 131; and

a VL comprising a LCDR1 having SEQ ID NO: 108, a LCDR2 having SEQUENCE A15 (of Figure 1) and a LCDR3 having SEQ ID NO: 39, optionally wherein the VL comprises SEQ ID NO: 154 or 275;

(s) a VH comprising a HCDR1 having SEQ ID NO: 87, a HCDR2 having SEQ ID NO: 64 and a HCDR3 having SEQ ID NO: 18, optionally wherein the VH comprises SEQ ID NO: 133; and

a VL comprising a LCDR1 having SEQ ID NO: 110, a LCDR2 having SEQUENCE A17 (of Figure 1) and a LCDR3 having SEQ ID NO: 41, optionally wherein the VL comprises SEQ ID NO: 156 or 277;

(t) a VH comprising a HCDR1 having SEQ ID NO: 89, a HCDR2 having SEQ ID NO: 66 and a HCDR3 having SEQ ID NO: 20, optionally wherein the VH comprises SEQ ID NO: 135; and

a VL comprising a LCDR1 having SEQ ID NO: 112, a LCDR2 having SEQUENCE A19 (of Figure 1) and a LCDR3 having SEQ ID NO: 43, optionally wherein the VL comprises SEQ ID NO: 158 or 279;

(u) a VH comprising a HCDR1 having SEQ ID NO: 90, a HCDR2 having SEQ ID NO: 67 and a HCDR3 having SEQ ID NO: 21, optionally wherein the VH comprises SEQ ID NO: 136; and

a VL comprising a LCDR1 having SEQ ID NO: 113, a LCDR2 having SEQUENCE A20 (of Figure 1) and a LCDR3 having SEQ ID NO: 44, optionally wherein the VL comprises SEQ ID NO: 159 or 280;

(v) a VH comprising a HCDR1 having SEQ ID NO: 91, a HCDR2 having SEQ ID NO: 68 and a HCDR3 having SEQ ID NO: 22, optionally wherein the VH comprises SEQ ID NO: 137; and

a VL comprising a LCDR1 having SEQ ID NO: 114, a LCDR2 having SEQUENCE A21 (of Figure 1) and a LCDR3 having SEQ ID NO: 45, optionally wherein the VL comprises SEQ ID NO: 160 or 281;

and/or

(w) a VH comprising a HCDR1 having SEQ ID NO: 93, a HCDR2 having SEQ ID NO: 70 and a HCDR3 having SEQ ID NO: 24, optionally wherein the VH comprises SEQ ID NO: 139; and

a VL comprising a LCDR1 having SEQ ID NO: 116, a LCDR2 having SEQUENCE A23 (of Figure 1) and a LCDR3 having SEQ ID NO: 47, optionally wherein the VL comprises SEQ ID NO: 162 or 283,

to a cell population comprising Vγ4 T cells.

46. The method as defined in any one of clauses 33 to 45, wherein the VH and VL region are joined by a linker, such as a polypeptide linker.

47. The method as defined in clause 46, wherein the linker comprises a (Gly4Ser)n format, where n = 1 to 8.

48. The method as defined in clause 46 or 47, wherein the linker comprises SEQ ID NO: 186.

49. The method as defined in clause 48, wherein the linker consists of SEQ ID NO: 186.

50. An *ex vivo* method of modulating gamma variable 4 (Vγ4) T cells comprising administering an anti-Vγ4 antibody or fragment thereof which comprises an amino acid sequence having at least 80% sequence identity with any one of SEQ ID NOs: 163-185, to a cell population comprising Vγ4 T cells.

51. The method as defined in clause 50, wherein the antibody or fragment thereof comprises an amino acid sequence of any one of SEQ ID NOs: 163-185.

52. The method as defined in clause 50 or clause 51, wherein the antibody or fragment thereof comprises SEQ ID NO: 171, 165, 175, 176, 178, 180 or 184.

53. An *ex vivo* method of modulating gamma variable 4 (Vγ4) T cells comprising administering an anti-Vγ4 antibody or fragment thereof which comprises an amino acid sequence having at least 80% sequence identity with any one of SEQ ID NOs: 233-255, to a cell population comprising Vγ4 T cells.

54. The method as defined in clause 53, which comprises an amino acid sequence of any one of SEQ ID NOs: 233-255.

55. The method as defined in clause 53 or clause 54, which comprises SEQ ID NO: 235, 241, 245, 246 or 254.

56. An *ex vivo* method of modulating gamma variable 4 (Vγ4) T cells comprising administering anti-Vγ4 antibody or fragment thereof comprising a heavy chain amino acid sequence having at least 80% sequence identity with any one of SEQ ID NOs: 284-306 and/or a light chain amino acid sequence having at least 80% sequence identity with any one of SEQ ID NOs: 307-329, to a cell population comprising Vγ4 T cells.

57. The method as defined in clause 56, comprising a heavy chain amino acid sequence comprising any one of SEQ ID NOs: 284-306 and/or a light chain amino acid sequence comprising any one of SEQ ID NOs: 307-329.

58. The method as defined in any one of clauses 1-11, wherein the anti-Vγ4 antibody or fragment thereof binds to the same, or essentially the same, epitope as, or competes with, an antibody or fragment thereof as defined in any one of clauses 12-57.

59. The method as defined in any one of clauses 1-58, wherein the antibody or fragment thereof is an scFv, Fab, Fab', F(ab')2, Fv, variable domain (e.g. VH or VL), diabody, minibody or full length antibody.

60. The method as defined in clause 59, wherein the antibody or fragment thereof is an scFv or a full length antibody.

61. The method as defined in clause 60, wherein the antibody or fragment thereof is a full length antibody, such as an IgG1 antibody.

62. The method as defined in any one of clauses 1-61, wherein the antibody or fragment thereof is human.

63. The method as defined in any one of clauses 1 to 62, wherein the modulation comprises expansion of Vγ4 T cells.

64. The method as defined in clause 63, wherein the method provides an expanded population of Vγ4 T cells which contains greater than about 60% Vγ4 T cells, such as greater than about 70% Vγ4 T cells.

65. The method as defined in any one of clauses 1 to 64, wherein the method comprises culturing the cell population for at least 5 days.

66. The method as defined in any one of clauses 1 to 65, wherein the method comprises culturing the cell population in the presence of at least one cytokine.

67. The method as defined in clause 66, wherein the cytokine is selected from: interleukin-2 (IL-2), interleukin-4 (IL-4), interleukin-7 (IL-7), interleukin-9 (IL-9), interleukin-12 (IL-12), interleukin-15 (IL-15), interleukin-21 (IL-21) or mixtures thereof.

68. The method as defined in any one of clauses 1 to 67, wherein the method comprises culturing the cell population in the presence of IL-2 and/or IL-15.

69. The method as defined in any one of clauses 65 to 68, wherein the cell population is not in direct contact with stromal and/or epithelial cells during culture.

70. The method as defined in clause 69, wherein the cell population is not in direct contact with fibroblasts during culture.

71. The method as defined in any one of clauses 65 to 70, wherein the cell population is not in direct contact with tumour cells and/or feeder cells during culture.

72. The method as defined in any one of clauses 1 to 71, wherein the method comprises culturing the cell population in serum-free media.

73. The method as defined in any one of clauses 1 to 72, wherein the cell population is enriched for T cells prior to administration of the antibody or fragment thereof.

74. The method as defined in any one of clauses 1 to 73, wherein the cell population is enriched for $\gamma\delta$ T cells prior to administration of the antibody or fragment thereof.

75. The method as defined in any one of clauses 1 to 74, wherein the cell population is depleted of $\alpha\beta$ T cells or NK cells prior to administration of the antibody or fragment thereof.

76. The method as defined in any one of clauses 1 to 75, wherein the cell population is obtained from a haematopoietic sample or a fraction thereof.

77. The method as defined in clause 76, wherein the haematopoietic sample is selected from peripheral blood, umbilical cord blood, lymphoid tissue, thymus, bone marrow, lymph node tissue or fractions thereof.

78. The method as defined in clause 76 or clause 77, wherein the haematopoietic sample consists of peripheral blood mononuclear cells (PBMCs) or low density mononuclear cells (LDMCs).

79. The method as defined in any one of clauses 1 to 75, wherein the cell population is obtained from a non-haematopoietic tissue sample, such as a skin, colon, gut, mammary gland, lung, prostate, liver, spleen, pancreas, uterus, vagina or other cutaneous, mucosal or serous membrane sample.

80. The method as defined in any one of clauses 1 to 75, wherein the cell population is obtained from a cancer tissue sample.

81. The method as defined in any one of clauses 1 to 80, wherein the cell population is obtained from human or non-human animal tissue.

82. The method as defined in any one of clauses 1 to 81, wherein the cell population is isolated from a sample prior to administering the anti-V$\gamma$4 antibody or fragment thereof.

83. A V$\gamma$4 T cell population obtained by the *ex vivo* method as defined in any one of clauses 1 to 82.

84. A composition comprising the V$\gamma$4 T cell population as defined in clause 83.

85. A pharmaceutical composition comprising the V$\gamma$4 T cell population as defined in clause 83, together with a pharmaceutically acceptable diluent or carrier.

86. The pharmaceutical composition as defined in clause 85, for use as a medicament.

87. The pharmaceutical composition as defined in clause 86 for use in the treatment of cancer, an infectious disease or an inflammatory disease.

88. A method of treating a cancer, an infectious disease or an inflammatory disease in a subject in need thereof, comprising administering a therapeutically effective amount of the V$\gamma$4 T cell population as defined in clause 83 or the pharmaceutical composition as defined in clause 85.

Other features and advantages of the present invention will be apparent from the description provided herein. It should be understood, however, that the description and the specific examples while indicating preferred embodiments of the invention are given by way of illustration only, since various changes and modifications will become apparent to those skilled in the art. The invention will now be described using the following, non-limiting examples:

**EXAMPLES**

**EXAMPLE 1. Materials and Methods**

Antigen preparation

The design of the soluble $\gamma\delta$ TCR heterodimers comprising the TCR$\alpha$ and TCR $\beta$ constant regions used in the below Examples were generated according to Xu et al. (2011) PNAS 108: 2414-2419. V$\gamma$ or V$\delta$ domains were fused in-frame to a TCR$\alpha$ or TCR$\beta$ constant region lacking the transmembrane domain, followed by a leucine zipper sequence or an Fc sequence, and a histidine tag/linker.

The expression construct was transiently transfected in mammalian EXPI HEK293 suspension cells (either as single or co-transfections for heterodimer). Secreted recombinant proteins were recovered and purified from culture supernatant by affinity chromatography. To ensure good recovery of monomer antigen, samples were further purified using preparative size exclusion chromatography (SEC). Purified antigens were analysed for purity by SDS-PAGE and aggregation state by analytical SEC.

Selected scFvs were subcloned into IgG1 frameworks using commercially available plasmids. expi293F suspension cells were transfected with said plasmids for antibody expression. For convenience, unless otherwise noted, the antibodies characterised in these Examples refer to IgG1 formatted antibodies selected from phage display as scFv. However, the antibodies may be in any antibody format as previously discussed.

Antibody purification

IgG antibodies were batch purified from supernatants using protein A chromatography. Quality of purified IgG was analysed using ELISA, SDS-PAGE and SEC-HPLC.

Antigen binding

Phage display selection outputs were subcloned into the scFv expression vector pSANG10 (Martin et al. (2006) BMC Biotechnol. 6: 46). Soluble scFv were expressed and screened for binding in dissociation-enhanced lanthanide fluorescence immunoassay (DELFIA) on directly immobilised targets. Hits were defined as a DELFIA signal above 3000 fluorescence units.

A DELFIA ELISA binding method was also employed to assess binding of antibody supernatants or further protein-A purified antibody. In brief, MaxiSorp plates were coated with 3 $\mu$g/ml of antigen BSA or L1 (DV1-GV4), L2 (DV1-GV2), L3 (DV1-GV8), or L4 (DV2-GV4) recombinant TCR antigen. Plates were then washed with PBS, blocked with PBS/skimmed milk and then test article added and incubated for 1 hour at room temperature. Thereafter, plates were washed with PBS-Tween and DELFIA Eu-N1-anti-human IgG (Perkin Elmer # 1244-330) added for 1 hour at room temperature prior to further washing, addition of DELFIA enhancement solution (Perkin Elmer #4001-0010), and reading on a Pherastar microplate reader.

D1.3 hIgG1 (described in England et al. (1999) J. Immunol. 162: 2129-2136) was used as a negative control and REA173 (Miltenyi) and TS8.2 (ThermoFisher, No. TCR1730) were used as comparator antibodies.

Antibody studies with recombinant JRT3-TCR cells

The recombinant JRT3-TCR cells employed in the antibody binding, the TCR downregulation, and the CD69 upregulation studies are described previously (see Melandri et al. (2018) Nature Immunology 19(12): 1352-1365, and Willcox et al. (2019) Immunity 51(5): 813-825.e4).

For the antibody binding studies, primary staining of either 100,000 non-transduced JRT3 controls or JRT3-TCR cells were undertaken in PBS 5% FCS for 30 minutes at 4°C with either a standard 1.0 $\mu$g/ml if the amount is not indicated or the amount indicated, such as 0.08, 0.4, 2 or 10 $\mu$g/ml in **Figure 6** of each lead antibody. Secondary staining was then carried out with A647 anti-human IgG (Biolegend). Additionally BV421 anti-CD3$\epsilon$ (Biolegend) or PE-Cy7 IMMU510 anti-$\gamma\delta$TCR (Beckman Coulter) staining was undertaken as/where indicated. Cells were then washed twice in PBS 5% FCS and flow analysis undertaken on a FACS Canto II 3L.

For TCR downregulation / CD69 upregulation studies, 96 flat well plates were first pre-coated by adding to each well 20 $\mu$g/ml secondary antibodies, specifically either anti-human IgG-Fc (for the human D1.3 and V$\gamma$4 antibodies) or anti-mouse IgG (for murine anti-CD3e or anti-Pan TCRgd) and then incubated for 2 h at 37°C. Test antibodies as indicated were first diluted to 0.01, 0.1, 1, and 10 $\mu$g/ml final concentrations. 50 $\mu$l of each concentration was then added to a well of the pre-coated plate prior to overnight incubation at 4°C. Unbound antibody was then washed twice with PBS before addition of saturating PBS 5% FCS for 1 hour at 37°C. 100,000 cells per well were then plated by 400g spinning. Cells were then incubated for 5 hours at 37°C, 5% $CO_2$ and then transferred to a 96 well round-bottom plate for staining. Staining antibodies employed included BV421 anti-CD3$\epsilon$ diluted 1:400 (clone OKT-3 Biolegend); PE-Cy7 anti-$\gamma\delta$TCR diluted 1:200 (clone IMMU510 Beckman Coulter); and A647 anti-CD69 diluted 1:200 (clone FN50 Biolegend). All staining undertaken in PBS 5% FCS for 30 minutes at 4°C.

Antibody studies with primary cells (PBMC)

24 well plates were first pre-coated by adding to each well 20 μg/ml (250 μl per well) anti-human IgG-Fc (Biolegend) and then incubated for 2 hours at 37°C. Unbound secondary antibody was washed twice with PBS, and isotype control (human IgG1, Biolegend) or anti-Vγ4 (clone G4_12) were first diluted to 0.1, 1, and 10 μg/ml final concentrations. 250 μl of each concentration was then added to a well of the pre-coated plate prior to overnight incubation at 4°C. Unbound antibodies were then washed twice with PBS before addition of saturating PBS 5% FCS for 1 hour at 37°C. 500,000 PBMC resuspended at $10^6$ cells per ml in complete media (RPMI supplemented with 5% heat-inactivated human AB serum [PAA laboratories], Sodium Pyruvate [1 mM] and Penicillin/Streptomycin [ThermoFisher]) were then added to each well. Cells were then incubated at 37°C, 5% $CO_2$. IL-2 or IL-2 + IL-15 (100 U/ml and 10 ng/ml final concentrations, respectively) were added after 24 hours and fresh complete media supplemented with IL-2 or IL-2 + IL-15 was added every 2-3 days. On days 7 and 14 of the culture, cells were transferred to a 96 well round-bottom plate for staining. Staining antibodies employed included biotin anti-TCRVy2/3/4 (Clone 23D12) diluted to 1 μg/ml, PE streptavidin diluted 1:100 (Biolegend); BV421 anti-CD3ε diluted 1:400 (clone OKT-3 Biolegend); PE-Cy7 anti-TCRγδ diluted 1:200 (clone IMMU510 Beckman Coulter); FITC anti-Vδ2 (Clone B6 Biolegend); and A647 anti-Vγ4 (clone G4_18) diluted to 1 μg/ml. All staining undertaken in PBS 5% FCS for 30 minutes at 4°C.

MS-based epitope mapping

CovalX 'Ultrafast Conformation/Linear Epitope Mapping' methodology was employed. First both protein antigen L1 (DV1-GV4) plus antibody G4_3 (1139_P01_A04) were analyzed for protein integrity and aggregation level using a high-mass MALDI. In order to determine the binding epitope of the L1(DV1-GV4)/G4_3 complex with high resolution, the complexes were incubated with deuterated cross-linkers and subjected to multi-enzymatic proteolysis using trypsin, chymotrypsin, Asp-N, elastase and thermolysin. After enrichment of the cross-linked peptides, the samples were analyzed by high resolution mass spectrometry (nLC-LTQ-Orbitrap MS) and the data generated were analyzed using XQuest and Stavrox software.

γδ T cell binding assay

The binding of antibodies to γδ T cells may be tested by incubating a fixed concentration of purified antibodies with 250000 γδ T cells. This incubation may be performed under blocking conditions, such as by the addition of huFc fragments or Ig to prevent unspecific binding of antibodies via the Fc receptor. Detection may be performed by addition of a secondary, fluorescent dye-conjugated antibody against human IgG1. For negative controls, cells may be prepared with a) an isotype antibody only (recombinant human IgG), b) the fluorescent dye-conjugated anti-human IgG antibody only and c) a combination of a) and b). A control well of completely unstained cells may be also prepared and analysed. As positive controls, a purified murine monoclonal IgG2 anti-human CD3 antibody may be used in two different concentrations and stained with a fluorescent dye-conjugated goat anti-mouse secondary antibody. The assay may be accepted if the lower concentration positive controls' mean fluorescence intensity in the FITC channel was at least tenfold as high as the highest negative control.

SPR Analysis

A MASS-2 instrument with an amine high capacity chip (both from Sierra Sensors, Germany) may be used to perform SPR analysis. 15 nM IgG may be captured via protein G to an amine high capacity chip (100 nM for TS8.2). L1 (DV1-GV4) antigen may be flown over the cell at a 1:2 dilution series from 2000 nM to 15.625 nM with the following parameters: 180 s association, 600 s dissociation, flowrate 30 μL/min, running buffer PBS + 0.02 % Tween 20. All experiments were performed at room temperature on MASS-2 instrument. Steady state fitting may be determined according to Langmuir 1:1 binding using software Sierra Analyzer 3.2.

γδ TCR downregulation and degranulation assay

THP-1 (TIB-202™, ATCC) target cells loaded or not with test antibodies may be labelled with CellTracker™ Orange CMTMR (ThermoFisher, C2927) and incubated with γδ T cells at 2:1 ratio in the presence of CD107a antibody (Anti-human CD107a BV421 (clone H4A3) BD Biosciences 562623). After 2 hours of incubation, the surface expression of γδ TCR (to measure TCR downregulation) and expression of CD107a (to measure degranulation) on γδ T cells may be evaluated using flow cytometry.

Killing assay

Gamma delta T cell-mediated killing activity and effect of test antibodies on the killing activity of γδ T cells may be accessed by flow cytometry. After 4 hours of *in vitro* co-culture at 20:1 ratio of γδ T cells and CellTracker™ Orange CMTMR (ThermoFisher, C2927) labelled THP-1 cells (loaded or not with the antibody) may be stained with Viability Dye eFluor™ 520 (ThermoFisher, 520 65-0867-14) to distinguish between live and dead target THP-1 cells. During sample acquisition, target cells may be gated on the CellTracker™ Orange CMTMR positivity and examined for cell death based on the uptake of Viability Dye. CMTMR and eFluor™ 520 double positive cells may be recognized as the dead target cells. The killing activity of γδ T cells may be presented as a % of the dead target cells.

## EXAMPLE 2. Antigen design

Gamma delta (γδ) T cells are polyclonal with CDR3 polyclonality. In order to avoid a situation where generated antibodies would be selected against the CDR3 sequence (as the CDR3 sequence will differ from TCR clone to TCR clone), the antigen design involved maintaining a consistent CDR3 in different formats. This design aimed to generate antibodies recognising a sequence within the variable domain, which is germline encoded and therefore the same in all clones, thus providing antibodies which recognise a wider subset of γδ T cells.

Another important aspect of the antigen preparation process was to design antigens which are suitable for expression as a protein. The γδ TCR is a complex protein involving a heterodimer with inter-chain and intra-chain disulphide bonds. A leucine zipper (LZ) format and Fc format were used to generate soluble TCR antigens to be used in the phage display selections. Both the LZ and Fc formats expressed well and successfully displayed the TCR (particularly heterodimeric TCRs, *e.g.* Vδ1Vγ4).

It was found that the CDR3 sequence from a public database entry for the γδ TCR expressed well as proteins (RSCB Protein Data Bank entry: 4MNH). This was therefore selected for antigen preparation.

Antigens containing the gamma variable 4 chain were expressed in LZ formats as a heterodimer (*i.e.* in combination with different delta variable chains - *e.g.* DV1-GV4, a heterodimer composed of a delta variable 1 chain and a gamma variable 4 chain, [ termed "L1"] and DV2-GV4, a heterodimer composed of a delta variable 2 chain and a gamma variable 4 chain, [ termed "L4"]) and in Fc format either as a heterodimer or as a homodimer (*i.e.* in combination with another gamma variable 4 chain - GV4-GV4, a homodimer composed of two gamma variable 4 chains, [termed "Fc4/4"]). All gamma variable 4 chains of the antigens contained the 4MNH CDR3. Another series of γδ TCR antigens using similar formats were designed containing different gamma variable chains (such as gamma variable 2 and gamma variable 8) and used to deselect antibodies with non-specific or off target binding (e.g. DV1-GV2, a heterodimer composed of a delta variable 1 chain and a gamma variable 2 chain, [termed "L2"] or DV1-GV8, a heterodimer composed of a delta variable 1 chain and a gamma variable 8 chain, [termed "L3"]). These antigens were also designed to include the 4MNH CDR3 to ensure that antibodies binding in the CDR3 region were also deselected.

## EXAMPLE 3. Phage Display

Phage display selections were performed against libraries of human scFvs using either heterodimeric LZ TCR format in round 1 and 2, with deselections on heterodimeric LZ TCR in both rounds. Or round 1 was performed using homodimeric Fc fusion TCR with deselection on human IgG1 Fc followed by round 2 on heterodimeric LZ TCR with deselection on heterodimeric LZ TCR (see **Table 1).**

Table 1. Overview phage display selections

| Target | Round 1 selection | Round 1 deselection | Round 2 selection | Round 2 deselection |
|--------|-------------------|---------------------|-------------------|---------------------|
| GV4 | bt-L1 (DV1-GV4) | L2 (DV1-GV2) | bt-L4 (DV2-GV4) | L2 (DV1-GV2) |
| GV4 | bt-Fc4/4 (GV4-GV4) | Fc | bt-L4 (DV2-GV4) | L2 (DV1-GV2) |
| bt = biotin. | | | | |

Selections were performed in solution phase using 100 nM biotinylated proteins. Deselections were performed using 1 μM non-biotinylated proteins.

## EXAMPLE 4. Antibody selection

Hits obtained in Example 3 were sequenced (using standard methods known in the art). 130 unique clones were identified, which showed a unique combination of VH and VL CDR3. Of these 130 unique clones, 129 showed a unique VH CDR3 and 116 showed a unique VL CDR3.

Unique clones were re-arrayed and specificity was analysed by ELISA (DELFIA). A panel of 42 unique human scFv binders which bind TRGV4 but not TRGV2 or TRGV8, were identified from the selections.

Affinity ranking of the selected binders was included to aid the choice of clones going forward. A large number of binders showed affinities in the nanomolar range, reacting with 25 to 100 nM biotinylated antigen (L1). A handful of binders showed a strong reaction with 5 nM antigen, indicating possible single digit nanomolar affinities. Some binders showed no reaction with 100 nM antigen, indicating affinities in the micromolar range.

For the selection of clones to proceed with to IgG conversion, the aim was to include as many germline lineages and as many different CDR3s as possible. Further, sequence liabilities like glycosylation, integrin binding sites, CD11c/CD18 binding sites, unpaired cysteines were avoided. In addition, a variety of affinities was included. The clones chosen to be converted to IgG are shown in **Figure 1.** The results from the ELISA binding (values in Fluorescence Units (FU)) are shown in **Figure 2A.** Results indicate that all 23 antibodies exhibit the desired gamma 4 chain specific profile and regardless of partner delta chain. The same data is also expressed in **Figure 2B** and further shows the fold-change increase in binding of each clone to the human Vγ4 chain versus the human Vy2 chain. Fold-change increases in binding to the human Vγ4 chain versus the human Vy2 chain ranged from an 80-fold (clone G4_26) to a 98387-fold increase (clone G4_18).

Antibody binding studies were also conducted using recombinant Jurkat (JRT3-hu17) cells. Comparison of the results from the ELISA data and flow cytometry data are shown in **Figure 3A.** Antibody clones which were identified as binding to both DV1-GV4 antigen via Delfia ELISA (Y-axis) and to JRT3-hu17 cells (X-axis) were chosen for further investigation.

## EXAMPLE 5. Investigation of Vγ4 antibody binding

The capacity of the antibodies chosen in Example 4 to stain Vγ4 TCRs bearing different CDR3 sequences (hu17 vs. hu20, both Vγ4Vδ1) or delta chains (hu20γ/huPBδ, Vγ4Vδ2; LES, Vγ4Vδ5) was investigated. Results are shown in **Figure 4A** and indicate that all tested antibodies show significantly increased binding to one or more of the Vγ4 TCRs used in the study relative to the D1.3 isotype control. In particular, five exemplary antibodies (G4_3, G4_12, G4_16, G4_18 and G4_27) bind all Vγ4 TCRs expressed in this study and regardless of CDR3 sequence or partner delta chain, exhibiting markedly enhanced binding signals over and above D1.3 isotype control. By way of illustration, example flow data for two of the antibodies in this study are shown in **Figure 4B** and illustrate the difference between G4_3 binding (stains positive for all Vγ4 TCRs) compared to G4_4 binding (stains positively for both hu17 and LES, whereas staining against both hu20 [different CDR3 sequences compared to hu17] and hu20g/huPBd [Vγ4Vδ2] is reduced).

## EXAMPLE 6. Epitope mapping using chimeric hu17 TCRs

hu17 is a Vγ4/Vδ1 TCR for which the paired CDR3 sequences were cloned from a BTNL3+8-reactive human colon intraepithelial lymphocyte by single-cell PCR (as described in Melandri et al. (2018) Nat. Immunol. 19: 1352-1365). Different chimeric hu17 TCR constructs were prepared as summarised in **Figure 5A.** These constructs were derived from hu17 and were all described in Melandri et al. (2018) Nat. Immunol. 19: 1352-1365 and Willcox et al. (2019) Immunity 51: 813-825 (both of which are incorporated herein by reference).

Antibody binding was then investigated by flow cytometry against the chimeric hu17 TCRs expressed on JRT3 cells. A summary table of the reactivity of each antibody to the indicated chimeric TCR constructs is shown in **Figure 5B.** The results highlight individual antibody relative binding specificity to the individual TCRs expressed on JRT3 cells. Antibodies G4_3, G4_12, G4_16, G4_18 and G4_27 were all indicated to specifically bind in or around the HV4 region because no staining or reduced staining was observed when hu17 TCR constructs containing the Vy2 sequences in the HV4 region were used.

Example flow data of epitope mapping to illustrate the differential binding signals observed in this study is shown in **Figure 5C.** In this instance, and as an example of this epitope mapping approach, G4_12 binding to the various recombinant chimeric TCRs is shown. First, G4_12 exhibits strong binding to starting hu17 TCR (far left panel). Strong binding is also observed against hu17 when the CDR1+2 sequences are exchanged in-frame for Vy2 equivalent CDRs (centre left panel) or when the hu17 is HV4 modified to Vγ2 sequence DG>YA (centre right panel). However, a noticeable drop in binding by G4_12 is observed with alternative Vγ4 to Vy2 amino acid substitutions (DGKM>YANL; centre panel) or KM>NL (far right panel), respectively. Hence in this instance the epitope recognized by G4_12 is located in the HV4 region (amino acids 67-82 of SEQ ID NO. 1 [KYDTYGSTRKNLRMIL]), and is heavily impacted by modifications of the underlined K and M residues to the equivalent resides found at this position in the Vγ2 HV4 region.

**EXAMPLE 7. Titration of investigated antibodies in staining and functional assays**

Results from titration of investigated antibodies for staining and analysis by flow cytometry to JRT3-hu17 cells (concentrations ranging from 0.08 to 10 μg/mL, 5-fold dilution steps) are shown in **Figure 6A.** Non-transduced JRT3 cells (no TCR) employed as a negative control. Results show all antibodies were capable of binding to JRT3 cells expressing Vγ4 TCRs.

Functional assays were then conducted by investigating TCR turnover and CD69 upregulation by titrated antibodies versus turnover conferred by anti-CD3ε binding or anti-pan-TCRγδ antibodies. The results are shown for five of the antibodies in **Figures 6B** and **6C** and results for a wider selection of antibodies within the original cohort are summarised in **Table 2.**

Table 2. TCR downregulation and activation of selected antibodies

| Antibody (μg/ml) | Vγ4 TCR downregulation | Conferred Activation (CD69 fold-change increase) |
|---|---|---|
| G4_3 | + | ++ |
| G4_4 | - | - |
| G4_7 | - | - |
| G4_10 | - | - |
| G4_12 | +++ | +++ |
| G4_16 | ++ | ++ |
| G4_18 | + | + |
| G4_27 | +/- | - |

All of the listed antibodies in **Table 2** have been shown to bind to the Vγ4 chain of a γδ TCR. However, as shown in the table, some of these antibodies are capable of activating the Vγ4 TCR as measured via Vγ4 TCR downregulation and/or increased CD69 expression (indicated as '+', '++' or '+++' with '+++' meaning highest relative levels of activation), whilst other antibodies show no appreciable ability to activate the Vγ4 TCR (indicated as '-').

**EXAMPLE 8. MS-based epitope mapping**

In order to determine the epitope of antigen/antibody complexes with high resolution, the protein complexes were incubated with deuterated cross-linkers and subjected to multi-enzymatic cleavage. After enrichment of the cross-linked peptides, the samples were analysed by high resolution mass spectrometry (nLC-LTQ-Orbitrap MS) and the data generated were analysed using XQuest (version 2.0) and Stavrox (version 3.6) software.

After trypsin, chymotrypsin, Asp-N, elastase and thermolysin proteolysis of the protein complex L1(DV1-GV4)/1139_P01_A04 with deuterated d0d12, the nLC-orbitrap MS/MS analysis detected 11 cross-linked peptides between L1(DV1-GV4) and the antibody 1139_P01_A04 (G4_3). Results of the epitope mapping results is presented in **Table 3.**

Table 3. Results of epitope mapping for antigen/antibody complexes

| Clone ID | Epitope mapping, amino acid numbering of SEQ ID NO: 1 |
|---|---|
| **1139_P01_A04** (G4_3) | 71, 73, 75, 76, 79 |

This epitope mapping data correlates with the experiments above, indicating that this antibody binds within the HV4 region of the γ4 chain.

**EXAMPLE 9. Anti-Vγ4 antibody targeting and modulation of primary Vγ4-positive cells.**

Further studies were undertaken to demonstrate anti-Vγ4 antibody targeting of primary Vγ4+ cells derived from skin, blood and gut, including cells derived from healthy and diseased patient samples.

Binding to primary $V\gamma4^+$ cells derived from skin

Firstly, anti-$V\gamma4$ antibodies were tested for binding to primary $V\gamma4^+$ T cells expanded from the skin of two individual donors. Skin samples were prepared by removing subcutaneous fat and a 3mm biopsy punch used to make multiple punches. Punches were placed on carbon matrix grids and placed in the well of a G-REX6 (Wilson Wolf). Each well was filled with complete isolation medium containing AIM-V media (Gibco, Life Technologies), CTS Immune Serum Replacement (Life Technologies), IL-2 and IL-15. For the first 7 days of culture, complete isolation medium containing Amphotericin B (Life Technologies) was used ("+AMP"). Media was changed every 7 days by gently aspirating the upper media and replacing with 2X complete isolation medium (without AMP), trying not to disturb the cells at the bottom of the plate or bioreactor. Beyond three weeks in culture, the resulting egressed cells were then passaged into fresh tissue culture vessels and fresh media (e.g. AIM-V media or TexMAX media (Miltenyi)) plus recombinant IL-2, IL-4, IL-15 and IL-21 before harvest. $\alpha\beta$ T cells also present within the culture were then removed with aid of $\alpha\beta$ T cell depletion kits and associated protocols, such as those provided by Miltenyi. For further reference see WO2020/095059.

Following isolation, $\gamma\delta$ T cells were first stained with viability dye in the presence of Fc block for 20 minutes at 4°C. $\gamma\delta$ T cells were then incubated with fixed concentrations of exemplary anti-$V\gamma4$ antibodies (0.046 - 100 $\mu$g/ml) or isotype control (IgG1 anti-respiratory syncytial virus (RSV) antibody) for 30 minutes at 4°C. Detection was performed by addition of a secondary, fluorescent dye-conjugated antibody against human IgG1 (IS11-12E4.23.20). Cells were then fixed and acquired on the MACSQuant16 flow cytometer. Cells were gated as single, live, IgG1 ($V\gamma4$)$^+$. Data shown are the median fluorescent intensity (MFI) of secondary detection antibody detected bound to $V\gamma4^+$ cells.

The results are shown in **Figure 7A.** These data confirmed that all of the tested anti-$V\gamma4$ antibodies were capable of binding to primary skin-derived $V\gamma4^+$ T cells in a dose-dependent manner. No binding was observed with the isotype control.

Binding to primary $V\gamma4^+$ cells derived from peripheral blood mononuclear cells (PBMCs)

In brief, human PBMCs (Lonza, product code CC-2702) were first stained with viability dye in the presence of Fc block for 20 minutes at 4°C. Cells were then incubated with 10 $\mu$g/ml anti-$V\gamma4$ antibodies or isotype control (RSV) for 30 minutes at 4°C, before being washed and stained extracellularly with anti-$V\delta1$ (REA173), anti-$V\delta2$ (REA771), anti-$\gamma\delta$ (REA591) and anti-human IgG1 (IS11-12E4.23.20) for 20 minutes at 4°C. Cells were then fixed and acquired on the MACSQuant16 flow cytometer. Cells were gated as single, live, $\gamma\delta^+$ $V\delta2^-$ IgG1 ($V\gamma4$)$^+$.

The results are shown in **Figure 7B.** Data shown are % $V\gamma4^+$ cells of $\gamma\delta^+$ $V\delta2^-$ cells detected using each individual antibody bound by the conjugated secondary anti-human IgG1 antibody. These data highlight the ability of substantially all of the anti-$V\gamma4$ antibodies to bind primary blood-derived $V\gamma4^+$ T cells. The strongest signals were detected using antibody G4_23, G4_3, G4_12, G4_18 or G4_20.

Binding to primary $V\gamma4^+$ cells from gut-derived intraepithelial lymphocytes (IELs) obtained from colorectal cancer (CRC) patients

For this study, human CRC tumour biopsy was shipped fresh and processed upon receipt. The biopsy was cut into pieces measuring ~2mm$^2$ and tumour-infiltrating lymphocytes (TILs) were obtained using an adaptation of the method originally described by Kupper and Clarke (Clarke et al., 2006, J. Invest. Dermatol. 126, 1059-1070). Specifically, up to four 2mm$^2$ biopsies were placed on 9mm x 9mm x 1.5mm Cellfoam matrices, and one matrix was placed per well on a 24-well plate. Biopsies were then cultured in 2 ml Iscove's Modified Dulbecco's Medium (IMDM) supplemented with 4% human plasma, $\beta$-mercaptoethanol (50 $\mu$M), penicillin (100 U/ml), streptomycin (100 $\mu$g/ml), gentamicin (20 $\mu$g/ml), metronidazole (1 $\mu$g/ml), amphotericin B (2.5 $\mu$g/ml), HEPES (10 mM), Na Pyruvate (1 mM), MEM Non-Essential Amino Acids Solution (1X) and IL-15 (20 ng/ml, Miltenyi Biotech). 1 ml of medium was aspirated every 3 days and replaced with 1 ml complete medium containing 2x concentrated IL-15. TILs were harvested 10 days later, passed through a 70 $\mu$M nylon cell strainer, centrifuged at 300 x g for 5 minutes and resuspended in complete medium for phenotyping. TILs were first stained with live/dead viability dye in the presence of Fc block for 20 minutes at 4°C. Cells were then incubated with 10 $\mu$g/ml anti-$V\gamma4$ antibodies or isotype control (RSV) for 30 minutes at 4°C, before being washed and stained extracellularly with anti-$V\delta1$ (REA173), anti-$\gamma\delta$ (REA591) and anti-human IgG (IS11-12E4.23.20) for 20 minutes at 4°C. Cells were then fixed and acquired on the MACSQuant16 flow cytometer. Cells were gated as single, live, $\gamma\delta^+$, IgG1$^+$ ($V\gamma4$)$^+$.

The results are shown in **Figure 7C.** Data shown are FACS plots illustrating binding of anti-$V\gamma4$ antibodies, G4_3, G4_12 and G4_18, to primary gut-derived $V\gamma4^+$ cells detected via the conjugated secondary anti-human IgG1 antibody. The data demonstrates the ability of the antibodies of the invention to bind to $V\gamma4^+$ cells obtained from CRC tumour tissue.

Detection and TCR downregulation of human gut-derived γδ T cells conferred by anti-Vγ4 antibody

A further study was undertaken to explore modulation of human gut-derived γδ T cells conferred by an anti-Vγ4 antibody. For these studies, normal adjacent tissue (NAT) biopsies from the colon of CRC patients were shipped fresh and processed upon receipt to obtain a single cell suspension. Specifically, the tissue was chopped in pieces measuring ~2mm² and up to 1 g of tissue was placed into a Miltenyi C tube along with 4.7 ml RPMI with enzymes from Miltenyi's Tumour Dissociation Kit at concentrations recommended by the manufacturer aside from Enzyme R which was used at 0.2X concentration to prevent cleavage of pertinent cell surface molecules. C-Tubes were placed on the gentleMACS™ Octo Dissociator with heating blocks attached. Program 37C_h_TDK_1 for the dissociation of soft tumours was selected. After 1 hour the digest was filtered through a 70 μM filter and complete IMDM containing 4% human plasma was added to quench enzymatic activity. Cells were then washed twice and resuspended in complete IMDM for counting. At this point, cells were plated for stimulation with anti-Vγ4 antibodies, or were used for phenotyping.

In one series of experiments, the phenotype of Vγ4⁺ γδ T cells in the gut digest before stimulation with anti-Vγ4 antibodies was determined. In brief, cells were stained with live/dead viability dye in the presence of Fc block for 20 minutes at 4°C. Cells were then incubated with 10 μg/ml G4_18 clone for 30 minutes at 4°C, before being washed and stained extracellularly with anti-Vδ1 (REA173), anti-γδ (REA591), anti-CD69 (REA824), anti-CD103 (Ber-Act8) and anti-human IgG1 (IS11-12E4.23.20) for 20 minutes at 4°C. Cells were then fixed and acquired on the MACSQuant16 flow cytometer. As shown in **Figure 7D,** 1.4% of live, single cells were Vδ1⁺. Of these, 44.2% were paired with Vγ4, and these all displayed markers of gut tissue residency (CD69⁺ CD103⁺) as expected for γδ T cells from the gut. These results confirm that the antibodies described herein, in this case example antibody G4_18, can be used to specifically detect Vγ4⁺ γδ T cells isolated from human gut tissue.

A next series of experiments measured the impact of stimulating the cells with an anti-Vγ4 antibody. 2x10⁶ cells were plated per well in a 48-well plate and were stimulated with G4_12, G4_18 or RSV IgG1 isotype control antibodies in the presence of IL-15 at a concentration of 2 ng/ml. Intraepithelial lymphocytes (IELs) isolated by enzymatic digestion were analysed by flow cytometry 24 hours post mAb stimulation. Following 24 hour stimulation, cells were stained with viability dye in the presence of Fc block for 20 minutes at 4°C. Cells were then stained extracellularly for γδTCR (REA591), fixed, and acquired on a MACSQuant16 flow cytometer. Live, single cells were gated as γδTCR⁺. **Figure 7E** shows conferred γδTCR downregulation following 24 hours stimulation with G4_12 or G4_18 clones, compared with RSV isotype control, accompanied by representative FACS plots. Both anti-Vγ4 antibodies, G4_12 and G4_18, induced γδTCR downregulation relative to the RSV isotype control, with the greatest downregulation observed with G4_12.

**EXAMPLE 10. Further studies measuring the binding affinity (KD) to human Vγ4 as measured by surface plasmon resonance (SPR) of example anti-Vγ4 antibodies of the invention.**

In addition, to the SPR binding studies described in **Example 4** (method described in **Example 1)** in respect of scFv binders, additional studies were undertaken to measure the binding affinity of select example clones to the human Vγ4 chain when clones were expressed as full IgG1 monoclonal antibodies.

In brief, the binding affinity of the antibodies to target (i.e. the human Vγ4 chain of a γδ TCR) was established by SPR analysis using a Reichert 4SPR instrument (Reichert Technologies). Antigen (L1 (DV1-GV4)) was coupled onto a Carboxymethyl Dextran Chip (Reichert Technologies) at 10ug/ml, which resulted in an increase from baseline of approximately 750 uRIU, respectively. Antibody was flown over the cell at a 1:2 dilution series from 500 nM to 31.25 nM with the following parameters: 180 s association, 300 s dissociation, flowrate 25 μL/min, running buffer PBS + 0.05 % Tween 20. All experiments were performed at room temperature, with the samples kept at 4°C before flowing over the chip. Steady state fitting was determined according to Langmuir 1:1 binding using software TraceDrawer (Reichert Technologies).

The results are shown in **Table 4** and represent the average of 2 experiments per antibody (except where indicated).

Table 4. Binding affinity of example antibodies for human Vγ4

| Antibody clone | Experiment 1: KD (nM) | Experiment 2: KD (nM) | Average KD (nM) |
|---|---|---|---|
| G4_3 | 3.69 | 2.03 | 2.86 |
| G4_12 | 17.8 | 20.2 | 19 |
| G4_18 | ND | 19.9 | 19.9 |
| G4_20 | 43 | 62.1 | 52.55 |
| G4_23 | 109 | 178 | 143.5 |

(continued)

| Antibody clone | Experiment 1: KD (nM) | Experiment 2: KD (nM) | Average KD (nM) |
|---|---|---|---|
| G4_27 | 261 | ND | 261 |
| *ND - not determined | | | |

A range of binding affinities was determined, as expected, thus enabling a particular antibody to be selected for a particular circumstance depending on the binding affinity required. In particular, binding affinities ranged from approximately 260 nM - 2.8 nM, as shown. This was consistent with the scFv studies described in **Example 4.**

**EXAMPLE 11. Use of Vγ4-specific antibodies to increase the number of primary human Vγ4 T cells.**

The antibody displaying the highest stimulatory activity on JRT3-hu17 cells (Clone G4_12, Figure 5B,C) was further tested for its capacity to stimulate primary Vγ4+ T cells. The increase in the percentage of Vγ4 T cells in PBMC cultures following plate-bound stimulation with G4_12 compared to isotype control was analysed by flow cytometry using a panel of antibodies including A647-conjugated anti-Vγ4 clone G4_18 and is shown in Figure 8. At days 7 and 14, the proportion of Vg4 positive cells in the presence of G4_12 antibody was greater than in cultures where the isotype control was present.

**SEQUENCES**

| SEQ ID NO. | Description | Sequence |
|---|---|---|
| 1 | Vγ4 chain of RSCB Protein Data Bank entry: 4MNH | SSNLEGRTKSVIRQTGSSAEITCDLAEGSTGYIHWYLHQEGKAP QRLLYYDSYTSSVVLESGISPGKYDTYGSTRKNLRMILRNLIEND SGVYYCATWDEKYYKKLFGSGTTLVVTEDLKNVFPPEVAVFEP SEAEISHTQKATLVCLATGFYPDHVELSWWVNGKEVHSGVCTD PQPLKEQPALNDSRYALSSRLRVSATFWQNPRNHFRCQVQFY GLSENDEWTQDRAKPVTQIVSAEAWGRADSRGGLEVLFQ |
| 2-24 | CDR3 heavy sequences | See Figure 1 |
| 25-47 | CDR3 light sequences | See Figure 1 |
| 48-70 | CDR2 heavy sequences | See Figure 1 |
| A1-A23 | CDR2 light sequences | See Figure 1 |
| 71-93 | CDR1 heavy sequences | See Figure 1 |
| 94-116 | CDR1 light sequences | See Figure 1 |
| 117 | TRGV4 full heavy variable sequence G4_1 | EVQLLESGGGVVQPGRPLRLSCAASGFTFSSYSMNWVRQAPG KGLEWVSSISSSSSYIYYADSVKGRFTISRDNSKNTLYLQMNSL RAEDTAVYYCAKGHWYFDLWGRGTLVTVSS |
| 118 | TRGV4 full heavy variable sequence G4_2 | QMQLVQSGAEVKKPGATVKISCKVSGYPFTDYYIHWVQQAPGK GLEWMGLVDPEDGQSRSAERFQGRVTITADTSTDTAYMELSSL RSEDTAVYYCATFPVAGFYGMDVWGQGTLVTVSS |
| 119 | TRGV4 full heavy variable sequence G4_3 | EVQLVESGGGLVQPGGSLRLSCAASGFTFSSYSMNWVRQAPG KGLEWVSSISSSSSYIYYADSVKGRFTISRDNSKNTLYLQMNSL RAEDTAVYYCARGGWLYDYWGQGTLVTVSS |
| 120 | TRGV4 full heavy variable sequence G4_4 | QVQLVESGGGLVQPGGSLRLSCAASGFTFSSYAMSWVRQAPG KGLEWVSAISGSGGSTYYADSVKGRFTISRDNSKNTLYLQMSSL RVEDTAVYYCAKSSVGWWSFDYWGQGTMVTVSS |

(continued)

| SEQ ID NO. | Description | Sequence |
|---|---|---|
| 121 | TRGV4 full heavy variable sequence G4_5 | EVQLVQSGAEVKKPGSSVKVSCKASGYTFTSYGISWVRQAPGQGLEWMGWIGAYNGNTNYAQKLQGRVTMSTDTSTSTAYMELRSPRSDDTAVYYCARGGTGGDHVFAYWGQGTTVTVSS |
| 122 | TRGV4 full heavy variable sequence G4_6 | EVQLVESGGGLVQPGGPLRLSCAASGFTFSSYAMNWVRQAPGKGLEWVSAISGSGGSTYYADSVKGRFTISRDNSKNTLYLQMNSLRAEDTAVYYCAKADYGVVYYFDYWGQGTMVTVSS |
| 123 | TRGV4 full heavy variable sequence G4_7 | EVQLVESGGGWVQSGGSLRPSCAASGFTFSHYWMSWVRQAPGKGLEWVANIKQDGSIIYYADSVKGRFTISRDNAKNSVYLQMNSLRAEDTAVYYCARIGYSSSSFDYWGRGTLVTVSS |
| 124 | TRGV4 full heavy variable sequence G4_10 | QVQLVESGGGVVQPGRPLRLSCAASGFTFSSYAMHWVRQAPGKGLEWVSAISGSGGSTYYADSVKGRFTISRDNSKNTLYLQMNSLRAEDTAVYYCAKDGAVDFWRNGMDVWGRGTLVTVSS |
| 125 | TRGV4 full heavy variable sequence G4_12 | EVQLLESGGGLVQPGGSLRLSCAASGFTVSSNYMSWVRQAPGKGLEWVSVIYSGGSTYYADSVKGRFTISRHNSKNTLYLQMNSLRAEDTAVYYCARVANGDFLDYWGRGTLVTVSS |
| 126 | TRGV4 full heavy variable sequence G4_13 | QVQLVESGAEVKKPGGSLRLSCAASGFTFSSYSMNWVRQAPGKGLEWVSSISGTSSYIYYADSVKGRFTISRDNAKNSLYLQMSSLRAEDTAVYYCARGGLGMVDPWGQGTLVTVSS |
| 127 | TRGV4 full heavy variable sequence G4_14 | EVQLVQSGAEVKKPGESLRISCKGSGYSFTSYWISWVRQMPGKGLEWMGRIDPSDSYTNYSPSFPGHVTISADKSISTAYLQWSSLKASDTAMYYCAADTAHGMDVWGRGTLVTVSS |
| 128 | TRGV4 full heavy variable sequence G4_15 | EVQLVQSEAEVKKPGASVKVSCKASGYTFTRHYMHWVRQAPGQGLEWMGLINPSGSSTVYAQKFQGRVTLTRDTSTSTDYMELSSLRSEDTAVYYCARDNSHLDQVWWFDPWGQGTLVTVSS |
| 129 | TRGV4 full heavy variable sequence G4_16 | EVQLLESGAEVKKPGASVKVSCKASGYTFTSYGISWVRQAPGQGLEWMGWISAYNGNTNYAQKLQGRVTMTTDTSTSTAYMELRSLRSDDTAVYYCARDYGDFYGMDVWGQGTLVTVSS |
| 130 | TRGV4 full heavy variable sequence G4_18 | EVQLVESGAEVKKPGASVKVSCKASGYTFTGYYMHWVRQAPGQGLEWMGRINPNSGGTNYAQKFQGRVTMTRDASISTAYMELSRLRSDDTAVYYCARDLDLSSLDYWGRGTLVTVSS |
| 131 | TRGV4 full heavy variable sequence G4_19 | EVQLVQSGAEVKKPGASVKVSCKASGYTLTSYYMHWVRQAPGQGLEWMGIINPSGGSTSYAQKFQGRVTMTRDTSTSTVYMELSSLRSEDTAVYYCARERGYSYGDGMDVWGQGTTVTVSS |
| 132 | TRGV4 full heavy variable sequence G4_20 | QVQLVESGAEVKKPGASVKVSCKASGGTFSSYAISWVRQAPGQGLEWMGGIIPIFGTANYAQKFQGRVTITVDKSTRTAYMELSSLRSKDTAVYYCARGNSRSDAFDIWGQGTMVTVSS |
| 133 | TRGV4 full heavy variable sequence G4_22 | QVQLVESGGGLVQPGGSLRLSCAASGFTFSTYAMSWVRQAPGKGLEWVSTVSGSGGTTYYADSVKGRFTISRDNSKNTLYLQMNSLRAEDTAVYYCAKDSTAVTDWFDPWGRGTLVTVSS |
| 134 | TRGV4 full heavy variable sequence G4_23 | EVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARGEVAALYYFDYWGQGTLVTVSS |

(continued)

| SEQ ID NO. | Description | Sequence |
|---|---|---|
| 135 | TRGV4 full heavy variable sequence G4_24 | QVQLQQSGPGLVKPSQTLSLTCAISGASVSSNSVAWNWIRQSP SRGLEWLGRTYYRSRWYNDYALSVKSRIIINPDTSKNQFSLQLN SVTPEDTAVYYCARDWSSTRSFDYWGRGTLVTVSS |
| 136 | TRGV4 full heavy variable sequence G4_25 | EVQLVESGAEVKKPGSSVKVSCKASGGTFSSYAISWVRQAPGQ GLEWMGGIIPIFGTANYAQKFQGRVTITADKSTSTAYMELSSLRS EDTAVYYCARSLRDGYNYIGSLGYWGQGTLVTVSS |
| 137 | TRGV4 full heavy variable sequence G4_26 | QVQLVQSGAEVKKPGSSVKVSCKASGGTFSSYAISWVRQAPG QGLEWMGGIIPIFGTANYAQKFQGRVTITADESTSTAYMELSSL RSEDTAVYYCASSRGSGWFPLGYWGQGTLVTVSS |
| 138 | TRGV4 full heavy variable sequence G4_27 | QVQLVQSGAEVKKPGESLKISCKSSGYSFTSYWIGWVRQMPGK GLEWMGIIYPGDSDTRYSPSFQGQVTFSADESISTAYLQWSSLK ASDTAMYYCARHGAYGDYPDTFDIWGQGTLVTVSS |
| 139 | TRGV4 full heavy variable sequence G4_28 | QVQLVESGGGLVQPGGSLRLSCAASGFTFDDYAMHWVRQAPG KGLEWVSGISAGGGSTNYAGSVKGRFTVSRDTSKNTLYLQMNS LRAEDTAVYYCVKSYVDTAMRYYYYYMDVWGQGTMVTVSS |
| 140 | TRGV4 full light variable sequence G4_1 | ASDIQMTQSPSSLSASVGDRVTITCQASQDISNYLNWYQQKPG KAPKLLIYDASNLETGVPSRFSGSGSGTDFTLTISSLQPEDFATY YCQQSYSTPVTFGPGTKVEIK |
| 141 | TRGV4 full light variable sequence G4_2 | ASDIQMTQSPSSLSASVGDRVTITCQASQDISNYLNWYQQKPG KAPKLLIYAASSLQSGVPSRFSGSGSGTDFTLTISGLQPEDFATY YCLEDYNYLWTFGQGTKLEIK |
| 142 | TRGV4 full light variable sequence G4_3 | ASDIQMTQSPSSLSASVGDRVTITCQASQDISNYLNWYQQKPG KAPKLLIYDASNLETGVPSRFSGSGSGTDFTLTISSLQPEDFATY FCQQSYSTPQTFGQGTKVDIK |
| 143 | TRGV4 full light variable sequence G4_4 | ASDIVMTQSPDSLAVSLGERATINCKSSQSVLSSSNNNNYLAWY QQRPGQPPKLLFYWASTRESGVPDRFSGSGSGTSFTLTITSLQ AEDVAVYYCQQYYSTPLTFGGGTKLEIK |
| 144 | TRGV4 full light variable sequence G4_5 | ASDIQMTQSPSSLSASVGDRVTITCRASQSISSYLNWYQQKPGK APKLLIYAASSLQSGVPSRFSGSGSGTDFTLTISSLQPEDFATYY CQQSYSTPYTFGQGTKVEIK |
| 145 | TRGV4 full light variable sequence G4_6 | ASDIQMTQSPSSLSASVGDRVTITCRASQSISTYLNWYQQKPGK APKLLIYAASSLQSGVPSRFSGSGSGTDFTLTISSPQPEDFATYY CQQSYSTPYTFGQGTKVEIK |
| 146 | TRGV4 full light variable sequence G4_7 | ASDIVMTQSPLSLPVTPGEPASISCRSSQSLLHSNRFNYLDWYL QKPGQSPQLLIYLGSNRASGVPDRFSGSGSGTDFTLKISRVEAE DVGVYYCMQGLQTPYTFGQGTKVDIK |
| 147 | TRGV4 full light variable sequence G4_10 | ASDIVMTQPPLSLPVTLGHPASISCKSSQSLEYSDGNTYLNWFQ QRPGQSPRRLIYKVSNRDSGAPDRFSGSGSGTDFTLEISRVEA EDVGVYYCMQGTLWPPTFGQGTKVDIK |
| 148 | TRGV4 full light variable sequence G4_12 | ASQSVLTQPASVSGSPGQSITISCTGTSSDVGGYNFVSWYQQH PGKAPKLMIYEVTNRPSGVPDRFSGSKSGNTASLTISGLQAEDE ADYYCSSHASPRVFGTGTKVTVL |

(continued)

| SEQ ID NO. | Description | Sequence |
|---|---|---|
| 149 | TRGV4 full light variable sequence G4_13 | ASNFMLTQPHSVSESPGKTVTISCTRSSGSIASNYVQWYQQRP GSSPSTVIYEDNQRPSGVPDRFSGSIDSSSNSASLTISGLRTED EADYYCQSYDSSIYVVFGGGTKLTVL |
| 150 | TRGV4 full light variable sequence G4_14 | ASNFMLTQPHSVSESPGKTVTISCTRSRGSIAGNYVHWYQQRP GRAPTTVIYRDKERPSGVPDRISGSIDSSSNSASLTISGLKTEDE ADYYCQSYDSSTHVVFGGGTKLTVL |
| 151 | TRGV4 full light variable sequence G4_15 | ASQSALTQPASVSGSPGQSITISCTGTSSDVGGYNYVSWYQQH PGKAPKLMIYDVSNRPSGVSNRFSGSKSGNTASLTISGLQAEDE ADYYCSSYGSGSVFGTGTKLTVL |
| 152 | TRGV4 full light variable sequence G4_16 | ASQSVLTQPPSASGSPGQSVTFSCTGTSSDIGAFNSVSWYQQH PGKAPKLLIYEITKRPSGVPDRFSGSKSGNTASLTISVLQAEDEA DYYCTSYAGSNTLIFGGGTKVTVL |
| 153 | TRGV4 full light variable sequence G4_18 | ASSYELTQPPSVTESPGQTARITCSGDALAKQYAYWYQQKPGQ APVLVIYRDSERPSEIPERFSGSSSGTTVTLTISGVQAEDEADYY CQSADSSGTYTVFGGGTKLTVL |
| 154 | TRGV4 full light variable sequence G4_19 | ASNFMLTQPHSVSESPGKTVTISCTRSSGSIASNYVQWYQQRP GSPPITLIYDDDQRPSGVPHRFSGSIDTSSNPASLTISGLKTEDE ADYYCQSYDSSNHVVFGGGTKLTVL |
| 155 | TRGV4 full light variable sequence G4_20 | ASSYELTHPPSVSVSPGQTASITCSGDKLGDKFVSWYHQKPGQ SPVLVIYQDSKRPSGIPERFSGSNSGNTATLTISGTRAMDEADY YCQAWDSSTVVFGGGTKLTVL |
| 156 | TRGV4 full light variable sequence G4_22 | ASDIQMTQSPSSLSASVGDRVTITCQASQDISNYLNWYQQKPG KAPKLLIYAASSLQSGVPSRFSVSGSGTDFTLTISNLQPEDFATY YCQQSYSIPWTFGQGTKVEIK |
| 157 | TRGV4 full light variable sequence G4_23 | ASDIQMTQSPSSLSASVGDRVTITCRASQGISNSLAWYQQKPG KAPKLLLYAASRLESGVPSRFSGSGSGTDYTLTISSLQPEDFATY YCQQYYSTPRTFGGGTKLEIK |
| 158 | TRGV4 full light variable sequence G4_24 | ASDIVMTQSPDSLAVSLGERATINCKSSQSVLYSSNNKNYLAWY QQKPGQPPKLLISWASTRESGVPDRFSGSGSGTDFTLTINSLQS EDVAIYYCQQYYSTPPTFGQGTKLEIK |
| 159 | TRGV4 full light variable sequence G4_25 | ASQSALTQPRSVSGSPGQSVTISCTGTSSDVGGYNYVSWYQQ HPGKAPKLMIYEVSNRPSGVSNRFSGSKSGNTASLTISGLQAED EADYYCSSYGSGSVFGTGTKLTVL |
| 160 | TRGV4 full light variable sequence G4_26 | ASQSGLTQPASVSGSPGQSITISCTGTSSDVGSYNLVSWYQQH PGKAPKLMIYEVSKRPSGVSNRFSGSKSGNTASLTISGLQAEDE ADYYCSSFGSGSIFGTGTKLTVL |
| 161 | TRGV4 full light variable sequence G4_27 | ASSYELTQDPAVSVALGQTVSITCQGDSLRNFYANWYQQKPGQ APVLVIYGKNNRPSGIPDRFSGSSSGNTASLTITGAQAEDEADY YCNSRDSSGNHLVFGGGTQLTVL |
| 162 | TRGV4 full light variable sequence G4_28 | ASSYELTQDPAVSVALGQTVTITCQGDSLRNYYASWYRQKPGQ TPVLVVYGKNNRPSGIPDRFSVSASGNTASLTITGAQAEDEGDY YCNSRDSSGVVFGGGTKVTVL |

(continued)

| SEQ ID NO. | Description | Sequence |
|---|---|---|
| 163 | scFv sequence G4_1 | EVQLLESGGGVVQPGRPLRLSCAASGFTFSSYSMNWVRQAPG KGLEWVSSISSSSSYIYYADSVKGRFTISRDNSKNTLYLQMNSL RAEDTAVYYCAKGHWYFDLWGRGTLVTVSSGGGGSGGGGSG GGASDIQMTQSPSSLSASVGDRVTITCQASQDISNYLNWYQQK PGKAPKLLIYDASNLETGVPSRFSGSGSGTDFTLTISSLQPEDFA TYYCQQSYSTPVTFGPGTKVEIKRTAAASAHHHHHHKLDYKDH DGDYKDHDIDYKDDDDK |
| 164 | scFv sequence G4_2 | QMQLVQSGAEVKKPGATVKISCKVSGYPFTDYYIHWVQQAPGK GLEWMGLVDPEDGQSRSAERFQGRVTITADTSTDTAYMELSSL RSEDTAVYYCATFPVAGFYGMDVWGQGTLVTVSSGGGGSGG GGSGGGASDIQMTQSPSSLSASVGDRVTITCQASQDISNYLNW YQQKPGKAPKLLIYAASSLQSGVPSRFSGSGSGTDFTLTISGLQ PEDFATYYCLEDYNYLWTFGQGTKLEIKRTAAASAHHHHHHKL DYKDHDGDYKDHDIDYKDDDDK |
| 165 | scFv sequence G4_3 | EVQLVESGGGLVQPGGSLRLSCAASGFTFSSYSMNWVRQAPG KGLEWVSSISSSSSYIYYADSVKGRFTISRDNSKNTLYLQMNSL RAEDTAVYYCARGGWLYDYWGQGTLVTVSSGGGGSGGGGSG GGASDIQMTQSPSSLSASVGDRVTITCQASQDISNYLNWYQQK PGKAPKLLIYDASNLETGVPSRFSGSGSGTDFTLTISSLQPEDFA TYFCQQSYSTPQTFGQGTKVDIKRTAAASAHHHHHHKLDYKDH DGDYKDHDIDYKDDDDK |
| 166 | scFv sequence G4_4 | QVQLVESGGGLVQPGGSLRLSCAASGFTFSSYAMSWVRQAPG KGLEWVSAISGSGGSTYYADSVKGRFTISRDNSKNTLYLQMSSL RVEDTAVYYCAKSSVGWWSFDYWGQGTMVTVSSGGGGSGG GGSGGGASDIVMTQSPDSLAVSLGERATINCKSSQSVLSSSNN NNYLAWYQQRPGQPPKLLFYWASTRESGVPDRFSGSGSGTSF TLTITSLQAEDVAVYYCQQYYSTPLTFGGGTKLEIKRTAAASAHH HHHHKLDYKDHDGDYKDHDIDYKDDDDK |
| 167 | scFv sequence G4_5 | EVQLVQSGAEVKKPGSSVKVSCKASGYTFTSYGISWVRQAPGQ GLEWMGWIGAYNGNTNYAQKLQGRVTMSTDTSTSTAYMELRS PRSDDTAVYYCARGGTGGDHVFAYWGQGTTVTVSSGGGGSG GGGSGGGASDIQMTQSPSSLSASVGDRVTITCRASQSISSYLN WYQQKPGKAPKLLIYAASSLQSGVPSRFSGSGSGTDFTLTISSL QPEDFATYYCQQSYSTPYTFGQGTKVEIKRTAAASAHHHHHHK LDYKDHDGDYKDHDIDYKDDDDK |
| 168 | scFv sequence G4_6 | EVQLVESGGGLVQPGGPLRLSCAASGFTFSSYAMNWVRQAPG KGLEWVSAISGSGGSTYYADSVKGRFTISRDNSKNTLYLQMNSL RAEDTAVYYCAKADYGVVYYFDYWGQGTMVTVSSGGGGSGG GGSGGGASDIQMTQSPSSLSASVGDRVTITCRASQSISTYLNW YQQKPGKAPKLLIYAASSLQSGVPSRFSGSGSGTDFTLTISSPQ PEDFATYYCQQSYSTPYTFGQGTKVEIKRTAAASAHHHHHHKL DYKDHDGDYKDHDIDYKDDDDK |
| 169 | scFv sequence G4_7 | EVQLVESGGGWVQSGGSLRPSCAASGFTFSHYWMSWVRQAP GKGLEWVANIKQDGSIIYYADSVKGRFTISRDNAKNSVYLQMNS LRAEDTAVYYCARIGYSSSSFDYWGRGTLVTVSSGGGGSGGG GSGGGASDIVMTQSPLSLPVTPGEPASISCRSSQSLLHSNRFNY LDWYLQKPGQSPQLLIYLGSNRASGVPDRFSGSGSGTDFTLKIS RVEAEDVGVYYCMQGLQTPYTFGQGTKVDIKRTAAASAHHHHH HKLDYKDHDGDYKDHDIDYKDDDDK |

(continued)

| SEQ ID NO. | Description | Sequence |
|---|---|---|
| 170 | scFv sequence G4_10 | QVQLVESGGGVVQPGRPLRLSCAASGFTFSSYAMHWVRQAPG KGLEWVSAISGSGGSTYYADSVKGRFTISRDNSKNTLYLQMNSL RAEDTAVYYCAKDGAVDFWRNGMDVWGRGTLVTVSSGGGGS GGGGSGGGASDIVMTQPPLSLPVTLGHPASISCKSSQSLEYSD GNTYLNWFQQRPGQSPRRLIYKVSNRDSGAPDRFSGSGSGTD FTLEISRVEAEDVGVYYCMQGTLWPPTFGQGTKVDIKRTAAASA HHHHHHKLDYKDHDGDYKDHDIDYKDDDDK |
| 171 | scFv sequence G4_12 | EVQLLESGGGLVQPGGSLRLSCAASGFTVSSNYMSWVRQAPG KGLEWVSVIYSGGSTYYADSVKGRFTISRHNSKNTLYLQMNSLR AEDTAVYYCARVANGDFLDYWGRGTLVTVSSGGGGSGGGGS GGGASQSVLTQPASVSGSPGQSITISCTGTSSDVGGYNFVSWY QQHPGKAPKLMIYEVTNRPSGVPDRFSGSKSGNTASLTISGLQA EDEADYYCSSHASPRVFGTGTKVTVLRTAAASAHHHHHHKLDY KDHDGDYKDHDIDYKDDDDK |
| 172 | scFv sequence G4_13 | QVQLVESGAEVKKPGGSLRLSCAASGFTFSSYSMNWVRQAPG KGLEWVSSISGTSSYIYYADSVKGRFTISRDNAKNSLYLQMSSL RAEDTAVYYCARGGLGMVDPWGQGTLVTVSSGGGGSGGGGS GGGASNFMLTQPHSVSESPGKTVTISCTRSSGSIASNYVQWYQ QRPGSSPSTVIYEDNQRPSGVPDRFSGSIDSSSNSASLTISGLR TEDEADYYCQSYDSSIYVVFGGGTKLTVLRTAAASAHHHHHHKL DYKDHDGDYKDHDIDYKDDDDK |
| 173 | scFv sequence G4_14 | EVQLVQSGAEVKKPGESLRISCKGSGYSFTSYWISWVRQMPGK GLEWMGRIDPSDSYTNYSPSFPGHVTISADKSISTAYLQWSSLK ASDTAMYYCAADTAHGMDVWGRGTLVTVSSGGGGSGGGGSG GGASNFMLTQPHSVSESPGKTVTISCTRSRGSIAGNYVHWYQQ RPGRAPTTVIYRDKERPSGVPDRISGSIDSSSNSASLTISGLKTE DEADYYCQSYDSSTHVVFGGGTKLTVLRTAAASAHHHHHHKLD YKDHDGDYKDHDIDYKDDDDK |
| 174 | scFv sequence G4_15 | EVQLVQSEAEVKKPGASVKVSCKASGYTFTRHYMHWVRQAPG QGLEWMGLINPSGSSTVYAQKFQGRVTLTRDTSTSTDYMELSS LRSEDTAVYYCARDNSHLDQVWWFDPWGQGTLVTVSSGGGG SGGGGSGGGASQSALTQPASVSGSPGQSITISCTGTSSDVGGY NYVSWYQQHPGKAPKLMIYDVSNRPSGVSNRFSGSKSGNTAS LTISGLQAEDEADYYCSSYGSGSVFGTGTKLTVLRTAAASAHHH HHHKLDYKDHDGDYKDHDIDYKDDDDK |
| 175 | scFv sequence G4_16 | EVQLLESGAEVKKPGASVKVSCKASGYTFTSYGISWVRQAPGQ GLEWMGWISAYNGNTNYAQKLQGRVTMTTDTSTSTAYMELRS LRSDDTAVYYCARDYGDFYGMDVWGQGTLVTVSSGGGGSGG GGSGGGASQSVLTQPPSASGSPGQSVTFSCTGTSSDIGAFNSV SWYQQHPGKAPKLLIYEITKRPSGVPDRFSGSKSGNTASLTISVL QAEDEADYYCTSYAGSNTLIFGGGTKVTVLRTAAASAHHHHHH KLDYKDHDGDYKDHDIDYKDDDDK |
| 176 | scFv sequence G4_18 | EVQLVESGAEVKKPGASVKVSCKASGYTFTGYYMHWVRQAPG QGLEWMGRINPNSGGTNYAQKFQGRVTMTRDASISTAYMELS RLRSDDTAVYYCARDLDLSSLDYWGRGTLVTVSSGGGGSGGG GSGGGASSYELTQPPSVTESPGQTARITCSGDALAKQYAYWYQ QKPGQAPVLVIYRDSERPSEIPERFSGSSSGTTVTLTISGVQAED EADYYCQSADSSGTYTVFGGGTKLTVLRTAAASAHHHHHHKLD YKDHDGDYKDHDIDYKDDDDK |

(continued)

| SEQ ID NO. | Description | Sequence |
|---|---|---|
| 177 | scFv sequence G4_19 | EVQLVQSGAEVKKPGASVKVSCKASGYTLTSYYMHWVRQAPG QGLEWMGIINPSGGSTSYAQKFQGRVTMRDTSTSTVYMELSS LRSEDTAVYYCARERGYSYGDGMDVWGQGTTVTVSSGGGGS GGGGSGGGASNFMLTQPHSVSESPGKTVTISCTRSSGSIASNY VQWYQQRPGSPPITLIYDDDQRPSGVPHRFSGSIDTSSNPASLT ISGLKTEDEADYYCQSYDSSNHVVFGGGTKLTVLRTAAASAHH HHHHKLDYKDHDGDYKDHDIDYKDDDDK |
| 178 | scFv sequence G4_20 | QVQLVESGAEVKKPGASVKVSCKASGGTFSSYAISWVRQAPG QGLEWMGGIIPIFGTANYAQKFQGRVTITVDKSTRTAYMELSSL RSKDTAVYYCARGNSRSDAFDIWGQGTMVTVSSGGGGSGGG GSGGGASSYELTHPPSVSVSPGQTASITCSGDKLGDKFVSWYH QKPGQSPVLVIYQDSKRPSGIPERFSGSNSGNTATLTISGTRAM DEADYYCQAWDSSTVVFGGGTKLTVLRTAAASAHHHHHHKLD YKDHDGDYKDHDIDYKDDDDK |
| 179 | scFv sequence G4_22 | QVQLVESGGGLVQPGGSLRLSCAASGFTFSTYAMSWVRQAPG KGLEWVSTVSGSGGTTYYADSVKGRFTISRDNSKNTLYLQMNS LRAEDTAVYYCAKDSTAVTDWFDPWGRGTLVTVSSGGGGSGG GGSGGGASDIQMTQSPSSLSASVGDRVTITCQASQDISNYLNW YQQKPGKAPKLLIYAASSLQSGVPSRFSVSGSGTDFTLTISNLQ PEDFATYYCQQSYSIPWTFGQGTKVEIKRTAAASAHHHHHHKL DYKDHDGDYKDHDIDYKDDDDK |
| 180 | scFv sequence G4_23 | EVQLVESGGGVVQPGRSLRLSCAASGFTFSSYGMHWVRQAPG KGLEWVAVIWYDGSNKYYADSVKGRFTISRDNSKNTLYLQMNS LRAEDTAVYYCARGEVAALYYFDYWGQGTLVTVSSGGGGSGG GGSGGGASDIQMTQSPSSLSASVGDRVTITCRASQGISNSLAW YQQKPGKAPKLLLYAASRLESGVPSRFSGSGSGTDYTLTISSLQ PEDFATYYCQQYYSTPRTFGGGTKLEIKRTAAASAHHHHHHKL DYKDHDGDYKDHDIDYKDDDDK |
| 181 | scFv sequence G4_24 | QVQLQQSGPGLVKPSQTLSLTCAISGASVSSNSVAWNWIRQSP SRGLEWLGRTYYRSRWYNDYALSVKSRIIINPDTSKNQFSLQLN SVTPEDTAVYYCARDWSSTRSFDYWGRGTLVTVSSGGGGSGG GGSGGGASDIVMTQSPDSLAVSLGERATINCKSSQSVLYSSNN KNYLAWYQQKPGQPPKLLISWASTRESGVPDRFSGSGSGTDFT LTINSLQSEDVAIYYCQQYYSTPPTFGQGTKLEIKRTAAASAHHH HHHKLDYKDHDGDYKDHDIDYKDDDDK |
| 182 | scFv sequence G4_25 | EVQLVESGAEVKKPGSSVKVSCKASGGTFSSYAISWVRQAPGQ GLEWMGGIIPIFGTANYAQKFQGRVTITADKSTSTAYMELSSLRS EDTAVYYCARSLRDGYNYIGSLGYWGQGTLVTVSSGGGGSGG GGSGGGASQSALTQPRSVSGSPGQSVTISCTGTSSDVGGYNY VSWYQQHPGKAPKLMIYEVSNRPSGVSNRFSGSKSGNTASLTI SGLQAEDEADYYCSSYGSGSVFGTGTKLTVLRTAAASAHHHHH HKLDYKDHDGDYKDHDIDYKDDDDK |
| 183 | scFv sequence G4_26 | QVQLVQSGAEVKKPGSSVKVSCKASGGTFSSYAISWVRQAPG QGLEWMGGIIPIFGTANYAQKFQGRVTITADESTSTAYMELSSL RSEDTAVYYCASSRGSGWFPLGYWGQGTLVTVSSGGGGSGG GGSGGGASQSGLTQPASVSGSPGQSITISCTGTSSDVGSYNLV SWYQQHPGKAPKLMIYEVSKRPSGVSNRFSGSKSGNTASLTIS GLQAEDEADYYCSSFGSGSIFGTGTKLTVLRTAAASAHHHHHH KLDYKDHDGDYKDHDIDYKDDDDK |

(continued)

| SEQ ID NO. | Description | Sequence |
|---|---|---|
| 184 | scFv sequence G4_27 | QVQLVQSGAEVKKPGESLKISCKSSGYSFTSYWIGWVRQMPGK GLEWMGIIYPGDSDTRYSPSFQGQVTFSADESISTAYLQWSSLK ASDTAMYYCARHGAYGDYPDTFDIWGQGTLVTVSSGGGGSGG GGSGGGGASSYELTQDPAVSVALGQTVSITCQGDSLRNFYANW YQQKPGQAPVLVIYGKNNRPSGIPDRFSGSSSGNTASLTITGAQ AEDEADYYCNSRDSSGNHLVFGGGTQLTVLRTAAASAHHHHH HKLDYKDHDGDYKDHDIDYKDDDDK |
| 185 | scFv sequence G4_28 | QVQLVESGGGLVQPGGSLRLSCAASGFTFDDYAMHWVRQAPG KGLEWVSGISAGGGSTNYAGSVKGRFTVSRDTSKNTLYLQMNS LRAEDTAVYYCVKSYVDTAMRYYYYYMDVWGQGTMVTVSSGG GGSGGGGSGGGASSYELTQDPAVSVALGQTVTITCQGDSLRN YYASWYRQKPGQTPVLVVYGKNNRPSGIPDRFSVSASGNTASL TITGAQAEDEGDYYCNSRDSSGVVFGGGTKVTVLRTAAASAHH HHHHKLDYKDHDGDYKDHDIDYKDDDDK |
| 186 | Linker | GGGGSGGGGSGGG |
| 187 | Nucleotide VH sequence G4_1 | GAGGTGCAGCTGTTGGAGTCTGGGGGAGGCGTGGTCCAGC CTGGGAGGCCCCTGAGACTCTCCTGTGCAGCGTCTGGATTC ACCTTCAGTAGCTATAGCATGAACTGGGTCCGCCAGGCTCCA GGGAAGGGGCTGGAGTGGGTCTCATCCATTAGTAGTAGTAG TAGTTACATATACTACGCAGACTCAGTGAAGGGCCGATTCAC CATCTCCAGAGACAATTCCAAGAACACGCTGTATCTGCAAAT GAACAGCCTGAGAGCCGAGGACACGGCCGTATATTACTGTG CGAAAGGACACTGGTACTTCGATCTCTGGGGCCGTGGCACC CTGGTCACCGTCTCGAGT |
| 188 | Nucleotide VH sequence G4_2 | CAGATGCAGCTGGTGCAGTCTGGGGCTGAGGTGAAGAAGCC TGGGGCTACAGTGAAAATCTCCTGCAAGGTTTCTGGATACCC TTTCACCGACTACTATATCCACTGGGTGCAACAGGCCCCTGG AAAAGGGCTTGAGTGGATGGGACTTGTTGATCCTGAGGATG GGCAAAGTAGATCCGCGGAGAGGTTCCAGGGCAGAGTCACC ATAACCGCGGACACGTCTACAGACACAGCCTACATGGAGCT GAGCAGCCTGAGATCTGAGGACACGGCCGTGTATTACTGTG CAACATTCCCAGTGGCTGGATTCTACGGTATGGACGTCTGGG GCCAGGGAACCCTGGTCACCGTCTCGAGT |
| 189 | Nucleotide VH sequence G4_3 | GAGGTGCAGCTGGTGGAGTCCGGGGGAGGCTTGGTCCAGC CGGGGGGGGGTCCCTGAGACTCTCCTGTGCAGCCTCTGGATTC ACCTTCAGTAGCTATAGCATGAACTGGGTCCGCCAGGCTCCA GGGAAGGGGCTGGAGTGGGTCTCATCCATTAGTAGTAGTAG TAGTTACATATACTACGCAGACTCCGTGAAGGGCCGGTTCAC CATCTCCAGAGACAATTCCAAGAACACGCTGTATCTGCAAAT GAACAGCCTGAGAGCCGAGGACACGGCCGTGTATTACTGTG CGAGAGGAGGGTGGCTATATGACTACTGGGGCCAAGGAACC CTGGTCACCGTCTCGAGT |
| 190 | Nucleotide VH sequence G4_4 | CAGGTGCAGCTGGTGGAGTCTGGGGGAGGCTTGGTACAGC CTGGGGGGTCCCTGAGACTCTCCTGTGCAGCCTCTGGATTC ACCTTTAGCAGCTATGCCATGAGCTGGGTCCGCCAGGCTCC AGGGAAGGGGCTGGAGTGGGTCTCAGCTATTAGTGGTAGTG GTGGTAGCACATACTACGCAGACTCCGTGAAGGGCCGGTTC ACCATCTCCAGAGACAATTCCAAAAACACCCTGTATCTGCAA ATGAGCAGCCTGAGAGTCGAAGACACGGCCGTATATTATTGT GCGAAATCGTCGGTGGGCTGGTGGTCTTTTGACTACTGGGG CCAAGGGACAATGGTCACCGTCTCGAGT |

(continued)

| SEQ ID NO. | Description | Sequence |
|---|---|---|
| 191 | Nucleotide VH sequence G4_5 | GAAGTGCAGCTGGTGCAGTCTGGGGGCTGAGGTGAAGAAGCC TGGGTCCTCGGTGAAGGTCTCCTGCAAGGCTTCTGGTTACAC CTTTACCAGCTACGGTATCAGCTGGGTGCGACAGGCCCCTG GACAAGGGCTTGAGTGGATGGGATGGATCGGCGCTTACAAT GGTAACACAAACTATGCACAGAAGCTCCAGGGCAGAGTCAC CATGAGCACAGACACATCCACGAGCACAGCCTACATGGAGC TGAGGAGCCCGAGATCTGACGACACGGCCGTGTATTACTGT GCGAGAGGCGGGACGGGGGGTGACCACGTCTTTGCCTACT GGGGGCAAGGGACCACGGTCACCGTCTCGAGT |
| 192 | Nucleotide VH sequence G4_6 | GAGGTGCAGCTGGTGGAGTCTGGGGGAGGCTTGGTACAGC CTGGGGGGCCCCTGAGACTCTCCTGTGCAGCCTCTGGATTC ACCTTTAGCAGCTATGCCATGAACTGGGTCCGCCAGGCTCCA GGGAAGGGGCTGGAGTGGGTCTCAGCTATTAGTGGTAGTGG TGGTAGCACATACTACGCAGACTCCGTGAAGGGCCGGTTCA CCATCTCCAGAGACAATTCCAAGAACACGCTGTATCTGCAAA TGAACAGCCTGAGAGCCGAGGACACGGCCGTATATTACTGT GCGAAAGCCGACTACGGGGTGGTCTACTACTTTGACTACTG GGGCCAAGGGACAATGGTCACCGTCTCGAGT |
| 193 | Nucleotide VH sequence G4_7 | GAGGTGCAGCTGGTGGAGTCTGGGGGAGGCTGGGTCCAGT CTGGGGGGTCCCTGAGACCCTCCTGTGCAGCCTCTGGATTC ACCTTTAGTCACTATTGGATGAGTTGGGTCCGCCAGGCTCCA GGGAAGGGGCTGGAGTGGGTGGCCAACATAAAGCAAGATG GAAGTATCATATACTATGCAGACTCTGTGAAGGGCCGATTCA CCATCTCCAGGGACAACGCCAAGAACTCAGTGTATCTGCAAA TGAACAGCCTGAGAGCCGAGGACACGGCTGTGTATTACTGT GCGAGAATTGGGTATAGCAGCTCGTCTTTTGACTACTGGGGC CGTGGCACCCTGGTCACCGTCTCGAGT |
| 194 | Nucleotide VH sequence G4_10 | CAGGTGCAGCTGGTGGAGTCTGGGGGAGGCGTGGTCCAGC CTGGGAGGCCCCTGAGACTCTCCTGTGCAGCCTCTGGATTC ACCTTCAGTAGCTATGCTATGCACTGGGTCCGCCAGGCTCCA GGGAAGGGGCTGGAGTGGGTCTCAGCTATTAGTGGTAGTGG TGGTAGCACATACTACGCAGACTCCGTGAAGGGCCGGTTCA CCATCTCCAGAGACAATTCCAAGAACACGCTGTATCTGCAAA TGAACAGCCTGAGAGCCGAGGACACGGCCGTATATTACTGT GCGAAAGATGGGGCCGTGGATTTTTGGCGAAACGGTATGGA CGTCTGGGGCCGTGGCACCCTGGTCACCGTCTCGAGT |
| 195 | Nucleotide VH sequence G4_12 | GAGGTGCAGCTGTTGGAGTCTGGAGGAGGCTTGGTCCAGCC TGGGGGGTCCCTGAGACTCTCCTGTGCAGCCTCTGGGTTCA CCGTCAGTAGCAACTACATGAGCTGGGTCCGCCAGGCTCCA GGGAAGGGGCTGGAGTGGGTCTCAGTTATTTATAGCGGTGG TAGCACATACTACGCAGACTCCGTGAAGGGCCGATTCACCAT CTCCCGACACAATTCCAAGAACACGCTGTATCTTCAAATGAA CAGCCTGAGAGCTGAGGACACGGCCGTGTATTACTGTGCGA GAGTAGCGAACGGTGACTTTCTTGACTACTGGGGCCGTGGC ACCCTGGTCACCGTCTCGAGT |

(continued)

| SEQ ID NO. | Description | Sequence |
|---|---|---|
| 196 | Nucleotide VH sequence G4_13 | CAGGTGCAGCTGGTGGAGTCTGGGGCTGAGGTGAAGAAGC CTGGGGGGTCCCTGAGACTCTCCTGTGCAGCCTCTGGATTC ACCTTCAGTAGCTATAGCATGAACTGGGTCCGCCAGGCTCCA GGGAAGGGGCTGGAGTGGGTCTCATCCATTAGTGGTACTAG TAGTTACATATACTACGCAGACTCTGTGAAGGGCCGATTCAC CATCTCCAGAGACAACGCCAAGAACTCACTGTATCTGCAAAT GAGCAGCCTGAGAGCCGAGGACACGGCTGTTTATTACTGTG CGAGAGGAGGGCTCGGGATGGTCGACCCCTGGGGCCAGGG AACCCTGGTCACCGTCTCGAGT |
| 197 | Nucleotide VH sequence G4_14 | GAAGTGCAGCTGGTGCAGTCCGGAGCAGAGGTGAAAAAGCC CGGGGAGTCTCTGAGGATCTCCTGTAAGGGTTCTGGATACA GCTTTACCAGCTACTGGATCAGCTGGGTGCGCCAGATGCCC GGGAAAGGCCTGGAGTGGATGGGGAGGATTGATCCTAGTGA CTCTTATACCAACTACAGCCCGTCCTTCCCAGGCCACGTCAC CATCTCAGCTGACAAGTCCATCAGCACTGCCTACCTGCAGTG GAGCAGCCTGAAGGCCTCGGACACCGCCATGTATTACTGTG CGGCGGATACAGCTCACGGTATGGACGTCTGGGGCCGTGG CACCCTGGTCACCGTCTCGAGT |
| 198 | Nucleotide VH sequence G4_15 | GAAGTGCAGCTGGTGCAGTCTGAGGCTGAGGTGAAGAAGCC TGGGGCCTCAGTGAAGGTTTCCTGCAAGGCCTCTGGATACA CCTTCACCAGGCATTATATGCACTGGGTGCGACAGGCCCCC GGACAAGGGCTTGAGTGGATGGGACTAATCAACCCTAGTGG TAGTAGCACAGTCTACGCACAGAAGTTCCAGGGCAGAGTCA CCTTGACCAGGGACACGTCCACGAGCACAGACTACATGGAG CTGAGCAGCCTGAGATCTGAGGACACGGCCGTCTATTATTGT GCGAGAGATAATAGTCACCTCGACCAGGTTTGGTGGTTCGAC CCCTGGGGCCAGGGCACCCTGGTCACCGTCTCGAGT |
| 199 | Nucleotide VH sequence G4_16 | GAGGTGCAGCTGTTGGAGTCTGGAGCTGAGGTGAAGAAGCC TGGGGCCTCAGTGAAGGTCTCCTGCAAGGCTTCTGGTTACA CCTTTACCAGCTATGGTATCAGCTGGGTGCGACAGGCCCCT GGACAAGGGCTTGAGTGGATGGGATGGATCAGCGCTTACAA TGGTAACACAAACTATGCACAGAAGCTCCAGGGCAGAGTCAC CATGACCACAGACACATCCACGAGCACAGCCTACATGGAGC TGAGGAGCCTGAGATCTGACGACACGGCCGTGTATTACTGT GCGAGAGACTACGGTGACTTCTACGGTATGGACGTCTGGGG CCAAGGAACCCTGGTCACCGTCTCGAGT |
| 200 | Nucleotide VH sequence G4_18 | GAGGTGCAGCTGGTGGAGTCTGGGGCTGAGGTGAAGAAGC CTGGGGCCTCAGTGAAGGTCTCCTGCAAGGCTTCTGGATAC ACCTTCACCGGCTACTATATGCACTGGGTGCGACAGGCCCC TGGACAAGGGCTTGAGTGGATGGGACGGATCAACCCTAACA GTGGTGGCACAAACTATGCACAGAAGTTTCAGGGCAGGGTC ACCATGACCAGGGACGCGTCCATCAGCACAGCCTACATGGA GCTGAGCAGGCTGAGATCTGACGACACGGCCGTGTATTACT GTGCGAGAGATCTTGATCTATCCTCCCTTGACTACTGGGGCC GTGGCACCCTGGTCACCGTCTCGAGT |

(continued)

| SEQ ID NO. | Description | Sequence |
|---|---|---|
| 201 | Nucleotide VH sequence G4_19 | GAAGTGCAGCTGGTGCAGTCTGGGGCTGAGGTGAAGAAGCC TGGGGCCTCAGTGAAGGTTTCCTGCAAGGCATCTGGATACA CCCTCACCAGCTACTATATGCACTGGGTGCGACAGGCCCCT GGACAAGGGCTTGAGTGGATGGGAATAATCAACCCTAGTGG TGGTAGCACAAGCTACGCACAGAAGTTCCAGGGCAGAGTCA CCATGACCAGGGACACGTCCACGAGCACAGTCTACATGGAG CTGAGCAGCCTGAGATCTGAGGACACGGCCGTGTATTACTG TGCGAGAGAGCGTGGATACAGCTATGGTGACGGTATGGACG TCTGGGGGCAAGGGACCACGGTCACCGTCTCGAGT |
| 202 | Nucleotide VH sequence G4_20 | CAGGTGCAGCTGGTGGAGTCTGGAGCTGAGGTGAAGAAGCC TGGGGCCTCGGTGAAGGTCTCCTGCAAGGCTTCTGGAGGCA CCTTCAGCAGCTATGCTATCAGCTGGGTGCGACAGGCCCCT GGACAAGGGCTTGAGTGGATGGGAGGGATCATCCCTATCTT TGGTACAGCAAACTACGCACAGAAGTTCCAGGGCAGAGTCA CGATTACCGTGGACAAATCCACGCGCACAGCCTACATGGAG CTGAGCAGCCTGAGATCTAAGGACACGGCCGTGTATTACTGT GCGAGGGGGAATAGCAGAAGTGATGCTTTTGATATCTGGGG CCAAGGGACAATGGTCACCGTCTCGAGT |
| 203 | Nucleotide VH sequence G4_22 | CAGGTGCAGTTGGTGGAGTCTGGGGGAGGCTTGGTACAGCC TGGGGGGTCCCTGAGACTCTCCTGTGCAGCCTCTGGATTCA CCTTTAGCACCTATGCCATGAGCTGGGTCCGCCAGGCTCCA GGGAAGGGGCTGGAGTGGGTCTCAACTGTTAGTGGTAGTGG TGGTACCACATACTACGCAGACTCCGTGAAGGGCCGGTTCA CCATCTCCAGAGACAATTCCAAGAACACGCTGTATCTGCAAA TGAACAGCCTGAGAGCCGAAGACACGGCCGTATATTACTGT GCGAAAGATTCAACGGCGGTGACTGACTGGTTCGACCCCTG GGGCCGTGGCACCCTGGTCACCGTCTCGAGT |
| 204 | Nucleotide VH sequence G4_23 | GAGGTGCAGCTGGTGGAGTCTGGGGGAGGCGTGGTCCAGC CTGGGAGGTCCCTGAGACTCTCCTGTGCAGCGTCTGGATTC ACCTTCAGTAGCTATGGCATGCACTGGGTCCGCCAGGCTCC AGGCAAGGGGCTGGAGTGGGTGGCAGTTATATGGTATGATG GAAGTAATAAATACTATGCAGACTCCGTGAAGGGCCGATTCA CCATCTCCAGAGACAATTCCAAGAACACGCTGTATCTGCAAA TGAACAGCCTGAGAGCCGAGGACACGGCTGTGTATTACTGT GCGAGAGGGGAAGTGGCTGCCTTGTACTACTTTGACTACTG GGGCCAGGGAACCCTGGTCACCGTCTCGAGT |
| 205 | Nucleotide VH sequence G4_24 | CAGGTACAGCTGCAGCAGTCAGGTCCAGGACTGGTGAAGCC CTCGCAGACCCTCTCACTCACCTGTGCCATCTCCGGGGCCA GTGTCTCTAGCAACAGTGTTGCTTGGAACTGGATCAGGCAGT CCCCATCGAGAGGCCTTGAGTGGCTGGGGAGGACATACTAC AGGTCCAGGTGGTATAATGATTATGCATTATCTGTGAAAAGTC GAATAATCATCAACCCAGACACATCCAAGAACCAGTTCTCCC TGCAGCTGAACTCTGTGACCCCCGAGGACACGGCTGTGTAT TACTGTGCAAGAGATTGGAGCAGCACCCGATCCTTTGACTAC TGGGGCCGTGGCACCCTGGTCACCGTCTCGAGT |

(continued)

| SEQ ID NO. | Description | Sequence |
|---|---|---|
| 206 | Nucleotide VH sequence G4_25 | GAGGTGCAGCTGGTGGAGTCTGGGGGCTGAGGTGAAGAAGCCTGGGTCCTCGGTGAAGGTCTCCTGCAAGGCTTCTGGAGGCACCTTCAGCAGCTATGCTATCAGCTGGGTGCGACAGGCCCCTGGACAAGGGCTTGAGTGGATGGGAGGGATCATCCCTATCTTTGGTACAGCAAACTACGCACAGAAGTTCCAGGGCAGAGTCACGATTACCGCGGACAAATCCACGAGCACAGCCTACATGGAGCTGAGCAGCCTGAGATCTGAGGACACGGCCGTGTATTACTGTGCGAGATCTCTTAGAGATGGCTACAATTACATCGGAAGTTTAGGCTACTGGGGCCAGGGCACCCTGGTCACCGTCTCGAGT |
| 207 | Nucleotide VH sequence G4_26 | CAGGTCCAGCTTGTGCAGTCTGGGGGCTGAGGTGAAGAAGCCTGGATCCTCGGTGAAGGTCTCCTGCAAGGCTTCTGGAGGCACCTTCAGCAGCTATGCTATCAGCTGGGTGCGACAGGCCCCTGGACAAGGGCTTGAGTGGATGGGAGGGATCATCCCTATCTTTGGTACAGCAAACTACGCACAGAAGTTCCAGGGCAGAGTCACGATTACCGCGGACGAATCCACGAGCACAGCTTACATGGAGCTGAGCAGCCTGAGATCTGAAGACACGGCTGTGTATTACTGTGCGAGCTCCCGGGGGCAGTGGCTGGTTTCCTTTGGGTTACTGGGGCCAAGGAACCCTGGTCACCGTCTCGAGT |
| 208 | Nucleotide VH sequence G4_27 | CAGGTCCAGCTGGTACAGTCTGGAGCAGAGGTGAAAAAGCCCGGGGAGTCTCTGAAGATCTCCTGTAAGAGTTCTGGATACAGCTTTACCAGCTACTGGATCGGCTGGGTGCGCCAGATGCCCGGGAAAGGCCTGGAGTGGATGGGGATCATCTATCCTGGTGACTCTGATACCAGATACAGCCCGTCCTTCCAAGGCCAGGTCACCTTCTCAGCCGACGAGTCCATCAGTACCGCCTACCTGCAGTGGAGCAGCCTGAAGGCCTCGGACACCGCCATGTATTACTGTGCGAGACATGGCGCCTACGGTGACTACCCGGATACTTTTGATATCTGGGGCCAGGGCACCCTGGTCACCGTCTCGAGT |
| 209 | Nucleotide VH sequence G4_28 | CAGGTGCAGCTGGTGGAGTCTGGGGGAGGCTTGGTACAGCCTGGGGGGTCCCTGAGACTCTCCTGTGCAGCCTCTGGATTCACCTTTGATGATTATGCCATGCACTGGGTCCGGCAAGCTCCAGGGAAGGGGCTGGAGTGGGTCTCAGGTATTAGTGCTGGTGGTGGTAGCACAAACTACGCAGGCTCCGTGAAGGGCCGGTTCACCGTCTCCAGGGACACGTCCAAGAACACACTTTATCTGCAAATGAACAGCCTGAGAGCCGAGGACACGGCCGTGTATTACTGTGTGAAGTCCTACGTGGATACAGCTATGCGCTACTACTACTACATGGACGTCTGGGGCCAAGGGACAATGGTCACCGTCTCGAGT |
| 210 | Nucleotide VL sequence G4_1 | GACATCCAGATGACCCAGTCTCCATCCTCCCTGTCTGCATCTGTAGGAGACAGAGTCACCATCACTTGCCAGGCGAGTCAGGACATTAGCAACTATTTAAATTGGTATCAGCAGAAACCAGGGAAAGCCCCTAAGCTCCTGATCTACGATGCATCCAATTTGGAAACAGGGGTCCCATCAAGGTTCAGTGGAAGTGGATCTGGGACAGATTTCACTCTCACCATCAGCAGTCTGCAACCTGAAGATTTTGCAACTTACTACTGTCAACAGAGTTACAGTACCCCCGTCACTTTCGGCCCTGGGACCAAGGTGGAAATCAAA |

(continued)

| SEQ ID NO. | Description | Sequence |
|---|---|---|
| 211 | Nucleotide VL sequence G4_2 | GACATCCAGATGACCCAGTCTCCATCCTCCCTGTCTGCATCT GTAGGAGACAGAGTCACCATCACTTGCCAGGCGAGTCAGGA CATTAGCAACTATTTAAATTGGTATCAGCAGAAACCAGGGAAA GCCCCTAAGCTCCTGATCTATGCTGCATCCAGTTTGCAAAGT GGGGTCCCATCAAGGTTCAGTGGCAGTGGATCTGGGACAGA TTTCACTCTCACCATCAGCGGCCTGCAGCCTGAAGATTTTGC AACTTACTACTGTCTAGAAGATTACAACTACCTGTGGACGTTC GGCCAAGGGACCAAGCTGGAGATCAAA |
| 212 | Nucleotide VL sequence G4_3 | GACATCCAGATGACCCAGTCTCCATCCTCCCTGTCTGCATCT GTAGGAGACAGAGTCACCATCACTTGCCAGGCGAGTCAGGA CATTAGCAACTATTTAAATTGGTATCAGCAGAAACCAGGGAAA GCCCCTAAGCTCCTGATCTACGATGCATCCAATTTGGAAACA GGGGTCCCATCAAGGTTCAGTGGAAGTGGGTCTGGGACAGA TTTCACTCTCACCATCAGCAGTCTGCAACCTGAAGATTTTGCA ACTTACTTCTGTCAACAGAGTTACAGTACCCCCCAGACGTTC GGCCAAGGGACCAAAGTGGATATCAAA |
| 213 | Nucleotide VL sequence G4_4 | GATATTGTGATGACCCAGTCTCCAGACTCCCTGGCTGTGTCT CTGGGCGAGAGGGCCACCATCAACTGCAAGTCCAGCCAGAG TGTTTTGTCCAGCTCCAACAATAACAACTACTTAGCTTGGTAC CAACAGAGACCAGGACAGCCTCCTAAGCTGCTCTTTTACTGG GCATCTACCCGGGAATCGGGGGGTCCCTGACCGATTCAGTGG CAGCGGGTCTGGAACATCTTTCACTCTCACCATCACCAGCCT GCAGGCTGAAGATGTGGCGGTTTATTACTGTCAGCAATATTA TTCCACTCCTCTCACTTTCGGCGGAGGGACCAAGCTGGAGAT CAAA |
| 214 | Nucleotide VL sequence G4_5 | GACATCCAGATGACCCAGTCTCCATCCTCCCTGTCTGCATCT GTAGGAGACAGAGTCACCATCACTTGCCGGGCAAGTCAGAG CATTAGCAGCTATTTAAATTGGTATCAGCAGAAACCAGGGAA AGCCCCTAAGCTCCTGATCTATGCTGCATCCAGTTTGCAAAG TGGGGTCCCATCAAGGTTCAGCGGCAGTGGATCTGGGACAG ATTTCACTCTCACTATCAGCAGCCTGCAGCCTGAAGATTTTG CAACTTACTACTGTCAACAGAGTTACAGTACCCCCTACACTTT TGGCCAGGGGACCAAGGTGGAAATCAAA |
| 215 | Nucleotide VL sequence G4_6 | GACATCCAGATGACCCAGTCTCCATCCTCCCTGTCTGCATCT GTAGGAGACAGAGTCACCATCACTTGCCGGGCAAGTCAGAG CATTAGCACCTATTTAAATTGGTATCAGCAGAAACCAGGGAA AGCCCCTAAGCTCCTGATCTATGCTGCATCCAGTTTGCAGAG TGGGGTCCCATCAAGGTTCAGTGGCAGTGGATCTGGGACAG ATTTCACTCTCACCATCAGCAGTCCGCAACCTGAAGATTTTG CAACTTACTACTGTCAACAGAGTTACAGTACCCCGTACACTTT TGGCCAGGGGACCAAGGTGGAAATCAAA |
| 216 | Nucleotide VL sequence G4_7 | GATATTGTGATGACGCAGTCTCCACTCTCCCTGCCCGTCACC CCTGGAGAGCCGGCCTCCATCTCCTGCAGGTCCAGTCAGAG CCTCCTGCATAGTAATAGATTCAACTATTTGGATTGGTACCTG CAGAAGCCAGGGCAGTCTCCACAGCTCCTGATCTATTTGGGT TCTAATCGGGCCTCCGGGGGTCCCTGACAGGTTCAGTGGCAG TGGATCTGGCACAGATTTTACACTGAAAATCAGCAGAGTGGA GGCTGAGGATGTTGGGGTTTATTACTGCATGCAAGGTCTACA AACTCCGTACACTTTTGGCCAGGGGACCAAAGTGGATATCAA A |

(continued)

| SEQ ID NO. | Description | Sequence |
|---|---|---|
| 217 | Nucleotide VL sequence G4_10 | GATATTGTGATGACGCAGCCTCCACTCTCCCTGCCCGTCACCCTTGGACATCCGGCCTCCATCTCCTGCAAGTCTAGTCAAAGCCTCGAATATAGTGATGGAAACACCTACTTGAATTGGTTTCAGCAGAGGCCAGGCCAATCTCCAAGGCGCCTCATTTATAAGGTTTCTAACCGGGACTCTGGGGCCCCCGACAGATTCAGCGGGAGTGGGTCAGGCACTGATTTCACACTGGAAATCAGCAGGGTGGAGGCTGAGGATGTTGGAGTTTATTACTGTATGCAAGGTACACTCTGGCCTCCCACGTTCGGCCAAGGGACCAAAGTGGATATCAAA |
| 218 | Nucleotide VL sequence G4_12 | CAGTCTGTGCTGACTCAGCCTGCCTCCGTGTCTGGGTCTCCTGGACAGTCGATCACCATCTCCTGCACTGGAACCAGCAGTGACGTTGGTGGTTATAACTTTGTCTCCTGGTACCAACAACACCCAGGCAAAGCCCCCAAACTCATGATTTATGAGGTCACTAATCGGCCCTCAGGGGTCCCTGATCGGTTCTCTGGCTCCAAGTCTGGCAACACGGCCTCCCTGACCATCTCTGGGCTCCAGGCTGAGGACGAGGCTGATTATTACTGCAGCTCACATGCAAGCCCCAGGGTCTTCGGAACTGGGACCAAGGTCACCGTCCTA |
| 219 | Nucleotide VL sequence G4_13 | AATTTTATGCTGACTCAGCCCCACTCTGTGTCGGAGTCTCCGGGGAAGACGGTAACCATCTCCTGCACCCGCAGCAGTGGCAGCATTGCCAGCAACTATGTGCAGTGGTACCAGCAGCGCCCGGGCAGTTCCCCCAGCACTGTGATCTATGAGGATAACCAAAGACCCTCAGGGGTCCCTGATCGGTTCTCTGGCTCCATCGACAGCTCCTCCAACTCTGCCTCCCTCACCATCTCTGGACTGAGGACTGAGGACGAGGCTGACTACTACTGTCAGTCTTATGATAGCAGCATTTATGTGGTATTCGGCGGAGGGACCAAGCTGACCGTCCTA |
| 220 | Nucleotide VL sequence G4_14 | AATTTTATGCTGACTCAGCCCCACTCTGTGTCGGAGTCTCCGGGGAAGACGGTAACCATATCCTGCACCCGCAGCCGTGGCAGCATTGCCGGCAACTATGTGCACTGGTACCAGCAGCGCCCAGGGCGTGCCCCCACCACTGTGATCTATCGGGATAAGGAAAGACCCTCTGGGGTCCCTGATCGAATCTCTGGCTCCATCGACAGCTCCTCCAACTCTGCCTCCCTCACCATCTCTGGACTGAAGACTGAGGACGAGGCTGATTACTATTGTCAGTCTTATGATAGCAGCACCCATGTGGTATTCGGCGGAGGGACCAAGCTGACCGTCCTA |
| 221 | Nucleotide VL sequence G4_15 | CAGTCTGCGCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCATGATTTATGACGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAGCTCGTATGGAAGCGGCAGCGTCTTCGGAACTGGGACCAAGCTGACCGTCCTA |
| 222 | Nucleotide VL sequence G4_16 | CAGTCTGTGCTGACTCAGCCTCCCTCCGCGTCCGGGTCTCCTGGACAGTCAGTCACCTTCTCCTGCACTGGAACCAGCAGTGACATTGGTGCTTTTAACTCTGTCTCTTGGTACCAACAGCACCCAGGCAAAGCCCCCAAACTCCTAATTTATGAGATCACTAAGCGGCCCTCAGGGGTCCCTGATCGCTTCTCTGGCTCCAAGTCTGGCAACACGGCCTCCCTGACCATCTCTGTGCTCCAGGCTGAAGATGAGGCTGATTATTACTGCACCTCATATGCAGGCAGCAACACTTTGATCTTCGGCGGAGGGACCAAGGTCACCGTCCTA |

(continued)

| SEQ ID NO. | Description | Sequence |
|---|---|---|
| 223 | Nucleotide VL sequence G4_18 | TCCTATGAGCTGACACAGCCACCCTCGGTGACAGAGTCCCCAGGACAGACGGCCAGGATCACCTGCTCTGGAGATGCATTGGCAAAGCAATATGCTTATTGGTACCAGCAGAAGCCAGGCCAGGCCCCTGTGTTGGTGATATATAGAGACAGTGAGAGGCCTTCAGAGATCCCTGAGCGATTCTCTGGCTCCAGCTCAGGGACAACAGTCACGTTGACCATCAGTGGAGTCCAGGCAGAAGACGAGGCTGACTATTACTGTCAATCAGCAGACAGCAGTGGTACTTATACAGTATTTGGCGGAGGGACCAAGCTGACCGTCCTA |
| 224 | Nucleotide VL sequence G4_19 | AATTTTATGCTGACTCAGCCCCACTCTGTGTCGGAGTCTCCGGGGAAGACGGTCACCATCTCCTGCACCCGCAGCAGTGGCAGCATTGCCAGCAACTATGTACAGTGGTACCAGCAGCGCCCGGGCAGTCCCCCCATCACTTTGATATATGATGATGACCAAAGACCCTCTGGGGTCCCTCATCGGTTCTCTGGCTCCATCGACACCTCATCCAACCCTGCCTCCCTCACCATCTCTGGACTGAAGACTGAGGACGAGGCTGACTACTACTGTCAGTCTTATGATAGCAGCAATCATGTGGTATTCGGCGGAGGGACCAAGCTGACCGTCCTA |
| 225 | Nucleotide VL sequence G4_20 | TCCTATGAGCTGACTCATCCACCCTCAGTGTCCGTGTCCCCAGGACAGACAGCCAGCATCACCTGCTCTGGAGATAAATTGGGGGATAAGTTTGTTTCCTGGTATCACCAAAAGCCAGGCCAGTCCCCTGTGCTGGTCATCTATCAAGATAGCAAGCGGCCCTCAGGGATCCCTGAGCGCTTCTCAGGCTCCAATTCTGGGAACACAGCCACTCTGACCATCAGCGGGACCCGGGCTATGGATGAGGCTGACTATTACTGTCAGGCGTGGGACAGCAGCACTGTGGTATTCGGCGGAGGGACCAAGCTGACCGTCCTA |
| 226 | Nucleotide VL sequence G4_22 | GACATCCAGATGACCCAGTCTCCATCCTCCCTGTCTGCATCTGTAGGAGACAGAGTCACCATCACTTGCCAGGCGAGTCAGGACATTAGCAACTATTTAAATTGGTATCAGCAGAAACCAGGGAAAGCCCCTAAGCTCCTGATCTATGCTGCATCCAGTCTGCAAAGTGGGGTCCCATCAAGGTTCAGCGTCAGTGGATCTGGGACAGATTTCACTCTCACCATCAGCAACCTGCAGCCTGAAGATTTTGCAACTTATTACTGTCAACAGAGTTACAGTATCCCGTGGACGTTCGGCCAAGGGACCAAGGTGGAGATCAAA |
| 227 | Nucleotide VL sequence G4_23 | GACATCCAGATGACCCAGTCTCCATCCTCCCTGTCTGCATCTGTAGGAGACAGAGTCACCATCACTTGCCGGGCGAGTCAGGGCATTAGCAATTCTTTAGCCTGGTATCAGCAGAAACCAGGGAAAGCCCCTAAGCTCCTGCTCTATGCTGCGTCCAGATTGGAAAGTGGGGTCCCATCCAGGTTTAGTGGCAGTGGATCTGGGACGGATTACACCCTCACCATCAGCAGCCTGCAGCCTGAAGATTTTGCAACTTATTACTGTCAACAGTATTATAGTACCCCTCGCACTTTCGGCGGAGGGACCAAGCTGGAGATCAAA |
| 228 | Nucleotide VL sequence G4_24 | GATATTGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAAGAACTACTTAGCTTGGTACCAGCAGAAACCAGGACAGCCTCCTAAGTTGTTGATTTCCTGGGCTTCTACCCGGGAATCTGGGGTCCCTGACCGATTCAGTGGCAGCGGGTCTGGGACAGATTTCACTCTCACCATCAACAGCCTACAGTCTGAAGATGTGGCAATTTATTACTGTCAGCAATATTATTCTACCCCTCCGACGTTCGGCCAGGGGACCAAGCTGGAGATCAAA |

(continued)

| SEQ ID NO. | Description | Sequence |
|---|---|---|
| 229 | Nucleotide VL sequence G4_25 | CAGTCTGCGCTGACTCAGCCTCGCTCAGTGTCCGGGTCTCCTGGACAGTCAGTCACCATCTCCTGCACTGGAACCAGCAGTGATGTTGGTGGTTATAACTATGTCTCCTGGTACCAACAGCACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAGCTCGTATGGAAGCGGCAGCGTCTTCGGAACTGGGACCAAGCTGACCGTCCTA |
| 230 | Nucleotide VL sequence G4_26 | CAGTCTGGGCTGACTCAGCCTGCCTCCGTGTCTGGGTCTCCTGGACAGTCGATCACCATCTCCTGCACTGGAACCAGCAGTGATGTTGGGAGTTATAACCTTGTCTCCTGGTACCAACAGCACCCAGGCAAAGCCCCCAAACTCATGATTTATGAGGTCAGTAAGCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAGCTCGTTTGGAAGCGGCAGCATCTTCGGAACTGGGACCAAGCTGACCGTCCTA |
| 231 | Nucleotide VL sequence G4_27 | TCCTATGAGCTGACTCAGGACCCAGCTGTGTCTGTGGCCCTGGGACAGACAGTCAGTATCACATGCCAAGGAGACAGCCTCAGAAACTTTTATGCAAACTGGTACCAGCAAAAGCCAGGACAGGCCCCTGTACTTGTCATCTATGGTAAAAACAACCGGCCCTCAGGGATCCCAGACCGATTCTCTGGCTCCAGCTCAGGAAACACAGCTTCCTTGACCATCACTGGGGCTCAGGCGGAAGATGAGGCTGACTATTACTGTAACTCCCGGGACAGCAGTGGTAACCATCTGGTATTCGGCGGAGGGACCCAGCTCACCGTCCTA |
| 232 | Nucleotide VL sequence G4_28 | TCCTATGAGCTGACTCAGGACCCTGCTGTGTCTGTGGCCTTGGGACAGACAGTCACGATCACATGCCAAGGAGACAGCCTCAGAAACTATTATGCAAGCTGGTACCGGCAGAAGCCAGGACAGACCCCTGTACTTGTCGTCTATGGTAAAAACAACCGGCCCTCAGGGATCCCAGACCGATTCTCTGTCTCCGCCTCAGGTAACACAGCTTCCTTGACCATCACTGGGGCTCAGGCGGAAGATGAGGGTGACTATTACTGTAACTCCCGGGACAGCAGTGGTGTGGTTTTCGGCGGAGGGACCAAGGTCACCGTCCTA |
| 233 | G4_1 IgG1 antibody sequence | ASDIQMTQSPSSLSASVGDRVTITCQASQDISNYLNWYQQKPGKAPKLLIYDASNLETGVPSRFSGSGSGTDFTLTISSLQPEDFATYYCQQSYSTPVTFGPGTKVEIKRTAAAPSVFIFPPSDEQLKSGTASVVCLLNNFYPREAKVQWKVDNALQSGNSQESVTEQDSKDSTYSLSSTLTLSKADYEKHKLYACEVTHQGLSSPVTKSFNRGECEVQLLESGGGVVQPGRPLRLSCAASGFTFSSYSMNWVRQAPGKGLEWVSSISSSSSYIYYADSVKGRFTISRDNSKNTLYLQMNSLRAEDTAVYYCAKGHWYFDLWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO. | Description | Sequence |
|---|---|---|
| 234 | G4_2 IgG1 antibody sequence | ASDIQMTQSPSSLSASVGDRVTITCQASQDISNYLNWYQQKPG KAPKLLIYAASSLQSGVPSRFSGSGSGTDFTLTISGLQPEDFATY YCLEDYNYLWTFGQGTKLEIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGECQ MQLVQSGAEVKKPGATVKISCKVSGYPFTDYYIHWVQQAPGKG LEWMGLVDPEDGQSRSAERFQGRVTITADTSTDTAYMELSSLR SEDTAVYYCATFPVAGFYGMDVWGQGTLVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQPR EPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 235 | G4_3 IgG1 antibody sequence | ASDIQMTQSPSSLSASVGDRVTITCQASQDISNYLNWYQQKPG KAPKLLIYDASNLETGVPSRFSGSGSGTDFTLTISSLQPEDFATY FCQQSYSTPQTFGQGTKVDIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGECEV QLVESGGGLVQPGGSLRLSCAASGFTFSSYSMNWVRQAPGKG LEWVSSISSSSSYIYYADSVKGRFTISRDNSKNTLYLQMNSLRAE DTAVYYCARGGWLYDYWGQGTLVTVSSASTKGPSVFPLAPSS KSTSGGTAALGCLVKDYFPEPVTVSWNSGALTSGVHTFPAVLQ SSGLYSLSSVVTVPSSSLGTQTYICNVNHKPSNTKVDKKVEPKS CDKTHTCPPCPAPELLGGPSVFLFPPKPKDTLMISRTPEVTCVV VDVSHEDPEVKFNWYVDGVEVHNAKTKPREEQYNSTYRVVSV LTVLHQDWLNGKEYKCKVSNKALPAPIEKTISKAKGQPREPQVY TLPPSRDELTKNQVSLTCLVKGFYPSDIAVEWESNGQPENNYKT TPPVLDSDGSFFLYSKLTVDKSRWQQGNVFSCSVMHEALHNHY TQKSLSLSPGK |
| 236 | G4_4 IgG1 antibody sequence | ASDIVMTQSPDSLAVSLGERATINCKSSQSVLSSSNNNNYLAWY QQRPGQPPKLLFYWASTRESGVPDRFSGSGSGTSFTLTITSLQ AEDVAVYYCQQYYSTPLTFGGGTKLEIKRTAAAPSVFIFPPSDE QLKSGTASVVCLLNNFYPREAKVQWKVDNALQSGNSQESVTE QDSKDSTYSLSSTLTLSKADYEKHKLYACEVTHQGLSSPVTKSF NRGECQVQLVESGGGLVQPGGSLRLSCAASGFTFSSYAMSWV RQAPGKGLEWVSAISGSGGSTYYADSVKGRFTISRDNSKNTLY LQMSSLRVEDTAVYYCAKSSVGWWSFDYWGQGTMVTVSSAS TKGPSVFPLAPSSKSTSGGTAALGCLVKDYFPEPVTVSWNSGA LTSGVHTFPAVLQSSGLYSLSSVVTVPSSSLGTQTYICNVNHKP SNTKVDKKVEPKSCDKTHTCPPCPAPELLGGPSVFLFPPKPKDT LMISRTPEVTCVVVDVSHEDPEVKFNWYVDGVEVHNAKTKPRE EQYNSTYRVVSVLTVLHQDWLNGKEYKCKVSNKALPAPIEKTIS KAKGQPREPQVYTLPPSRDELTKNQVSLTCLVKGFYPSDIAVE WESNGQPENNYKTTPPVLDSDGSFFLYSKLTVDKSRWQQGNV FSCSVMHEALHNHYTQKSLSLSPGK |

(continued)

| SEQ ID NO. | Description | Sequence |
|---|---|---|
| 237 | G4_5 IgG1 antibody sequence | ASDIQMTQSPSSLSASVGDRVTITCRASQSISSYLNWYQQKPGK APKLLIYAASSLQSGVPSRFSGSGSGTDFTLTISSLQPEDFATYY CQQSYSTPYTFGQGTKVEIKRTAAAPSVFIFPPSDEQLKSGTAS VVCLLNNFYPREAKVQWKVDNALQSGNSQESVTEQDSKDSTY SLSSTLTLSKADYEKHKLYACEVTHQGLSSPVTKSFNRGECEVQ LVQSGAEVKKPGSSVKVSCKASGYTFTSYGISWVRQAPGQGLE WMGWIGAYNGNTNYAQKLQGRVTMSTDTSTSTAYMELRSPRS DDTAVYYCARGGTGGDHVFAYWGQGTTVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQPR EPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 238 | G4_6 IgG1 antibody sequence | ASDIQMTQSPSSLSASVGDRVTITCRASQSISTYLNWYQQKPGK APKLLIYAASSLQSGVPSRFSGSGSGTDFTLTISSPQPEDFATYY CQQSYSTPYTFGQGTKVEIKRTAAAPSVFIFPPSDEQLKSGTAS VVCLLNNFYPREAKVQWKVDNALQSGNSQESVTEQDSKDSTY SLSSTLTLSKADYEKHKLYACEVTHQGLSSPVTKSFNRGECEVQ LVESGGGLVQPGGPLRLSCAASGFTFSSYAMNWVRQAPGKGL EWVSAISGSGGSTYYADSVKGRFTISRDNSKNTLYLQMNSLRA EDTAVYYCAKADYGVVYYFDYWGQGTMVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQPR EPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 239 | G4_7 IgG1 antibody sequence | ASDIVMTQSPLSLPVTPGEPASISCRSSQSLLHSNRFNYLDWYL QKPGQSPQLLIYLGSNRASGVPDRFSGSGSGTDFTLKISRVEAE DVGVYYCMQGLQTPYTFGQGTKVDIKRTAAAPSVFIFPPSDEQL KSGTASVVCLLNNFYPREAKVQWKVDNALQSGNSQESVTEQD SKDSTYSLSSTLTLSKADYEKHKLYACEVTHQGLSSPVTKSFNR GECEVQLVESGGGWVQSGGSLRPSCAASGFTFSHYWMSWVR QAPGKGLEWVANIKQDGSIIYYADSVKGRFTISRDNAKNSVYLQ MNSLRAEDTAVYYCARIGYSSSSFDYWGRGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSRDELTKNQVSLTCLVKGFYPSDIAVEWES NGQPENNYKTTPPVLDSDGSFFLYSKLTVDKSRWQQGNVFSC SVMHEALHNHYTQKSLSLSPGK |

(continued)

| SEQ ID NO. | Description | Sequence |
|---|---|---|
| 240 | G4_10 IgG1 antibody sequence | ASDIVMTQPPLSLPVTLGHPASISCKSSQSLEYSDGNTYLNWFQ QRPGQSPRRLIYKVSNRDSGAPDRFSGSGSGTDFTLEISRVEA EDVGVYYCMQGTLWPPTFGQGTKVDIKRTAAAPSVFIFPPSDE QLKSGTASVVCLLNNFYPREAKVQWKVDNALQSGNSQESVTE QDSKDSTYSLSSTLTLSKADYEKHKLYACEVTHQGLSSPVTKSF NRGECQVQLVESGGGVVQPGRPLRLSCAASGFTFSSYAMHWV RQAPGKGLEWVSAISGSGGSTYYADSVKGRFTISRDNSKNTLY LQMNSLRAEDTAVYYCAKDGAVDFWRNGMDVWGRGTLVTVS SASTKGPSVFPLAPSSKSTSGGTAALGCLVKDYFPEPVTVSWN SGALTSGVHTFPAVLQSSGLYSLSSVVTVPSSSLGTQTYICNVN HKPSNTKVDKKVEPKSCDKTHTCPPCPAPELLGGPSVFLFPPKP KDTLMISRTPEVTCVVVDVSHEDPEVKFNWYVDGVEVHNAKTK PREEQYNSTYRVVSVLTVLHQDWLNGKEYKCKVSNKALPAPIE KTISKAKGQPREPQVYTLPPSRDELTKNQVSLTCLVKGFYPSDIA VEWESNGQPENNYKTTPPVLDSDGSFFLYSKLTVDKSRWQQG NVFSCSVMHEALHNHYTQKSLSLSPGK |
| 241 | G4_12 IgG1 antibody sequence | ASQSVLTQPASVSGSPGQSITISCTGTSSDVGGYNFVSWYQQH PGKAPKLMIYEVTNRPSGVPDRFSGSKSGNTASLTISGLQAEDE ADYYCSSHASPRVFGTGTKVTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECSE VQLLESGGGLVQPGGSLRLSCAASGFTVSSNYMSWVRQAPGK GLEWVSVIYSGGSTYYADSVKGRFTISRHNSKNTLYLQMNSLRA EDTAVYYCARVANGDFLDYWGRGTLVTVSSASTKGPSVFPLAP SSKSTSGGTAALGCLVKDYFPEPVTVSWNSGALTSGVHTFPAV LQSSGLYSLSSVVTVPSSSLGTQTYICNVNHKPSNTKVDKKVEP KSCDKTHTCPPCPAPELLGGPSVFLFPPKPKDTLMISRTPEVTC VVVDVSHEDPEVKFNWYVDGVEVHNAKTKPREEQYNSTYRVV SVLTVLHQDWLNGKEYKCKVSNKALPAPIEKTISKAKGQPREPQ VYTLPPSRDELTKNQVSLTCLVKGFYPSDIAVEWESNGQPENN YKTTPPVLDSDGSFFLYSKLTVDKSRWQQGNVFSCSVMHEALH NHYTQKSLSLSPGK |
| 242 | G4_13 IgG1 antibody sequence | ASNFMLTQPHSVSESPGKTVTISCTRSSGSIASNYVQWYQQRP GSSPSTVIYEDNQRPSGVPDRFSGSIDSSSNSASLTISGLRTED EADYYCQSYDSSIYVVFGGGTKLTVLGQPAAAPSVTLFPPSSEE LQANKATLVCLISDFYPGAVTVAWKADSSPVKAGVETTTPSKQS NNKYAASSYLSLTPEQWKSHRSYSCQVTHEGSTVEKTVAPTEC SQVQLVESGAEVKKPGGSLRLSCAASGFTFSSYSMNWVRQAP GKGLEWVSSISGTSSYIYYADSVKGRFTISRDNAKNSLYLQMSS LRAEDTAVYYCARGGLGMVDPWGQGTLVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQPR EPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |

(continued)

| SEQ ID NO. | Description | Sequence |
|---|---|---|
| 243 | G4_14 IgG1 antibody sequence | ASNFMLTQPHSVSESPGKTVTISCTRSRGSIAGNYVHWYQQRP GRAPTTVIYRDKERPSGVPDRISGSIDSSSNSASLTISGLKTEDE ADYYCQSYDSSTHVVFGGGTKLTVLGQPAAAPSVTLFPPSSEE LQANKATLVCLISDFYPGAVTVAWKADSSPVKAGVETTTPSKQS NNKYAASSYLSLTPEQWKSHRSYSCQVTHEGSTVEKTVAPTEC SEVQLVQSGAEVKKPGESLRISCKGSGYSFTSYWISWVRQMPG KGLEWMGRIDPSDSYTNYSPSFPGHVTISADKSISTAYLQWSSL KASDTAMYYCAADTAHGMDVWGRGTLVTVSSASTKGPSVFPL APSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPELLGGPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPRE PQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGK |
| 244 | G4_15 IgG1 antibody sequence | ASQSALTQPASVSGSPGQSITISCTGTSSDVGGYNYVSWYQQH PGKAPKLMIYDVSNRPSGVSNRFSGSKSGNTASLTISGLQAEDE ADYYCSSYGSGSVFGTGTKLTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECSE VQLVQSEAEVKKPGASVKVSCKASGYTFTRHYMHWVRQAPGQ GLEWMGLINPSGSSTVYAQKFQGRVTLTRDTSTSTDYMELSSL RSEDTAVYYCARDNSHLDQVWWFDPWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSRDELTKNQVSLTCLVKGFYPSDIAVEWES NGQPENNYKTTPPVLDSDGSFFLYSKLTVDKSRWQQGNVFSC SVMHEALHNHYTQKSLSLSPGK |
| 245 | G4_16 IgG1 antibody sequence | ASQSVLTQPPSASGSPGQSVTFSCTGTSSDIGAFNSVSWYQQH PGKAPKLLIYEITKRPSGVPDRFSGSKSGNTASLTISVLQAEDEA DYYCTSYAGSNTLIFGGGTKVTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECSE VQLLESGAEVKKPGASVKVSCKASGYTFTSYGISWVRQAPGQG LEWMGWISAYNGNTNYAQKLQGRVTMTTDTSTSTAYMELRSL RSDDTAVYYCARDYGDFYGMDVWGQGTLVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVDK KVEPKSCDKTHTCPPCPAPELLGGPSVFLFPPKPKDTLMISRTP EVTCVVVDVSHEDPEVKFNWYVDGVEVHNAKTKPREEQYNST YRVVSVLTVLHQDWLNGKEYKCKVSNKALPAPIEKTISKAKGQP REPQVYTLPPSRDELTKNQVSLTCLVKGFYPSDIAVEWESNGQ PENNYKTTPPVLDSDGSFFLYSKLTVDKSRWQQGNVFSCSVMH EALHNHYTQKSLSLSPGK |

(continued)

| SEQ ID NO. | Description | Sequence |
|---|---|---|
| 246 | G4_18 IgG1 antibody sequence | ASSYELTQPPSVTESPGQTARITCSGDALAKQYAYWYQQKPGQ APVLVIYRDSERPSEIPERFSGSSSGTTVTLTISGVQAEDEADYY CQSADSSGTYTVFGGGTKLTVLGQPAAAPSVTLFPPSSEELQA NKATLVCLISDFYPGAVTVAWKADSSPVKAGVETTTPSKQSNNK YAASSYLSLTPEQWKSHRSYSCQVTHEGSTVEKTVAPTECSEV QLVESGAEVKKPGASVKVSCKASGYTFTGYYMHWVRQAPGQG LEWMGRINPNSGGTNYAQKFQGRVTMTRDASISTAYMELSRLR SDDTAVYYCARDLDLSSLDYWGRGTLVTVSSASTKGPSVFPLA PSSKSTSGGTAALGCLVKDYFPEPVTVSWNSGALTSGVHTFPA VLQSSGLYSLSSVVTVPSSSLGTQTYICNVNHKPSNTKVDKKVE PKSCDKTHTCPPCPAPELLGGPSVFLFPPKPKDTLMISRTPEVT CVVVDVSHEDPEVKFNWYVDGVEVHNAKTKPREEQYNSTYRV VSVLTVLHQDWLNGKEYKCKVSNKALPAPIEKTISKAKGQPREP QVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |
| 247 | G4_19 IgG1 antibody sequence | ASNFMLTQPHSVSESPGKTVTISCTRSSGSIASNYVQWYQQRP GSPPITLIYDDDQRPSGVPHRFSGSIDTSSNPASLTISGLKTEDE ADYYCQSYDSSNHVVFGGGTKLTVLGQPAAAPSVTLFPPSSEE LQANKATLVCLISDFYPGAVTVAWKADSSPVKAGVETTTPSKQS NNKYAASSYLSLTPEQWKSHRSYSCQVTHEGSTVEKTVAPTEC SEVQLVQSGAEVKKPGASVKVSCKASGYTLTSYYMHWVRQAP GQGLEWMGIINPSGGSTSYAQKFQGRVTMTRDTSTSTVYMELS SLRSEDTAVYYCARERGYSYGDGMDVWGQGTTVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPELLGGPSVFLFPPKPKDTLMI SRTPEVTCVVVDVSHEDPEVKFNWYVDGVEVHNAKTKPREEQ YNSTYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| 248 | G4_20 IgG1 antibody sequence | ASSYELTHPPSVSVSPGQTASITCSGDKLGDKFVSWYHQKPGQ SPVLVIYQDSKRPSGIPERFSGSNSGNTATLTISGTRAMDEADY YCQAWDSSTVVFGGGTKLTVLGQPAAAPSVTLFPPSSEELQAN KATLVCLISDFYPGAVTVAWKADSSPVKAGVETTTPSKQSNNKY AASSYLSLTPEQWKSHRSYSCQVTHEGSTVEKTVAPTECSQVQ LVESGAEVKKPGASVKVSCKASGGTFSSYAISWVRQAPGQGLE WMGGIIPIFGTANYAQKFQGRVTITVDKSTRTAYMELSSLRSKDT AVYYCARGNSRSDAFDIWGQGTMVTVSSASTKGPSVFPLAPSS KSTSGGTAALGCLVKDYFPEPVTVSWNSGALTSGVHTFPAVLQ SSGLYSLSSVVTVPSSSLGTQTYICNVNHKPSNTKVDKKVEPKS CDKTHTCPPCPAPELLGGPSVFLFPPKPKDTLMISRTPEVTCVV VDVSHEDPEVKFNWYVDGVEVHNAKTKPREEQYNSTYRVVSV LTVLHQDWLNGKEYKCKVSNKALPAPIEKTISKAKGQPREPQVY TLPPSRDELTKNQVSLTCLVKGFYPSDIAVEWESNGQPENNYKT TPPVLDSDGSFFLYSKLTVDKSRWQQGNVFSCSVMHEALHNHY TQKSLSLSPGK |

(continued)

| SEQ ID NO. | Description | Sequence |
|---|---|---|
| 249 | G4_22 IgG1 antibody sequence | ASDIQMTQSPSSLSASVGDRVTITCQASQDISNYLNWYQQKPG KAPKLLIYAASSLQSGVPSRFSVSGSGTDFTLTISNLQPEDFATY YCQQSYSIPWTFGQGTKVEIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGECQV QLVESGGGLVQPGGSLRLSCAASGFTFSTYAMSWVRQAPGKG LEWVSTVSGSGGTTYYADSVKGRFTISRDNSKNTLYLQMNSLR AEDTAVYYCAKDSTAVTDWFDPWGRGTLVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQPR EPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 250 | G4_23 IgG1 antibody sequence | ASDIQMTQSPSSLSASVGDRVTITCRASQGISNSLAWYQQKPG KAPKLLLYAASRLESGVPSRFSGSGSGTDYTLTISSLQPEDFATY YCQQYYSTPRTFGGGTKLEIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGECEV QLVESGGGVVQPGRSLRLSCAASGFTFSSYGMHWVRQAPGK GLEWVAVIWYDGSNKYYADSVKGRFTISRDNSKNTLYLQMNSL RAEDTAVYYCARGEVAALYYFDYWGQGTLVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVDK KVEPKSCDKTHTCPPCPAPELLGGPSVFLFPPKPKDTLMISRTP EVTCVVVDVSHEDPEVKFNWYVDGVEVHNAKTKPREEQYNST YRVVSVLTVLHQDWLNGKEYKCKVSNKALPAPIEKTISKAKGQP REPQVYTLPPSRDELTKNQVSLTCLVKGFYPSDIAVEWESNGQ PENNYKTTPPVLDSDGSFFLYSKLTVDKSRWQQGNVFSCSVMH EALHNHYTQKSLSLSPGK |
| 251 | G4_24 IgG1 antibody sequence | ASDIVMTQSPDSLAVSLGERATINCKSSQSVLYSSNNKNYLAWY QQKPGQPPKLLISWASTRESGVPDRFSGSGSGTDFTLTINSLQS EDVAIYYCQQYYSTPPTFGQGTKLEIKRTAAAPSVFIFPPSDEQL KSGTASVVCLLNNFYPREAKVQWKVDNALQSGNSQESVTEQD SKDSTYSLSSTLTLSKADYEKHKLYACEVTHQGLSSPVTKSFNR GECQVQLQQSGPGLVKPSQTLSLTCAISGASVSSNSVAWNWIR QSPSRGLEWLGRTYYRSRWYNDYALSVKSRIIINPDTSKNQFSL QLNSVTPEDTAVYYCARDWSSTRSFDYWGRGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPSN TKVDKKVEPKSCDKTHTCPPCPAPELLGGPSVFLFPPKPKDTLM ISRTPEVTCVVVDVSHEDPEVKFNWYVDGVEVHNAKTKPREEQ YNSTYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |

(continued)

| SEQ ID NO. | Description | Sequence |
|---|---|---|
| 252 | G4_25 IgG1 antibody sequence | ASQSALTQPRSVSGSPGQSVTISCTGTSSDVGGYNYVSWYQQ HPGKAPKLMIYEVSNRPSGVSNRFSGSKSGNTASLTISGLQAED EADYYCSSYGSGSVFGTGTKLTVLGQPAAAPSVTLFPPSSEEL QANKATLVCLISDFYPGAVTVAWKADSSPVKAGVETTTPSKQSN NKYAASSYLSLTPEQWKSHRSYSCQVTHEGSTVEKTVAPTECS EVQLVESGAEVKKPGSSVKVSCKASGGTFSSYAISWVRQAPGQ GLEWMGGIIPIFGTANYAQKFQGRVTITADKSTSTAYMELSSLRS EDTAVYYCARSLRDGYNYIGSLGYWGQGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 253 | G4_26 IgG1 antibody sequence | ASQSGLTQPASVSGSPGQSITISCTGTSSDVGSYNLVSWYQQH PGKAPKLMIYEVSKRPSGVSNRFSGSKSGNTASLTISGLQAEDE ADYYCSSFGSGSIFGTGTKLTVLGQPAAAPSVTLFPPSSEELQA NKATLVCLISDFYPGAVTVAWKADSSPVKAGVETTTPSKQSNNK YAASSYLSLTPEQWKSHRSYSCQVTHEGSTVEKTVAPTECSQV QLVQSGAEVKKPGSSVKVSCKASGGTFSSYAISWVRQAPGQG LEWMGGIIPIFGTANYAQKFQGRVTITADESTSTAYMELSSLRSE DTAVYYCASSRGSGWFPLGYWGQGTLVTVSSASTKGPSVFPL APSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPELLGGPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPRE PQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGK |
| 254 | G4_27 IgG1 antibody sequence | ASSYELTQDPAVSVALGQTVSITCQGDSLRNFYANWYQQKPGQ APVLVIYGKNNRPSGIPDRFSGSSSGNTASLTITGAQAEDEADY YCNSRDSSGNHLVFGGGTQLTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECSQ VQLVQSGAEVKKPGESLKISCKSSGYSFTSYWIGWVRQMPGKG LEWMGIIYPGDSDTRYSPSFQGQVTFSADESISTAYLQWSSLKA SDTAMYYCARHGAYGDYPDTFDIWGQGTLVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVDK KVEPKSCDKTHTCPPCPAPELLGGPSVFLFPPKPKDTLMISRTP EVTCVVVDVSHEDPEVKFNWYVDGVEVHNAKTKPREEQYNST YRVVSVLTVLHQDWLNGKEYKCKVSNKALPAPIEKTISKAKGQP REPQVYTLPPSRDELTKNQVSLTCLVKGFYPSDIAVEWESNGQ PENNYKTTPPVLDSDGSFFLYSKLTVDKSRWQQGNVFSCSVMH EALHNHYTQKSLSLSPGK |

(continued)

| SEQ ID NO. | Description | Sequence |
|---|---|---|
| 255 | G4_28 IgG1 antibody sequence | ASSYELTQDPAVSVALGQTVTITCQGDSLRNYYASWYRQKPGQ TPVLVVYGKNNRPSGIPDRFSVSASGNTASLTITGAQAEDEGDY YCNSRDSSGVVFGGGTKVTVLGQPAAAPSVTLFPPSSEELQAN KATLVCLISDFYPGAVTVAWKADSSPVKAGVETTTPSKQSNNKY AASSYLSLTPEQWKSHRSYSCQVTHEGSTVEKTVAPTECSQVQ LVESGGGLVQPGGSLRLSCAASGFTFDDYAMHWVRQAPGKGL EWVSGISAGGGSTNYAGSVKGRFTVSRDTSKNTLYLQMNSLRA EDTAVYYCVKSYVDTAMRYYYYYMDVWGQGTMVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPELLGGPSVFLFPPKPKDTLMI SRTPEVTCVVVDVSHEDPEVKFNWYVDGVEVHNAKTKPREEQ YNSTYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| 256 | TRGV4(4MNH) TRAC antigen sequence | SSNLEGRTKSVIRQTGSSAEITCDLAEGSTGYIHWYLHQEGKAP QRLLYYDSYTSSVVLESGISPGKYDTYGSTRKNLRMILRNLIEND SGVYYCATWDEKYYKKLFGSGTTLVVTEDLKNVFPPEVAVFEP SEAEISHTQKATLVCLATGFYPDHVELSWWVNGKEVHSGVSTD PQPLKEQPALNDSRYALSSRLRVSATFWQNPRNHFRCQVQFY GLSENDEWTQDRAKPVTQIVSAEAWGRADCTTAPSAQLEKELQ ALEKENAQLE |
| 257 | Vγ4 - (4MNH)GV4TRB C leucine zipper heterodimer antigen sequence | SSNLEGRTKSVIRQTGSSAEITCDLAEGSTGYIHWYLHQEGKAP QRLLYYDSYTSSVVLESGISPGKYDTYGSTRKNLRMILRNLIEND SGVYYCATWDEKYYKKLFGSGTTLVVTEDLKNVFPPEVAVFEP SEAEISHTQKATLVCLATGFYPDHVELSWWVNGKEVHSGVSTD PQPLKEQPALNDSRYALSSRLRVSATFWQNPRNHFRCQVQFY GLSENDEWTQDRAKPVTQIVSAEAWGRADCTTAPSAQLEKELQ ALEKENAQLEWELQALEKELAQ |
| 258 | Vγ4 - TRGV4(4MNH) Fc heterodimer antigen sequence | SSNLEGRTKSVIRQTGSSAEITCDLAEGSTGYIHWYLHQEGKAP QRLLYYDSYTSSVVLESGISPGKYDTYGSTRKNLRMILRNLIEND SGVYYCATWDEKYYKKLFGSGTTLVVTEDAAADKTHTCPPCPA PELLGGPSVFLFPPKPKDTLMISRTPEVTCVVVDVSHEDPEVKF NWYVDGVEVHNAKTKPREEQYNSTYRVVSVLTVLHQDWLNGK EYKCKVSNKALPAPIEKTISKAKGQPREPQVYTLPPSRDELTKN QVSLTCLVKGFYPSDIAVEWESNGQPENNYKTTPPVLDSDGSF FLYSKLTVDKSRWQQGNVFSCSVSHEALHSHHTQKSLSLSPGK |
| 259 | Vy2 - (4MNH)GV2TRB C leucine zipper heterodimer antigen sequence | SSNLEGRTKSVIRQTGSSAEITCDLAEGSNGYIHWYLHQEGKAP QRLQYYDSYNSKVVLESGVSPGKYYTYASTRNNLRLILRNLIEN DSGVYYCATWDEKYYKKLFGSGTTLVVTEDLKNVFPPEVAVFE PSEAEISHTQKATLVCLATGFYPDHVELSWWVNGKEVHSGVST DPQPLKEQPALNDSRYALSSRLRVSATFWQNPRNHFRCQVQF YGLSENDEWTQDRAKPVTQIVSAEAWGRADCTTAPSAQLEKEL QALEKENAQLEWELQALEKELAQ |

(continued)

| SEQ ID NO. | Description | Sequence |
|---|---|---|
| 260 | Vy2 - TRGV2(4MNH) Fc heterodimer antigen sequence | SSNLEGRTKSVIRQTGSSAEITCDLAEGSNGYIHWYLHQEGKAP QRLQYYDSYNSKVVLESGVSPGKYYTYASTRNNLRLILRNLIEN DSGVYYCATWDEKYYKKLFGSGTTLVVTEDAAADKTHTCPPCP APELLGGPSVFLFPPKPKDTLMISRTPEVTCVVVDVSHEDPEVK FNWYVDGVEVHNAKTKPREEQYNSTYRVVSVLTVLHQDWLNG KEYKCKVSNKALPAPIEKTISKAKGQPREPQVYTLPPSRDELTKN QVSLTCLVKGFYPSDIAVEWESNGQPENNYKTTPPVLDSDGSF FLYSKLTVDKSRWQQGNVFSCSVSHEALHSHHTQKSLSLSPGK |
| 261 | TRGV4 full light variable sequence G4_1 - no N-terminal AS | DIQMTQSPSSLSASVGDRVTITCQASQDISNYLNWYQQKPGKA PKLLIYDASNLETGVPSRFSGSGSGTDFTLTISSLQPEDFATYYC QQSYSTPVTFGPGTKVEIK |
| 262 | TRGV4 full light variable sequence G4_2 - no N-terminal AS | DIQMTQSPSSLSASVGDRVTITCQASQDISNYLNWYQQKPGKA PKLLIYAASSLQSGVPSRFSGSGSGTDFTLTISGLQPEDFATYYC LEDYNYLWTFGQGTKLEIK |
| 263 | TRGV4 full light variable sequence G4_3 - no N-terminal AS | DIQMTQSPSSLSASVGDRVTITCQASQDISNYLNWYQQKPGKA PKLLIYDASNLETGVPSRFSGSGSGTDFTLTISSLQPEDFATYFC QQSYSTPQTFGQGTKVDIK |
| 264 | TRGV4 full light variable sequence G4_4 - no N-terminal AS | DIVMTQSPDSLAVSLGERATINCKSSQSVLSSSNNNNYLAWYQ QRPGQPPKLLFYWASTRESGVPDRFSGSGSGTSFTLTITSLQA EDVAVYYCQQYYSTPLTFGGGTKLEIK |
| 265 | TRGV4 full light variable sequence G4_5 - no N-terminal AS | DIQMTQSPSSLSASVGDRVTITCRASQSISSYLNWYQQKPGKAP KLLIYAASSLQSGVPSRFSGSGSGTDFTLTISSLQPEDFATYYCQ QSYSTPYTFGQGTKVEIK |
| 266 | TRGV4 full light variable sequence G4_6 - no N-terminal AS | DIQMTQSPSSLSASVGDRVTITCRASQSISTYLNWYQQKPGKAP KLLIYAASSLQSGVPSRFSGSGSGTDFTLTISSPQPEDFATYYCQ QSYSTPYTFGQGTKVEIK |
| 267 | TRGV4 full light variable sequence G4_7 - no N-terminal AS | DIVMTQSPLSLPVTPGEPASISCRSSQSLLHSNRFNYLDWYLQK PGQSPQLLIYLGSNRASGVPDRFSGSGSGTDFTLKISRVEAEDV GVYYCMQGLQTPYTFGQGTKVDIK |
| 268 | TRGV4 full light variable sequence G4_10 - no N-terminal AS | DIVMTQPPLSLPVTLGHPASISCKSSQSLEYSDGNTYLNWFQQR PGQSPRRLIYKVSNRDSGAPDRFSGSGSGTDFTLEISRVEAEDV GVYYCMQGTLWPPTFGQGTKVDIK |
| 269 | TRGV4 full light variable sequence G4_12 - no N-terminal AS | QSVLTQPASVSGSPGQSITISCTGTSSDVGGYNFVSWYQQHPG KAPKLMIYEVTNRPSGVPDRFSGSKSGNTASLTISGLQAEDEAD YYCSSHASPRVFGTGTKVTVL |
| 270 | TRGV4 full light variable sequence G4_13 - no N-terminal AS | NFMLTQPHSVSESPGKTVTISCTRSSGSIASNYVQWYQQRPGS SPSTVIYEDNQRPSGVPDRFSGSIDSSSNSASLTISGLRTEDEAD YYCQSYDSSIYVVFGGGTKLTVL |
| 271 | TRGV4 full light variable sequence G4_14 - no N-terminal AS | NFMLTQPHSVSESPGKTVTISCTRSRGSIAGNYVHWYQQRPGR APTTVIYRDKERPSGVPDRISGSIDSSSNSASLTISGLKTEDEAD YYCQSYDSSTHVVFGGGTKLTVL |
| 272 | TRGV4 full light variable sequence G4_15 - no N-terminal AS | QSALTQPASVSGSPGQSITISCTGTSSDVGGYNYVSWYQQHPG KAPKLMIYDVSNRPSGVSNRFSGSKSGNTASLTISGLQAEDEAD YYCSSYGSGSVFGTGTKLTVL |

EP 4 591 882 A2

(continued)

| SEQ ID NO. | Description | Sequence |
|---|---|---|
| 273 | TRGV4 full light variable sequence G4_16 - no N-terminal AS | QSVLTQPPSASGSPGQSVTFSCTGTSSDIGAFNSVSWYQQHPGKAPKLLIYEITKRPSGVPDRFSGSKSGNTASLTISVLQAEDEADYYCTSYAGSNTLIFGGGTKVTVL |
| 274 | TRGV4 full light variable sequence G4_18 - no N-terminal AS | SYELTQPPSVTESPGQTARITCSGDALAKQYAYWYQQKPGQAPVLVIYRDSERPSEIPERFSGSSSGTTVTLTISGVQAEDEADYYCQSADSSGTYTVFGGGTKLTVL |
| 275 | TRGV4 full light variable sequence G4_19 - no N-terminal AS | NFMLTQPHSVSESPGKTVTISCTRSSGSIASNYVQWYQQRPGSPPITLIYDDDQRPSGVPHRFSGSIDTSSNPASLTISGLKTEDEADYYCQSYDSSNHVVFGGGTKLTVL |
| 276 | TRGV4 full light variable sequence G4_20 - no N-terminal AS | SYELTHPPSVSVSPGQTASITCSGDKLGDKFVSWYHQKPGQSPVLVIYQDSKRPSGIPERFSGSNSGNTATLTISGTRAMDEADYYCQAWDSSTVVFGGGTKLTVL |
| 277 | TRGV4 full light variable sequence G4_22 - no N-terminal AS | DIQMTQSPSSLSASVGDRVTITCQASQDISNYLNWYQQKPGKAPKLLIYAASSLQSGVPSRFSVSGSGTDFTLTISNLQPEDFATYYCQQSYSIPWTFGQGTKVEIK |
| 278 | TRGV4 full light variable sequence G4_23 - no N-terminal AS | DIQMTQSPSSLSASVGDRVTITCRASQGISNSLAWYQQKPGKAPKLLLYAASRLESGVPSRFSGSGSGTDYTLTISSLQPEDFATYYCQQYYSTPRTFGGGTKLEIK |
| 279 | TRGV4 full light variable sequence G4_24 - no N-terminal AS | DIVMTQSPDSLAVSLGERATINCKSSQSVLYSSNNKNYLAWYQQKPGQPPKLLISWASTRESGVPDRFSGSGSGTDFTLTINSLQSEDVAIYYCQQYYSTPPTFGQGTKLEIK |
| 280 | TRGV4 full light variable sequence G4_25 - no N-terminal AS | QSALTQPRSVSGSPGQSVTISCTGTSSDVGGYNYVSWYQQHPGKAPKLMIYEVSNRPSGVSNRFSGSKSGNTASLTISGLQAEDEADYYCSSYGSGSVFGTGTKLTVL |
| 281 | TRGV4 full light variable sequence G4_26 - no N-terminal AS | QSGLTQPASVSGSPGQSITISCTGTSSDVGSYNLVSWYQQHPGKAPKLMIYEVSKRPSGVSNRFSGSKSGNTASLTISGLQAEDEADYYCSSFGSGSIFGTGTKLTVL |
| 282 | TRGV4 full light variable sequence G4_27 - no N-terminal AS | SYELTQDPAVSVALGQTVSITCQGDSLRNFYANWYQQKPGQAPVLVIYGKNNRPSGIPDRFSGSSSGNTASLTITGAQAEDEADYYCNSRDSSGNHLVFGGGTQLTVL |
| 283 | TRGV4 full light variable sequence G4_28 - no N-terminal AS | SYELTQDPAVSVALGQTVTITCQGDSLRNYYASWYRQKPGQTPVLVVYGKNNRPSGIPDRFSVSASGNTASLTITGAQAEDEGDYYCNSRDSSGVVFGGGTKVTVL |
| 284 | G4_1 IgG1 antibody heavy chain sequence | EVQLLESGGGVVQPGRPLRLSCAASGFTFSSYSMNWVRQAPGKGLEWVSSISSSSSYIYYADSVKGRFTISRDNSKNTLYLQMNSLRAEDTAVYYCAKGHWYFDLWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

77

(continued)

| SEQ ID NO. | Description | Sequence |
|---|---|---|
| 285 | G4_2 IgG1 antibody heavy chain sequence | QMQLVQSGAEVKKPGATVKISCKVSGYPFTDYYIHWVQQAPGK GLEWMGLVDPEDGQSRSAERFQGRVTITADTSTDTAYMELSSL RSEDTAVYYCATFPVAGFYGMDVWGQGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 286 | G4_3 IgG1 antibody heavy chain sequence | EVQLVESGGGLVQPGGSLRLSCAASGFTFSSYSMNWVRQAPG KGLEWVSSISSSSSYIYYADSVKGRFTISRDNSKNTLYLQMNSL RAEDTAVYYCARGGWLYDYWGQGTLVTVSSASTKGPSVFPLA PSSKSTSGGTAALGCLVKDYFPEPVTVSWNSGALTSGVHTFPA VLQSSGLYSLSSVVTVPSSSLGTQTYICNVNHKPSNTKVDKKVE PKSCDKTHTCPPCPAPELLGGPSVFLFPPKPKDTLMISRTPEVT CVVVDVSHEDPEVKFNWYVDGVEVHNAKTKPREEQYNSTYRV VSVLTVLHQDWLNGKEYKCKVSNKALPAPIEKTISKAKGQPREP QVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |
| 287 | G4_4 IgG1 antibody heavy chain sequence | QVQLVESGGGLVQPGGSLRLSCAASGFTFSSYAMSWVRQAPG KGLEWVSAISGSGGSTYYADSVKGRFTISRDNSKNTLYLQMSSL RVEDTAVYYCAKSSVGWWSFDYWGQGTMVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 288 | G4_5 IgG1 antibody heavy chain sequence | EVQLVQSGAEVKKPGSSVKVSCKASGYTFTSYGISWVRQAPGQ GLEWMGWIGAYNGNTNYAQKLQGRVTMSTDTSTSTAYMELRS PRSDDTAVYYCARGGTGGDHVFAYWGQGTTVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSRDELTKNQVSLTCLVKGFYPSDIAVEWESN GQPENNYKTTPPVLDSDGSFFLYSKLTVDKSRWQQGNVFSCSV MHEALHNHYTQKSLSLSPGK |

(continued)

| SEQ ID NO. | Description | Sequence |
|---|---|---|
| 289 | G4_6 IgG1 antibody heavy chain sequence | EVQLVESGGGLVQPGGPLRLSCAASGFTFSSYAMNWVRQAPG KGLEWVSAISGSGGSTYYADSVKGRFTISRDNSKNTLYLQMNSL RAEDTAVYYCAKADYGVVYYFDYWGQGTMVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 290 | G4_7 IgG1 antibody heavy chain sequence | EVQLVESGGGWVQSGGGSLRPSCAASGFTFSHYWMSWVRQAP GKGLEWVANIKQDGSIIYYADSVKGRFTISRDNAKNSVYLQMNS LRAEDTAVYYCARIGYSSSSFDYWGRGTLVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVDK KVEPKSCDKTHTCPPCPAPELLGGPSVFLFPPKPKDTLMISRTP EVTCVVVDVSHEDPEVKFNWYVDGVEVHNAKTKPREEQYNST YRVVSVLTVLHQDWLNGKEYKCKVSNKALPAPIEKTISKAKGQP REPQVYTLPPSRDELTKNQVSLTCLVKGFYPSDIAVEWESNGQ PENNYKTTPPVLDSDGSFFLYSKLTVDKSRWQQGNVFSCSVMH EALHNHYTQKSLSLSPGK |
| 291 | G4_10 IgG1 antibody heavy chain sequence | QVQLVESGGGVVQPGRPLRLSCAASGFTFSSYAMHWVRQAPG KGLEWVSAISGSGGSTYYADSVKGRFTISRDNSKNTLYLQMNSL RAEDTAVYYCAKDGAVDFWRNGMDVWGRGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSRDELTKNQVSLTCLVKGFYPSDIAVEWES NGQPENNYKTTPPVLDSDGSFFLYSKLTVDKSRWQQGNVFSC SVMHEALHNHYTQKSLSLSPGK |
| 292 | G4_12 IgG1 antibody heavy chain sequence | EVQLLESGGGLVQPGGSLRLSCAASGFTVSSNYMSWVRQAPG KGLEWVSVIYSGGSTYYADSVKGRFTISRHNSKNTLYLQMNSLR AEDTAVYYCARVANGDFLDYWGRGTLVTVSSASTKGPSVFPLA PSSKSTSGGTAALGCLVKDYFPEPVTVSWNSGALTSGVHTFPA VLQSSGLYSLSSVVTVPSSSLGTQTYICNVNHKPSNTKVDKKVE PKSCDKTHTCPPCPAPELLGGPSVFLFPPKPKDTLMISRTPEVT CVVVDVSHEDPEVKFNWYVDGVEVHNAKTKPREEQYNSTYRV VSVLTVLHQDWLNGKEYKCKVSNKALPAPIEKTISKAKGQPREP QVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |

(continued)

| SEQ ID NO. | Description | Sequence |
|---|---|---|
| 293 | G4_13 IgG1 antibody heavy chain sequence | QVQLVESGAEVKKPGGSLRLSCAASGFTFSSYSMNWVRQAPG KGLEWVSSISGTSSYIYYADSVKGRFTISRDNAKNSLYLQMSSL RAEDTAVYYCARGGLGMVDPWGQGTLVTVSSASTKGPSVFPL APSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPELLGGPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPRE PQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGK |
| 294 | G4_14 IgG1 antibody heavy chain sequence | EVQLVQSGAEVKKPGESLRISCKGSGYSFTSYWISWVRQMPGK GLEWMGRIDPSDSYTNYSPSFPGHVTISADKSISTAYLQWSSLK ASDTAMYYCAADTAHGMDVWGRGTLVTVSSASTKGPSVFPLA PSSKSTSGGTAALGCLVKDYFPEPVTVSWNSGALTSGVHTFPA VLQSSGLYSLSSVVTVPSSSLGTQTYICNVNHKPSNTKVDKKVE PKSCDKTHTCPPCPAPELLGGPSVFLFPPKPKDTLMISRTPEVT CVVVDVSHEDPEVKFNWYVDGVEVHNAKTKPREEQYNSTYRV VSVLTVLHQDWLNGKEYKCKVSNKALPAPIEKTISKAKGQPREP QVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |
| 295 | G4_15 IgG1 antibody heavy chain sequence | EVQLVQSEAEVKKPGASVKVSCKASGYTFTRHYMHWVRQAPG QGLEWMGLINPSGSSTVYAQKFQGRVTLTRDTSTSTDYMELSS LRSEDTAVYYCARDNSHLDQVWWFDPWGQGTLVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPELLGGPSVFLFPPKPKDTLMI SRTPEVTCVVVDVSHEDPEVKFNWYVDGVEVHNAKTKPREEQ YNSTYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| 296 | G4_16 IgG1 antibody heavy chain sequence | EVQLLESGAEVKKPGASVKVSCKASGYTFTSYGISWVRQAPGQ GLEWMGWISAYNGNTNYAQKLQGRVTMTTDTSTSTAYMELRS LRSDDTAVYYCARDYGDFYGMDVWGQGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |

(continued)

| SEQ ID NO. | Description | Sequence |
|---|---|---|
| 297 | G4_18 IgG1 antibody heavy chain sequence | EVQLVESGAEVKKPGASVKVSCKASGYTFTGYYMHWVRQAPG QGLEWMGRINPNSGGTNYAQKFQGRVTMTRDASISTAYMELS RLRSDDTAVYYCARDLDLSSLDYWGRGTLVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVDK KVEPKSCDKTHTCPPCPAPELLGGPSVFLFPPKPKDTLMISRTP EVTCVVVDVSHEDPEVKFNWYVDGVEVHNAKTKPREEQYNST YRVVSVLTVLHQDWLNGKEYKCKVSNKALPAPIEKTISKAKGQP REPQVYTLPPSRDELTKNQVSLTCLVKGFYPSDIAVEWESNGQ PENNYKTTPPVLDSDGSFFLYSKLTVDKSRWQQGNVFSCSVMH EALHNHYTQKSLSLSPGK |
| 298 | G4_19 IgG1 antibody heavy chain sequence | EVQLVQSGAEVKKPGASVKVSCKASGYTLTSYYMHWVRQAPG QGLEWMGIINPSGGSTSYAQKFQGRVTMTRDTSTSTVYMELSS LRSEDTAVYYCARERGYSYGDGMDVWGQGTTVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSRDELTKNQVSLTCLVKGFYPSDIAVEWES NGQPENNYKTTPPVLDSDGSFFLYSKLTVDKSRWQQGNVFSC SVMHEALHNHYTQKSLSLSPGK |
| 299 | G4_20 IgG1 antibody heavy chain sequence | QVQLVESGAEVKKPGASVKVSCKASGGTFSSYAISWVRQAPG QGLEWMGGIIPIFGTANYAQKFQGRVTITVDKSTRTAYMELSSL RSKDTAVYYCARGNSRSDAFDIWGQGTMVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQPR EPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 300 | G4_22 IgG1 antibody heavy chain sequence | QVQLVESGGGLVQPGGSLRLSCAASGFTFSTYAMSWVRQAPG KGLEWVSTVSGSGGTTYYADSVKGRFTISRDNSKNTLYLQMNS LRAEDTAVYYCAKDSTAVTDWFDPWGRGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |

(continued)

| SEQ ID NO. | Description | Sequence |
|---|---|---|
| 301 | G4_23 IgG1 antibody heavy chain sequence | EVQLVESGGGVVQPGRSLRLSCAASGFTFSSYGMHWVRQAPG KGLEWVAVIWYDGSNKYYADSVKGRFTISRDNSKNTLYLQMNS LRAEDTAVYYCARGEVAALYYFDYWGQGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 302 | G4_24 IgG1 antibody heavy chain sequence | QVQLQQSGPGLVKPSQTLSLTCAISGASVSSNSVAWNWIRQSP SRGLEWLGRTYYRSRWYNDYALSVKSRIIINPDTSKNQFSLQLN SVTPEDTAVYYCARDWSSTRSFDYWGRGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSRDELTKNQVSLTCLVKGFYPSDIAVEWESN GQPENNYKTTPPVLDSDGSFFLYSKLTVDKSRWQQGNVFSCSV MHEALHNHYTQKSLSLSPGK |
| 303 | G4_25 IgG1 antibody heavy chain sequence | EVQLVESGAEVKKPGSSVKVSCKASGGTFSSYAISWVRQAPGQ GLEWMGGIIPIFGTANYAQKFQGRVTITADKSTSTAYMELSSLRS EDTAVYYCARSLRDGYNYIGSLGYWGQGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 304 | G4_26 IgG1 antibody heavy chain sequence | QVQLVQSGAEVKKPGSSVKVSCKASGGTFSSYAISWVRQAPG QGLEWMGGIIPIFGTANYAQKFQGRVTITADESTSTAYMELSSL RSEDTAVYYCASSRGSGWFPLGYWGQGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |

(continued)

| SEQ ID NO. | Description | Sequence |
|---|---|---|
| 305 | G4_27 IgG1 antibody heavy chain sequence | QVQLVQSGAEVKKPGESLKISCKSSGYSFTSYWIGWVRQMPGK GLEWMGIIYPGDSDTRYSPSFQGQVTFSADESISTAYLQWSSLK ASDTAMYYCARHGAYGDYPDTFDIWGQGTLVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 306 | G4_28 IgG1 antibody heavy chain sequence | QVQLVESGGGLVQPGGSLRLSCAASGFTFDDYAMHWVRQAPG KGLEWVSGISAGGGSTNYAGSVKGRFTVSRDTSKNTLYLQMNS LRAEDTAVYYCVKSYVDTAMRYYYYYMDVWGQGTMVTVSSAS TKGPSVFPLAPSSKSTSGGTAALGCLVKDYFPEPVTVSWNSGA LTSGVHTFPAVLQSSGLYSLSSVVTVPSSSLGTQTYICNVNHKP SNTKVDKKVEPKSCDKTHTCPPCPAPELLGGPSVFLFPPKPKDT LMISRTPEVTCVVVDVSHEDPEVKFNWYVDGVEVHNAKTKPRE EQYNSTYRVVSVLTVLHQDWLNGKEYKCKVSNKALPAPIEKTIS KAKGQPREPQVYTLPPSRDELTKNQVSLTCLVKGFYPSDIAVE WESNGQPENNYKTTPPVLDSDGSFFLYSKLTVDKSRWQQGNV FSCSVMHEALHNHYTQKSLSLSPGK |
| 307 | G4_1 IgG1 antibody light chain sequence | ASDIQMTQSPSSLSASVGDRVTITCQASQDISNYLNWYQQKPG KAPKLLIYDASNLETGVPSRFSGSGSGTDFTLTISSLQPEDFATY YCQQSYSTPVTFGPGTKVEIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGEC |
| 308 | G4_2 IgG1 antibody light chain sequence | ASDIQMTQSPSSLSASVGDRVTITCQASQDISNYLNWYQQKPG KAPKLLIYAASSLQSGVPSRFSGSGSGTDFTLTISGLQPEDFATY YCLEDYNYLWTFGQGTKLEIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGEC |
| 309 | G4_3 IgG1 antibody light chain sequence | ASDIQMTQSPSSLSASVGDRVTITCQASQDISNYLNWYQQKPG KAPKLLIYDASNLETGVPSRFSGSGSGTDFTLTISSLQPEDFATY FCQQSYSTPQTFGQGTKVDIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGEC |
| 310 | G4_4 IgG1 antibody light chain sequence | ASDIVMTQSPDSLAVSLGERATINCKSSQSVLSSSNNNYLAWY QQRPGQPPKLLFYWASTRESGVPDRFSGSGSGTSFTLTITSLQ AEDVAVYYCQQYYSTPLTFGGGTKLEIKRTAAAPSVFIFPPSDE QLKSGTASVVCLLNNFYPREAKVQWKVDNALQSGNSQESVTE QDSKDSTYSLSSTLTLSKADYEKHKLYACEVTHQGLSSPVTKSF NRGEC |
| 311 | G4_5 IgG1 antibody light chain sequence | ASDIQMTQSPSSLSASVGDRVTITCRASQSISSYLNWYQQKPGK APKLLIYAASSLQSGVPSRFSGSGSGTDFTLTISSLQPEDFATYY CQQSYSTPYTFGQGTKVEIKRTAAAPSVFIFPPSDEQLKSGTAS VVCLLNNFYPREAKVQWKVDNALQSGNSQESVTEQDSKDSTY SLSSTLTLSKADYEKHKLYACEVTHQGLSSPVTKSFNRGEC |

(continued)

| SEQ ID NO. | Description | Sequence |
|---|---|---|
| 312 | G4_6 IgG1 antibody light chain sequence | ASDIQMTQSPSSLSASVGDRVTITCRASQSISTYLNWYQQKPGK APKLLIYAASSLQSGVPSRFSGSGSGTDFTLTISSPQPEDFATYY CQQSYSTPYTFGQGTKVEIKRTAAAPSVFIFPPSDEQLKSGTAS VVCLLNNFYPREAKVQWKVDNALQSGNSQESVTEQDSKDSTY SLSSTLTLSKADYEKHKLYACEVTHQGLSSPVTKSFNRGEC |
| 313 | G4_7 IgG1 antibody light chain sequence | ASDIVMTQSPLSLPVTPGEPASISCRSSQSLLHSNRFNYLDWYL QKPGQSPQLLIYLGSNRASGVPDRFSGSGSGTDFTLKISRVEAE DVGVYYCMQGLQTPYTFGQGTKVDIKRTAAAPSVFIFPPSDEQL KSGTASVVCLLNNFYPREAKVQWKVDNALQSGNSQESVTEQD SKDSTYSLSSTLTLSKADYEKHKLYACEVTHQGLSSPVTKSFNR GEC |
| 314 | G4_10 IgG1 antibody light chain sequence | ASDIVMTQPPLSLPVTLGHPASISCKSSQSLEYSDGNTYLNWFQ QRPGQSPRRLIYKVSNRDSGAPDRFSGSGSGTDFTLEISRVEA EDVGVYYCMQGTLWPPTFGQGTKVDIKRTAAAPSVFIFPPSDE QLKSGTASVVCLLNNFYPREAKVQWKVDNALQSGNSQESVTE QDSKDSTYSLSSTLTLSKADYEKHKLYACEVTHQGLSSPVTKSF NRGEC |
| 315 | G4_12 IgG1 antibody light chain sequence | ASQSVLTQPASVSGSPGQSITISCTGTSSDVGGYNFVSWYQQH PGKAPKLMIYEVTNRPSGVPDRFSGSKSGNTASLTISGLQAEDE ADYYCSSHASPRVFGTGTKVTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECS |
| 316 | G4_13 IgG1 antibody light chain sequence | ASNFMLTQPHSVSESPGKTVTISCTRSSGSIASNYVQWYQQRP GSSPSTVIYEDNQRPSGVPDRFSGSIDSSSNSASLTISGLRTED EADYYCQSYDSSIYVVFGGGTKLTVLGQPAAAPSVTLFPPSSEE LQANKATLVCLISDFYPGAVTVAWKADSSPVKAGVETTTPSKQS NNKYAASSYLSLTPEQWKSHRSYSCQVTHEGSTVEKTVAPTEC S |
| 317 | G4_14 IgG1 antibody light chain sequence | ASNFMLTQPHSVSESPGKTVTISCTRSRGSIAGNYVHWYQQRP GRAPTTVIYRDKERPSGVPDRISGSIDSSSNSASLTISGLKTEDE ADYYCQSYDSSTHVVFGGGTKLTVLGQPAAAPSVTLFPPSSEE LQANKATLVCLISDFYPGAVTVAWKADSSPVKAGVETTTPSKQS NNKYAASSYLSLTPEQWKSHRSYSCQVTHEGSTVEKTVAPTEC S |
| 318 | G4_15 IgG1 antibody light chain sequence | ASQSALTQPASVSGSPGQSITISCTGTSSDVGGYNYVSWYQQH PGKAPKLMIYDVSNRPSGVSNRFSGSKSGNTASLTISGLQAEDE ADYYCSSYGSGSVFGTGTKLTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECS |
| 319 | G4_16 IgG1 antibody light chain sequence | ASQSVLTQPPSASGSPGQSVTFSCTGTSSDIGAFNSVSWYQQH PGKAPKLLIYEITKRPSGVPDRFSGSKSGNTASLTISVLQAEDEA DYYCTSYAGSNTLIFGGGTKVTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECS |

(continued)

| SEQ ID NO. | Description | Sequence |
|---|---|---|
| 320 | G4_18 IgG1 antibody light chain sequence | ASSYELTQPPSVTESPGQTARITCSGDALAKQYAYWYQQKPGQ APVLVIYRDSERPSEIPERFSGSSSGTTVTLTISGVQAEDEADYY CQSADSSGTYTVFGGGTKLTVLGQPAAAPSVTLFPPSSEELQA NKATLVCLISDFYPGAVTVAWKADSSPVKAGVETTTPSKQSNNK YAASSYLSLTPEQWKSHRSYSCQVTHEGSTVEKTVAPTECS |
| 321 | G4_19 IgG1 antibody light chain sequence | ASNFMLTQPHSVSESPGKTVTISCTRSSGSIASNYVQWYQQRP GSPPITLIYDDDQRPSGVPHRFSGSIDTSSNPASLTISGLKTEDE ADYYCQSYDSSNHVVFGGGTKLTVLGQPAAAPSVTLFPPSSEE LQANKATLVCLISDFYPGAVTVAWKADSSPVKAGVETTTPSKQS NNKYAASSYLSLTPEQWKSHRSYSCQVTHEGSTVEKTVAPTEC S |
| 322 | G4_20 IgG1 antibody light chain sequence | ASSYELTHPPSVSVSPGQTASITCSGDKLGDKFVSWYHQKPGQ SPVLVIYQDSKRPSGIPERFSGSNSGNTATLTISGTRAMDEADY YCQAWDSSTVVFGGGTKLTVLGQPAAAPSVTLFPPSSEELQAN KATLVCLISDFYPGAVTVAWKADSSPVKAGVETTTPSKQSNNKY AASSYLSLTPEQWKSHRSYSCQVTHEGSTVEKTVAPTECS |
| 323 | G4_22 IgG1 antibody light chain sequence | ASDIQMTQSPSSLSASVGDRVTITCQASQDISNYLNWYQQKPG KAPKLLIYAASSLQSGVPSRFSVSGSGTDFTLTISNLQPEDFATY YCQQSYSIPWTFGQGTKVEIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGEC |
| 324 | G4_23 IgG1 antibody light chain sequence | ASDIQMTQSPSSLSASVGDRVTITCRASQGISNSLAWYQQKPG KAPKLLLYAASRLESGVPSRFSGSGSGTDYTLTISSLQPEDFATY YCQQYYSTPRTFGGGTKLEIKRTAAAPSVFIFPPSDEQLKSGTA SVVCLLNNFYPREAKVQWKVDNALQSGNSQESVTEQDSKDST YSLSSTLTLSKADYEKHKLYACEVTHQGLSSPVTKSFNRGEC |
| 325 | G4_24 IgG1 antibody light chain sequence | ASDIVMTQSPDSLAVSLGERATINCKSSQSVLYSSNNKNYLAWY QQKPGQPPKLLISWASTRESGVPDRFSGSGSGTDFTLTINSLQS EDVAIYYCQQYYSTPPTFGQGTKLEIKRTAAAPSVFIFPPSDEQL KSGTASVVCLLNNFYPREAKVQWKVDNALQSGNSQESVTEQD SKDSTYSLSSTLTLSKADYEKHKLYACEVTHQGLSSPVTKSFNR GEC |
| 326 | G4_25 IgG1 antibody light chain sequence | ASQSALTQPRSVSGSPGQSVTISCTGTSSDVGGYNYVSWYQQ HPGKAPKLMIYEVSNRPSGVSNRFSGSKSGNTASLTISGLQAED EADYYCSSYGSGSVFGTGTKLTVLGQPAAAPSVTLFPPSSEEL QANKATLVCLISDFYPGAVTVAWKADSSPVKAGVETTTPSKQSN NKYAASSYLSLTPEQWKSHRSYSCQVTHEGSTVEKTVAPTECS |
| 327 | G4_26 IgG1 antibody light chain sequence | ASQSGLTQPASVSGSPGQSITISCTGTSSDVGSYNLVSWYQQH PGKAPKLMIYEVSKRPSGVSNRFSGSKSGNTASLTISGLQAEDE ADYYCSSFGSGSIFGTGTKLTVLGQPAAAPSVTLFPPSSEELQA NKATLVCLISDFYPGAVTVAWKADSSPVKAGVETTTPSKQSNNK YAASSYLSLTPEQWKSHRSYSCQVTHEGSTVEKTVAPTECS |
| 328 | G4_27 IgG1 antibody light chain sequence | ASSYELTQDPAVSVALGQTVSITCQGDSLRNFYANWYQQKPGQ APVLVIYGKNNRPSGIPDRFSGSSSGNTASLTITGAQAEDEADY YCNSRDSSGNHLVFGGGTQLTVLGQPAAAPSVTLFPPSSEELQ ANKATLVCLISDFYPGAVTVAWKADSSPVKAGVETTTPSKQSNN KYAASSYLSLTPEQWKSHRSYSCQVTHEGSTVEKTVAPTECS |

(continued)

| SEQ ID NO. | Description | Sequence |
|---|---|---|
| 329 | G4_28 IgG1 antibody light chain sequence | ASSYELTQDPAVSVALGQTVTITCQGDSLRNYYASWYRQKPGQ TPVLVVYGKNNRPSGIPDRFSVSASGNTASLTITGAQAEDEGDY YCNSRDSSGVVFGGGTKVTVLGQPAAAPSVTLFPPSSEELQAN KATLVCLISDFYPGAVTVAWKADSSPVKAGVETTTPSKQSNNKY AASSYLSLTPEQWKSHRSYSCQVTHEGSTVEKTVAPTECS |
| 330 | Human Kappa light constant sequence (preferred allotype) | RTVAAPSVFIFPPSDEQLKSGTASVVCLLNNFYPREAKVQWKVD NALQSGNSQESVTEQDSKDSTYSLSSTLTLSKADYEKHKVYAC EVTHQGLSSPVTKSFNRGEC |
| 331 | Human Lambda light constant sequence (IGLC2) | GQPKAAPSVTLFPPSSEELQANKATLVCLISDFYPGAVTVAWKA DSSPVKAGVETTTPSKQSNNKYAASSYLSLTPEQWKSHRSYSC QVTHEGSTVEKTVAPTECS |
| 332 | Human IgG1 constant domain | ASTKGPSVFPLAPSSKSTSGGTAALGCLVKDYFPEPVTVSWNS GALTSGVHTFPAVLQSSGLYSLSSVVTVPSSSLGTQTYICNVNH KPSNTKVDKKVEPKSCDKTHTCPPCPAPELLGGPSVFLFPPKPK DTLMISRTPEVTCVVVDVSHEDPEVKFNWYVDGVEVHNAKTKP REEQYNSTYRVVSVLTVLHQDWLNGKEYKCKVSNKALPAPIEKT ISKAKGQPREPQVYTLPPSRDELTKNQVSLTCLVKGFYPSDIAV EWESNGQPENNYKTTPPVLDSDGSFFLYSKLTVDKSRWQQGN VFSCSVMHEALHNHYTQKSLSLSPGK |
| 333 | Human IgG1 constant domain with LAGA substitution | ASTKGPSVFPLAPSSKSTSGGTAALGCLVKDYFPEPVTVSWNS GALTSGVHTFPAVLQSSGLYSLSSVVTVPSSSLGTQTYICNVNH KPSNTKVDKKVEPKSCDKTHTCPPCPAPELAGAPSVFLFPPKPK DTLMISRTPEVTCVVVDVSHEDPEVKFNWYVDGVEVHNAKTKP REEQYNSTYRVVSVLTVLHQDWLNGKEYKCKVSNKALPAPIEKT ISKAKGQPREPQVYTLPPSRDELTKNQVSLTCLVKGFYPSDIAV EWESNGQPENNYKTTPPVLDSDGSFFLYSKLTVDKSRWQQGN VFSCSVMHEALHNHYTQKSLSLSPGK |
| 334 | human TRGV4 | SSNLEGRTKSVIRQTGSSAEITCDLAEGSTGYIHWYLHQEGKAP QRLLYYDSYTSSVVLESGISPGKYDTYGSTRKNLRMILRNLIEND SGVYYCATWD |
| 335 | human TRGV2 | SSNLEGRTKSVIRQTGSSAEITCDLAEGSNGYIHWYLHQEGKAP QRLQYYDSYNSKVVLESGVSPGKYYTYASTRNNLRLILRNLIEN DSGVYYCATWD |
| 336 | human TRGV8 | SSNLEGRTKSVTRPTGSSAVITCDLPVENAVYTHWYLHQEGKA PQRLLYYDSYNSRVVLESGISREKYHTYASTGKSLKFILENLIER DSGVYYCATWD |
| 337 | human TRDV1 | AQKVTQAQSSVSMPVRKAVTLNCLYETSWWSYYIFWYKQLPSK EMIFLIRQGSDEQNAKSGRYSVNFKKAAKSVALTISALQLEDSAK YFCALGE |
| 338 | human TRDV2 | AIELVPEHQTVPVSIGVPATLRCSMKGEAIGNYYINWYRKTQGN TITFIYREKDIYGPGFKDNFQGDIDIAKNLAVLKILAPSERDEGSY YCACDT |

**Claims**

**1.** An *ex vivo* method of modulating gamma variable 4 (Vγ4) T cells comprising administering an antibody or fragment thereof, which specifically binds to a Vγ4 chain of a γδ T cell receptor (TCR) and not to a gamma variable 2 (Vγ2) chain of a γδ TCR, to a cell population comprising Vγ4 T cells.

2. The method as defined in claim 1, wherein:

(i) the Vγ4 chain of the γδ TCR is human Vγ4 and the Vy2 chain of the γδ TCR is human Vγ2;
(ii) the antibody or fragment thereof binds to an epitope of the Vγ4 chain of the γδ TCR comprising one or more amino acid residues within amino acid region 67-82 of SEQ ID NO: 1, optionally wherein the epitope comprises at least one of amino acid residues 71, 73, 75, 76, 79 of SEQ ID NO: 1;
(iii) the epitope comprises or consists of K76 and/or M80 of SEQ ID NO: 1; and/or
(iv) the epitope is an activating epitope of a γδ T cell, optionally wherein binding of the activating epitope: (i) downregulates the γδ TCR; (ii) activates degranulation of the γδ T cell; (iii) activates γδ T cell-mediated killing; and/or (iv) activates or increases Vγ4 chain-mediated cell signalling.

3. An *ex vivo* method of modulating gamma variable 4 (Vγ4) T cells comprising administering an anti-Vγ4 antibody or fragment thereof, which comprises one or more of:

a CDR3 comprising a sequence having at least 80% sequence identity with any one of SEQ ID NOs: 2-47, preferably with SEQ ID NO: 10 and/or 33;
a CDR2 comprising a sequence having at least 80% sequence identity with any one of SEQ ID NOs: 48-70 and SEQUENCES: A1-A23 (of Figure 1), preferably with SEQ ID NO: 56 and/or SEQUENCE A9; and/or
a CDR1 comprising a sequence having at least 80% sequence identity with any one of SEQ ID NOs: 71-116, preferably with SEQ ID NO: 79 and/or 102,

to a cell population comprising Vγ4 T cells.

4. The method as defined in claim 3, wherein:

(i) the antibody or fragment thereof comprises a VH region comprising a CDR3 comprising a sequence having at least 80% sequence identity with any one of SEQ ID NOs: 2-24, such as SEQ ID NOs: 10, 4, 14, 15, 17, 19 or 23; and/or
(ii) the antibody or fragment thereof comprises a VL region comprising a CDR3 comprising a sequence having at least 80% sequence identity with any one of SEQ ID NOs: 25-47, such as SEQ ID NOs: 33, 27, 37, 38, 40, 42 or 46.

5. An *ex vivo* method of modulating gamma variable 4 (Vγ4) T cells comprising administering an anti-Vγ4 antibody or fragment thereof, which comprises an amino acid sequence having at least 80% sequence identity with any one of SEQ ID NOs: 117-162 or 261-283, to a cell population comprising Vγ4 T cells.

6. An *ex vivo* method of modulating gamma variable 4 (Vγ4) T cells comprising administering an anti-Vγ4 antibody or fragment thereof, which comprises one or more of:

(a) a VH comprising a HCDR1 having SEQ ID NO: 79, a HCDR2 having SEQ ID NO: 56 and a HCDR3 having SEQ ID NO: 10, optionally wherein the VH comprises SEQ ID NO: 125; and
a VL comprising a LCDR1 having SEQ ID NO: 102, a LCDR2 having SEQUENCE A9 (of Figure 1) and a LCDR3 having SEQ ID NO: 33, optionally wherein the VL comprises SEQ ID NO: 148 or 269;
(b) a VH comprising a HCDR1 having SEQ ID NO: 86, a HCDR2 having SEQ ID NO: 63 and a HCDR3 having SEQ ID NO: 17, optionally wherein the VH comprises SEQ ID NO: 132; and
a VL comprising a LCDR1 having SEQ ID NO: 109, a LCDR2 having SEQUENCE A16 (of Figure 1) and a LCDR3 having SEQ ID NO: 40, optionally wherein the VL comprises SEQ ID NO: 155 or 276;
(c) a VH comprising a HCDR1 having SEQ ID NO: 73, a HCDR2 having SEQ ID NO: 50 and a HCDR3 having SEQ ID NO: 4, optionally wherein the VH comprises SEQ ID NO: 119; and
a VL comprising a LCDR1 having SEQ ID NO: 96, a LCDR2 having SEQUENCE A3 (of Figure 1) and a LCDR3 having SEQ ID NO: 27, optionally wherein the VL comprises SEQ ID NO: 142 or 263;
(d) a VH comprising a HCDR1 having SEQ ID NO: 83, a HCDR2 having SEQ ID NO: 60 and a HCDR3 having SEQ ID NO: 14, optionally wherein the VH comprises SEQ ID NO: 129; and
a VL comprising a LCDR1 having SEQ ID NO: 106, a LCDR2 having SEQUENCE A13 (of Figure 1) and a LCDR3 having SEQ ID NO: 37, optionally wherein the VL comprises SEQ ID NO: 152 or 273;
(e) a VH comprising a HCDR1 having SEQ ID NO: 84, a HCDR2 having SEQ ID NO: 61 and a HCDR3 having SEQ ID NO: 15, optionally wherein the VH comprises SEQ ID NO: 130; and

a VL comprising a LCDR1 having SEQ ID NO: 107, a LCDR2 having SEQUENCE A14 (of Figure 1) and a LCDR3 having SEQ ID NO: 38, optionally wherein the VL comprises SEQ ID NO: 153 or 274;

(f) a VH comprising a HCDR1 having SEQ ID NO: 88, a HCDR2 having SEQ ID NO: 65 and a HCDR3 having SEQ ID NO: 19, optionally wherein the VH comprises SEQ ID NO: 134; and

a VL comprising a LCDR1 having SEQ ID NO: 111, a LCDR2 having SEQUENCE A18 (of Figure 1) and a LCDR3 having SEQ ID NO: 42, optionally wherein the VL comprises SEQ ID NO: 157 or 278;

(g) a VH comprising a HCDR1 having SEQ ID NO: 92, a HCDR2 having SEQ ID NO: 69 and a HCDR3 having SEQ ID NO: 23, optionally wherein the VH comprises SEQ ID NO: 138; and

a VL comprising a LCDR1 having SEQ ID NO: 115, a LCDR2 having SEQUENCE A22 (of Figure 1) and a LCDR3 having SEQ ID NO: 46, optionally wherein the VL comprises SEQ ID NO: 161 or 282;

(h) a VH comprising a HCDR1 having SEQ ID NO: 71, a HCDR2 having SEQ ID NO: 48 and a HCDR3 having SEQ ID NO: 2, optionally wherein the VH comprises SEQ ID NO: 117; and

a VL comprising a LCDR1 having SEQ ID NO: 94, a LCDR2 having SEQUENCE A1 (of Figure 1) and a LCDR3 having SEQ ID NO: 25, optionally wherein the VL comprises SEQ ID NO: 140 or 261;

(i) a VH comprising a HCDR1 having SEQ ID NO: 72, a HCDR2 having SEQ ID NO: 49 and a HCDR3 having SEQ ID NO: 3, optionally wherein the VH comprises SEQ ID NO: 118; and

a VL comprising a LCDR1 having SEQ ID NO: 95, a LCDR2 having SEQUENCE A2 (of Figure 1) and a LCDR3 having SEQ ID NO: 26, optionally wherein the VL comprises SEQ ID NO: 141 or 262;

(j) a VH comprising a HCDR1 having SEQ ID NO: 74, a HCDR2 having SEQ ID NO: 51 and a HCDR3 having SEQ ID NO: 5, optionally wherein the VH comprises SEQ ID NO: 120; and

a VL comprising a LCDR1 having SEQ ID NO: 97, a LCDR2 having SEQUENCE A4 (of Figure 1) and a LCDR3 having SEQ ID NO: 28, optionally wherein the VL comprises SEQ ID NO: 143 or 264;

(k) a VH comprising a HCDR1 having SEQ ID NO: 75, a HCDR2 having SEQ ID NO: 52 and a HCDR3 having SEQ ID NO: 6, optionally wherein the VH comprises SEQ ID NO: 121; and

a VL comprising a LCDR1 having SEQ ID NO: 98, a LCDR2 having SEQUENCE A5 (of Figure 1) and a LCDR3 having SEQ ID NO: 29, optionally wherein the VL comprises SEQ ID NO: 144 or 265;

(l) a VH comprising a HCDR1 having SEQ ID NO: 76, a HCDR2 having SEQ ID NO: 53 and a HCDR3 having SEQ ID NO: 7, optionally wherein the VH comprises SEQ ID NO: 122; and

a VL comprising a LCDR1 having SEQ ID NO: 99, a LCDR2 having SEQUENCE A6 (of Figure 1) and a LCDR3 having SEQ ID NO: 30, optionally wherein the VL comprises SEQ ID NO: 145 or 266;

(m) a VH comprising a HCDR1 having SEQ ID NO: 77, a HCDR2 having SEQ ID NO: 54 and a HCDR3 having SEQ ID NO: 8, optionally wherein the VH comprises SEQ ID NO: 123; and

a VL comprising a LCDR1 having SEQ ID NO: 100, a LCDR2 having SEQUENCE A7 (of Figure 1) and a LCDR3 having SEQ ID NO: 31, optionally wherein the VL comprises SEQ ID NO: 146 or 267;

(n) a VH comprising a HCDR1 having SEQ ID NO: 78, a HCDR2 having SEQ ID NO: 55 and a HCDR3 having SEQ ID NO: 9, optionally wherein the VH comprises SEQ ID NO: 124; and

a VL comprising a LCDR1 having SEQ ID NO: 101, a LCDR2 having SEQUENCE A8 (of Figure 1) and a LCDR3 having SEQ ID NO: 32, optionally wherein the VL comprises SEQ ID NO: 147 or 268;

(o) a VH comprising a HCDR1 having SEQ ID NO: 80, a HCDR2 having SEQ ID NO: 57 and a HCDR3 having SEQ ID NO: 11, optionally wherein the VH comprises SEQ ID NO: 126; and

a VL comprising a LCDR1 having SEQ ID NO: 103, a LCDR2 having SEQUENCE A10 (of Figure 1) and a LCDR3 having SEQ ID NO: 34, optionally wherein the VL comprises SEQ ID NO: 149 or 270;

(p) a VH comprising a HCDR1 having SEQ ID NO: 81, a HCDR2 having SEQ ID NO: 58 and a HCDR3 having SEQ ID NO: 12, optionally wherein the VH comprises SEQ ID NO: 127; and

a VL comprising a LCDR1 having SEQ ID NO: 104, a LCDR2 having SEQUENCE A11 (of Figure 1) and a LCDR3 having SEQ ID NO: 35, optionally wherein the VL comprises SEQ ID NO: 150 or 271;

(q) a VH comprising a HCDR1 having SEQ ID NO: 82, a HCDR2 having SEQ ID NO: 59 and a HCDR3 having SEQ ID NO: 13, optionally wherein the VH comprises SEQ ID NO: 128; and

a VL comprising a LCDR1 having SEQ ID NO: 105, a LCDR2 having SEQUENCE A12 (of Figure 1) and a LCDR3 having SEQ ID NO: 36, optionally wherein the VL comprises SEQ ID NO: 151 or 272;

(r) a VH comprising a HCDR1 having SEQ ID NO: 85, a HCDR2 having SEQ ID NO: 62 and a HCDR3 having SEQ ID NO: 16, optionally wherein the VH comprises SEQ ID NO: 131; and

a VL comprising a LCDR1 having SEQ ID NO: 108, a LCDR2 having SEQUENCE A15 (of Figure 1) and a LCDR3 having SEQ ID NO: 39, optionally wherein the VL comprises SEQ ID NO: 154 or 275;

(s) a VH comprising a HCDR1 having SEQ ID NO: 87, a HCDR2 having SEQ ID NO: 64 and a HCDR3 having SEQ ID NO: 18, optionally wherein the VH comprises SEQ ID NO: 133; and

a VL comprising a LCDR1 having SEQ ID NO: 110, a LCDR2 having SEQUENCE A17 (of Figure 1) and a LCDR3 having SEQ ID NO: 41, optionally wherein the VL comprises SEQ ID NO: 156 or 277;

(t) a VH comprising a HCDR1 having SEQ ID NO: 89, a HCDR2 having SEQ ID NO: 66 and a HCDR3 having SEQ ID NO: 20, optionally wherein the VH comprises SEQ ID NO: 135; and

a VL comprising a LCDR1 having SEQ ID NO: 112, a LCDR2 having SEQUENCE A19 (of Figure 1) and a LCDR3 having SEQ ID NO: 43, optionally wherein the VL comprises SEQ ID NO: 158 or 279;

(u) a VH comprising a HCDR1 having SEQ ID NO: 90, a HCDR2 having SEQ ID NO: 67 and a HCDR3 having SEQ ID NO: 21, optionally wherein the VH comprises SEQ ID NO: 136; and

a VL comprising a LCDR1 having SEQ ID NO: 113, a LCDR2 having SEQUENCE A20 (of Figure 1) and a LCDR3 having SEQ ID NO: 44, optionally wherein the VL comprises SEQ ID NO: 159 or 280;

(v) a VH comprising a HCDR1 having SEQ ID NO: 91, a HCDR2 having SEQ ID NO: 68 and a HCDR3 having SEQ ID NO: 22, optionally wherein the VH comprises SEQ ID NO: 137; and

a VL comprising a LCDR1 having SEQ ID NO: 114, a LCDR2 having SEQUENCE A21 (of Figure 1) and a LCDR3 having SEQ ID NO: 45, optionally wherein the VL comprises SEQ ID NO: 160 or 281;

and/or

(w) a VH comprising a HCDR1 having SEQ ID NO: 93, a HCDR2 having SEQ ID NO: 70 and a HCDR3 having SEQ ID NO: 24, optionally wherein the VH comprises SEQ ID NO: 139; and

a VL comprising a LCDR1 having SEQ ID NO: 116, a LCDR2 having SEQUENCE A23 (of Figure 1) and a LCDR3 having SEQ ID NO: 47, optionally wherein the VL comprises SEQ ID NO: 162 or 283,

to a cell population comprising Vγ4 T cells.

7. An *ex vivo* method of modulating gamma variable 4 (Vγ4) T cells comprising:

(i) administering an anti-Vγ4 antibody or fragment thereof which comprises an amino acid sequence having at least 80% sequence identity with any one of SEQ ID NOs: 163-185, to a cell population comprising Vγ4 T cells;
(ii) administering an anti-Vγ4 antibody which comprises an amino acid sequence having at least 80% sequence identity with any one of SEQ ID NOs: 233-255, to a cell population comprising Vγ4 T cells; or
(iii) administering an anti-Vγ4 antibody or fragment thereof comprising a heavy chain amino acid sequence having at least 80% sequence identity with any one of SEQ ID NOs: 284-306 and/or a light chain amino acid sequence having at least 80% sequence identity with any one of SEQ ID NOs: 307-329, to a cell population comprising Vγ4 T cells.

8. The method as defined in any one of claims 1 to 7, wherein:

(i) the anti-Vγ4 antibody or fragment thereof is:

(a) an scFv or a full length antibody; and/or
(b) a human antibody or fragment thereof;

(ii) the modulation comprises expansion of Vγ4 T cells, optionally wherein the method provides an expanded population of Vγ4 T cells which contains greater than about 60% Vγ4 T cells, such as greater than about 70% Vγ4 T cells;
(iii) the method comprises culturing the cell population for at least 5 days; and/or
(iv) the method comprises culturing the cell population in the presence of IL-2 and/or IL-15.

9. The method as defined in any one of claims 1 to 8, wherein the cell population is obtained from:

(i) a haematopoietic sample or a fraction thereof, optionally wherein the haematopoietic sample consists of peripheral blood mononuclear cells (PBMCs) or low density mononuclear cells (LDMCs); or
(ii) a non-haematopoietic tissue sample, such as a skin, colon, gut, mammary gland, lung, prostate, liver, spleen, pancreas, uterus, vagina or other cutaneous, mucosal or serous membrane sample.

10. The method as defined in any one of claims 1 to 9, wherein the cell population is obtained from human or non-human animal tissue.

11. A Vγ4 T cell population obtained or obtainable by the *ex vivo* method as defined in any one of claims 1 to 10.

12. A composition comprising the Vγ4 T cell population as defined in claim 11.

13. A pharmaceutical composition comprising the Vγ4 T cell population as defined in claim 11, together with a pharmaceutically acceptable diluent or carrier.

14. The pharmaceutical composition as defined in claim 13, for use as a medicament.

15. The Vγ4 T cell population as defined in claim 11 or the pharmaceutical composition as defined in claim 13, for use in the treatment of cancer, an infectious disease or an inflammatory disease.

**FIGURE 1**

| Clone ID | Alias | Heavy CDR1 | SEQ ID NO. | Heavy CDR2 | SEQ ID NO. | Heavy CDR3 | SEQ ID NO. | Light CDR1 | SEQ ID NO. | Light CDR2 | SEQ ID NO. | Light CDR3 | SEQ ID NO. |
|---|---|---|---|---|---|---|---|---|---|---|---|---|---|
| 1138_P01_F06 | G4_1 | GFTFSSYS | 71 | SSSSSYI | 48 | GHWYFDL | 2 | QDISNY | 94 | DAS | A1 | QQSYSTPVT | 25 |
| 1139_P01_B03 | G4_2 | GYPFTDYY | 72 | VDPEDGQS | 49 | FPVAGFYGMDV | 3 | QDISNY | 95 | AAS | A2 | LEDYNYLWT | 26 |
| 1139_P01_A04 | G4_3 | GFTFSSYS | 73 | SSSSSYI | 50 | GGWLYDY | 4 | QDISNY | 96 | DAS | A3 | QQSYSTPQT | 27 |
| 1247_P01_E02 | G4_4 | GFTFSSYA | 74 | SGSGGST | 51 | SSVGWWSFDY | 5 | QSVLSSSNNNNY | 97 | WAS | A4 | QQYYSTPLT | 28 |
| 1247_P02_H01 | G4_5 | GYTFTSYG | 75 | GAYNGNT | 52 | GGTGGDHVFAY | 6 | QSISSY | 98 | AAS | A5 | QQSYSTPYT | 29 |
| 1247_P02_A03 | G4_6 | GFTFSSYA | 76 | SGSGGST | 53 | ADYGWYYFDY | 7 | QSISTY | 99 | AAS | A6 | QQSYSTPYT | 30 |
| 1247_P02_A04 | G4_7 | GFTFSHYW | 77 | KQDGSII | 54 | IGYSSSSFDY | 8 | QSLLHSNRFNY | 100 | LGS | A7 | MQGLQTPYT | 31 |
| 1253_P02_H04 | G4_10 | GFTFSSYA | 78 | SGSGGST | 55 | DGAVDFWRNGMDV | 9 | QSLEYSDGNTY | 101 | KVS | A8 | MQGTLWPPT | 32 |
| 1140_P01_G08 | G4_12 | GFTVSSNY | 79 | YSGGST | 56 | VANGDFLDY | 10 | SSDVGGYNF | 102 | EVT | A9 | SSHASPRV | 33 |
| 1248_P01_B01 | G4_13 | GFTFSSYS | 80 | SGTSSYI | 57 | GGLGMVDP | 11 | SGSIASNY | 103 | EDN | A10 | QSYDSSIYVV | 34 |
| 1248_P01_C01 | G4_14 | GYSFTSYW | 81 | DFSDSYT | 58 | DTAHGMDV | 12 | RGSIAGNY | 104 | RDK | A11 | QSYDSSTHVV | 35 |
| 1248_P02_D06 | G4_15 | GYTFTRHY | 82 | NPSGSST | 59 | DNSHLDQVWWFDP | 13 | SSDVGGYNY | 105 | DVS | A12 | SSYGSGSV | 36 |
| 1248_P02_D10 | G4_16 | GYTFTSYG | 83 | SAYNGNT | 60 | DYGDFYGMDV | 14 | SSDIGAFNS | 106 | EIT | A13 | TSYAGSNTLI | 37 |
| 1254_P01_H04 | G4_18 | GYTFTGYY | 84 | NPNSGGT | 61 | DLDLSSLDY | 15 | ALAKQY | 107 | RDS | A14 | QSADSSGTYTV | 38 |
| 1254_P01_G06 | G4_19 | GYTLTSYY | 85 | NPSGGST | 62 | ERGYSYGDGMDV | 16 | SGSIASNY | 108 | DDD | A15 | QSYDSSNHVV | 39 |
| 1254_P02_G02 | G4_20 | GGTFSSYA | 86 | IPIFGTA | 63 | GNSRSDAFDI | 17 | KLGDKF | 109 | QDS | A16 | QAWDSSTVV | 40 |
| 1247_P02_B10 | G4_22 | GFTFSTYA | 87 | VSGSGGTT | 64 | DSTAVTDWFDP | 18 | QDISNY | 110 | AAS | A17 | QQSYSIPWT | 41 |
| 1253_P03_H05 | G4_23 | GFTFSSYG | 88 | WYDGSNK | 65 | GEVAALYYFDY | 19 | QGISNS | 111 | AAS | A18 | QQYYSTPRT | 42 |
| 1247_P01_B04 | G4_24 | GASVSSNSVA | 89 | TYYRSRWYN | 66 | DWSSTRSFDY | 20 | QSVLYSSNNKNY | 112 | WAS | A19 | QQYYSTPPT | 43 |
| 1248_P02_E05 | G4_25 | GGTFSSYA | 90 | IPIFGTA | 67 | SLRDGYNYIGSLGY | 21 | SSDVGGYNY | 113 | EVS | A20 | SSYGSGSV | 44 |
| 1248_P02_C01 | G4_26 | GGTFSSYA | 91 | IPIFGTA | 68 | SRGSGWFPLGY | 22 | SSDVGSYNL | 114 | EVS | A21 | SSFGSGSI | 45 |
| 1248_P02_C10 | G4_27 | GYSFTSYW | 92 | YPGDSDT | 69 | HGAYGDYPDTFDI | 23 | SLRNFY | 115 | GKN | A22 | NSRDSSGNHLV | 46 |
| 1248_P02_F12 | G4_28 | GFTFDDYA | 93 | SAGGGST | 70 | SYVDTAMRYYYYYMDV | 24 | SLRNYY | 116 | GKN | A23 | NSRDSSGVV | 47 |

91

**FIGURE 2A**

EP 4 591 882 A2

| Alias | Clone ID | Antigen | | | | | DV1-GV4 specificity over DV1-GV2 (fold improvement after background D1.3 subtraction) | DV1-GV4 specificity over DV1-GV2 (% fold improvement after background subtraction) |
| | | BSA | L1 (DV1-GV4) | L2 (DV1-GV2) | L3 (DV1-GV8) | L4 (DV2-GV4) | | |
|---|---|---|---|---|---|---|---|---|
| G4_1 | 1138_P01_F06 | 78 | 202446 | 100 | 244 | 151594 | 5622 | 562167 |
| G4_2 | 1139_P01_B03 | 56 | 200571 | 86 | 270 | 66611 | 9114 | 911386 |
| G4_3 | 1139_P01_A04 | 88 | 213198 | 216 | 239 | 167686 | 1402 | 140202 |
| G4_4 | 1247_P01_E02 | 121 | 197877 | 90 | 190 | 115097 | 7607 | 760715 |
| G4_5 | 1247_P02_H01 | 86 | 162563 | 93 | 173 | 53057 | 5602 | 560248 |
| G4_6 | 1247_P02_A03 | 122 | 153210 | 112 | 256 | 123562 | 3190 | 318998 |
| G4_7 | 1247_P02_A04 | 164 | 163326 | 168 | 238 | 51157 | 1570 | 156957 |
| G4_10 | 1253_P02_H04 | 57 | 145186 | 109 | 187 | 54803 | 3224 | 322433 |
| G4_12 | 1140_P01_G08 | 80 | 236812 | 229 | 252 | 195126 | 1435 | 143467 |
| G4_13 | 1248_P01_B01 | 78 | 196024 | 94 | 270 | 100595 | 6531 | 653110 |
| G4_14 | 1248_P01_C01 | 135 | 208474 | 199 | 229 | 146754 | 1544 | 154358 |
| G4_15 | 1248_P02_D06 | 63 | 119133 | 844 | 156 | 38839 | 153 | 15262 |
| G4_16 | 1248_P02_D10 | 69 | 178442 | 84 | 118 | 89921 | 8918 | 891755 |
| G4_18 | 1254_P01_H04 | 67 | 196864 | 66 | 185 | 144733 | 98387 | 9838650 |
| G4_19 | 1254_P01_G06 | 55 | 142432 | 73 | 192 | 54072 | 15816 | 1581567 |
| G4_20 | 1254_P02_G02 | 132 | 184142 | 147 | 239 | 140291 | 2218 | 221778 |
| D1.3 (Assay 1) | Anti-Chick Lysozyme (D1.3) | 66 | 91 | 64 | 120 | 83 | NA | NA |
| G4_22 | 1247_P02_B10 | 47 | 109257 | 567 | 980 | 50342 | 670 | 67017 |
| G4_23 | 1253_P03_H05 | 53 | 196478 | 616 | 1029 | 248899 | 928 | 92791 |
| G4_24 | 1247_P01_B04 | 53 | 154317 | 534 | 960 | 125859 | 1191 | 119091 |
| G4_25 | 1248_P02_E05 | 40 | 21670 | 486 | 885 | 8811 | 259 | 25901 |
| G4_26 | 1248_P02_C01 | 51 | 22796 | 680 | 870 | 18504 | 80 | 8039 |
| G4_27 | 1248_P02_C10 | 40 | 138240 | 579 | 1686 | 91330 | 791 | 79052 |
| G4_28 | 1248_P02_F12 | 65 | 227097 | 576 | 984 | 217278 | 1324 | 132402 |
| D1.3 (Assay 2) | Anti-Chick Lysozyme (D1.3) | 51 | 690 | 405 | 717 | 43 | NA | NA |

**FIGURE 2B**

93

**FIGURE 3**

**FIGURE 4**

A

**FIGURE 5**

**B**

|  | G4_3 | G4_12 | G4_16 | G4_18 | G4_27 |
|---|---|---|---|---|---|
| hu17 | | | | | |
| hu17.Vγ2$^{CDR1}$ | ░ | | | | |
| hu17.Vγ2$^{CDR2}$ | | | | | |
| hu17.Vγ2$^{CDR1+2}$ | ░ | | | | |
| hu17$^{DG\,KM>YA\,NL}$ | ▓ | ▓ | ▓ | ▓ | ▓ |
| hu17$^{DG>YA}$ | ▓ | ░ | ▓ | ▓ | ▓ |
| hu17$^{KM>NL}$ | ▓ | ░ | | ▓ | ▓ |
| hu17.Vγ2 | ▓ | ▓ | ▓ | ▓ | ▓ |
| hu17.Vγ2$^{YA>DG}$ | ▓ | ▓ | ▓ | ▓ | ▓ |
| hu17.Vγ3 | ▓ | ▓ | ▓ | ▓ | ▓ |
| hu17.Vγ3-Vγ4$^{HV4}$ | ▓ | ░ | ▓ | ▓ | |
| hu17.Vγ3-Vγ4$^{CDR2-HV4}$ | ▓ | ░ | | ░ | |

☐ Staining equivalent to hu17

▨ Weaker staining compared to hu17 (gMFI reduced by <50%)

▓ No staining

**C**

FIGURE 5 (contd.)

FIGURE 6

**FIGURE 7**

**FIGURE 7 (contd.)**

FIGURE 8

**EP 4 591 882 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020095059 A

### Non-patent literature cited in the description

- **ADAMS et al.** *Cell Immunol.*, 2015, vol. 296, 30-40
- **BARROS et al.** *Cell*, 2016, vol. 167, 203-218
- **PADLAN**. *Mol. Immunol.*, 1994, vol. 31, 169-217
- **KABAT et al.** Sequences of Proteins of Immunological Interest. U.S. Department of Health and Human Services, 1991
- **CHOTHIA et al.** *Nature*, 1989, vol. 342, 877-883
- **MELANDRI et al.** *Nat. Immunol.*, 2018, vol. 19, 1352-1365
- **LUOMA et al.** *Immunity*, 2013, vol. 39, 1032-1042
- **JEFFERIS** ; **LEFRANC**. *MAbs*, 2009, vol. 1 (4), 332-8
- **GREEN** ; **SAMBROOK**. Molecular Cloning: A Laboratory Manual. Cold Spring Harbour Laboratory Press, 2012
- **XU et al.** *PNAS*, 2011, vol. 108, 2414-2419
- **MARTIN et al.** *BMC Biotechnol*, 2006, vol. 6, 46
- **ENGLAND et al.** *J. Immunol.*, 1999, vol. 162, 2129-2136
- **MELANDRI et al.** *Nature Immunology*, 2018, vol. 19 (12), 1352-1365
- **WILLCOX et al.** *Immunity*, 2019, vol. 51 (5), 813-825
- **WILLCOX et al.** *Immunity*, 2019, vol. 51, 813-825
- **CLARKE et al.** *J. Invest. Dermatol.*, 2006, vol. 126, 1059-1070